# EUROPEAN PATENT SPECIFICATION

(11) **EP 1 363 705 B1**
(45) Date of publication and mention of the grant of the patent: **13.06.2012**
(21) Application number: 02707413.7
(22) Date of filing: 02.01.2002
(51) Int. Cl.: A61P 31/18, A61P 31/12, A61K 31/496, A61K 31/501, A61K 31/53, A61K 31/506, C07D 401/14, C07D 403/14, C07D 241/02, C07D 253/08, C07D 251/02, C07D 241/36, C07D 471/02

(54) **COMPOSITION AND ANTIVIRAL ACTIVITY OF SUBSTITUTED AZAINDOLEOXOACETIC PIPERAZINE DERIVATIVES**
ZUSAMMENSETZUNG UND ANTIVIRALE WIRKUNG SUBSTITUIERTER AZAINDOLOXOESSIGSÄUREPIPERAZIN- DERIVATIVE
COMPOSITION ET ACTIVITE ANTIVIRALE DE DERIVES DE PIPERAZINE AZAINDOLEOXOACETIQUE SUBSTITUES

(30) Priority: 02.02.2001 US 266183 P; 23.08.2001 US 314406 P
(43) Date of publication of application: 26.11.2003
(73) Proprietor: Bristol-Myers Squibb Company, Princeton NJ 08543-4000 (US)
(72) Inventor: WANG, Tao, Middletown, CT 06457 (US); ZHANG, Zhongxing, Madison, CT 06443 (US); MEANWELL, Nicholas, A., East Hampton, CT 06424 (US); KADOW, John, F., Wallingford, CT 06492 (US); YIN, Zhiwei, Meriden, CT 06450 (US)
(74) Representative: Reitstötter - Kinzebach
(86) International application number: PCT/US2002/000455
(87) International publication number: WO 2002/062423

(56) References cited:
- WO-A1-00/76521
- WO-A1-01/62255
- WO-A1-02/04440
- WO-A1-96/11929
- WANG TAO ET AL: "Discovery of 4-benzoyl-1-[(4-methoxy-1H- pyrrolo[2,3-b]pyridin-3-yl)o xoacetyl]-2- (R)-methylpiperazine (BMS-378806): a novel HIV-1 attachment inhibitor that interferes with CD4-gp120 interactions." JOURNAL OF MEDICINAL CHEMISTRY 25 SEP 2003, vol. 46, no. 20, 25 September 2003 (2003-09-25), pages 4236-4239, XP002533377 ISSN: 0022-2623

## Description

### REFERENCE TO RELATED APPLICATIONS

This application claims the benefit of U.S. Provisional Application Serial Numbers 60/314,406 filed August 23, 2001 and 60/266,183 filed February 2, 2001.

### BACKGROUND OF THE INVENTION

### Field of the Invention

This invention provides compounds having drug and bio-affecting properties, their pharmaceutical compositions and method of use. In particular, the invention is concerned with azaindole piperazine diamide derivatives that possess unique antiviral activity. More particularly, the present invention relates to compounds useful for the treatment of HIV and AIDS.

### Background Art

HIV-1 (human immunodeficiency virus -1) infection remains a major medical problem, with an estimated 33.6 million people infected worldwide. The number of cases of HIV and AIDS (acquired immunodeficiency syndrome) has risen rapidly. In 1999, 5.6 million new infections were reported, and 2.6 million people died from AIDS. Currently available drugs for the treatment of HIV include six nucleoside reverse transcriptase (RT) inhibitors (zidovudine, didanosine, stavudine, lamivudine, zalcitabine and abacavir), three non-nucleoside reverse transcriptase inhibitors (nevirapine, delavirdine and efavirenz), and six peptidomimetic protease inhibitors (saquinavir, indinavir, ritonavir, nelfinavir, amprenavir and lopinavir). Each of these drugs can only transiently restrain viral replication if used alone. However, when used in combination, these drugs have a profound effect on viremia and disease progression. In fact, significant reductions in death rates among AIDS patients have been recently documented as a consequence of the widespread application of combination therapy. However, despite these impressive results, 30 to 50% of patients ultimately fail combination drug therapies. Insufficient drug potency, non-compliance, restricted tissue penetration and drug-specific limitations within certain cell types (e.g. most nucleoside analogs cannot be phosphorylated in resting cells) may account for the incomplete suppression of sensitive viruses. Furthermore, the high replication rate and rapid turnover of HIV-1 combined with the frequent incorporation of mutations, leads to the appearance of drug-resistant variants and treatment failures when sub-optimal drug concentrations are present (Larder and Kemp; Gulick; Kuritzkes; Morris-Jones *et al*; Schinazi *et al*; Vacca and Condra; Flexner; Berkhout and Ren *et al*; (Ref. 6-14)). Therefore, novel anti-HIV agents exhibiting distinct resistance patterns, and favorable pharmacokinetic as well as safety profiles are needed to provide more treatment options.

Currently marketed HIV-1 drugs are dominated by either nucleoside reverse transcriptase inhibitors or peptidomimetic protease inhibitors. Non-nucleoside reverse transcriptase inhibitors (NNRTIs) have recently gained an increasingly important role in the therapy of HIV infections (Pedersen & Pedersen, Ref 15). At least 30 different classes of NNRTI have been described in the literature (De Clercq, Ref. 16) and several NNRTIs have been evaluated in clinical trials. Dipyridodiazepinone (nevirapine), benzoxazinone (efavirenz) and bis(heteroaryl) piperazine derivatives (delavirdine) have been approved for clinical use. However, the major drawback to the development and application of NNRTIs is the propensity for rapid emergence of drug resistant strains, both in tissue cell culture and in treated individuals, particularly those subject to monotherapy. As a consequence, there is considerable interest in the identification of NNRTIs less prone to the development of resistance (Pedersen & Pedersen, Ref 15).

Several indole derivatives including indole-3-sulfones, piperazino indoles, pyrazino indoles, and 5H-indolo[3,2-b][1,5]benzothiazepine derivatives have been reported as HIV-1 reverse transciptase inhibitors (Greenlee et al, Ref. 1; Williams et al, Ref. 2; Romero et al, Ref. 3; Font et al, Ref. 17; Romero et al, Ref. 18; Young et al, Ref. 19; Genin et al, Ref. 20; Silvestri et al, Ref. 21). Indole 2-carboxamides have also been described as inhibitors of cell adhesion and HIV infection (Boschelli et al, US 5,424,329, Ref. 4). Finally, 3-substituted indole natural products (Semicochliodinol A and B, didemethylasterriquinone and isocochliodinol) were disclosed as inhibitors of HIV-1 protease (Fredenhagen et al, Ref. 22). Other indole derivatives exhibiting antiviral activity useful for treating HIV are disclosed in PCT WO 00/76521 (Ref. 93). Also, indole derivatives are disclosed in PCT WO 00/71535 (Ref. 94).

Structurally related aza-indole amide derivatives have been disclosed previously (Kato et al, Ref. 23; Levacher et al, Ref. 24; Dompe Spa, WO-09504742, Ref. 5(a); SmithKline Beecham PLC, WO-09611929, Ref. 5(b); Schering Corp., US-05023265, Ref. 5(c)). However, these structures differ from those claimed herein in that they are aza-indole mono-amide rather than unsymmetrical aza-indole piperazine diamide derivatives, and there is no mention of the use of these compounds for treating viral infections, particularly HIV. Other azaindoles have been also disclosed by Wang et al, Ref. 95. Nothing in these references can be construed to disclose or suggest the novel compounds of this invention and their use to inhibit HIV infection.

### REFERENCES CITED

### Patent documents

1. Greenlee, W.J.; Srinivasan, P.C. Indole reverse transcriptase inhibitors. U.S. Patent 5,124,327.
2. Williams, T.M.; Ciccarone, T.M.; Saari, W. S.; Wai, J.S.; Greenlee, W.J.; Balani, S.K.; Goldman, M.E.; Theohrides, A.D. Indoles as inhibitors of HIV reverse transcriptase. European Patent 530907.
3. Romero, D.L.; Thomas, R.C.; Preparation of substituted indoles as anti-AIDS pharmaceuticals. PCT WO 93 /01181.
4. Boschelli, D.H.; Connor, D.T.; Unangst, P.C. Indole-2-carboxamides as inhibitors of cell adhesion. U.S. Patent 5,424,329.
5. (a) Mantovanini, M.; Melillo, G.; Daffonchio, L. Tropyl 7-azaindol-3-ylcarboxyamides as antitussive agents. PCT WO 95/04742 (Dompe Spa). (b) Cassidy, F.; Hughes, I.; Rahman, S.; Hunter, D. J. Bisheteroaryl-carbonyl and carboxamide derivatives with 5HT 2C/2B antagonists activity. PCT WO 96/11929. (c) Scherlock, M. H.; Tom, W. C. Substituted 1*H*-pyrrolopyridine-3-carboxamides. U. S. Patent 5,023,265.

### Other Publications

6. Larder, B.A.; Kemp, S.D. Multiple mutations in the HIV-1 reverse transcriptase confer high-level resistance to zidovudine (AZT). Science, 1989, 246,1155-1158.
7. Gulick, R.M. Current antiretroviral therapy: An overview. Quality of Life Research, 1997, 6, 471-474.
8. Kuritzkes, D.R. HIV resistance to current therapies. Antiviral Therapy, 1997, 2 (Supplement 3), 61-67.
9. Morris-Jones, S.; Moyle, G.; Easterbrook, P.J. Antiretroviral therapies in HIV-1 infection. Expert Opinion on Investigational Drugs, 1997, 6(8),1049-1061.
10. Schinazi, R.F.; Larder, B.A.; Mellors, J.W. Mutations in retroviral genes associated with drug resistance. International Antiviral News, 1997, 5,129-142,.
11. Vacca, J.P.; Condra, J.H. Clinically effective HIV-1 protease inhibitors. Drug Discovery Today, 1997, 2, 261-272.
12. Flexner, D. HIV-protease inhibitors. Drug Therapy, 1998, 338, 1281-1292.
13. Berkhout, B. HIV-1 evolution under pressure of protease inhibitors: Climbing the stairs of viral fitness. J. Biomed. Sci., 1999, 6, 298-305.
14. Ren, S.; Lien, E. J. Development of HIV protease inhibitors: A survey. Prog. Drug Res., 1998, 51, 1-31.
15. Pedersen, O.S.; Pedersen, E.B. Non-nucleoside reverse transcriptase inhibitors: the NNRTI boom. Antiviral Chem. Chemother. 1999,10, 285-314.
16. (a) De Clercq, E. The role of non-nucleoside reverse transcriptase inhibitors (NNRTIs) in the therapy of HIV-1 infection. Antiviral Research, 1998, 38, 153-179. (b) De Clercq, E. Perspectives of non-nucleoside reverse transcriptase inhibitors (NNRTIs) in the therapy of HIV infection. IL. Farmaco, 1999, 54, 26-45.
17. Font, M.; Monge, A.; Cuartero, A.; Elorriaga, A.; Martinez-Irujo, J.J.; Alberdi, E.; Santiago, E.; Prieto, I.; Lasarte, J.J.; Sarobe, P. and Borras, F. Indoles and pyrazino[4,5-b]indoles as nonnucleoside analog inhibitors of HIV-1 reverse transcriptase. Eur. J. Med. Chem., 1995, 30, 963-971.
18. Romero, D.L.; Morge, R.A.; Genin, M.J.; Biles, C.; Busso, M,; Resnick, L.; Althaus, I.W.; Reusser, F.; Thomas, R.C and Tarpley, W.G. Bis(heteroaryl)piperazine (BHAP) reverse transcriptase inhibitors: structure-activity relationships of novel substituted indole analogues and the identification of 1-[(5-methanesulfonamido-1H-indol-2-yl)-carbonyl]-4-[3-[1-methylethyl)amino]-pyridinyl]piperazine momomethansulfonate (U-90152S), a second generation clinical candidate. J. Med. Chem., 1993, 36, 1505-1508.
19. Young, S.D.; Amblard, M.C.; Britcher, S.F.; Grey, V.E.; Tran, L.O.; Lumma, W.C.; Huff, J.R.; Schleif, W.A.; Emini, E.E.; O'Brien, J.A.; Pettibone, D.J. 2-Heterocyclic indole-3-sulfones as inhibitors of HIV-reverse transcriptase. Bioorg. Med. Chem. Lett., 1995, 5, 491-496.
20. Genin, M.J.; Poel, T.J.; Yagi, Y.; Biles, C.; Althaus, I.; Keiser, B.J.; Kopta, L.A.; Friis, J.M.; Reusser, F.; Adams, W.J.; Olmsted, R.A.; Voorman, R.L.; Thomas, RC. and Romero, D.L. Synthesis and bioactivity of novel bis(heteroaryl)piperazine (BHAP) reverse transcriptase inhibitors: structure-activity relationships and increased metabolic stability of novel substituted pyridine analogs. J. Med Chem., 1996, 39, 5267-5275.
21. Silvestri, R.; Artico, M.; Bruno, B.; Massa, S.; Novellino, E.; Greco, G.; Marongiu, M.E.; Pani, A.; De Montis, A and La Colla, P. Synthesis and biological evaluation of 5H-indolo[3,2-b][1,5]benzothiazepine derivatives, designed as conformationally constrained analogues of the human immunodeficiency virus type 1 reverse transcriptase inhibitor L-737,126. Antiviral Chem. Chemother. 1998, 9, 139-148.
22. Fredenhagen, A.; Petersen, F.; Tintelnot-Blomley, M.; Rosel, J.; Mett, H and Hug, P. J. Semicochliodinol A and B: Inhibitors of HIV-1 protease and EGF-R protein Tyrosine Kinase related to Asterriquinones produced by the fungus Chrysosporium nerdarium. Antibiotics, 1997, 50, 395-401.
23. Kato, M.; Ito, K.; Nishino, S.; Yamakuni, H.; Takasugi, H. New 5-HT3 (Serotonin-3) receptor antagonists. IV. Synthesis and structure-activity relationships of azabicycloalkaneacetamide derivatives. Chem. Pharm. Bull., 1995, 43, 1351-1357.
24. Levacher, V.; Benoit, R.; Duflos, J; Dupas, G.; Bourguignon, J.; Queguiner, G. Broadening the scope of NADH models by using chiral and non chiral pyrrolo [2,3-b] pyridine derivatives. Tetrahedron, 1991, 47, 429-440.
25. Shadrina, L.P.; Dormidontov, Yu.P.; Ponomarev, V,G.; Lapkin, I.I. Reactions of organomagnesium derivatives of 7-aza- and benzoindoles with diethyl oxalate and the reactivity of ethoxalylindoles. Khim. Geterotsikl. Soedin., 1987, 1206-1209.
26. Sycheva, T.V.; Rubtsov, N.M.; Sheinker, Yu.N.; Yakhontov, L.N. Some reactions of 5-cyano-6-chloro-7-azaindoles and lactam-lactim tautomerism in 5-cyano-6-hydroxy-7-azaindolines. Khim. Geterotsikl. Soedin., 1987, 100-106.
27. (a) Desai, M.; Watthey, J.W.H.; Zuckerman, M. A convenient preparation of 1-aroylpiperazines. Org. Prep. Proced Int., 1976, 8, 85-86. (b) Adamczyk, M.; Fino, J.R. Synthesis of procainamide metabolites. N-acetyl desethylprocainamide and desethylprocainamide. Org. Prep. Proced Int. 1996, 28, 470-474. (c) Rossen, K.; Weissman, S.A.; Sager, J.; Reamer, R.A.; Askin, D.; Volante, R.P.; Reider, P.J. Asymmetric Hydrogenation of tetrahydropyrazines: Synthesis of (S)-piperazine 2-tert-butylcarboxamide, an intermediate in the preparation of the HIV protease inhibitor Indinavir. Tetrahedron Lett., 1995, 36, 6419-6422. (d) Wang, T.; Zhang, Z.; Meanwell, N.A. Benzoylation of Dianions: Preparation of mono-Benzoylated Symmetric Secondary Diamines. J. Org. Chem., 1999, 64,7661-7662.
28. Li, H.; Jiang, X.; Ye, Y.-H.; Fan, C.; Romoff, T.; Goodman, M. 3-(Diethoxyphosphoryloxy)-1,2,3-benzotriazin-4(3H)-one (DEPBT): A new coupling reagent with remarkable resistance to racemization. Organic Lett., 1999, 1, 91-93.
29. Harada, N.; Kawaguchi, T.; Inoue, I.; Ohashi, M.; Oda, K.; Hashiyama, T.; Tsujihara, K. Synthesis and antitumor activity of quaternary salts of 2-(2'-oxoalkoxy)-9-hydroxyellipticines. Chem. Pharm. Bull., 1997, 45, 134-137.
30. Schneller, S. W.; Luo, J.-K. Synthesis of 4-amino-1H-pyrrolo[2,3-b]pyridine (1,7-Dideazaadenine) and 1H-pyrrolo[2,3-b]pyridin-4-ol (1,7-Dideazahypoxanthine). J. Org. Chem., 1980, 45, 4045-4048.
31. Shiotani, S.; Tanigochi, K. Furopyridines. XXII [1]. Elaboration of the C-substitutents alpha to the heteronitrogen atom of furo[2,3-b]-, -[3.2-b]-, -[2.3-c]- and -[3,2-c]pyridine. J. Het. Chem., 1997, 34, 901-907.
32. Minakata, S.; Komatsu, M.; Ohshiro, Y. Regioselective functionalization of 1H-pyrrolo[2,3-b]pyridine via its N-oxide. Synthesis, 1992, 661-663.
33. Klemm, L. H.; Hartling, R. Chemistry of thienopyridines. XXIV. Two transformations of thieno[2,3-b]pyridine 7-oxide (1). J Het. Chem., 1976, 13, 1197-1200.
34. Antonini, I.; Claudi, F.; Cristalli, G.; Franchetti, P.; Crifantini, M.; Martelli, S. Synthesis of 4-amino-1-□-D-ribofuranosyl-1H-pyrrolo[2,3-b]pyridine (1-Deazatubercidin) as a potential antitumor agent. J. Med. Chem., 1982, 25, 1258-1261.
35. (a) Regnouf De Vains, J.B.; Papet, A.L.; Marsura, A. New symmetric and unsymmetric polyfunctionalized 2,2'-bipyridines. J. Het. Chem., 1994, 31, 1069-1077. (b) Miura, Y.; Yoshida, M.; Hamana, M. Synthesis of 2,3-fused quinolines from 3-substituted quinoline 1-oxides. Part II, Heterocycles, 1993, 36, 1005-1016. (c) Profft, V.E.; Rolle, W. Uber 4-merkaptoverbindungendes 2-methylpyridins. J. Prakt. Chem., 1960, 283 (11), 22-34.
36. Nesi, R.; Giomi, D.; Turchi, S.; Tedeschi, P., Ponticelli, F. A new one step synthetic approach to the isoxazolo[4,5-b]pyridine system. Synth. Comm., 1992, 22, 2349-2355.
37. (a) Walser, A.; Zenchoff, G.; Fryer, R.I. Quinazolines and 1,4-benzodiazepines. 75. 7-Hydroxyaminobenzodiazepines and derivatives. J. Med. Chem., 1976, 19, 1378-1381. (b) Barker, G.; Ellis, G.P. Benzopyrone. Part I. 6-Amino- and 6-hydroxy-2-subtituted chromones. J. Chem. Soc., 1970, 2230-2233.
38. Ayyangar, N.R; Lahoti, R J.; Daniel, T. An alternate synthesis of 3,4-diaminobenzophenone and mebendazole. Org. Prep. Proced Int.,1991, 23, 627-631.
39. Mahadevan, I.; Rasmussen, M. Ambident heterocyclic reactivity: The alkylation of pyrrolopyridines (azaindoles, diazaindenes). Tetrahedron, 1993, 49, 7337-7352.
40. Chen, B.K.; Saksela, K.; Andino, R.; Baltimore, D. Distinct modes of human immunodeficiency type 1 proviral latency revealed by superinfection of nonproductively infected cell lines with recombinant luciferase-encoding viruses. J. Virol., 1994, 68, 654-660.
41. Bodanszky, M.; Bodanszky, A. "The Practice of Peptide Synthesis" 2nd Ed., Springer-Verlag: Berlin Heidelberg, Germany, 1994.
42. Albericio, F. et al. J. Org. Chem. 1998, 63, 9678.
43. Knorr, R. et al. Tetrahedron Lett. 1989, 30, 1927.
44. (a) Jaszay Z. M. et al. Synth. Commun., 1998 28, 2761 and references cited therein; (b) Bernasconi, S. et al. Synthesis, 1980, 385.
45. (a) Jaszay Z. M. et al. Synthesis, 1989, 745 and references cited therein; (b) Nicolaou, K. C. et al. Angew. Chem. Int. Ed 1999, 38, 1669.
46. Ooi, T. et al. Synlett. 1999, 729.
47. Ford, R. E. et al. J. Med. Chem. 1986, 29, 538.
48. (a) Yeung, K.-S. et al. Bristol-Myers Squibb Unpublished Results. (b) Wang, W. et al. Tetrahedron Lett. 1999, 40, 2501.
49. Brook, M. A. et al. Synthesis, 1983, 201.
50. Yamazaki, N. et al. Tetrahedron Lett. 1972, 5047.
51. Barry A. Bunin "The Combinatorial Index" 1998 Academic Press, San Diego / London pages 78-82.
52. Richard C. Larock Comprehensive Organic Transormations 2nd Ed. 1999, John Wiley and Sons New York.
53. M.D. Mullican et.al. J.Med. Chem. 1991, 34, 2186-2194.
54. Protective groups in organic synthesis 3rd ed. / Theodora W. Greene and Peter G.M. Wuts. New York : Wiley, 1999.
55. Katritzky, Alan R. Lagowski, Jeanne M. The principles of heterocyclic ChemistryNew York : Academic Press, 1968
56. Paquette, Leo A. Principles of modern heterocyclic chemistry New York : Benjamin.
57. Katritzky, Alan R.; Rees, Charles W.; Comprehensive heterocyclic chemistry : the structure, reactions, synthesis, and uses of heterocyclic compounds 1 st ed.Oxford (Oxfordshire) ; New York : Pergamon Press, 1984. 8 v.
58. Katritzky, Alan RHandbook of heterocyclic 1st edOxford (Oxfordshire) ; New York : Pergamon Press, 1985.
59. Davies, David I Aromatic Heterocyclic Oxford; New York : Oxford University Press, 1991.
60. Ellis, G. P. Synthesis of fused Chichester [Sussex] ; New York : Wiley, c1987-c1992. Chemistry of heterocyclic compounds ; v. 47.
61. Joule, J. A Mills, K. ,Smith, G. F. Heterocyclic Chemistry , 3rd ed London ;New York Chapman & Hall, 1995.
62. Katritzky, Alan R., Rees, Charles W. , Scriven, Eric F. V. Comprehensive heterocyclic chemistry II : a review of the literature 1982-1995.
63. The structure, reactions, synthesis, and uses of heterocyclic compounds 1 st ed. Oxford ; New York : Pergamon, 1996. 11 v. in 12 : ill. ; 28 cm.
64. Eicher, Theophil, Hauptmann, Siegfried. The chemistry of heterocycles : structure, reactions, syntheses, and applications Stuttgart; New York: G. Thieme, 1995.
65. Grimmett, M. R. Imidazole and benzimidazole Synthesis London; San Diego : Academic Press, 1997.
66. Advances in heterocyclic chemistry. Published in New York by Academic Press, starting in 1963- present.
67. Gilchrist, T. L. (Thomas Lonsdale) Heterocyclic chemistry 3rd ed. Harlow, Essex : Longman, 1997. 414 p. : ill. ; 24 cm.
68. Farina, Vittorio; Roth, Gregory P. Recent advances in the Stille reaction; Adv. Met.-Org. Chem. 1996, 5, 1-53.
69. Farina, Vittorio; Krishnamurthy, Venkat; Scott, William J. The Stille reaction ; Org. React. (N. Y.) (1997), 50, 1-652.
70. Stille, J. K. Angew. Chem. Int. Ed. Engl. 1986, 25, 508-524.
71. Norio Miyaura and Akiro Suzuki Chem Rev. 1995, 95, 2457.
72. Home, D.A. Heterocycles 1994, 39, 139.
73. Kamitori, Y. et.al. Heterocycles, 1994, 37(1), 153.
74. Shawali, J. Heterocyclic Chem. 1976, 13, 989.
75. a) Kende, A.S.et al. Org. Photochem. Synth. 1972, 1, 92. b) Hankes, L.V.; Biochem. Prep. 1966, 11, 63. c) Synth. Meth. 22, 837.
76. Hulton et. al. Synth. Comm. 1979, 9, 789.
77. Pattanayak, B.K. et.al. Indian J. Chem. 1978, 16, 1030.
78. Chemische Berichte 1902, 35, 1545.
79. Chemische Berichte Ibid 1911, 44, 493.
80. Moubarak, I., Vessiere, R. Synthesis 1980, Vol. 1, 52-53.
81. Ind J. Chem. 1973, 11, 1260.
82. Roomi et.al. Can J. Chem. 1970, 48, 1689.
83. Sorrel, T.N. J. Org. Chem. 1994, 59, 1589.
84. Nitz, T.J. et. al. J. Org. Chem. 1994, 59, 5828-5832.
85. Bowden, K. et.al. J. Chem. Soc. 1946, 953.
86. Nitz, T.J. et. al. J. Org. Chem. 1994, 59, 5828-5832.
87. Scholkopf et. al. Angew. Int. Ed Engl. 1971, 10(5), 333.
88. (a) Behun, J. D.; Levine, R. J. Org. Chem. 1961, 26, 3379. (b) Rossen, K.; Weissman, S.A.; Sager, J.; Reamer, R.A.; Askin, D.; Volante, R.P.; Reider, P.J. Asymmetric Hydrogenation of tetrahydropyrazines: Synthesis of (S)-piperazine 2-tert-butylcarboxamide, an intermediate in the preparation of the HIV protease inhibitor Indinavir. Tetrahedron Lett., 1995, 36, 6419-6422. (c) Jenneskens, L. W.; Mahy, J.; den Berg, E. M. M. de B.-v.; Van der Hoef, I.; Lugtenburg, J. Recl. Trav. Chim. Pays-Bas 1995, 114, 97.
89. Wang, T.; Zhang, Z.; Meanwell, N.A. Benzoylation of Dianions: Preparation of mono-Benzoylated Symmetric Secondary Diamines. J. Org. Chem., 1999, 64, 7661-7662.
90. (a) Adamczyk, M.; Fino, J.R. Synthesis of procainamide metabolites. N-acetyl desethylprocainamide and desethylprocainamide. Org. Prep. Proced Int. 1996, 28, 470-474. (b) Wang, T.; Zhang, Z.; Meanwell, N.A. Regioselective mono-Benzoylation of Unsymmetrical Piperazines. J. Org. Chem., in press.
91. Masuzawa, K.; Kitagawa, M.; Uchida, H. Bull Chem. Soc. Jpn. 1967, 40, 244-245.
92. Furber, M.; Cooper, M. E.; Donald, D. K. Tetrahedron Lett. 1993, 34, 1351-1354.
93. Blair, W. S. et al, PCT WO 00/76521 published December 21, 2000.
94. Mavunkel, B. J. et al, PCT WO 00/71535 published November 30,2000.
95. Wang, T. et al, PCT WO 01/62255 published August 30, 2001. 96. Wallace et al., PCT WO 02/04440 published January 17, 2002.

### SUMMARY DESCRIPTION OF THE INVENTION

The present invention comprises compounds of Formula I, or pharmaceutically acceptable salts thereof, which are effective antiviral agents, particularly as inhibitors of HIV.

The invention relates to compounds of Formula I, including pharmaceutically acceptable salts thereof, 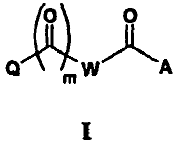 wherein:
Q is either 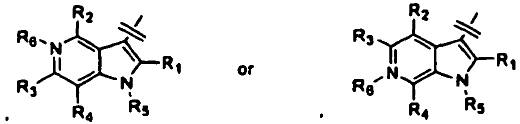 and then R² is selected from the group consisting of hydrogen, halogen and methoxy; and
R₃ is hydrogen;
or Q is: 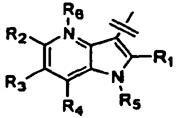 and R² is halogen or hydrogen and R³ is hydrogen;
R⁴ is B;
B is selected from the group consisting of substituted phenyl, heteroaryl, and C(O)R⁷ wherein said heteroaryl is optionally substituted and phenyl is substituted with one to three same or different halogens or from one to two same or different substituents selected from the group F;
F is selected from the group consisting of (C₁₋₆)alkyl, (C₃₋₆)cycloalkyl, aryl, heteroaryl, heteroalicyclic, hydroxy, (C₁₋₆)alkoxy, (C₁₋₆)thioalkoxy, cyano, halogen, carbonyl, benzyl, - NR⁴²C(O)-(C₁₋₆)alkyl, -NR⁴²C(O)-(C₃₋₆)cycloalkyl, -NR⁴²C(O)-aryl, -NR⁴²C(O)-heteroaryl, - NR⁴²C(O)-heteroalicyclic, a cyclic N-amido, -NR⁴²S(O)₂-(C₁₋₆)alkyl, -NR⁴²S(O)₂-(C₃₋₆)cycloalkyl, -NR⁴²S(O)₂-aryl, -NR²S(O)₂-heteroaryl, -NR⁴²S(O)₂-heteroalicyclic, -S(O)₂ NR⁴²R⁴³, NR⁴²R⁴³, (C₁₋₆)alkylC(O)NR⁴²R⁴³, C(O)NR⁴²R⁴³, NHC(O)NR⁴²R⁴³, OC(O)NR⁴²R⁴³, NHC(O)OR^{54'}, (C₁₋₆)alkylNR⁴²R⁴³, COOR⁵⁴, and (C₁₋₆)alkylCOOR⁵⁴ wherein said (C₁₋₆)alkyl, (C₃₋₆)cycloalkyl, aryl, heteroaryl, heteroalicyclic, (C₁₋₆)alkoxy, are optionally substituted with one to three same or different halogens or from one to two same or different substituents selected from the group G;
G is selected from the group consisting of (C₁₋₆)alkyl, (C₃₋₇)cycloalkyl, aryl, heteroaryl, hcteroalicyclic, hydroxy, (C₁₋₆)alkoxy, (C₁₋₆)thioalkoxy, thioaryloxy, cyano, halogen, nitro, carbonyl, thiocarbonyl, benzyl, -NR⁴⁸C(O)-(C₁₋₆)alkyl, -NR⁴⁸C(O)-(C₃₋₆)cycloalkyl, - NR⁴⁸C(O)-aryl, -NR⁴⁸C(O)-heteroaryl, -NR⁴⁸C(O)-heteroalicyclic, a cyclic N-amido, - NR⁴⁸S(O)₂-(C₁₋₆)alkyl, - -NR⁴⁸S(O)₂-(C₃₋₆)cycloalkyl, -NR⁴⁸S(O)₂-aryl, -NR⁴⁸S(O)₂-heteroaryl, -NR⁴⁸S(O)₂-heteroalicyclic, sulfonyl, -S(O)₂ NR⁴⁸R⁴⁹, NR⁴⁸R⁴⁹, (C₁₋₆)alkyl C(O)NR⁴⁸R⁴⁹, C(O)NR⁴⁸R⁴⁹, NHC(O)NR⁴⁸R⁴⁹, OC(O)NR⁴⁸R⁴⁹, NHC(O)OR^{54'}, (C₁₋₆)alkylNR⁴⁸R⁴⁹, COOR⁵⁴, and (C₁₋₆)alkylCOOR^{54'};
R¹ is hydrogen;
m is 2;
R⁵ is hydrogen;
R⁶ does not exist;
R⁷ is selected from the group consisting of aryl, heteroaryl, and heteroalicyclic wherein said aryl, heteroaryl, and heteroalicyclic are optionally substituted with one to three same or different halogens or with from one to two same or different substituents selected from the group F;
A is selected from the group consisting of phenyl and heteroaryl; wherein heteroaryl is pyridinyl, furanyl or thienyl; and said phenyl or said heteroaryl is optionally substituted with one to two of the same or different amino, C₁₋₆alkyl, hydroxy, or halogen;
-W- is 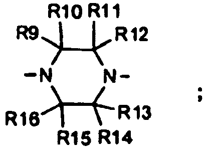
R⁹, R¹⁰, R¹¹, R¹², R¹³, and R¹⁴ are each hydrogen; and
R¹⁵ and R¹⁶ are each independently hydrogen or methyl with the proviso that only one is methyl;
R⁴² and R⁴³ are independently selected from the group consisting of hydrogen, (C₁₋₆)alkyl, (C₁₋₆)alkoxy, (C₃₋₇)cycloalkyl, aryl, heteroaryl and heteroalicyclic; or R⁴² and R⁴³ taken together with the nitrogen to which they are attached form a heteroaryl ring or a heteroalicyclic ring which may contain up to 2 additional heteroatoms selected from N, O, S(O)ₘ, wherein m' is 0, 1, or 2; and wherein said (C₁₋₆)alkyl, (C_{1-ó})alkoxy, (C₃₋₇cycloalkyl, aryl, heteroaryl, and heteroalicyclic are optionally substituted with one to three same or different halogens or from one to two same or different substituents selected from the group G;
R⁴⁸ and R⁴⁹ are independently selected from the group consisting of hydrogen, (C₁₋₆)alkyl, (C₁₋₆)alkoxy, (C₃₋₇)cycloalkyl, allyl, aryl, heteroaryl, heteroalicyclic or R⁴⁸ and R⁴⁹ taken together with the nitrogen to which they are attached form a heteroaryl ring or a heteroalicyclic ring which may contain up to two additional heteroatoms selected from N, O, S(O)ₘ, wherein m' is 0, 1, or 2;
R⁵⁴ is selected from the group consisting of hydrogen, (C₁₋₆)alkyl, (C₃₋₇)cycloalkyl, aryl, heteroaryl, and heteroalicyclic wherein said (C₁₋₆)alkyl, (C₃₋₇)cycloalkyl, aryl, heteroaryl, and heteroalicyclic are optionally substituted with one to three same or different halogens or from one to two same or different substituents selected from the group consisting of: amino, OH, and NR⁵⁵R⁵⁶;
R^{54'} is selected from the group consisting of (C₁₋₆)alkyl, (C₃₋₇)cycloalky), aryl, heteroaryl, and heteroalicyclic wherein said (C₁₋₆)alkyl, (C₃₋₇)cycloalkyl, aryl, heteroaryl, and heteroalicyclic are optionally substituted with one to three same or different halogens or from one to two same or different substituents selected from the group consisting of: amino, OH, and NR⁵⁵R⁵⁶;
R⁵⁵ and R⁵⁶ are independently selected from the group consisting of hydrogen, (C₁₋₆)alkyl, or (C₃₋₇)cycloalkyl.

A group of preferred compounds of Formula I, including pharmaceutically acceptable salts thereof,
Q is 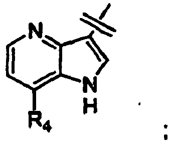
R⁴ is B;
A is Phenyl or 2-pyridyl;
B is heteroaryl, wherein said heteroaryl is optionally substituted with one to three same or different halogens or from one to two same or different substituents selected from the group F;
Most preferred among this group of preferred compounds are those where R⁴ is B;
A is Phenyl or 2-pyridyl and B is heteroaryl, wherein said heteroaryl is optionally substituted with one to three same or different halogens or from one to two same or different substituents selected from the group F;
Compounds where B is heteroaryl, wherein said heteroaryl is optionally substituted with one to three same or different halogens or from one to two same or different substituents selected from the group F claimed;
Preferred groups for B when B is heteroaryl are selected from the group consisting of thiazole, pyridazine, pyrazine, pyrazole, isoxazole, isothiazole, imidazole, furyl, thienyl, oxazole, oxadiazole, thiadiazole, pyrimidine, pyrazole, triazine, triazole, tetrazole, pyridyl, wherein said heteroaryl is optionally substituted with one to three same or different halogens or from one to two same or different substituents selected from the group F;
When B is heteroaryl, most preferred is when said heteroaryl is optionally substituted with one to three same or different halogens or a substituent selected from the group (C₁-C₆ alkyl), amino, -NHC(O)-(C₁-C₆ alkyl), -NHS(O)₂-(C₁-C₆ alkyl), Methoxy,-C(O)-NH₂, C(O)NHMe, C(O)NMe₂, trifluoromethyl, -NHC (C₁-C₆ alkyl), -N(C₁-C₆ alkyl)₂, -heteroaryl, cyclic N-amido; among the most prefered B is thienyl and when B is thienyl most preferred is when the thienyl is optionally substituted with one to three same or different halogens or a substituent selected from the group (C₁-C₆ alkyl), amino, -NHC(O)-(C₁-C₆ alkyl), -NHS(O)₂-(C₁-C₆ alkyl), methoxy, -C(O)-NH₂, C(O)NHMe, C(O)NMe₂, trifluoromethyl, -NHC(C₁-C₆ alkyl), -N(C₁-C₆ alkyl)₂, -heteroaryl, cyclic N-amido;
and even more preferred is when the thienyl is optionally substituted with one to three same or different halogens or a substituent selected from the group (C₁-C₆ alkyl), amino, -NHC(O)-(C₁-C₆ alkyl), -NHS(O)₂-(C₁-C₆ alkyl), methoxy, -C(O)-NH₂, C(O)NHMe, C(O)NMe2, trifluoromethyl, -NHC(C₁-C₆ alkyl), -N(C₁-C₆ alkyl)₂, -heteroaryl, cyclic N-amido;
A further group of preferred compounds of Formula I are, including pharmaceutically acceptable salts thereof wherein,
Q is 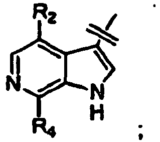
R² is selected from the group consisting of hydrogen, halogen, and methoxy;
R⁴ is B;
B is heteroaryl, wherein said heteroaryl is optionally substituted with one to three same or different halogens or from one to two same or different substituents selected from the group F;
Most preferred are compounds where A is Phenyl or 2-pyridyl;
Most preferred for B is as described above.
Preferred groups for B when B is heteroaryl are selected from the group consisting of thiazole, pyridazine, pyrazine, pyrazole, isoxazole, isothiazole, imidazole, furyl, thienyl, oxazole, oxadiazole, thiadiazole, pyrimidine, pyrazole, triazine, triazole, tetrazole, pyridyl, wherein said heteroaryl is optionally substituted with one to three same or different halogens or from one to two same or different substituents selected from the group F;
When B is heteroaryl, most preferred is when said heteroaryl is optionally substituted with one to three same or different halogens or a substituent selected from the group (C₁-C₆ alkyl), amino, -NHC(O)-(C₁-C₆ alkyl), -NHS(O)₂-(C₁-C₆ alkyl), methoxy,-C(O)-NH₂, C(O)NHMe, C(O)NMe2, trifluoromethyl, -NHC (C₁-C₆ alkyl), -N(C₁-C₆ alkyl)₂, -heteroaryl, cyclic N-amido;
   among the most preferred B is thienyl, pyrazole, or a six membered heteroaryl containing two ring nitrogens.
   and when B is one of these most preferred groups it is optionally substituted with one to three same or different halogens or a substituent selected from the group (C₁-C₆ alkyl), amino, -NHC(O)-(C₁-C₆ alkyl), -NHS(O)₂-(C₁-C₆ alkyl), methoxy, -C(O)-NH₂, C(O)NHMe, C(O)NMe2, trifluoromethyl, -NHC(C₁-C₆ alkyl), -N (C₁-C₆ alkyl)₂, -heteroaryl, cyclic N-amido;
   and even more preferred is when said heteroaryl is optionally substituted with one to three same or different halogens or a substituent selected from the group (C₁-C₆ alkyl), amino, NHC(O)-(C₁-C₆ alkyl), -NHS(O)₂-(C₁-C₆ alkyl), methoxy, -C(O)-NH₂, C(O)NHMe, C(O)NMe2, trifluoromethyl, NHC(C₁-C₆ alkyl), -N (C₁-C₆ alkyl)₂, -heteroaryl, cyclic N-amido;
Another embodiment of a preferred aspect of the invention are compounds of Formula I, including pharmaceutically acceptable salts thereof,
A is selected from the group consisting ofphenyl and heteroaryl in which said heteroaryl is selected from pyridinyl, furanyl and thienyl, and said phenyl or said heteroaryl is optionally substituted with one to two of the same or different amino, C₁₋₆alkyl, or halogen;
R⁹, R¹⁰, R¹¹, R¹², R¹³, and R¹⁴ are each hydrogen; and
R¹⁵ and R¹⁶ are each independently hydrogen or methyl with the proviso that only one is methyl.
Q is either 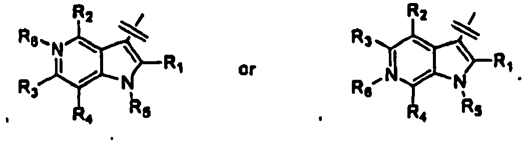 and then R² is selected from the group consisting of hydrogen, halogen and methoxy;
And R₃ is hydrogen;
Or Q is: 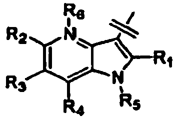 and R² is halogen or hydrogen and R³ is hydrogen;
R⁴ is B; and
F is selected from the group consisting of (C₁₋₆)alkyl, hydroxy, heteroaryl, heteroalicyclic, methoxy, methylthioalkoxy, halogen, carbonyl, C(O)NR⁴²R⁴³, -NR⁴²C(O)-(C₁₋₆)alkyl, -NR⁴²C(O)-(C₃₋₆)cycloalkyl, -NR⁴C(O)-aryl, -NR⁴²C(O)-heteroaryl, -NR⁴²C(O)-heteroalicyclic, a cyclic N-amido, -NR⁴²S(O)₂-(C₁₋₆)alkyl, -NR⁴²S(O)₂-(C₃₋₆)cycloalkyl, -NR⁴²S(O)2-aryL -NR⁴²S(O)₂-heteroaryl, -NR⁴²S(O)2-heteroalicyclic, NR⁴²R⁴³ COOH;
G is selected from the group consisting of (C₁₋₆)alkyl, (C₃₋₇)cycloalkyl, aryl, heteroaryl, heteroalicyclic, hydroxy, (C₁₋₆)alkoxy, (C₁₋₆)thioalkoxy, thioaryloxy, cyano, halogen, nitro, carbonyl, thiocarbonyl, benzyl, -NR⁴⁸C(O)-(C₁₋₆)alkyl, - NR⁴⁸C(O)-(C₃₋₆)cycloalkyl, -NR⁴⁸C(O)-aryl, -NR⁴⁸C(O)-heteroaryl, -NR⁴⁸C(O)-heteroalicyclic, a cyclic N-amido, -NR⁴⁸S(O)₂-(C₁₋₆)alkyl, -NR⁴⁸S(O)₂-(C₁₋₆)cycloalkyl, NR⁴⁸S(O)2-aryl, -NR⁴⁸S(O)₂-heteroaryl, NR⁴⁸S(O)2-heteroalicyclic, sulfonyl, -S(O)2 NR⁴⁸R⁴⁹, NR⁴⁸R⁴⁹, (C₁₋₆)alkyl C(O)NR⁴⁸R⁴⁹, C(O)NR⁴⁸R⁴⁹, NHC(O)NR⁴⁸R⁴⁹*,* OC(O)NR⁴⁸R⁴⁹, NHC(O)OR^{54'}, (C₁₋₆)alkylNR⁴⁸R⁴⁹, COOR⁵⁴, and (C₁₋₆)alkylCOOR^{54'};
R⁷ is selected from the group consisting of aryl, heteroaryl, and heteroalicyclic wherein said aryl, heteroaryl, and heteroalicyclic are optionally substituted with one to three same or different halogens or with from one to two same or different substituents selected from the group F;
R⁴² and R⁴³ are independently selected from the group consisting of hydrogen, (C₁₋₆)alkyl, (C₁₋₆)alkoxy, (C₃₋₇)cycloalkyl, aryl, heteroaryl, heteroalicyclic or R⁴² and R⁴³ taken together with the nitrogen to which they are attached form a heteroaryl ring or a heteroalicyclic ring which may contain up to two additional heteroatoms selected from N, O, S(O)ₘ, wherein m' is 0, 1, or 2; and wherein said (C₁₋₆)alkyl, (C₁₋₆)alkoxy, (C₃₋₇)cycloalkyl, (C₂₋₆)alkenyl, (C₃₋₇)cycloalkenyl, (C₂₋₆)alkynyl, aryl, heteroaryl, and heteroalicyclic are optionally substituted with one to three same or different halogens or from one to two same or different substituents selected from the group G;
R⁴⁸ and R⁴⁹ are independently selected from the group consisting of hydrogen, (C₁₋₆)alkyl, (C₁₋₆alkoxy, (C₃₋₇)cycloalkyl, aryl, heteroaryl, heteroalicyclic or R⁴⁸ and R⁴⁹ taken together with the nitrogen to which they are attached form a heteroaryl ring or a heteroalicyclic ring which may contain up to two additional heteroatoms selected from N, O, S(O)ₘ, wherein m' is 0, 1, or 2;
R⁵⁴ is selected from the group consisting of hydrogen, (C₁₋₆)alkyl, (C₃₋₇)cycloalkyl, aryl, heteroaryl, and heteroalicyclic wherein said (C₁₋₆)alkyl, (C₃₋₇)cycloalkyl, aryl, heteroaryl, and heteroalicyclic are optionally substituted with one to three same or different halogens or from one to two same or different substituents selected from the group consisting of: amino, OH, and NR⁵⁵R⁵⁶;
R^{54'} is selected from the group consisting of (C₁₋₆)alkyl, (C₃₋₇)cycloalkyl, aryl, heteroaryl, and heteroalicyclic wherein said (C₁₋₆)alkyl, (C₃₋₇)cycloalkyl, aryl, heteroaryl, and heteroalicyclic are optionally substituted with one to three same or different halogens or from one to two same or different substituents selected from the group consisting of: amino, OH, and NR⁵⁵R⁵⁶;
R⁵⁵ and R⁵⁶ are independently selected from the group consisting of hydrogen, (C₁₋₆)alkyl, or (C₃₋₇)cycloalkyl

A further group of preferred compounds is those wherein:
Q is 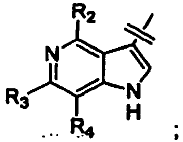
R² is selected from the group consisting of hydrogen or methoxy;
R³ is hydrogen;
R⁴ is B
B is heteroaryl, wherein said heteroaryl is optionally substituted with one to three same or different halogens or from one to two same or different substituents selected from the group F;

A final prefixed aspect of the invention are compounds depicted in Table 2 or Table 4 of the biology section.

A further embodiment is a compound of the present invention for use in a method for treating mammals infected with a virus, wherein said virus is HIV, comprising administering to said mammal an antiviral effective amount of a compound of Formula I.

A further embodiment is a compound of the present invention for use in a method for treating mammals infected with a virus, such as HIV, comprising administering to said mammal an antiviral effective amount of a compound of Formula I in combination with an antiviral effective amount of an AIDS treatment agent selected from the group consisting of: (a) an AIDS antiviral agent; (b) an anti-infective agent; (c) an immunomodulator, and (d) HIV entry inhibitors.

### DETAILED DESCRIPTION OF THE INVENTION

Since the compounds of the present invention, may possess asymmetric centers and therefore occur as mixtures of diastereomers and enantiomers, the present invention includes the individual diastereoisomeric and enantiomeric forms of the compounds of Formula I in addition to the mixtures thereof.

### DEFINITIONS

The term "C₁₋₆ alkyl" as used herein and in the claims (unless specified otherwise) mean straight or branched chain alkyl groups such as methyl, ethyl, propyl, isopropyl, butyl, isobutyl, t-butyl, amyl, hexyl and the like.

"Halogen," refers to chlorine, bromine, iodine or fluorine.

An "aryl" group refers to an all carbon monocyclic or fused-ring polycyclic (i.e., rings which share adjacent pairs of carbon atoms) groups having a completely conjugated pi-electron system. Examples, without limitation, of aryl groups are phenyl napthalenyl and anthracenyl. The aryl group may be substituted or unsubstituted. When substituted the substituted group(s) is preferably one or more selected from alkyl, cycloalkyl, aryl, heteroaryl, heteroalicyclic, hydroxy, alkoxy, aryloxy, heteroaryloxy, heteroalicycloxy, thiohydroxy, thioaryloxy, thioheteroaryloxy, thioheteroalicycloxy, cyano, halogen, nitro, carbonyl, O-carbamyl, N-carbamyl, C-amido, N-amido, C-carboxy, O-carboxy, sulfinyl, sulfonyl, sulfonamido, trihalomethyl, ureido, amino and -NR^{x}R^{y}, wherein R^{x} and R^{y} are independently selected from the group consisting of hydrogen, alkyl, cycloalkyl, aryl, carbonyl, C-carboxy, sulfonyl, trihalomethyl, and, combined, a five- or six-member heteroalicyclic ring.

As used herein, a "heteroaryl" group refers to a monocyclic or fused ring (i.e., rings which share an adjacent pair of atoms) group having in the ring(s) one or more atoms selected from the group consisting of nitrogen, oxygen and sulfur and, in addition, having a completely conjugated pi-electron system. It should be noted that the term heteroaryl is intended to encompass an N-oxide of the parent heteroaryl if such an N-oxide is chemically feasible as is known in the art. Examples, without limitation, of heteroaryl groups are furyl, thienyl, benzothienyl, thiazolyl, imidazolyl, oxazolyl, oxadiazolyl, thiadiazolyl, benzothiazolyl, triazolyl, tetrazolyl, isoxazolyl, isothiazolyl, pyrrolyl, pyranyl, tetrahydropyranyl, pyrazolyl, pyridyl, pyrimidinyl, quinolinyl, isoquinolinyl, purinyl, carbazolyl, benzoxazolyl, benzimidazolyl, indolyl, isoindolyl, pyrazinyl. diazinyl, pyrazine, triazinyltriazine, tetrazinyl, and tetrazolyl. When substituted the substituted group(s) is preferably one or more selected from alkyl, cycloalkyl, aryl, heteroaryl, heteroalicyclic, hydroxy, alkoxy, aryloxy, heteroaryloxy, heteroalicycloxy, thiohydroxy, thioaryloxy, thioheteroaryloxy, thioheteroalicycloxy, cyano, halogen, nitro, carbonyl, O-carbamyl, N-carbamyl, C-amido, N-amido, C-carboxy, O-carboxy, sulfinyl, sulfonyl, sulfonamido, trihalomethyl, ureido, amino, and -NR^{x}R^{y}, wherein R^{x} and R^{y} are as defined above.

As used herein, a "heteroalicyclic" group refers to a monocyclic or fused ring group having in the ring(s) one or more atoms selected from the group consisting of nitrogen, oxygen and sulfur. The rings may also have one or more double bonds. However, the rings do not have a completely conjugated pi-electron system. Examples, without limitation, of heteroalicyclic groups are azetidinyl, piperidyl, piperazinyl, imidazolinyl, thiazolidinyl, 3-pyrrolidin-1-yl, morpholinyl, thiomorpholinyl and tetrahydropyranyl. When substituted the substituted group(s) is preferably one or more selected from alkyl, cycloalkyl, aryl, heteroaryl, heteroalicyclic, hydroxy, alkoxy, aryloxy, heteroaryloxy, heteroalicycloxy, thiohydroxy, thioalkoxy, thioaryloxy, thioheteroaryloxy, thioheteroalicycloxy, cyano, halogen, nitro, carbonyl, thiocarbonyl, O-carbamyl, N-carbamyl, O-thiocarbamyl, N-thiocarbamyl, C-amido, C-thioamido, N-amido, C-carboxy, O-carboxy, sulfinyl, sulfonyl, sulfonamido, trihalomethanesulfonamido, trihalomethanesulfonyl, silyl, guanyl, guanidino, ureido, phosphonyl, amino and -NR^{x}R^{y}, wherein R^{x} and R^{y} are as defined above.

An "alkyl" group refers to a saturated aliphatic hydrocarbon including straight chain and branched chain groups. Preferably, the alkyl group has 1 to 20 carbon atoms (whenever a numerical range; e.g., "1-20", is stated herein, it means that the group, in this case the alkyl group may contain 1 carbon atom, 2 carbon atoms, 3 carbon atoms, etc. up to and including 20 carbon atoms). More preferably, it is a medium size alkyl having 1 to 10 carbon atoms. Most preferably, it is a lower alkyl having 1 to 4 carbon atoms. The alkyl group may be substituted or unsubstituted. When substituted, the substituent group(s) is preferably one or more individually selected from trihaloalkyl, cycloalkyl, aryl, heteroaryl, heteroalicyclic, hydroxy, alkoxy, aryloxy, heteroaryloxy, heteroalicycloxy, thiohydroxy, thioalkoxy, thioaryloxy, thioheteroaryloxy, thioheteroalicycloxy, cyano, halo, nitro, carbonyl, thiocarbonyl, O-carbamyl, N-carbamyl, O-thiocarbamyl, N-thiocarbamyl, C-amido, C-thioamido, N-amido, C-carboxy, O-carboxy, sulfinyl, sulfonyl, sulfonamido, trihalomethanesulfonamido, trihalomethanesulfonyl, and combined, a five- or six-member heteroalicyclic ring.

A "cycloalkyl" group refers to an all-carbon monocyclic or fused ring (i.e., rings which share and adjacent pair of carbon atoms) group wherein one or more rings does not have a completely conjugated pi-electron system. Examples, without limitation, of cycloalkyl groups are cyclopropane, cyclobutane, cyclopentane, cyclopentene, cyclohexane, cyclohexadiene, cycloheptane, cycloheptatriene and adamantane. A cycloalkyl group may be substituted or unsubstituted. When substituted, the substituent group(s) is preferably one or more individually selected from alkyl, aryl, heteroaryl, heteroalicyclic, hydroxy, alkoxy, aryloxy, heteroaryloxy, heteroalicycloxy, thiohydroxy, thioalkoxy, thioaryloxy, thioheteroarylloxy, thioheteroalicycloxy, cyano, halo, nitro, carbonyl, thiocarbonyl, O-carbamyl, N-carbamyl, O-thiocarbamyl, N-thiocarbamyl, C-amido, C-thioamido, N-amido, C-carboxy, O-carboxy, sulfinyl, sulfonyl, sulfonamido, trihalo- methanesulfonamido, trihalomethanesulfonyl, silyl, guanyl, guanidino, ureido, phosphonyl, amino and - NR^{x}R^{y} with R^{x} and R^{y} as defined above.

An "alkenyl" group refers to an alkyl group, as defined herein, consisting of at least two carbon atoms and at least one carbon-carbon double bond.

An "alkynyl" group refers to an alkyl group, as defined herein, consisting of at least two carbon atoms and at least one carbon-carbon triple bond.

A "hydroxy" group refers to an -OH group.

An "alkoxy" group refers to both an -O-alkyl and an -O-cycloalkyl group as defined herein.

An "aryloxy" group refers to both an -0-aryl and an -0-heteroaryl group, as defined herein.

A "heteroaryloxy" group refers to a heteroaryl-O- group with heteroaryl as defined herein.

A "heteroalicycloxy" group refers to a heteroalicyclic-O- group with heteroalicyclic as defined herein.

A "thiohydroxy" group refers to an -SH group.

A "thioalkoxy" group refers to both an S-alkyl and an -S-cycloalkyl group, as defined herein.

A "thioaryloxy" group refers to both an -S-aryl and an -S-heteroaryl group, as defined herein.

A "thioheteroaryloxy" group refers to a heteroaryl-S- group with heteroaryl as defined herein.

A "thioheteroalicycloxy" group refers to a heteroalicyclic-S- group with heteroalicyclic as defined herein.

A "carbonyl" group refers to a -C(=O)-R" group, where R" is selected from the group consisting of hydrogen, alkyl, alkenyl, alkynyl, cycloalkyl, aryl, heteroaryl (bonded through a ring carbon) and heteroalicyclic (bonded through a ring carbon), as each is defined herein.

An "aldehyde" group refers to a carbonyl group where R" is hydrogen.

A "thiocarbonyl" group refers to a -C(=S)-R" group, with R" as defined herein.

A "Keto" group refers to a -CC(=O)C- group wherein the carbon on either or both sides of the C=O may be alkyl, cycloalkyl, aryl or a carbon of a heteroaryl or heteroaliacyclic group.

A "trihalomethanecarbonyl" group refers to a Z₃CC(=O)- group with said Z being a halogen.

A "C-carboxy" group refers to a -C(=O)O-R" groups, with R" as defined herein.

An "O-carboxy" group refers to a R"C(-O)O-group, with R" as defined herein.

A "carboxylic acid" group refers to a C-carboxy group in which R" is hydrogen.

A "trihalomethyl" group refers to a -CZ₃, group wherein Z is a halogen group as defined herein.

A "trihalomethanesulfonyl" group refers to an Z₃CS(=O)₂- groups with Z as defined above.

A "trihalomethanesulfonamido" group refers to a Z₃CS(=O)₂NR^{x}- group with Z and R^{x} as defined herein.

A "sulfinyl" group refers to a -S(=O)-R" group, with R" as defined herein and, in addition, as a bond only; i.e., -S(O)-.

A "sulfonyl" group refers to a -S(=O)₂R" group with R" as defmed herein and, in addition as a bond only; i.e., -S(O)₂-.

A "S-sulfonamido" group refers to a -S(=O)2NR^{X}R^{Y}, with R^{X} and R^{Y} as defined herein.

A "N-Sulfonamido" group refers to a R"S(=O)₂NRₓ- group with Rₓ as defined herein.

A "O-carbamyl" group refers to a -OC(=O)NR^{x}R^{y} as defined herein.

A "N-carbamyl" group refers to a R^{x}OC(=O)NR^{y} group, with R^{x} and R^{y} as defined herein.

A "O-thiocarbamyl" group refers to a -OC(=S)NR^{x}R^{y} group with R^{x} and R^{y} as defined herein.

A "N-thiocarbamyl" group refers to a R^{x}OC(=S)NR^{y}- group with R^{x} and R^{y} as defined herein.

An "amino" group refers to an NH₂ group.

A "C-amido" group refers to a -C(=O)NR^{x}R^{y} group with R^{x} and R^{y} as defined herein.

A "C-thioamido" group refers to a -C(=S)NR^{x}R^{y} group, with R^{x} and R^{y} as defined herein.

A "N-amido" group refers to a R^{x}C(=O)NR^{y}- group, with R^{x} and R^{y} as defined herein.

An "ureido" group refers to a -NR^{x}C(=O)NR^{y}R^{y2} group with R^{x} and R^{y} as defined herein and R^{y2} defined the same as R^{x} and R^{y}.

A "guanidino" group refers to a -R^{x}NC(=N)NR^{y}R^{y2} group, with R^{x}, R^{y} and R^{y2} as defined herein.

A "guanyl" group refers to a R^{x}R^{y}NC(=N)- group, with R^{x} and R^{Y} as defined herein.

A "cyano" group refers to a-CN group.

A "silyl" group refers to a -Si(R")₃, with R" as defined herein.

A "phosphonyl" group refers to a P(=O)(OR^{x})₂ with R^{x} as defined herein.

A "hydrazino" group refers to a NR^{x}NR^{y}R^{y2} group with R^{x}, R^{y} and R^{y2} as defined herein.

Any two adjacent R groups may combine to form an additional aryl, cycloalkyl, heteroaryl or heterocyclic ring fused to the ring initially bearing those R groups.

It is known in the art that nitogen atoms in heteroaryl systems can be "participating in a heteroaryl ring double bond", and this refers to the form of double bonds in the two tautomeric structures which comprise five-member ring heteroaryl groups. This dictates whether nitrogens can be substituted as well understood by chemists in the art. The disclosure and claims of the present invention are based on the known general principles of chemical bonding. It is understood that the claims do not encompass structures known to be unstable or not able to exist based on the literature.

Physiologically acceptable salts and prodrugs of compounds disclosed herein are within the scope of this invention. The term "pharmaceutically acceptable salt" as used herein and in the claims is intended to include nontoxic base addition salts. Suitable salts include those derived from organic and inorganic acids such as, without limitation, hydrochloric acid, hydrobromic acid, phosphoric acid, sulfuric acid, methanesulfonic acid, acetic acid, tartaric acid, lactic acid, sulfinic acid, citric acid, maleic acid, fumaric acid, sorbic acid, aconitic acid, salicylic acid, phthalic acid, and the like. The term "pharmaceutically acceptable salt" as used herein is also intended to include salts of acidic groups, such as a carboxylate, with such counterions as ammonium, alkali metal salts, particularly sodium or potassium, alkaline earth metal salts, particularly calcium or magnesium, and salts with suitable organic bases such as lower alkylamines (methylamine, ethylamine, cyclohexylamine, and the like) or with substituted lower alkylamines (e.g. hydroxyl-substituted alkylamines such as diethanolamine, triethanolamine or tris(hydroxymethyl)- aminomethane), or with bases such as piperidine or morpholine.

In the method of the present invention, the term "antiviral effective amount" means the total amount of each active component of the method that is sufficient to show a meaningful patient benefit, i.e., healing of acute conditions characterized by inhibition of the HIV infection. When applied to an individual active ingredient, administered alone, the term refers to that ingredient alone. When applied to a combination, the term refers to combined amounts of the active ingredients that result in the therapeutic effect, whether administered in combination, serially or simultaneously. The terms "treat, treating, treatment" as used herein and in the claims means preventing or ameliorating diseases associated with HIV infection.

The present invention is also directed to combinations of the compounds with one or more agents useful in the treatment of AIDS. For example, the compounds of this invention may be effectively administered, whether at periods of pre-exposure and/or post-exposure, in combination with effective amounts of the AIDS antivirals, immunomodulators, antiinfectives, or vaccines, such as those in the following table.

### ANTIVIRALS

| Drug Name | Manufacturer | Indication |
|---|---|---|
| 097 | Hoechst/Bayer | HIV infection, |
| | | AIDS, ARC (non-nucleoside reverse transcriptase (RT) inhibitor) |
| | | |
| Amprenivir | Glaxo Wellcome | HIV infection, |
| 141 W94 | | AIDS, ARC |
| GW 141 | | (protease inhibitor) |
| | | |
| Abacavir (1592U89) | Glaxo Wellcome | HIV infection, |
| GW 1592 | | AIDS, ARC |
| | | (RT inhibitor) |
| | | |
| Acemannan | Carrington Labs (Irving, TX) | ARC |
| | | |
| Acyclovir | Burroughs Wellcome | HIV infection, AIDS, |
| | | ARC, in combination with AZT |
| | | |
| AD-439 | Tanox Biosystems | HIV infection, AIDS, |
| | | ARC |
| AD-519 | Tanox Biosystems | HIV infection, AIDS, |
| | | ARC |
| | | |
| Adefovir dipivoxil | Gilead Sciences | HIV infection |
| AL-721 | Ethigen | ARC, PGL |
| | (Los Angeles, CA) | HIV positive, AIDS |
| | | |
| Alpha Interferon | Glaxo Wellcome | Kaposi's sarcoma, |
| | | HIV in combination w/Retrovir |
| | | |
| Ansamycin | Adria Laboratories | ARC |
| LM 427 | (Dublin, OH) | |
| | Erbamont | |
| | (Stamford, CT) | |
| | | |
| Antibody which | Advanced Biotherapy | AIDS, ARC |
| Neutralizes pH | Concepts | |
| Labile alpha aberrant | (Rockville, MD) | |
| Interferon | | |
| | | |
| AR177 | Aronex Pharm | HIV infection, AIDS, |
| | | ARC |
| | | |
| Beta-fluoro-ddA | Nat'l Cancer Institute | AIDS-associated |
| | | diseases |
| | | |
| BMS-232623 | Bristol-Myers Squibb/ | HIV infection, |
| (CGP-73547) | Novartis | AIDS, ARC |
| | | (protease inhibitor) |
| | | |
| BMS-234475 | Bristol-Myers Squibb/ | HIV infection, |
| (CGP-61755) | Novartis | AIDS, ARC |
| | | (protease inhibitor) |
| | | |
| CI-1012 | Warner-Lambert | HIV-1 infection |
| | | |
| Cidofovir | Gilead Science | CMV retinitis, |
| | | herpes, papillomavirus |
| | | |
| Curdlan sulfate | AJI Pharma USA | HIV infection |
| | | |
| Cytomegalovirus Immune globin | MedImmune | CMV retinitis |
| Cytovene | Syntex | Sight threatening |
| Ganciclovir | | CMV |
| | | peripheral CMV |
| | | retinitis |
| | | |
| Delaviridine | Pharmacia-Upjohn | HIV infection, |
| | | AIDS, ARC |
| | | (RT inhibitor) |
| | | |
| Dextran Sulfate | Ueno Fine Chem. | AIDS, ARC, HIV |
| | Ind. Ltd. (Osaka, | positive |
| | Japan) | asymptomatic |
| | | |
| ddC | Hoffman-La Roche | HIV infection, AIDS, |
| Dideoxycytidine | | ARC |
| | | |
| ddI | Bristol-Myers Squibb | HIV infection, AIDS, |
| Dideoxyinosine | | ARC; combination |
| | | with AZT/d4T |
| | | |
| DMP-450 | AVID | HIV infection, |
| | (Camden, NJ) | AIDS, ARC |
| | | (protease inhibitor) |
| | | |
| Efavirenz | DuPont Merck | HIV infection, |
| (DMP 266) | | AIDS, ARC |
| (-)6-Chloro-4-(S)-cyclopropylethynyl-4(S)-trifluoro-methyl-1,4-dihydro-2H-3,1-benzoxazin-2-one, STOCRINE | | (non-nucleoside RT inhibitor) |
| | | |
| EL10 | Elan Corp, PLC | HIV infection |
| | (Gainesville, GA) | |
| | | |
| Famciclovir | Smith Kline | herpes zoster, |
| | | herpes simplex |
| FTC | Emory University | HIV infection, |
| | | AIDS, ARC |
| | | (reverse transcriptase inhibitor) |
| | | |
| GS 840 | Gilead | HIV infection, |
| | | AIDS, ARC |
| | | (reverse transcriptase inhibitor) |
| | | |
| HBY097 | Hoechst Marion | HIV infection, |
| | Roussel | AIDS, ARC |
| | | (non-nucleoside reverse transcriptase inhibitor) |
| | | |
| Hypericin | VIMRx Pharm. | HIV infection, AIDS, |
| | | ARC |
| | | |
| Recombinant Human | Triton Biosciences | AIDS, Kaposi's |
| Interferon Beta | (Almeda, CA) | sarcoma, ARC |
| | | |
| Interferon alfa-n3 | Interferon Sciences | ARC, AIDS |
| | | |
| Indinavir | Merck | HIV infection, AIDS, |
| | | ARC, asymptomatic |
| | | HIV positive, also in combination with |
| | | AZT/ddI/ddC |
| | | |
| ISIS 2922 | ISIS Pharmaceuticals | CMV retinitis |
| | | |
| KNI-272 | Nat'l Cancer Institute | HIV-assoc. diseases |
| | | |
| Lamivudine, 3TC | Glaxo Wellcome | HIV infection, |
| | | AIDS, ARC |
| | | (reverse transcriptase inhibitor); also with AZT |
| Lobucavir | Bristol-Myers Squibb | CMV infection |
| Nelfinavir | Agouron | HIV infection, |
| | Pharmaceuticals | AIDS, ARC |
| | | (protease inhibitor) |
| | | |
| Nevirapine | Boeheringer | HIV infection, |
| | Ingleheim | AIDS, ARC |
| | | (RT inhibitor) |
| | | |
| Novapren | Novaferon Labs, Inc. (Akron, OH) | HIV inhibitor |
| | | |
| Peptide T | Peninsula Labs | AIDS |
| Octapeptide | (Belmont, CA) | |
| Sequence | | |
| | | |
| Trisodium | Astra Pharm. | CMV retinitis, HIV |
| Phosphonoformate | Products, Inc. | infection, other CMV |
| | | infections |
| | | |
| PNU-140690 | Pharmacia Upjohn | HIV infection, |
| | | AIDS, ARC |
| | | (protease inhibitor) |
| | | |
| Probucol | Vyrex | HIV infection, AIDS |
| | | |
| RBC-CD4 | Sheffield Med. | HIV infection, |
| | Tech (Houston, TX) | AIDS, ARC |
| | | |
| Ritonavir | Abbott | HIV infection, |
| | | AIDS, ARC |
| | | (protease inhibitor) |
| | | |
| Saquinavir | Hoffmann- | HIV infection, |
| | LaRoche | AIDS, ARC |
| | | (protease inhibitor) |
| | | |
| Stavudine; d4T | Bristol-Myers Squibb | HIV infection, AIDS, |
| Didehydrodeoxy- | | ARC |
| thymidine | | |
| | | |
| Valaciclovir | Glaxo Wellcome | Genital HSV & CMV infections |
| Virazole | Viratek/ICN | asymptomatic HIV |
| Ribavirin | (Costa Mesa, CA) | positive, LAS, ARC |
| | | |
| VX-478 | Vertex | HIV infection, AIDS, |
| | | ARC |
| | | |
| Zalcitabine | Hoffmann-LaRoche | HIV infection, AIDS, |
| | | ARC, with AZT |
| | | |
| Zidovudine; AZT | Glaxo Wellcome | HIV infection, AIDS, |
| | | ARC, Kaposi's |
| | | sarcoma, in combination with |
| | | other therapies |
| | | |

| IMMUNOMODULATORS | | |
|---|---|---|
| Drug Name | Manufacturer | Indication |
| AS-101 | Wyeth-Ayerst | AIDS |
| Bropirimine | Pharmacia Upjohn | Advanced AIDS |
| Acemannan | Carrington Labs, Inc. (Irving, TX) | AIDS, ARC |
| CL246,738 | American Cyanamid Lederle Labs | AIDS, Kaposi's sarcoma |
| EL 10 | Elan Corp, PLC (Gainesville, GA) | HIV infection |
| FP-21399 | Fuki ImmunoPharm | Blocks HIV fusion with CD4+ cells |
| Gamma Interferon | Genentech | ARC, in combination |
| | | w/TNF (tumor necrosis factor) |
| | | |
| Granulocyte | Genetics Institute | AIDS |
| Macrophage Colony | Sandoz | |
| Stimulating Factor | | |
| | | |
| Granulocyte | Hoechst-Roussel | AIDS |
| Macrophage Colony | Immunex | |
| Stimulating Factor | | |
| | | |
| Granulocyte | Schering-Plough | AIDS, |
| Macrophage Colony | | combination |
| Stimulating Factor | | w/AZT |
| | | |
| HIV Core Particle | Rorer | Seropositive HIV |
| Immunostimulant | | |
| | | |
| IL-2 | Cetus | AIDS, in combination |
| Interleukin-2 | | w/AZT |
| | | |
| IL-2 | Hoffman-LaRoche | AIDS, ARC, HIV, in |
| Interleukin-2 | Immunex | combination w/AZT |
| | | |
| IL-2 | Chiron | AIDS, increase in |
| Interleukin-2 | | CD4 cell counts |
| (aldeslukin) | | |
| | | |
| Immune Globulin | Cutter Biological | Pediatric AIDS, in |
| Intravenous | (Berkeley, CA) | combination w/AZT |
| (human) | | |
| | | |
| IMREG-1 | Imreg | AIDS, Kaposi's |
| | (New Orleans, LA) | sarcoma, ARC, PGL |
| | | |
| IMREG-2 | Imreg | AIDS, Kaposi's |
| | (New Orleans, LA) | sarcoma, ARC, PGL |
| | | |
| Imuthiol Diethyl | Merieux Institute | AIDS, ARC |
| Dithio Carbamate | | |
| | | |
| Alpha-2 | Schering Plough | Kaposi's sarcoma |
| Interferon | | w/AZT, AIDS |
| Methionine- | TNI Pharmaceutical | AIDS, ARC |
| Enkephalin | (Chicago, IL) | |
| | | |
| MTP-PE | Ciba-Geigy Corp. | Kaposi's sarcoma |
| Muramyl-Tripeptide | | |
| | | |
| Granulocyte | Amgen | AIDS, in combination |
| Colony Stimulating | | w/AZT |
| Factor | | |
| | | |
| Remune | Immune Response | Immunotherapeutic |
| | Corp. | |
| | | |
| rCD4 | Genentech | AIDS, ARC |
| Recombinant | | |
| Soluble Human CD4 | | |
| | | |
| rCD4-IgG | | AIDS, ARC |
| hybrids | | |
| | | |
| Recombinant | Biogen | AIDS, ARC |
| Soluble Human CD4 | | |
| | | |
| Interferon | Hoffman-La Roche | Kaposi's sarcoma |
| Alfa 2a | | AIDS, ARC, |
| | | in combination w/AZT |
| | | |
| SK&F 106528 | Smith Kline | HIV infection |
| Soluble T4 | | |
| | | |
| Thymopentin | Immunobiology | HIV infection |
| | Research Institute | |
| | (Annandale, NJ) | |
| | | |
| Tumor Necrosis | Genentech | ARC, in combination |
| Factor; TNF | | w/gamma Interferon |

### ANTI-INFECTIVES

| Drug Name | Manufacturer | Indication |
|---|---|---|
| Clindamycin with | Pharmacia Upjohn | PCP |
| Primaquine | | |
| | | |
| Fluconazole | Pfizer | Cryptococcal |
| | | meningitis, |
| | | candidiasis |
| | | |
| Pastille | Squibb Corp. | Prevention of |
| Nystatin Pastille | | oral candidiasis |
| | | |
| Ornidyl | Merrell Dow | PCP |
| Eflornithine | | |
| | | |
| Pentamidine | LyphoMed | PCP treatment |
| Isethionate (IM & IV) | (Rosemont, IL) | |
| | | |
| Trimethoprim | | Antibacterial |
| | | |
| Trimethoprim/sulfa | | Antibacterial |
| | | |
| Piritrexim | Burroughs Wellcome | PCP treatment |
| | | |
| Pentamidine | Fisons Corporation | PCP prophylaxis |
| Isethionate for | | |
| Inhalation | | |
| | | |
| Spiramycin | Rhone-Poulenc | Cryptosporidial |
| | diarrhea | |
| | | |
| Intraconazole- | Janssen-Pharm. | Histoplasmosis; |
| R51211 | | cryptococcal |
| | | meningitis |
| | | |
| Trimetrexate | Warner-Lambert | PCP |
| Daunorubicin | NeXstar, Sequus | Kaposi's sarcoma |
| | | |
| Recombinant Human | Ortho Pharm. Corp. | Severe anemia |
| Erythropoietin | | assoc. with AZT |
| | | therapy |
| | | |
| Recombinant Human | Serono | AIDS-related |
| Growth Hormone | | wasting, cachexia |
| | | |
| Megestrol Acetate | Bristol-Myers Squibb | Treatment of |
| | | anorexia assoc. |
| | | W/AIDS |
| | | |
| Testosterone | Alza, Smith Kline | AIDS-related wasting |
| | | |
| Total Enteral | Norwich Eaton | Diarrhea and |
| Nutrition | Pharmaceuticals | malabsorption |
| | | related to AIDS |

Additionally, the compounds of the invention herein may be used in combination with another class of agents for treating AIDS which are called HIV entry inhibitors. Examples of such HIV entry inhibitors are discussed in DRUGS OF THE FUTURE 1999,24(12), pp. 1355-1362; CELL, Vol. 9, pp. 243-246, Oct. 29, 1999; and DRUG DISCOVERY TODAY, Vol. 5, No. 5, May 2000, pp. 183-194.

It will be understood that the scope of combinations of the compounds of this invention with AIDS antivirals, immunomodulators, anti-infectives, HIV entry inhibitors or vaccines is not limited to the list in the above Table, but includes in principle any combination with any pharmaceutical composition useful for the treatment of AIDS.

Preferred combinations are simultaneous or alternating treatments of with a compound of the present invention and an inhibitor of HIV protease and/or a non-nucleoside inhibitor of HIV reverse transcriptase. An optional fourth component in the combination is a nucleoside inhibitor of HIV reverse transcriptase, such as AZT, 3TC, ddC or ddI. A preferred inhibitor of HIV protease is indinavir, which is the sulfate salt of N-(2(R)-hydroxy-1-(S)-indanyl)-2(R)-phenylmethyl-4-(S)-hydroxy-5-(1-(4-(3-pyridyl-methyl)-2(S)-N'-(t-butylcarboxamido)-piperazinyl))-pentaneamide ethanolate, and is synthesized according to U.S. 5,413,999. Indinavir is generally administered at a dosage of 800 mg three times a day. Other preferred protease inhibitors are nelfinavir and ritonavir. Another preferred inhibitor of HIV protease is saquinavir which is administered in a dosage of 600 or 1200 mg tid. Preferred non-nucleoside inhibitors of HIV reverse transcriptase include efavirenz. The preparation of ddC, ddI and AZT are also described in EPO 0,484,071. These combinations may have unexpected effects on limiting the spread and degree of infection of HIV. Preferred combinations include those with the following (1) indinavir with efavirenz, and, optionally, AZT and/or 3TC and/or ddI and/or ddC; (2) indinavir, and any of AZT and/or ddI and/or ddC and/or 3TC, in particular, indinavir and AZT and 3TC; (3) stavudine and 3TC and/or zidovudine; (4) zidovudine and lamivudine and 141W94 and 1592U89; (5) zidovudine and lamivudine.

In such combinations the compound of the present invention and other active agents may be administered separately or in conjunction. In addition, the administration of one element may be prior to, concurrent to, or subsequent to the administration of other agent(s).

The preparative procedures and anti-HIV-1 activity of the novel azaindole piperazine diamide analogs of Formula I are summarized below in Schemes 1-64.

### Abbreviations

The following abbreviations, most of which are conventional abbreviations well known to those skilled in the art, are used throughout the description of the invention and the examples. Some of the abbreviations used are as follows:
- h: = hour(s)
- rt: = room temperature
- mol: = mole(s)
- mmol: = millimole(s)
- g: = gram(s)
- mg: = milligram(s)
- mL: = milliliter(s)
- TFA: = Trifluoroacetic Acid

- DCE: = 1,2-Dichloroethane
- CH₂Cl₂: = Dichloromethane
- TPAP: = tetrapropylanunonium perruthenate
- THF: = Tetrahydofuran
- DEPBT: = 3-(Diethoxyphosphoryloxy)-1,2,3-benzotriazin-4(3H)-one
- DMAP: = 4-dimethylaminopyridine
- P-EDC: = Polymer supported 1-(3-dimethylaminopropyl)-3-ethylcarbodiimide
- EDC: = 1-(3-dimethylaminopropyl)-3-ethylcarbodiimide
- DMF: = *N,N*-dimethylformamide
- Hunig's Base: = *N,N*-Diisopropylethylamine
- mCPBA: = *meta*-Chloroperbenzoic Acid
- azaindole: = 1*H*-Pyrrolo-pyridine
- 4-azaindole: = 1*H*-pyrrolo[3,2-b]pyridine
- 5-azaindole =: 1*H*-Pyrrolo[3,2-c]pyridine
- 6-azaindole: = 1*H*-pyrrolo[2,3-c]pyridine
- 7-azaindole: = 1*H*-Pyrrolo[2,3-b]pyridine
- PMB: = 4-Methoxybenzyl
- DDQ: = 2, 3-Dichloro-5, 6-dicyano-1, 4-benzoquinone
- OTf: = Trifluoromethanesulfonoxy
- NMM: = 4-Methylmorpholine
- PIP-COPh: = 1-Benzoylpiperazine
- NaHMDS: = Sodium hexamethyldisilazide
- EDAC: = 1-(3-Dimethylaminopropyl)-3-ethylcarbodiimide
- TMS: = Trimethylsilyl
- DCM: = Dichloromethane
- DCE: = Dichloroethane

- MeOH: = Methanol
- THF: = Tetrahrdrofuran
- EtOAc: = Ethyl Acetate
- LDA: = Lithium diisopropylamide
- TMP-Li: = 2,2,6,6-tetramethylpiperidinyl lithium
- DME: = Dimethoxyethane
- DIBALH: = Diisobutylaluminum hydride
- HOBT: = 1-hydroxybenzotriazole
- CBZ: = Benzyloxycarbonyl
- PCC: = Pyridinium chlorochromate

### Chemistry

The present invention comprises compounds of Formula I, their pharmaceutical formulations, and their use in patients suffering from or susceptible to HIV infection. The compounds of Formula I include pharmaceutically acceptable salts thereof.

General procedures to construct substituted azaindole piperazine diamides of Formula I and intermediates useful for their synthesis are described in the following Schemes.

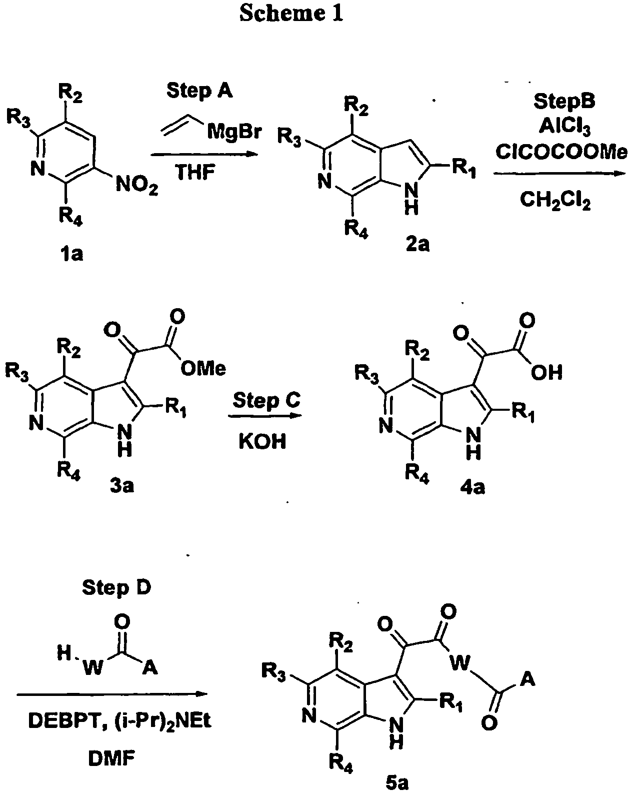

Step A in Scheme 1 depicts the synthesis of an aza indole intermediate, 2, via the well known Bartoli reaction in which vinyl magnesium bromide reacts with an aryl or heteroaryl nitro group, such as in 1, to form a five-membered nitrogen containing ring as shown. Some references for the above transformation include: Bartoli et al. a) Tetrahedron Lett. 1989, 30, 2129*. b)* J. Chem. Soc. Perkin Trans. 1 1991, 2757. c) J. Chem. Soc. Perkin Trans. II 1991, 657. d) Synthesis (1999), 1594. In the preferred procedure, a solution of vinyl Magnesium bromide in THF (typically 1.0M but from 0.25 to 3.0M) is added dropwise to a solution of the nitro pyridine in THF at -78° under an inert atmosphere of either nitrogen or Argon. After addition is completed, the reaction temperature is allowed to warm to -20° and then is stirred for approximately 12h before quenching with 20% aq ammonium chloride solution. The reaction is extracted with ethyl acetate and then worked up in a typical manner using a drying agent such as anhydrous magnesium sulfate or sodium sulfate. Products are generally purified using chromatography over Silica gel. Best results are generally achieved using freshly prepared vinyl Magnesium bromide. In some cases, vinyl Magnesium chloride may be substituted for vinyl Magnesium bromide.

Substituted azaindoles may be prepared by methods described in the literature or may be available from commercial sources. Thus there are many methods for carrying out step A in the literature and the specific examples are too numerous to even list Alternative syntheses of aza indoles and general methods for carrying out step A include, but are not limited to, those described in the following references (a-k below): a) Prokopov, A. A.; Yakhontov, L. N. Khim.-Farm. Zh. 1994, 28(7), 30-51; b) Lablache-Combier, A. Heteroaromatics. Photoinduced Electron Transfer 1988, Pt. C, 134-312; c) Saify, Zafar Said. Pak. J. Pharmacol. 1986, 2(2), 43-6; d) Bisagni, E. Jerusalem Symp. Quantum Chem. Biochem. 1972, 4, 439-45; e) Yakhontov, L. N. Usp. Khim. 1968, 37(7), 1258-87; f) Willette, R. E. Advan. Heterocycl. Chem. 1968, 9, 27-105; g) Mahadevan, I.; Rasmussen, M. Tetrahedron 1993, 49(33), 7337-52; h) Mahadevan, I.; Rasmussen, M. J. Heterocycl. Chem. 1992, 29(2), 359-67; i) Spivey, A. C.; Fekner, T.; Spey, S. E.; Adams, H. J. Org. Chem. 1999, 64(26), 9430-9443; j) Spivey, A.C.; Fekner, T.; Adams, H. Tetrahedron Lett. 1998, 39(48), 8919-8922; k) Advances in Heterocyclic Chemistry (Academic press) 1991, Vol. 52, pg 235-236 and references therein.

**Step B.** Intermediate 3 can be prepared by reaction of aza-indole, intermediate 2, with an excess of CICOCOOMe in the presence of AlCl₃ (aluminum chloride) (Sycheva et al, Ref. 26, Sycheva, T.V.; Rubtsov, N.M.; Sheinker, Yu.N.; Yakhontov, L.N. Some reactions of 5-cyano-6-chloro-7-azaindoles and lactam-lactim tautomerism in 5-cyano-6-hydroxy-7-azaindolines. Khim. Geterotsikl. Soedin., 1987, 100-106). Typically an inert solvent such as CH₂Cl₂ is used but others such as THF, Et₂O, DCE, dioxane, benzene, or toluene may find applicability either alone or in mixtures. Other oxalate esters such as ethyl or benzyl mono esters of oxalic acid could also suffice for either method shown above. More lipophilic esters ease isolation during aqueous extractions. Phenolic or substituted phenolic (such as pentafluorophenol) esters enable direct coupling of the HW(C=O)A group, such as a piperazine, in Step D without activation. Lewis acid catalysts, such as tin tetrachloride, titanium IV chloride, and aluminum chloride are employed in Step B with aluminum chloride being most preferred. Alternatively, the azaindole is treated with a Grignard reagent such as MeMgI (methyl magnesium iodide), methyl magnesium bromide or ethyl magnesium bromide and a zinc halide, such as ZnCl₂ (zinc chloride) or zinc bromide, followed by the addition of an oxalyl chloride mono ester, such as CICOCOOMe (methyl chlorooxoacetate) or another ester as above, to afford the aza-indole glyoxyl ester (Shadrina et al, Ref. 25). Oxalic acid esters such as methyl oxalate, ethyl oxalate or as above are used. Aprotic solvents such as CH₂Cl₂, Et₂O, benzene, toluene, DCE, or the like may be used alone or in combination for this sequence. In addition to the oxalyl chloride mono esters, oxalyl chloride itself may be reacted with the azaindole and then further reacted with an appropriate amine, such as a piperazine derivative (See Scheme 52, for example).

**Step C.** Hydrolysis of the methyl ester, (intermediate 3, Scheme 1) affords a potassium salt of intermediate 4, which is coupled with mono-benzoylated piperazine derivatives as shown in Step D of Scheme 1. Some typical conditions employ methanolic or ethanolic sodium hydroxide followed by careful acidification with aqueous hydrochloric acid of varying molarity but 1M HCl is preferred. The acidification is not utilized in many cases as described above for the preferred conditions. Lithium hydroxide or potassium hydroxide could also be employed and varying amounts of water could be added to the alcohols. Propanols or butanols could also be used as solvents. Elevated temperatures up to the boiling points of the solvents may be utilized if ambient temperatures do not suffice. Alternatively, the hydrolysis may be carried out in a non polar solvent such as CH₂Cl₂ or THF in the presence of Triton B. Temperatures of -78 °C to the boiling point of the solvent may be employed but -10 °C is preferred. Other conditions for ester hydrolysis are listed in reference 41 and both this reference and many of the conditions for ester hydrolysis are well known to chemists of average skill in the art.

### Alternative procedures for step B and C:

### Imidazolium Chloroaluminate:

We found that ionic liquid 1-alkyl-3-alkylimidazolium chloroaluminate is generally useful in promoting the Friedel-Crafts type acylation of indoles and azaindoles. The ionic liquid is generated by mixing 1-alkyl-3-alkylimidazolium chloride with aluminium chloride at room temperature with vigorous stirring. 1:2 or 1:3 molar ratio of 1-alkyl-3-alkylimidazolium chloride to aluminium chloride is preferred. One particular useful imidazolium chloroaluminate for the acylation of azaindole with methyl or ethyl chlorooxoacetate is the 1-ethyl-3-methylimidazolium chloroaluminate. The reaction is typically performed at ambient temperature and the azaindoleglyoxyl ester can be isolated. More conveniently, we found that the glyoxyl ester can be hydrolyzed *in situ* at ambient temperature on prolonged reaction time (typically overnight) to give the corresponding glyoxyl acid for amide formation **(Scheme 1)**.

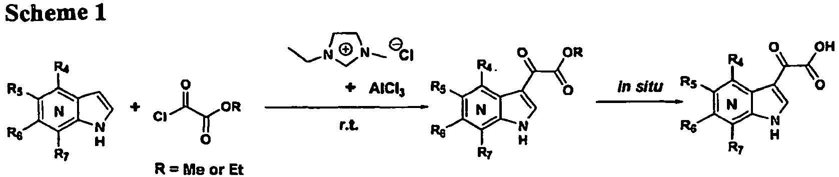

A representative experimental procedure is as follows: 1-ethyl-3-methylimidazolium chloride (2 equiv.; purchased from TCI; weighted under a stream of nitrogen) was stirred in an oven-dried round bottom flask at r.t. under a nitrogen atmosphere, and added aluminium chloride (6 equiv.; anhydrous powder packaged under argon in ampules purchased from Aldrich preferred; weighted under a stream of nitrogen). The mixture was vigorously stirred to form a liquid, which was then added azaindole (1 equiv.) and stirred until a homogenous mixture resulted. The reaction mixture was added dropwise ethyl or methyl chlorooxoacetate (2 equiv.) and then stirred at r.t. for 16 h. After which time, the mixture was cooled in an ice-water bath and the reaction quenched by carefully adding excess water. The precipitates were filtered, washed with water and dried under high vacuum to give the azaindoleglyoxyl acid. For some examples, 3 equivalents of 1-ethyl-3-methylimidazolium chloride and chlorooxoacetate may be required.

Related references: (1) Welton, T. Chem Rev. 1999, 99, 2071; (2) Surette, J. K. D.; Green, L.; Singer, R. D. Chem. Commun. 1996, 2753; (3) Saleh, R. Y. WO 0015594.

**Step D.** The acid intermediate, 4, from step C of Scheme 1 is coupled with an amine A(C=O)WH preferably in the presence of DEPBT (3-(diethoxyphosphoryloxy)-1,2,3-benzotriazin-4(3*H*)-one) and *N,N-*diisopropylethylamine, commonly known as Hunig's base, to provide azaindole piperazine diamides. DEPBT was prepared according to the procedure of Ref. 28, Li, H.; Jiang, X.; Ye, Y.-H.; Fan, C.; Romoff, T.; Goodman, M. Organic Lett., 1999, 1, 91-93. Typically an inert solvent such as DMF or THF is used but other aprotic solvents could be used. The group W as referred to herein is

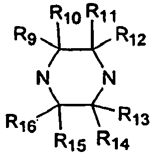

The amide bond construction reaction could be carried out using the preferred conditions described above, the EDC conditions described below, other coupling conditions described in this application, or alternatively by applying the conditions or coupling reagents for amide bond construction described later in this application for construction of substituents R₁-R₄. Some specific nonlimiting examples are given in this application.

The mono-substituted piperazine derivatives can be prepared according to well established procedures such as those described by Desai et al, Ref. 27(a), Adamczyk et al, Ref. 27(b), Rossen et al, Ref. 27(c), and Wang et al, 27(d).

Additional procedures for synthesizing, modifying and attaching groups : (C=O)ₘ-WC(O)-A are contained in PCT WO 00/71535.

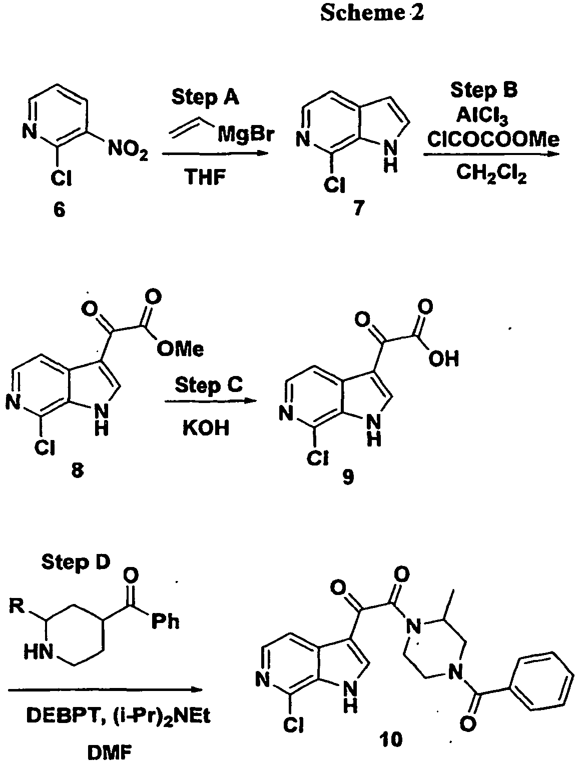

Scheme 2 provides a more specific example of the transformations previously described in Scheme 1. Intermediates 6-10 are prepared by the methodologies as described for intermediates 1a-5a in Scheme 1. Scheme 2A is another embodiment of the transformations described in Schemes 1 and 2. Conversion of the phenol to the chloride (Step S, Scheme 2A) may be accomplished according to the procedures described in Reimann, E.; Wichmann, P.; Hoefner, G.; Sci. Pharm. 1996, 64(3), 637-646; and Katritzky, A.R.; Rachwal, S.; Smith, T.P.; Steel, P.J.; J. Heterocycl. Chem. 1995, 32(3), 979-984. Step T of Scheme 2A can be carried out as described for Step A of Scheme 1. The bromo intermediate can then be converted into alkoxy, chloro, or fluoro intermediates as shown in Step U of Scheme 2A. Scheme 2A describes the preferred method for preparing intermediate 6c or other closely related compounds containing a 4 methoxy group in the 6-azaindole system. When step U is the conversion of the bromide into alkoxy derivatives, the conversion may be carried out by reacting the bromide with an excess of sodium methoxide in methanol with cuprous salts, such as copper I bromide, copper I iodide, and copper I cyanide. The temperature may be carried out at temperatures of between ambient and 175° but most likely will be around 115°C or 100°C. The reaction may be run in a pressure vessel or sealed tube to prevent escape of volatiles such as methanol. The preferred conditions utilize 3eq of sodium methoxide in methanol, CuBr as the reaction catalyst (0.2 to 3 equivalents with the preferred being 1 eq or less), and a reaction temperature of 115° C. The reaction is carried out in a sealed tube or sealed reaction vessel. The conversion of the bromide into alkoxy derivatives may also be carried out according to procedures described in Palucki, M.; Wolfe, J.P.; Buchwald, S.L.; J. Am. Chem. Soc. 1997, 119(14), 3395-3396; Yamato, T.; Komine, M.; Nagano, Y.; Org. Prep. Proc. Int. 1997, 29(3), 300-303; Rychnovsky, S.D.; Hwang, K.; J. Org. Chem. 1994, 59(18), 5414-5418. Conversion of the bromide to the fluoro derivative (Step U, Scheme 2A) may be accomplished according to Antipin, I.S.; Vigalok, A.I.; Konovalov, A.I.; Zh. Org. Khim. 1991, 27(7), 1577-1577; and Uchibori, Y.; Umeno, M.; Seto, H.; Qian, Z.; Yoshioka, H.; Synlett. 1992, 4, 345-346. Conversion of the bromide to the chloro derivative (Step U, Scheme 2A) may be accomplished according to procedures described in Gilbert, E.J.; Van Vranken, D.L.; J. Am. Chem. Soc. 1996, 118(23), 5500-5501; Mongin, F.; Mongin, O.; Trecourt, F.; Godard, A.; Queguiner, G.; Tetrahedron Lett. 1996, 37(37), 6695-6698; and O'Connor, K.J.; Burrows, C.J.; J. Org. Chem. 1991, 56(3), 1344-1346. Steps V, W and X of Scheme 2A are carried out according to the procedures previously described for Steps B, C, and D of Scheme 1, respectively. The steps of Scheme 2A may be carried out in a different order as shown in Scheme 2B and Scheme 2C.

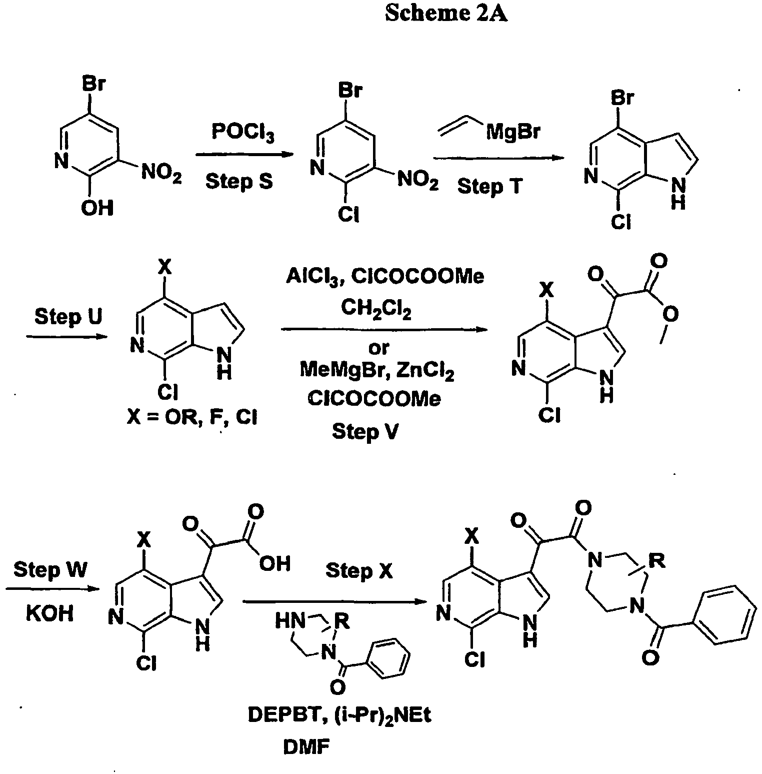

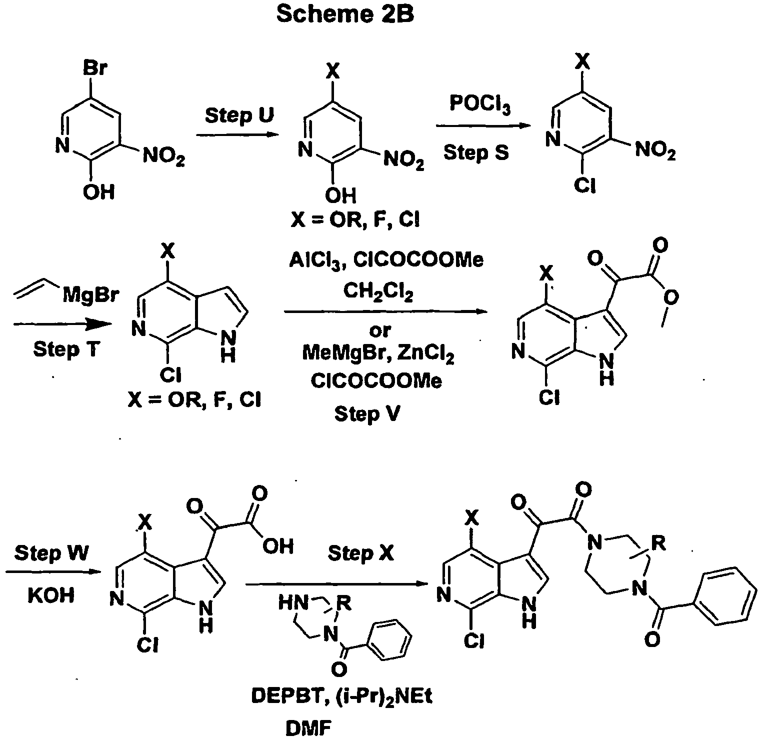

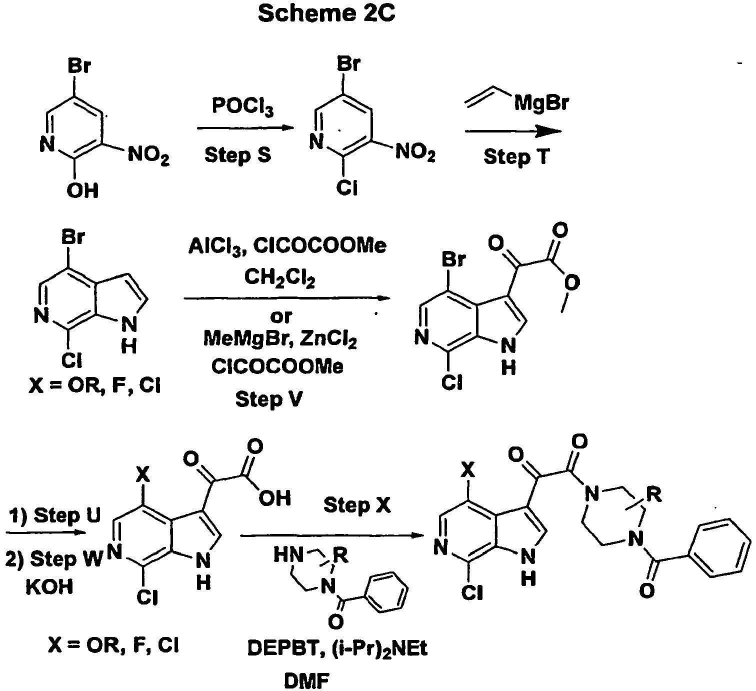

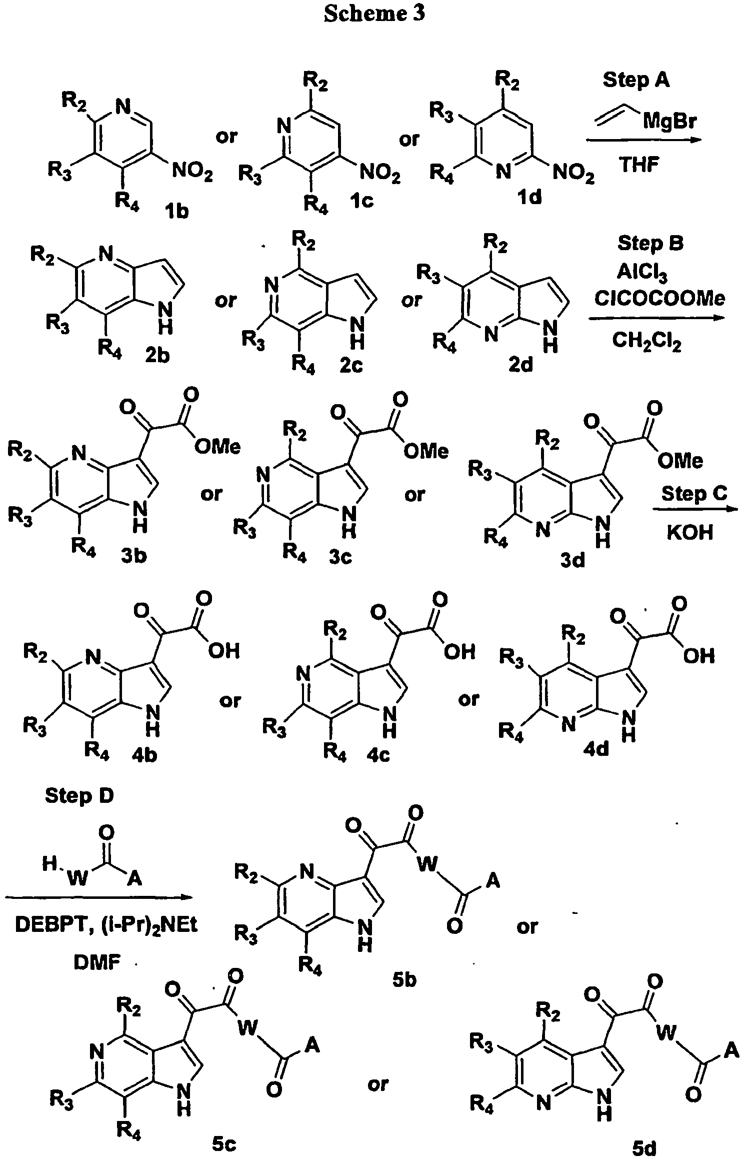

Scheme 3 shows the synthesis of 4-azaindole derivatives 1b-5b, 5-azaindole derivatives 1c-5c, and 7-azaindole derivatives 1d-5d. The methods used to synthesize 1b-5b, 1c-5c, and 1d-5d are the same methods described for the synthesis of 1a-5a as described in Scheme 1. It is understood, for the purposes of Scheme 3, that 1b is used to synthesize 2b-5b, 1c provides 2c-5c and 1d provides 2d-5d.

The compounds where there is a single carbonyl between the azaindole and group W can be prepared by the method of Kelarev, V. I.; Gasanov, S. Sh.; Karakhanov, R. A.; Polivin, Yu. N.; Kuatbekova, K. P.; Panina, M. E.; Zh. Org. Khim 1992, 28(12), 2561-2568. In this method azaindoles are reacted with trichloroacetyl chloride in pyridine and then subsequently with KOH in methanol to provide the 3-carbomethoxy azaindoles shown in Scheme 4 which can then be hydrolyzed to the acid and carried through the coupling sequence with HW(C=O)A to provide the compounds of Formula I wherein a single carbonyl links the azaindole moiety and group W.

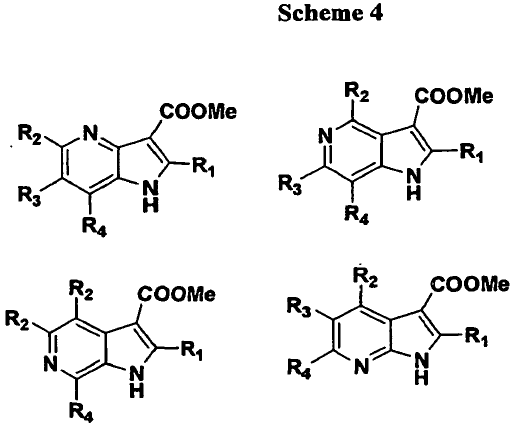

An alternative method for carrying out the sequence outlined in steps B-D (shown in Scheme 5) involves treating an azaindole, such as 11, obtained by procedures described in the literature or from commercial sources, with MeMgI and ZnCl₂, followed by the addition of ClCOCOCl (oxalyl chloride) in either THF or Et₂O to afford a mixture of a glyoxyl chloride azaindole, 12a, and an acyl chloride azaindole, 12b. The resulting mixture of glyoxyl chloride azaindole and acyl chloride azaindole is then coupled with mono-benzoylated piperazine derivatives under basic conditions to afford the products of step D as a mixture of compounds, 13a and 13b, where either one or two carbonyl groups link the azaindole and group W. Separation via chromatographic methods which are well known in the art provides the pure 13a and 13b. This sequence is summarized in Scheme 5, below.

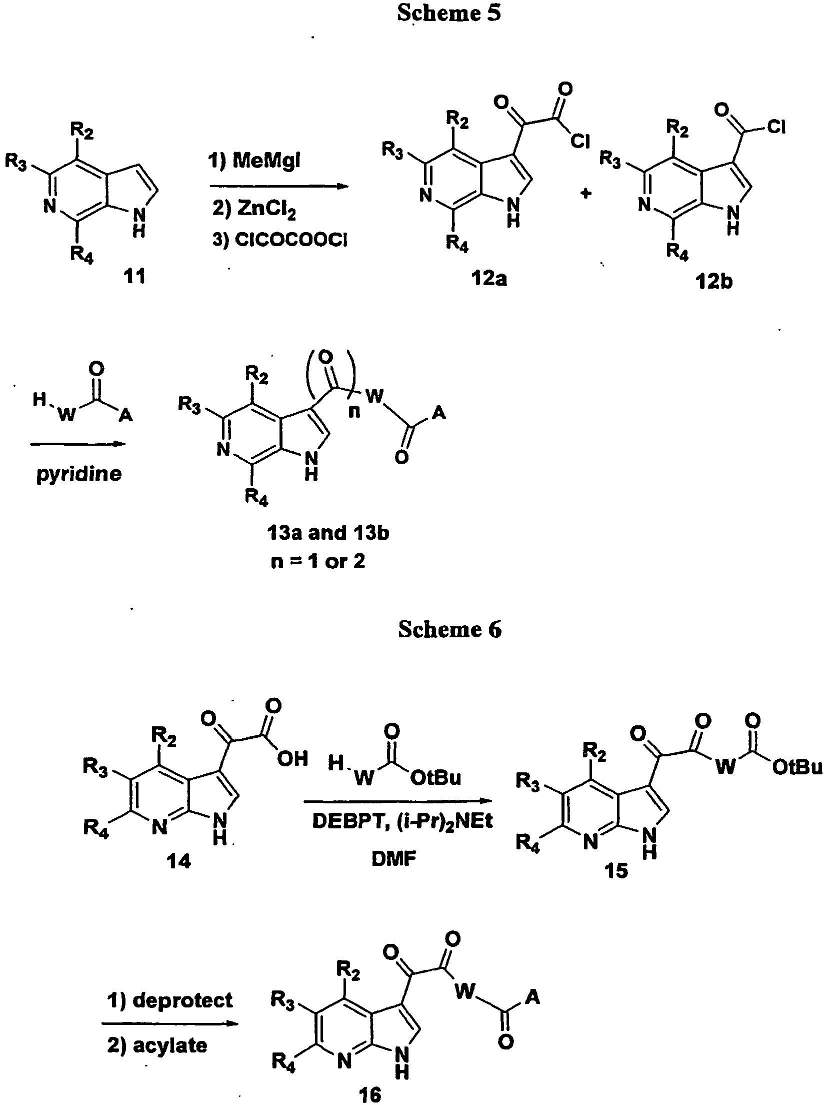

Scheme 6 depicts a general method for modifying the substituent A. Coupling of H-W-C(O)OtBu using the conditions described previously for W in Scheme 1, Step D provides Boc protected intermediate, 15. Intermediate 15 is then deprotected by treatment with an acid such as TFA, hydrochloric acid or formic acid using standard solvents or additives such as CH₂Cl₂ , dioxane, or anisole and temperatures between -78 °C and 100 °C. Other acids such as aq hydrochloric or perchloric may also be used for deprotection. Alternatively other nitrogen protecting groups on W such as Cbz or TROC, may be utilized and could be removed via hydrogenation or treatment with zinc respectively. A stable silyl protecting group such as phenyl dimethylsilyl could also be employed as a nitrogen protecting group on W and can be removed with fluoride sources such as tetrabutylammonium fluoride. Finally, the free amine is coupled to acid A-C(O)OH using standard amine-acid coupling conditions such as those used to attach group W or as shown below for amide formation on positions R₁-R₄ to provide compound 16.

Some specific examples of general methods for preparing functionalized azaindoles or for interconverting functionality on aza indoles which will be useful for preparing the compounds of this invention are shown in the following sections for illustrative purposes. It should be understood that this invention covers substituted 4, 5, 6, and 7 azaindoles and that the methodology shown below may be applicable to all of the above series while other shown below will be specific to one or more. A typical practioner of the art can make this distinction when not specifically delineated. Many methods are intended to be applicable to all the series, particularly functional group installations or interconversions. For example, a general strategy for providing further functionality of this invention is to position or install a halide such as bromo, chloro, or iodo, aldehyde, cyano, or a carboxy group on the azaindole and then to convert that functionality to the desired compounds. In particular, conversion to substituted heteroaryl, aryl, and amide groups on the ring are of particular interest.

General routes for functionalizing azaindole rings are shown in Schemes 7, 8 and 9. As depicted in Scheme 7, the azaindole, 17, can be oxidized to the corresponding *N*-oxide derivative, 18, by using mCPBA (meta-Chloroperbenzoic Acid) in acetone or DMF (eq. 1, Harada et al, Ref. 29 and Antonini et al, Ref. 34). The *N*-oxide, 18, can be converted to a variety of substituted azaindole derivatives by using well documented reagents such as phosphorus oxychloride (POCl₃) (eq. 2, Schneller et al, Ref. 30), tetramethylammonium fluoride (Me₄NF) (eq. 3), Grignard reagents RMgX (R = alkyl or aryl, X = Cl, Br or I) (eq. 4, Shiotani et al, Ref. 31), trimethylsilyl cyanide (TMSCN) (eq. 5, Minakata et al, Ref. 32) or Ac₂O (eq. 6, Klemm et al, Ref. 33). Under such conditions, a chlorine (in 19), fluorine (in 20), nitrile (in 22), alkyl (in 21), aromatic (in 21) or hydroxyl group (in 24) can be introduced to the pyridine ring. Nitration of azaindole *N*-oxides results in introduction of a nitro group to azaindole ring, as shown in Scheme 8 (eq. 7, Antonini et al, Ref. 34). The nitro group can subsequently be displaced by a variety of nucleophilic agents, such as OR, NR¹R² or SR, in a well established chemical fashion (eq. 8, Regnouf De Vains et al, Ref. 35(a), Miura et al, Ref. 35(b), Profft et al, Ref. 35(c)). The resulting N-oxides, 26, are readily reduced to the corresponding azaindole, 27, using phosphorus trichloride (PCl₃) (eq. 9, Antonini et al, Ref .34 and Nesi et al, Ref. 36). Similarly, nitro-substituted *N*-oxide, 25, can be reduced to the azaindole, 28, using phosphorus trichloride (eq. 10). The nitro group of compound 28 can be reduced to either a hydroxylamine (NHOH), as in 29, (eq. 11, Walser et al, Ref. 37(a) and Barker et al, Ref. 37(b)) or an amino (NH₂) group, as in 30, (eq. 12, Nesi et al , Ref. 36 and Ayyangar et al, Ref. 38) by carefully selecting different reducing conditions.

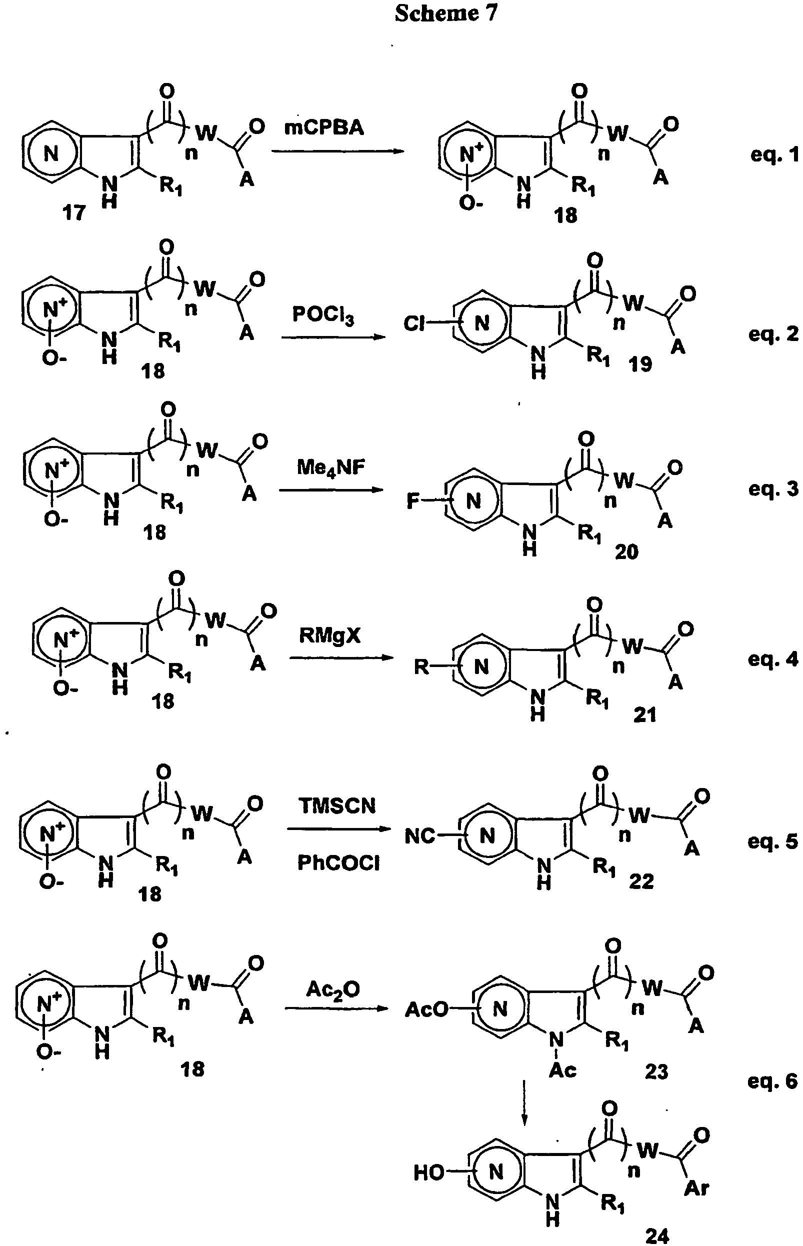

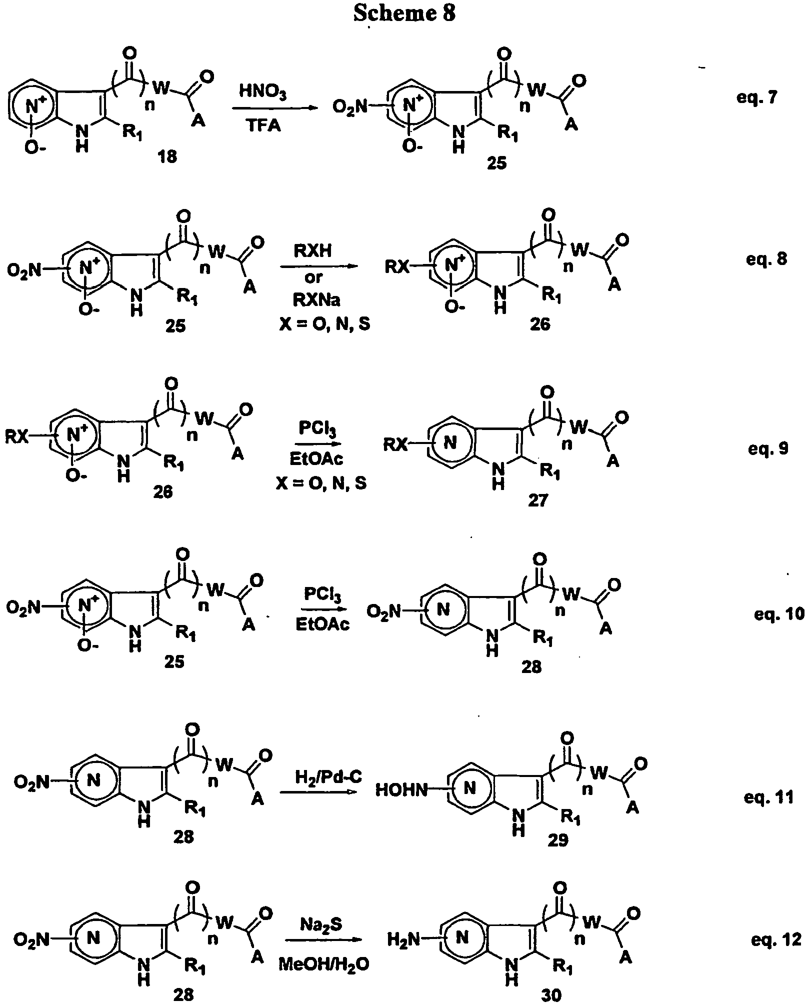

The alkylation of the nitrogen atom at position 1 of the azaindole derivatives can be achieved using NaH as the base, DMF as the solvent and an alkyl halide or sulfonate as alkylating agent, according to a procedure described in the literature (Mahadevan et al, Ref. 39) (Scheme 9).

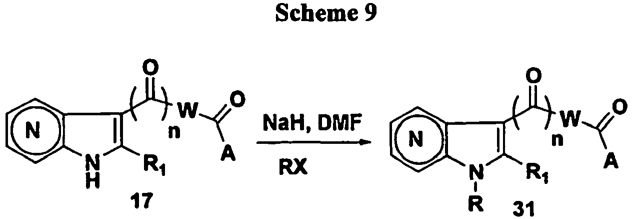

In the general routes for substituting the azaindole ring described above, each process can be applied repeatedly and combinations of these processes is permissible in order to provide azaindoles incorporating multiple substituents. The application of such processes provides additional compounds of Formula I.

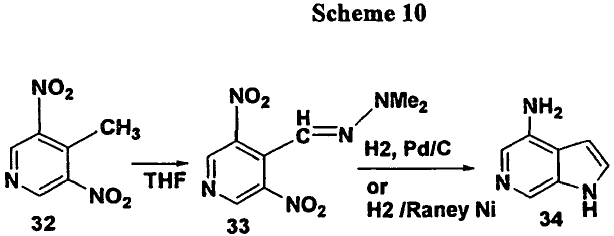

The synthesis of 4-aminoazaindoles which are useful precursors for 4, 5, and/or 7-substituted azaindoles is shown in Scheme 10 above.
The synthesis of 3, 5-dinitro-4-methylpyridine, 32, is described in the following two references by Achremowicz et.al.: Achremowicz, Lucjan. Pr. Nauk Inst. Chem. Org. Fiz. Politech. Wroclaw. 1982, 23, 3-128; Achremowicz, Lucjan. Synthesis 1975, 10, 653-4. In the first step of Scheme 10, the reaction with dimethylformamide dimethyl acetal in an inert solvent or neat under conditions for forming Batcho-Leimgruber precursors provides the cyclization precursor, 33, as shown. Although the step is anticipated to work as shown, the pyridine may be oxidized to the N-oxide prior to the reaction using a peracid such as MCPBA or a more potent oxidant like metatrifluoromethyl or meta nitro peroxy benzoic acids. In the second step of Scheme 10, reduction of the nitro group using for example hydrogenation over Pd/C catalyst in a solvent such as MeOH, EtOH, or EtOAc provides the cyclized product, 34. Alternatively the reduction may be carried out using tin dichloride and HCl, hydrogenation over Raney nickel or other catalysts, or by using other methods for nitro reduction such as described elsewhere in this application.

The amino indole, 34, can now be converted to compounds of Formula I via, for example, diazotization of the amino group, and then conversion of the diazonium salt to the fluoride, chloride or alkoxy group. See the discussion of such conversions in the descriotions for Schemes 17 and 18. The conversion of the amino moiety into desired functionality could then be followed by installation of the oxoacetopiperazine moiety by the standard methodology described above. 5 or 7-substitution of the azaindole can arise from N-oxide formation at position 6 and subsequent conversion to the chloro via conditions such as POCl₃ in chloroform, acetic anhydride followed by POCl₃ in DMF, or alternatively TsCl in DMF. Literature references for these and other conditions are provided in some of the later Schemes in this application. The synthesis of 4-bromo-7-hydroxy or protected hydroxy-4-azaindole is described below as this is a useful precursor for 4 and/or 7 substituted 6-aza indoles.

The synthesis of 5-bromo-2-hydroxy-4-methyl-3-nitro pyridine, 35, may be carried out as described in the following reference:Betageri, R.; Beaulieu, P.L.; Llinas-Brunet, M; Ferland, J.M.; Cardozo, M.; Moss, N.; Patel, U.; Proudfoot, J.R. PCT Int. Appl. WO 9931066, 1999. Intermediate 36 is prepared from 35 according to the method as described for Step 1 of Scheme 10. PG is an optional hydroxy protecting group such as triallylsilyl or the like. Intermediate 37 is then prepared from 36 by the selective reduction of the nitro group in the presence of bromide and subsequent cyclization as described in the second step of Scheme 10. Fe(OH)₂ in DMF with catalytic tetrabutylammonium bromide can also be utilized for the reduction of the nitro group. The bromide may then be converted to fluoride via displacement with fluoride anions or to other substituents. The compounds are then converted to compounds of Formula I as above.

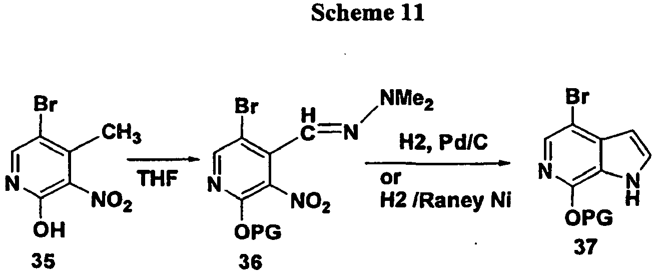

An alternate method for preparing substituted 6-azaindoles is shown below in Schemes 12 and 13. It should be recognized that slight modifications of the route depicted below are possible. For example, acylation reactions of the 3 position of what will become the azaindole five membered ring, prior to aromatization of the azaindole, may be carried out in order to obtain higher yields. In addition to a para-methoxybenzyl group (PMB), a benzyl group can be carried through the sequence and removed during azaindole formation by using TsOH, p-Chloranil, in benzene as the oxidant if DDQ is not optimal. The benzyl intermediate, 38, has been described by Ziegler et al. in J. Am. Chem. Soc. 1973, 95(22), 7458. The transformation of 38 to 40 is analogous to the transformation described in Heterocycles 1984, 22, 2313.

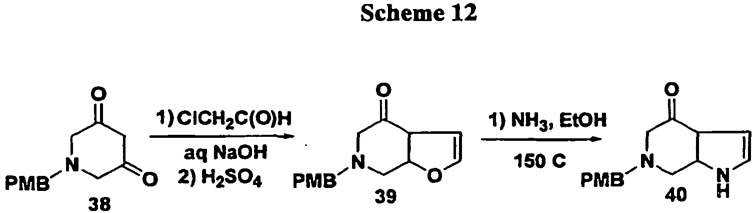

Scheme 13 describes various transformations of intermediate 40 which ultimately provide compounds of Formula I. The conversions of the phenol moiety to other functionality at position 4 (R₂ position in Scheme 13) may be carried out by the following methods: 1) conversion of a phenol to methoxy group with silver oxide and MeI or diazomethane; 2) conversion of a phenolic hydroxy group to chloro using cat ZnCl₂, and N,N dimethylaniline in CH₂Cl₂ or PCl₅ and POCl₃ together; 3) conversion of a phenolic hydroxy group to fluoro using diethylamine-SF₃ as in Org.Prep. Proc. Int. 1992, 24(1), 55-57. The method described in EP 427603, 1991, using the chloroformate and HF will also be useful. Other transformations are possible. For example the phenol can be converted to a triflate by standard methods and used in coupling chemistries described later in this application.

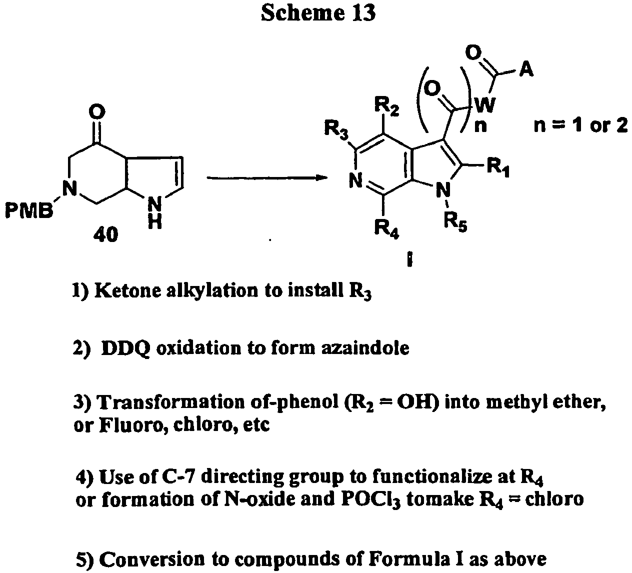

**Step E** Scheme 14 depicts the nitration of an azaindole, 41, (R₂ = H). Numerous conditions for nitration of the azaindole may be effective and have been described in the literature. N₂O₅ in nitromethane followed by aqueous sodium bisulfite according to the method of Bakke, J. M.; Ranes, E.; Synthesis 1997, 3, 281-283 could be utilized. Nitric acid in acetic may also be employed as described in Kimura, H.; Yotsuya, S.; Yuki, S.; Sugi, H.; Shigehara, I.; Haga, T.; Chem. Pharm. Bull. 1995, 43(10), 1696-1700. Sulfuric acid followed by nitric acid may be employed as in Ruefenacht, K.; Kristinsson, H.; Mattern, G.; Helv Chim Acta 1976, 59, 1593. Coombes, R. G.; Russell, L. W.; J. Chem. Soc., Perkin Trans. 1 1974, 1751 describes the use of a Titatanium based reagent system for nitration. Other conditions for the nitration of the azaindole can be found in the following references: Lever, O.W.J.; Werblood, H. M.; Russell, R. K.; Synth. Comm. 1993, 23(9), 1315-1320; Wozniak, M.; Van Der Plas, H. C.; J. Heterocycl Chem. 1978, 15, 731.

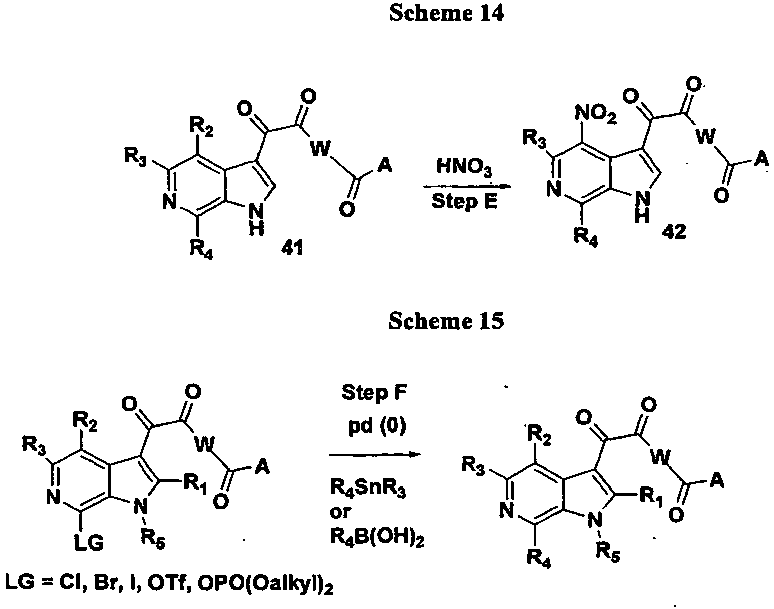

### Step F

As shown above in Scheme 15, Step F, substituted azaindoles containing a chloride, bromide, iodide, triflate, or phosphonate undergo coupling reactions with a boronate (Suzuki type reactions) or a stannane to provide substituted azaindoles. Stannanes and boronates are prepared via standard literature procedures or as described in the experimental section of this application. The substitututed indoles may undergo metal mediated coupling to provide compounds of Formula I wherein R₄ is aryl, heteroaryl, or heteroalicyclic for example. The bromoazaindole intermediates, (or azaindole triflates or iodides) may undergo Stille-type coupling with heteroarylstannanes as shown in Scheme 15. Conditions for this reaction are well known in the art and the following are three example references a) Farina, V.; Roth, G.P. Recent advances in the Stille reaction; Adv. Met.-Org. Chem. 1996, 5, 1-53. b) Farina, V.; Krishnamurthy, V.; Scott, W.J. The Stille reaction; Org. React. (N. Y.) 1997, 50, 1-652. and c) Stille, J. K. Angew. Chem. Int. Ed. Engl. 1986, 25, 508-524. Other references for general coupling conditions are also in the reference by Richard C. Larock Comprehensive Organic Transformations 2nd Ed. 1999, John Wiley and Sons New York. All of these references provide numerous conditions at the disposal of those skilled in the art in addition to the specific examples provided in Scheme 15 and in the specific embodiments. It can be well recognized that an indole stannane could also couple to a heterocyclic or aryl halide or triflate to construct compounds of Formula I. Suzuki coupling (Norio Miyaura and Akiro Suzuki Chem Rev.1995, 95, 2457.) between a triflate, bromo, or chloro azaindole intermediate and a suitable boronate could also be employed and some specific examples are contained in this application. Palladium catalyzed couplings of stannanes and boronates between chloro azaindole intermediates are also feasible and have been utilized extensively for this invention. Preferred procedures for coupling of a chloro azaindole and a stannane employ dioxane, stoichiometric or an excess of the tin reagent (up to 5 equivalents), 0.1 to 1 eq of Palladium (0) tetrakis triphenyl phosphine in dioxane heated for 5 to 15 h at 110 to 120°. Other solvents such as DMF, THF, toluene, or benzene could be employed. Preferred procedures for Suzuki coupling of a chloro azaindole and a boronate employ 1:1 DMF water as solvent, 2 equivalents of potassium carbonate as base stoichiometric or an excess of the boron reagent (up to 5 equivalents), 0.1 to 1 eq of Palladium (0) tetrakis triphenyl phosphine heated for 5 to 15 h at 110 to 120°. If standard conditions fail new specialized catalysts and conditions can be employed. Some references (and the references therein) describing catalysts which are useful for coupling with aryl and heteroaryl chlorides are:
Littke, A. F.; Dai, C.; Fu, G. C. J. Am. Chem. Soc. 2000, 122(17), 4020-4028; Varma, R. S.; Naicker, K. P. Tetrahedron Lett. 1999, 40(3), 439-442; Wallow, T. I.; Novak, B. M. J. Org. Chem. 1994, 59(17), 5034-7; Buchwald, S.; Old, D. W.; Wolfe, J. P.; Palucki, M.; Kamikawa, K.; Chieffi, A.; Sadighi, J. P.; Singer, R. A.; Ahman, J PCT Int. Appl. WO 0002887 2000**;** Wolfe, J. P.; Buchwald, S. L. Angew. Chem., Int. Ed. 1999, 38(23), 3415*;* Wolfe, J. P.; Singer, R. A.; Yang, B. H.; Buchwald, S. L. J. Am. Chem. Soc. 1999, 121(41), 9550-9561; Wolfe, J. P.; Buchwald, S. L. Angew. Chem., Int. Ed. 1999, 38(16), 2413-2416*;* Bracher, F.; Hildebrand, D.; Liebigs Ann. Chem. 1992, 12, 1315-1319; and Bracher, F.; Hildebrand, D.; Liebigs Ann. Chem. 1993, 8, 837-839.

Alternatively, the boronate or stannane may be formed on the azaindole via methods known in the art and the coupling performed in the reverse manner with aryl or heteroaryl based halogens or triflates.

Known boronate or stannane agents could be either purchased from commercial resources or prepared following disclosed documents. Additional examples for the preparation of tin reagents or boronate reagents are contained in the experimental section.

Novel stannane agents could be prepared from one of the following routes. 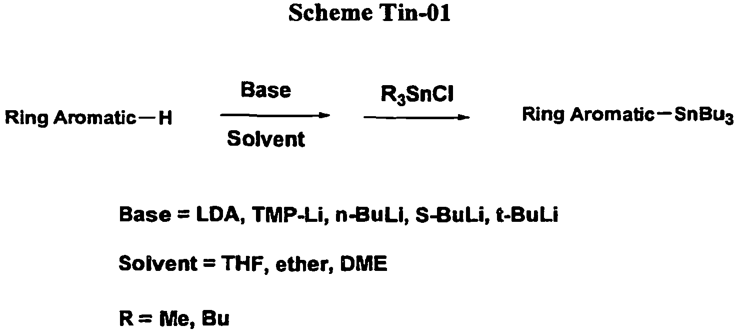 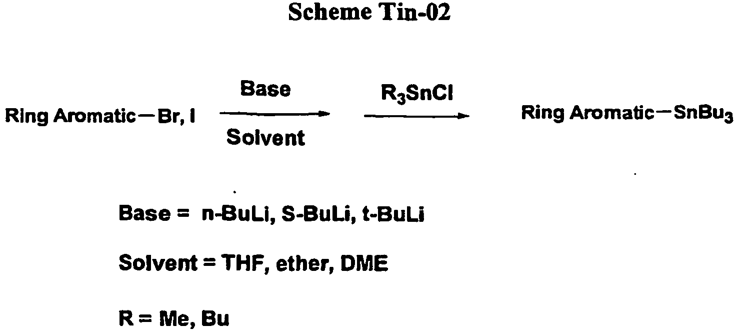 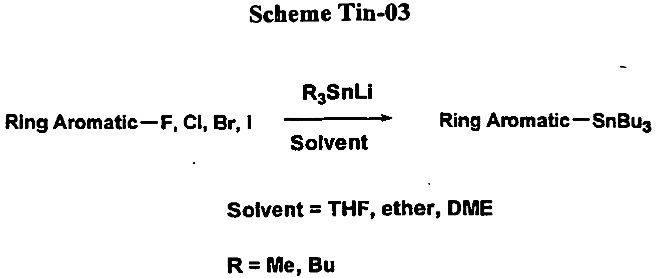 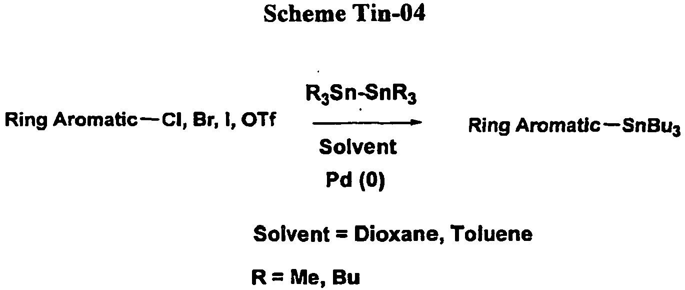 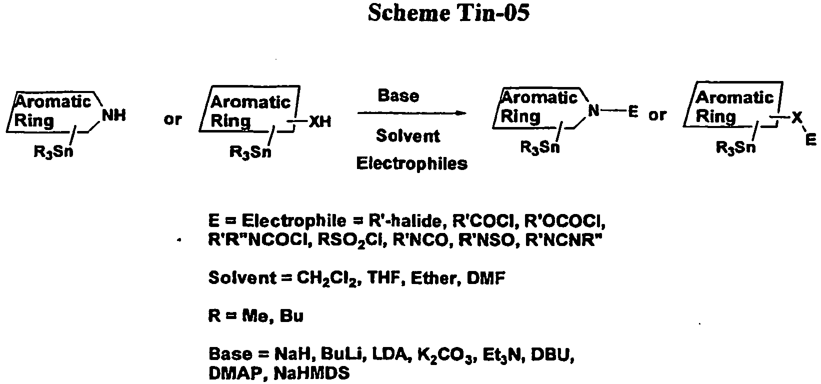 Boronate reagents are prepeared as described in reference 71. Reaction of lithium or Grignard reagents with trialkyl borates generates boronates. Alternatively, Palladium catalyzed couplings of alkoxy diboron or alkyl diboron reagents with aryl or heteroaryl halides can provide boron reagents for use in Suzuki type couplings. Some example conditions for coupling a halide with (MeO)BB(OMe)2 utilize PdCl2 (dppf), KOAc, DMSO, at 80°C until reaction is complete when followed by TLC or HPLC analysis.

Related examples are provided in the following experimental section.

Methods for direct addition of aryl or heteroaryl organometallic reagents to alpha chloro nitrogen containing heterocyles or the N-oxides of nitrogen containing heterocycles are known and applicable to the azaindoles. Some examples are Shiotani et. Al. J. Heterocyclic Chem. 1997, 34(3), 901-907; Fourmigue et.al. J.Org. Chem. 1991, 56(16), 4858-4864*.*

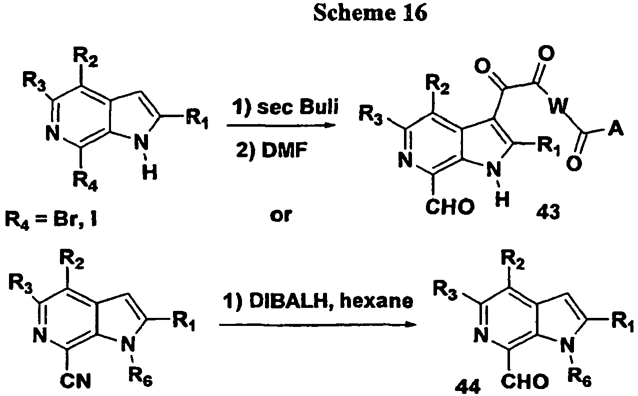

The preparation of a key aldehyde intermediate, 43, using a procedure adapted from the method of Gilmore et. Al. Synlett 1992, 79-80. Is shown in Scheme 16 above. The aldehyde substituent is shown only at the R₄ position for the sake of clarity, and should not be considered as a limitation of the methodology. The bromide or iodide intermediate is converted into an aldehyde intermediate, 43, by metal-halogen exchange and subsequent reaction with dimethylformamide in an appropriate aprotic solvent. Typical bases used include, but are not limited to, alkyl lithium bases such as n-butyl lithium, sec butyl lithium or tert butyl lithium or a metal such as lithium metal. A preferred aprotic solvent is THF. Typically the transmetallation is initiated at -78 °C. The reaction may be allowed to warm to allow the transmetalation to go to completion depending on the reactivity of the bromide intermediate. The reaction is then recooled to -78 °C and allowed to react with dimethylformamide. (allowing the reaction to warm may be required to enable complete reaction) to provide an aldehyde which is elaborated to compounds of . Formula I. Other methods for introduction of an aldehyde group to form intermediates of formula 43 include transition metal catalyzed carbonylation reactions of suitable bromo, trifluoromethane sulfonyl, or stannyl azaindoles. Alternative the aldehydes can be introduced by reacting indolyl anions or indolyl Grignard reagents with formaldehyde and then oxidizing with MnO₂ or TPAP/NMO or other suitable oxidants to provide intermediate 43.

The methodology described in T. Fukuda et.al. Tetrahedron 1999, 55, 9151 and M. Iwao et. Al. Heterocycles 1992, 34(5), 1031 provide methods for preparing indoles with substituents at the 7-position. The Fukuda references provide methods for functionalizing the C-7 position of indoles by either protecting the indole nitrogen with 2,2-diethyl propanoyl group and then deprotonating the 7-position with sec/Buli in TMEDA to give an anion. This anion may be quenched with DMF, formaldehyde, or carbon dioxide to give the aldehyde, benzyl alcohol, or carboxylic acid respectively and the protecting group removed with aqueous t butoxide. Similar tranformations can be achieved by converting indoles to indoline, lithiation at C-7 and then reoxidation to the indole such as described in the Iwao reference above. The oxidation level of any of these products may be adjusted by methods well known in the art as the interconversion of alcohol, aldehyde, and acid groups has been well studied. It is also well understood that a cyano group can be readily converted to an aldehyde. A reducing agent such as DIBALH in hexane such as used in Weyerstahl, P.; Schlicht, V.; Liebigs Ann/Recl. 1997, 1, 175-177 or alternatively catecholalane in THF such as used in Cha, J. S.; Chang, S. W.; Kwon, O. O.; Kim, J. M.; Synlett. 1996, 2, 165-166 will readily achieve this conversion to provide intermediates such as 44 (Scheme 16). Methods for synthesizing the nitriles are shown later in this application. It is also well understood that a protected alcohol, aldehyde, or acid group could be present in the starting azaindole and carried through the synthetic steps to a compound of Formula I in a protected form until they can be converted into the desired substituent at R₁ through R₄. For example, a benzyl alcohol can be protected as a benzyl ether or silyl ether or other alcohol protecting group; an aldehyde may be carried as an acetal, and an acid may be protected as an ester or ortho ester until deprotection is desired and carried out by literature methods.

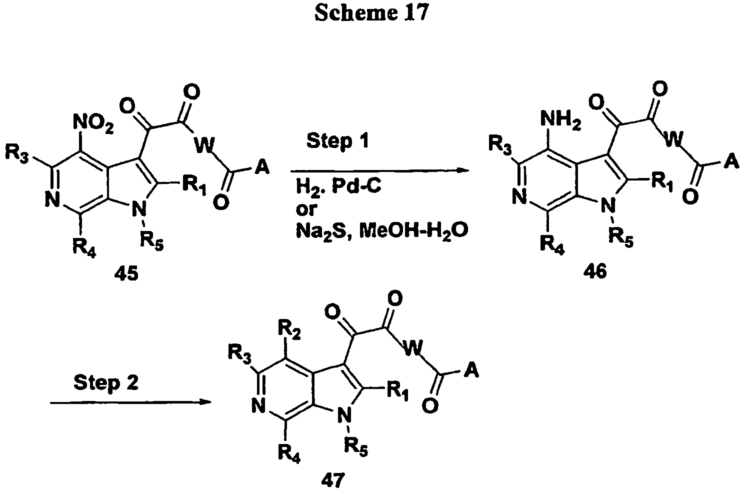

**Step G** Step 1 of Scheme 17 shows the reduction of a nitro group on 45 to the amino group of 46. Although shown on position 4 of the azaindole, the chemistry is applicable to other nitro isomers. The procedure described in Ciurla, H.; Puszko, A.; Khim Geterotsikl Soedin 1996, 10, 1366-1371 uses hydrazine Raney-Nickel for the reduction of the nitro group to the amine. Robinson, R. P.; DonahueO, K. M.; Son, P. S.; Wagy, S. D.; J. Heterocyc/. Chem. 1996, 33(2), 287-293 describes the use of hydrogenation and Raney Nickel for the reduction of the nitro group to the amine. Similar conditions are described by Nicolai, E.; Claude, S.; Teulon, J. M.; J. Heterocycl. Chem. 1994, 31(1), 73-75 for the same transformation. The following two references describe some trimethylsilyl sulfur or chloride based reagents which may be used for the reduction of a nitro group to an amine. Hwu, J.R.; Wong, F.F.; Shiao, M.J.; J. Org. Chem. 1992, 57(19), 5254-5255; Shiao, M.J.; Lai, L.L.; Ku, W.S.; Lin, P.Y.; Hwu, J.R.; J. Org. Chem. 1993, 58(17), 4742-4744.

Step 2 of Scheme 17 describes general methods for conversion of amino groups on azaindoles into other functionality. Scheme 18 also depicts transformations of an amino azaindole into various intermediates and compounds of Formula I.

The amino group at any position of the azaindole, such as 46 (Scheme 17), may be converted to a hydroxy group using sodium nitrite, sulfuric acid, and water via the method of Klemm, L. H.; Zell, R; J. Heterocycl. Chem. 1968, 5, 773. Bradsher, C. K.; Brown, F. C.; Porter, H. K.; J. Am. Chem. Soc. 1954, 76, 2357 describes how the hydroxy group may be alkylated under standard or Mitsonobu conditions to form ethers. The amino group may be converted directly into a methoxy group by diazotization (sodium nitrite and acid )and trapping with methanol.

The amino group of an azaindole, such as 46, can be converted to fluoro via the method of Sanchez using HPF₆, NaNO₂, and water by the method described in Sanchez, J. P.; Gogliotti, R. D.; J. Heterocycl. Chem. 1993, 30(4), 855-859. Other methods useful for the conversion of the amino group to fluoro are described in Rocca, P.; Marsais, F.; Godard, A.; Queguiner, G.; Tetrahedron Lett. 1993, 34(18), 2937-2940 and Sanchez, J. P.; Rogowski, J.W.; J. Heterocycl. Chem. 1987, 24, 215.

The amino group of the azaindole, 46, can also be converted to a chloride via diazotization and chloride displacement as described in Ciurla, H.; Puszko, A.; Khim Geterotsikl Soedin 1996, 10, 1366-1371 or the methods in Raveglia, L.F.; Giardina, G.A..; Grugni, M.; Rigolio, R.; Farina, C.; J. Heterocycl. Chem. 1997, 34(2), 557-559 or the methods in Matsumoto, J. I.; Miyamoto, T.; Minamida, A.; Mishimura, Y.; Egawa, H.; Mishimura, H.; J. Med. Chem. 1984, 27(3), 292; or as in Lee, T.C.; Salemnick, G.; J. Org. Chem.1975, 24, 3608.

The amino group of the azaindole, 46, can also be converted to a bromide via diazotization and displacement by bromide as described in Raveglia, L.F.; Giardina, G.A..; Grugni, M.; Rigolio, R.; Farina, C.; J. Heterocycl. Chem. 1997, 34(2), 557-559; Talik, T.; Talik, Z.; Ban-Oganowska, H.; Synthesis 1974, 293; and Abramovitch, R.A.; Saha, M.; Can. J. Chem. 1966, 44, 1765.

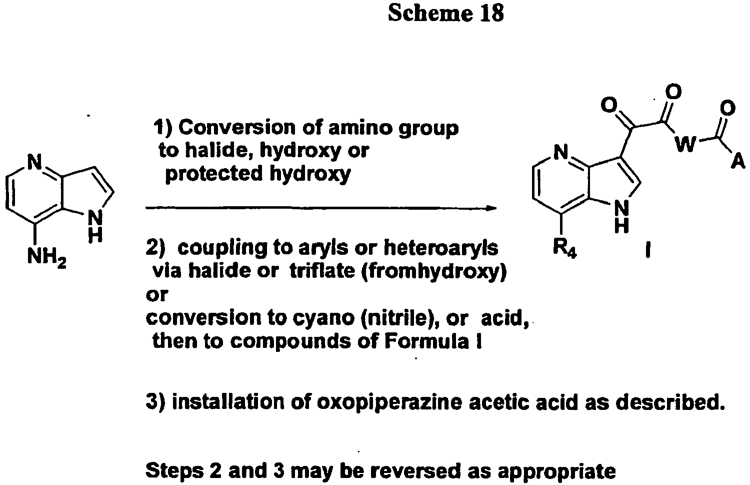

The preparation of 4-amino 4-azaindole and 7-methyl-4-azaindole is described by Mahadevan, I.; Rasmussen, M. J. Heterocycl. Chem. 1992, 29(2), 359-67. The amino group of the 4-amino 4-azaindole can be converted to halogens, hydroxy, protected hydroxy, triflate, as described above in Schemes 17-18 for the 4-amino compounds or by other methods known in the art. Protection of the indole nitrogen of the 7-methyl-4-azaindole via acetylation or other strategy followed by oxidation of the 7-methyl group with potassium permanganate or chromic acid provides the 7-acid /4-N-oxide. Reduction of the N-oxide, as described below, provides an intermediate from which to install various substituents at position R₄. Alternatively the parent 4-azaindole which was prepared as described in Mahadevan, I.; Rasmussen, M. J. Heterocycl. Chem. 1992, 29(2), 359-67 could be derivatized at nitrogen to provide the 1-(2,2-diethylbutanoyl)azaindole which could then be lithiated using TMEDA /sec BuLi as described in T. Fukuda et. Al. Tetrahedron 1999, 55, 9151-9162; followed by conversion of the lithio species to the 7-carboxylic acid or 7-halogen as described. Hydrolysis of the N-amide using aqueous tert-butoxide in THF regenerates the free NH indole which can now be converted to compounds of Formula I. The chemistry used to functionalize position 7 can also be applied to the 5 and 6 indole series.

Scheme 19 shows the preparation of a 7-chloro-4-azaindole, 50, which can be converted to compounds of Formula I by the chemistry previously described, especially the palladium catalyzed tin and boron based coupling methodology described above. The chloro nitro indole, 49, is commercially available or can be prepared from 48 according to the method of Delarge, J.; Lapiere, C. L. Pharm. Acta Helv. 1975,50(6),188-91*.*

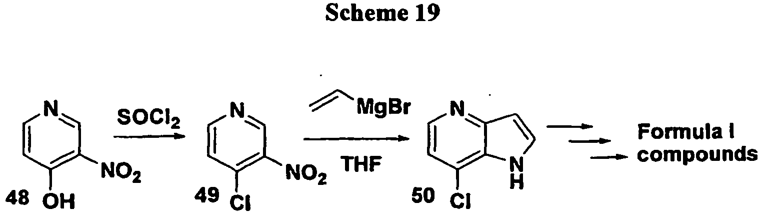

Scheme 20, below, shows another synthetic route to substituted 4-aza indoles. The 3-aminopyrrole, 51, was reacted to provide the pyrrolopyridinone, 52, which was then reduced to give the hydroxy azaindole, 53. The pyrrolo[2,3-b]pyridines described were prepared according to the method of Britten, A.Z.; Griffiths, G.W.G. Chem. Ind. (London) 1973, 6, 278. The hydroxy azaindole, 53, can then be converted to the triflate then further reacted to provide compounds of Formula I.

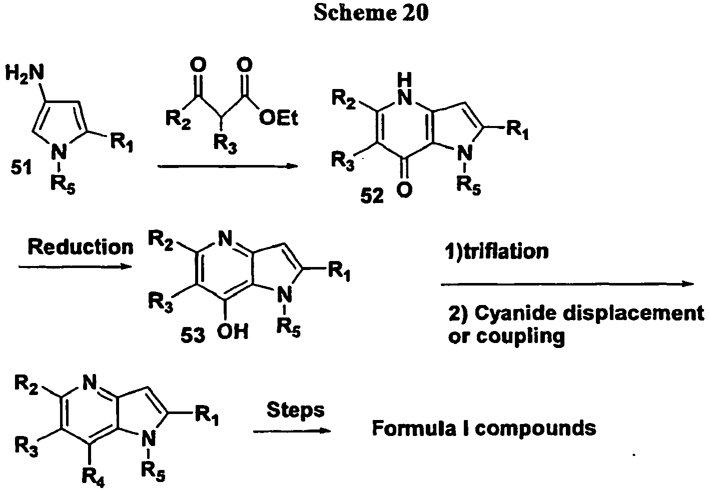

The following references describe the synthesis of 7-halo or 7 carboxylic acid, or 7-amido derivatives of 5-azaindoline which can be used to construct compounds of Formula I. Bychikhina, N. N.; Azimov, V. A.; Yakhontov, L.N. Khim. Geterotsikl. Soedin. 1983, 1, 58-62; Bychikhina, N. N.; Azimov, V. A.; Yakhontov, L. N. Khim. Geterotsikl. Soedin. 1982, 3, 356-60; Azimov, V. A.; Bychikhina, N. N.; Yakhontov, L. N. Khim. Geterotsikl. Soedin. 1981, 12, 1648-53; Spivey, A.C.; Fekner, T.; Spey, S.E.; Adams, H. J Org. Chem. 1999, 64(26), 9430-9443; Spivey, A.C.; Fekner, T.; Adams, H. Tetrahedron Lett. 1998, 39(48), 8919-8922. The methods described in Spivey et al. (preceding two references) for the preparation of 1-methyl-7-bromo-4-azaindoline can be used to prepare the 1-benzyl-7-bromo-4-azaindoline, 54, shown below in Scheme 21. This can be utilized in Stille or Suzuki couplings to provide 55, which is deprotected and dehydrogenated to provide 56. Other useful azaindole intermediates, such as the cyano derivatives, 57 and 58, and the aldehyde derivatives, 59 and 60, can then be further elaborated to compounds of Formula I.

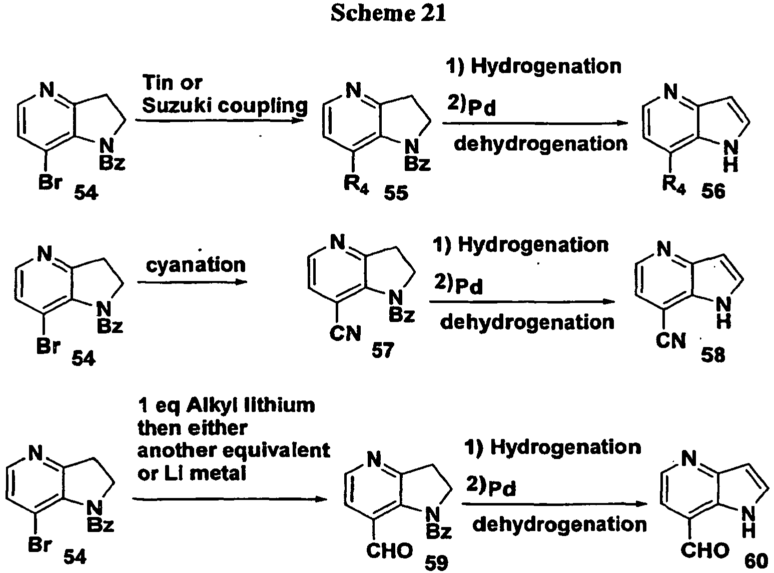

Alternatively the 7-functionalized 5-azaindole derivatives may be obtained by functionalization using the methodologies of T. Fukuda et.al. Tetrahedron 1999, 55, 9151 and M. Iwao et. Al. Heterocycles 1992, 34(5), 1031 described above for the 4 or 6 azaindoles. The 4 or 6 positions of the 5 aza indoles can be functionalized by using the azaindole N-oxide.

The conversion of indoles to indolines is well known in the art and can be carried out as shown or by the methods described in Somei, M.; Saida, Y.; Funamoto, T.; Ohta, T. Chem. Pharm. Bull. 1987, 35(8), 3146-54; M. Iwao et. Al. Heterocycles 1992, 34(5), 1031; andAkagi, M.; Ozaki, K. Heterocycles 1987, 26(1), 61-4.

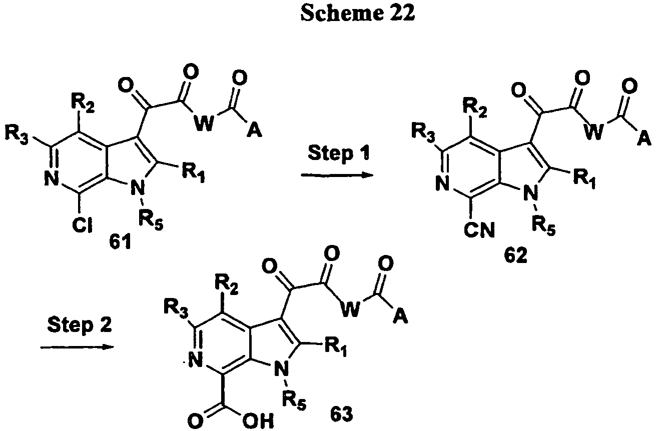

The preparation of azaindole oxoacetyl or oxo piperidines with carboxylic acids can be carried out from nitrile, aldehyde, or anion precursors via hydrolysis, oxidation, or trapping with CO₂ respectively. As shown in the Scheme 22, Step 1, or the scheme below step a12 one method for forming the nitrile intermediate, 62, is by cyanide displacement of a halide in the aza-indole ring. The cyanide reagent used can be sodium cyanide, or more preferably copper or zinc cyanide. The reactions may be carried out in numerous solvents which are well known in the art. For example DMF is used in the case of copper cyanide. Additional procedures useful for carrying out step 1 of Scheme 24 are Yamaguchi, S.; Yoshida, M.; Miyajima, I.; Araki, T.; Hirai, Y.; J Heterocycl. Chem. 1995, 32(5), 1517-1519 which describes methods for copper cyanide; Yutilov, Y.M.; Svertilova, I.A.; Khim Geterotsikl Soedin 1994, 8, 1071-1075 which utilizes potassium cyanide; and Prager, R.H.; Tsopelas, C.; Heisler, T.; Aust. J. Chem. 1991, 44 (2), 277-285 which utilizes copper cyanide in the presence of MeOS(O)₂F. The chloride or more preferably a bromide on the azaindole may be displaced by sodium cyanide in dioxane via the method described in Synlett. 1998, 3, 243-244. Alternatively, Nickel dibromide, Zinc, and triphenyl phosphine in can be used to activate aromatic and heteroaryl chlorides to displacement via potassium cyanide in THF or other suitable solvent by the methods described in Eur. Pat. Appl., 831083, 1998.

The conversion of the cyano intermediate, 62, to the carboxylic acid intermediate, 63, is depicted in step 2, Scheme 22 or in step a12, Scheme 23. Many methods for the conversion of nitriles to acids are well known in the art and may be employed. Suitable conditions for step 2 of Scheme 22 or the conversion of intermediate 65 to intermediate 66 below employ potassium hydroxide, water, and an aqueous alcohol such as ethanol. Typically the reaction must be heated at refluxing temperatures for one to 100 h. Other procedures for hydrolysis include those described in:
Shiotani, S.; Taniguchi, K.; J. Heterocycl. Chem. 1997, 34(2), 493-499; Boogaard; A. T.; Pandit, U. K.; Koomen, G.-J.; Tetrahedron 1994, 50(8), 2551-2560; Rivalle, C.; Bisagni, E.; Heterocycles 1994, 38(2), 391-397; Macor, J.E.; Post, R.; Ryan, K.; J. Heterocycl. Chem. 1992, 29(6), 1465-1467.

The acid intermediate, 66 (Scheme 23), may then be esterified using conditions well known in the art. For example, reaction of the acid with diazomethane in an inert solvent such as ether, dioxane, or THF would give the methyl ester. Intermediate 67 may then be converted to intermediate 68 according to the procedure described in Scheme 2. Intermediate 68 may then be hydrolyzed to provide intermediate 69.

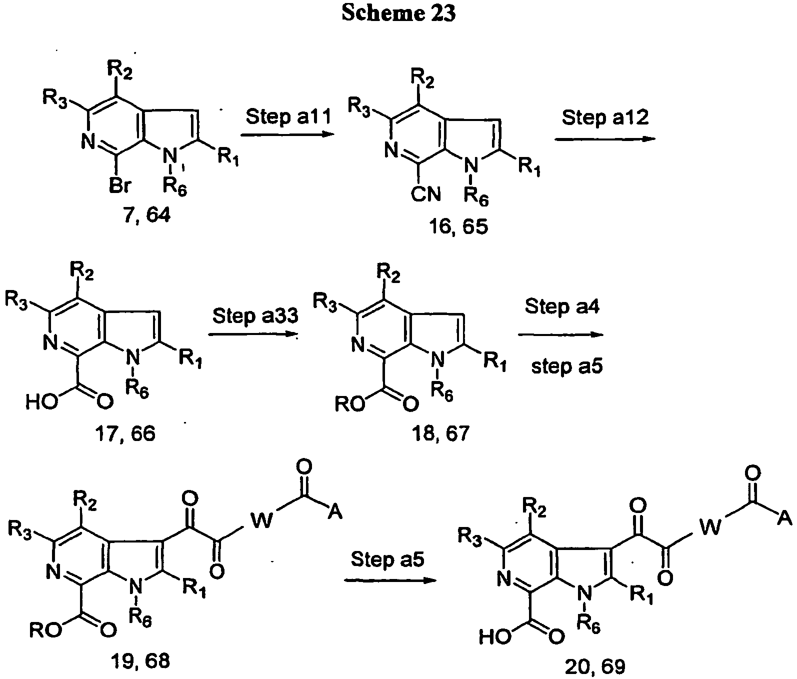

As shown in Scheme 24, step a13 another preparation of the indoleoxoacetylpiperazine 7-carboxylic acids, 69, is carried out by oxidation of the corresponding 7-carboxaldehyde, 70. Numerous oxidants are suitable for the conversion of aldehyde to acid and many of these are described in standard organic chemistry texts such as: Larock, Richard C., Comprehensive organic transformations : a guide to functional group preparations 2nd ed. New York : Wiley-VCH, 1999. One preferred method is the use of silver nitrate or silver oxide in a solvent such as aqueous or anhydrous methanol at a temperature of ~25 °C or as high as reflux. The reaction is typically carried out for one to 48 h and is typically monitored by TLC or LC/MS until complete conversion of product to starting material has occurred. Alternatively, KmnO₄ or CrO₃/H₂SO₄ could be utilized.

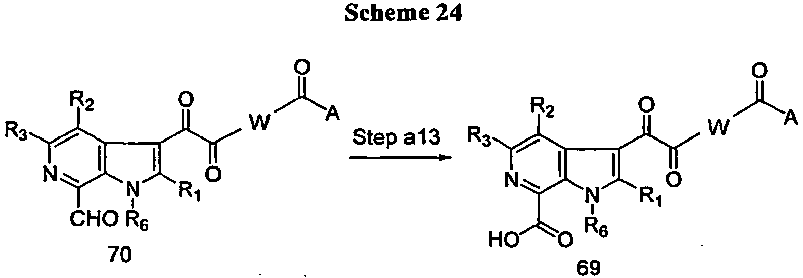

Scheme 25 gives a specific example of the oxidation of an aldehyde intermediate, 70a, to provide the carboxylic acid intermediate, 69a.

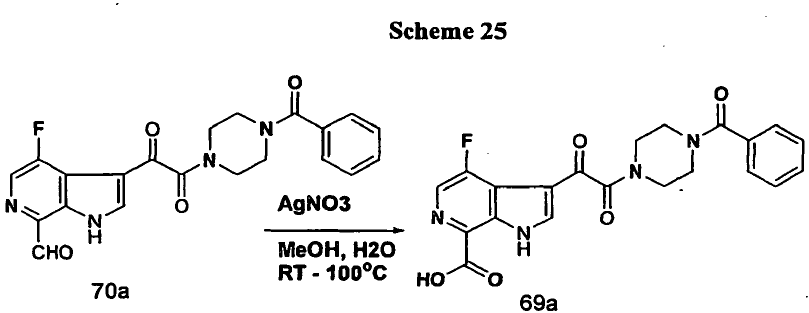

Alternatively, intermediate 69 can be prepared by the nitrile method of synthesis carried out in an alternative order as shown in Scheme 26. The nitrile hydrolyis step can be delayed and the nitrile carried through the synthesis to provide a nitrile which can be hydrolyzed to provide the free acid, 69, as above.

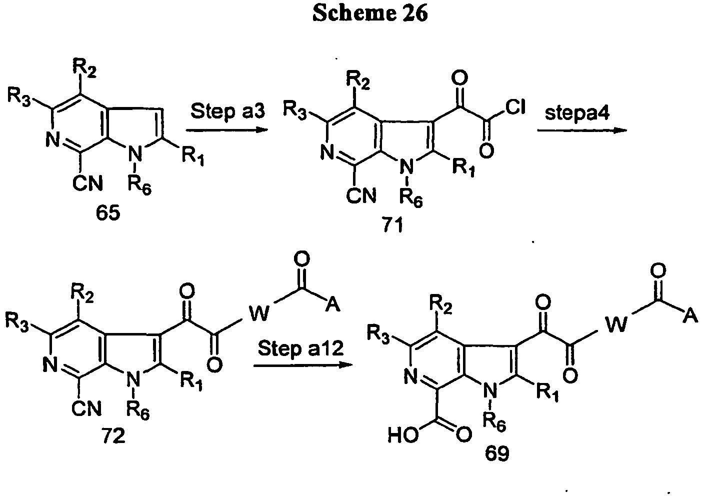

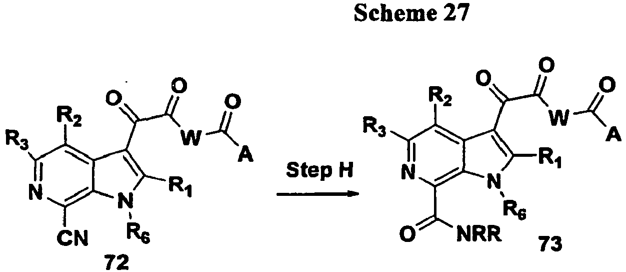

**Step H** The direct conversion of nitriles, such as 72, to amides, such as 73, shown in Scheme 27, Step H, can be carried out using the conditions as described in Shiotani, S.; Taniguchi, K.; J. Heterocycl. Chem. 1996, 33(4), 1051-1056 (describes the use of aqueous sulfuric acid); Memoli, K.A.; Tetrahedron Lett. 1996, 37(21), 3617-3618; Adolfsson, H.; Waemmark, K.; Moberg, C.; J. Org. Chem. 1994, 59(8), 2004-2009; and El Hadri, A.; Leclerc, G.; J. Heterocycl. Chem. 1993, 30(3), 631-635.

### Step I For NH2

Shiotani, S.; Taniguchi, K.; J. Heterocycl. Chem. 1997, 34(2), 493-499; Boogaard, A. T.; Pandit, U. K.; Koomen, G.-J.; Tetrahedron 1994, 50(8), 2551-2560; Rivalle, C.; Bisagni, E.; Heterocycles 1994, 38(2), 391-397;
Macor, J.E.; Post, R.; Ryan, K.; J. Heterocycl. Chem. 1992, 29(6), 1465-1467.

### Step J

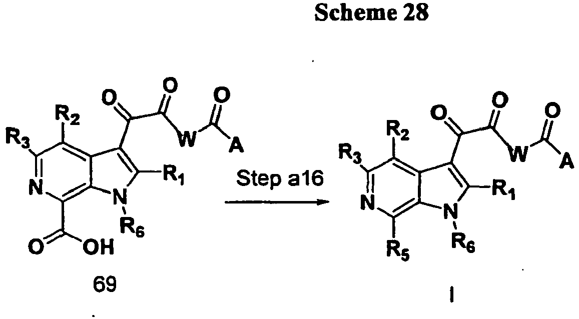

The following scheme (28A) shows an example for the preparation of 4-fluoro-7substituted azaindoles from a known starting materials. References for the Bartoli indole synthesis were mentioned earlier. The conditions for tranformation to the nitriles, acids, aldeheydes, heterocycles and amides have also been described in this application.

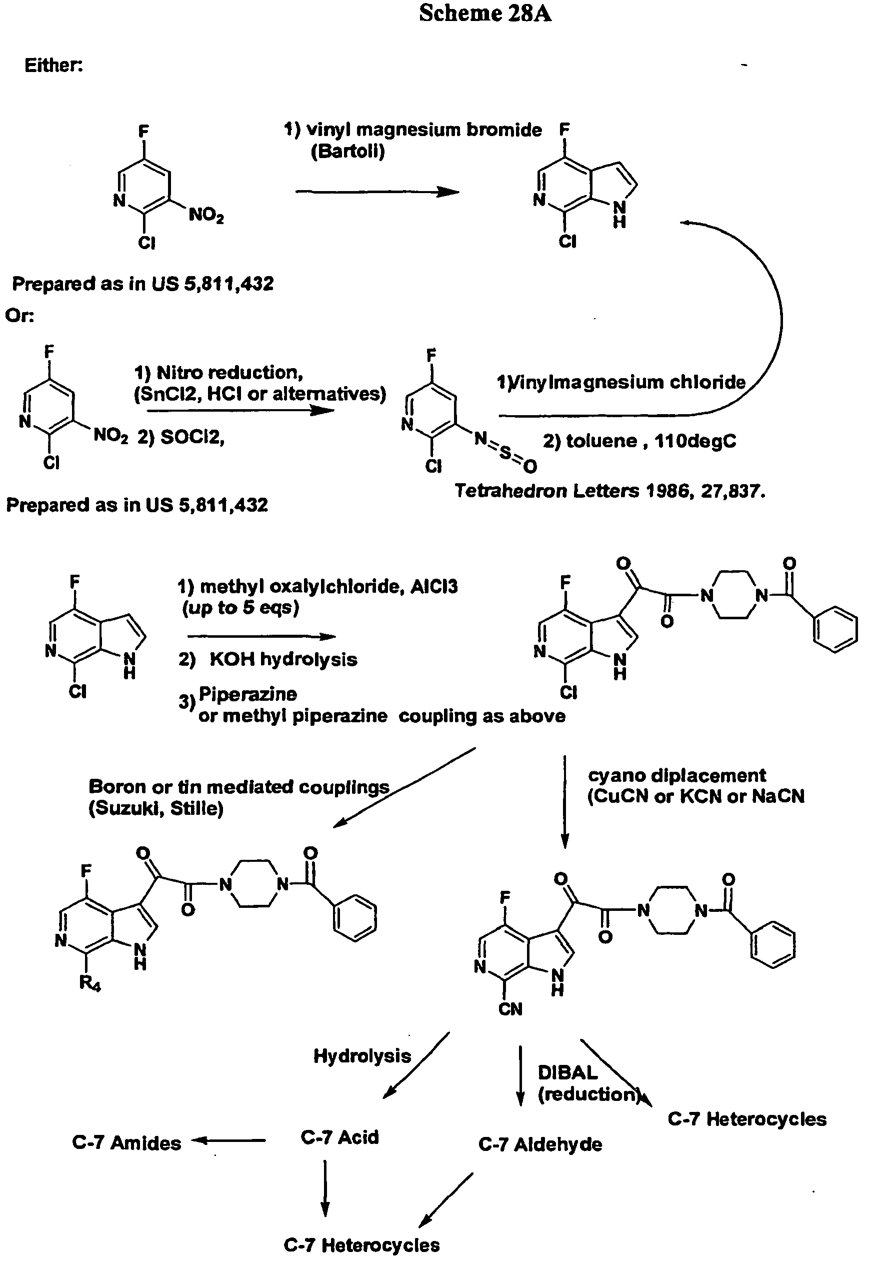

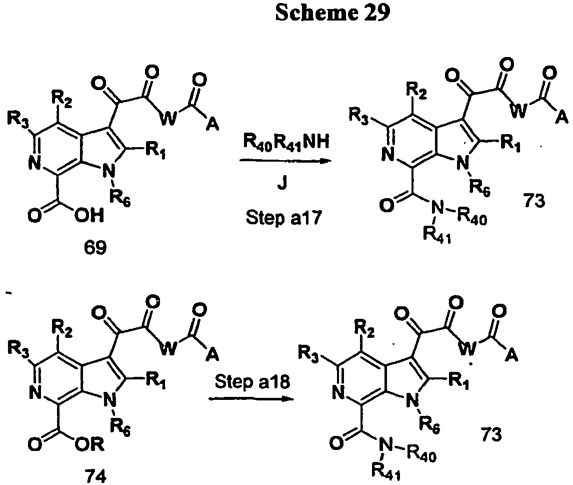

Steps a16, a17, and a18 encompasses reactions and conditions for 1°, 2° and 3° amide bond formation as shown in Schemes 28 and 29 which provide compounds such as those of Formula 73.

The reaction conditions for the formation of amide bonds encompass any reagents that generate a reactive intermediate for activation of the carboxylic acid to amide formation, for example (but not limited to), acyl halide, from carbodiimide, acyl iminium salt, symmetrical anhydrides, mixed anhydrides (including phosphonic/phosphinic mixed anhydrides), active esters (including silyl ester, methyl ester and thioester), acyl carbonate, acyl azide, acyl sulfonate and acyloxy N-phosphonium salt. The reaction of the indole carboxylic acids with amines to form amides may be mediated by standard amide bond forming conditions described in the art. Some examples for amide bond formation are listed in references 41-53 but this list is not limiting. Some carboxylic acid to amine coupling reagents which are applicable are EDC, Diisopropylcarbodiimide or other carbodiimides, PyBop (benzotriazolyloxytris(dimethylamino) phosphonium hexafluorophosphate), 2-(1H-benzotriazole-1-yl)-1, 1, 3, 3-tetramethyl uronium hexafluorophosphate (HBTU). A particularly useful method for azaindole 7-carboxylic acid to amide reactions is the use of carbonyl imidazole as the coupling reagent as described in reference 53. The temperature of this reaction may be lower than in the cited reference , from 80 °C (or possibly lower) to 150°C or higher. A more specific application is depicted in Scheme 30.

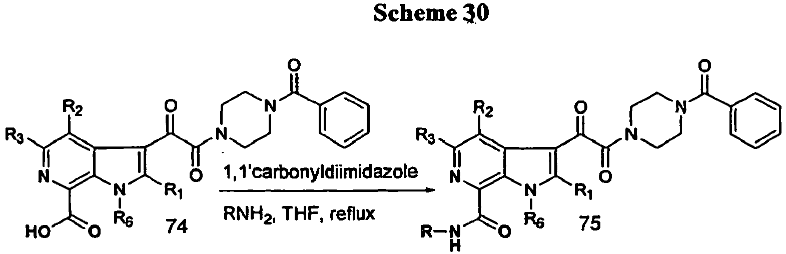

The following four general methods provide a more detailed description for the preparation of indolecarboamides and these methods were employed for the synthesis of compounds of Formula I.

### Method 1:

To a mixture of an acid intermediate, such as 69, (1 equiv., 0.48 mmol), an appropriate amine (4 equiv.) and DMAP (58 mg, 0.47 mmol) dissolved CH₂Cl₂ (1 mL) was added EDC (90 mg, 0.47 mmol). The resulting mixture was shaken at rt for 12h, and then evaporated *in vacuo.* The residue was dissolved in MeOH, and subjected to preparative reverse phase HPLC purification.

### Method 2:

To a mixture of an appropriate amine (4 equiv.) and HOBT (16 mg, 0.12 mmol) in THF (0.5 mL) was added an acid intermediate, such as 69, (25 mg, 0.06 mmol) and NMM (50 µl, 0.45 mmol), followed by EDC (23 mg, 0.12 mmol). The reaction mixture was shaken at rt for 12 h. The volatiles were evaporated *in vacuo;* and the residue dissolved in MeOH and subjected to preparative reverse phase HPLC purification.

### Method 3:

To a mixture of an acid intermediate, such as 69, (0.047 mmol), amine (4 equiv.) and DEPBT (prepared according to Li, H.; Jiang, X. Ye, Y.; Fan, C.; Todd, R; Goodman, M. Organic Letters 1999, 1, 91; 21 mg, 0.071 mmol) in DMF (0.5 mL) was added TEA (0.03 mL, 0.22 mmol). The resulting mixture was shaken at rt for 12 h; and then diluted with MeOH (2 mL) and purified by preparative reverse phase HPLC.

### Method 4:

A mixture of an acid intermediate, such as 69, (0.047mmol) and 8.5 mg (0.052mmol)) of 1,1-carbonyldiimidazole in anhydrous THF (2 mL) was heated to reflux under nitrogen. After 2.5h, 0.052 mmol of amine was added and heating continued. After an additional period of 3~20 h at reflux, the reaction mixture was cooled and concentrated in vacuo. The residue was purified by chromatography on silica gel to provide a compound of Formula I

In addition, the carboxylic acid may be converted to an acid chloride using reagents such as thionyl chloride (neat or in an inert solvent) or oxalyl chloride in a solvent such as benzene, toluene, THF, or CH₂Cl₂. The amides may alternatively, be formed by reaction of the acid chloride with an excess of ammonia, primary, or secondary amine in an inert solvent such as benzene, toluene, THF, or CH₂Cl₂ or with stoichiometric amounts of amines in the presence of a tertiary amine such as triethylamine or a base such as pyridine or 2,6-lutidine. Alternatively, the acid chloride may be reacted with an amine under basic conditions (Usually sodium or potassium hydroxide) in solvent mixtures containing water and possibly a miscible co solvent such as dioxane or THF. Scheme 25B depicts a typical preparation of an acid chloride and derivatization to an amide of Formula I. Additionally, the carboxylic acid may be converted to an ester preferably a methyl or ethyl ester and then reacted with an amine. The ester may be formed by reaction with diazomethane or alternatively trimethylsilyl diazomethane using standard conditions which are well known in the art. References and procedures for using these or other ester forming reactions can be found in reference 52 or 54.

Additional references for the formation of amides from acids are: Norman, M.H.; Navas, F. III; Thompson, J.B.; Rigdon, G.C.; J. Med Chem. 1996, 39(24), 4692-4703; Hong, F.; Pang, Y.-P.; Cusack, B.; Richelson, E.; J. Chem. Soc., Perkin Trans 1 1997, 14, 2083-2088; Langry, K.C.; Org. Prep. Proc. Int. 1994, 26(4), 429-438; Romero, D.L.; Morge, R.A.; Biles, C.; Berrios-Pena, N.; May, P.D.; Palmer, J.R; Johnson, P.D.; Smith, H.W.; Busso, M.; Tan, C.-K.; Voorman, R.L.; Reusser, F.; Althaus, I.W.; Downey, K.M.; et al.; J. Med. Chem. 1994, 37(7), 999-1014; Bhattacharjee, A.; Mukhopadhyay, R.; Bhattacharjya, A.; Indian J. Chem., Sect B 1994, 33(7), 679-682.

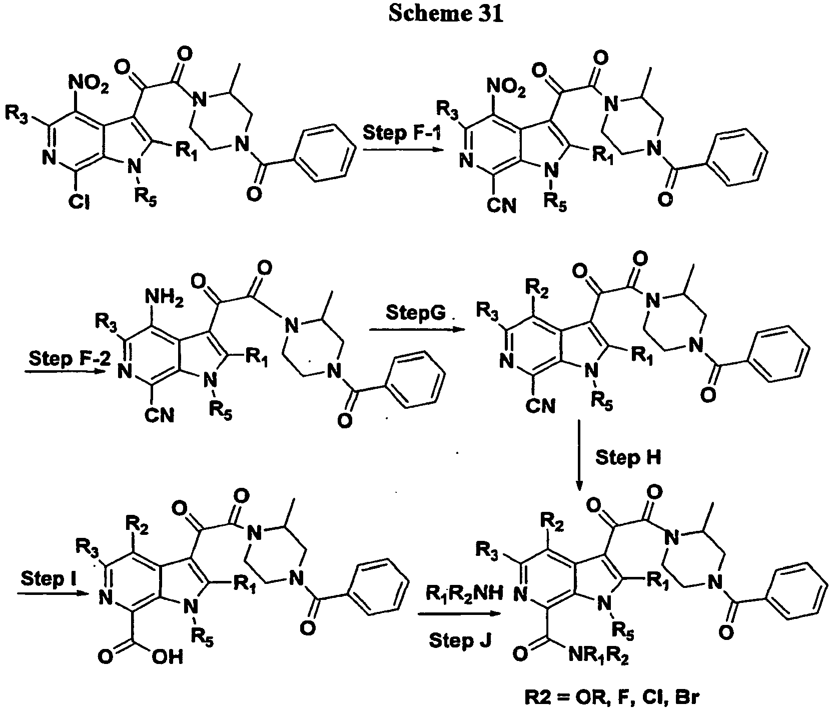

Scheme 31 shows synthetic transformations on a chloro nitro azaindole. Step F-1 of Scheme 31 can be carried may be carried out according to the following procedures: Yamaguchi, S.; Yoshida, M.; Miyajima, I.; Araki, T.; Hirai, Y.; J. Heterocycl. Chem. 1995, 32(5), 1517-1519;

Yutilov, Y.M.; Svertilova, I. A.; Khim Geterotsikl Soedin 1994, 8, 1071-1075; and Prager, RH.; Tsopelas, C.; Heisler, T.; Aust. J. Chem. 1991, 44(2), 277-285. Step F-2 of Scheme 31 may be accomplished according to the procedures set forth in: Ciurla, H.; Puszko, A.; Khim Geterotsikl Soedin 1996, 10, 1366-1371; Robinson, R.P.; Donahue, K.M.; Son, P.S.; Wagy, S.D.; J. Heterocycl. Chem. 1996, 33(2), 287-293; Nicolai, E.; Claude, S.; Teulon, J. M.; J. Heterocycl. Chem. 1994, 31(1), 73-75; Hwu, J.R; Wong, F.F.; Shiao, M.-J.; J. Org. Chem. 1992, 57(19), 5254-5255; Shiao, M.-J.; Lai, L.-L.; Ku, W.-S.; Lin, P.-Y.; Hwu, J.R.; J. Org. Chem. 1993, 58(17), 4742-4744.

The introduction of an alkoxy or aryloxy substituent onto the azaindole (Step G, Scheme 31, R₂ is alkoxy or aryloxy) may be accomplished by the f procedures described in Klemm, L.H.; Zell, R.; J. Heterocycl. Chem. 1968, 5, 773; Bradsher, C. K.; Brown, F. C.; Porter, H. K.; J. Am. Chem. Soc. 1954, 76, 2357; and Hodgson, H. H.; Foster, C. K.; J. Chem. Soc. 1942, 581.

The introduction of a fluorine substituent onto the azaindole (Step G, Scheme 31) may be accomplished according to the procedures as described in Sanchez, J. P.; Gogliotti, R. D.; J. Heterocycl. Chem. 1993, 30(4), 855-859; Rocca, P.; Marsais, F.; Godard, A.; Queguiner, G.; Tetrahedron Lett. 1993, 34(18), 2937-2940; and Sanchez, J.P.; Rogowski, J.W.; J. Heterocycl. Chem. 1987, 24, 215.

The introduction of a chlorine substituent onto the azaindole (Step G, Scheme 31) may be accomplished according to the procedures as described in Ciurla, H.; Puszko, A.; Khim Geterotsikl Soedin 1996, 10, 1366-1371; Raveglia, L.F.; Giardinal, G.A.M.; Grugni, M.; Rigolio, R.; Farina, C.; J. Heterocycl. Chem. 1997, 34(2), 557-559; Matsumoto, J.I.; Miyamoto, T.; Minamida, A.; Mishimura, Y.; Egawa, H.; Mishimura, H.; J. Med. Chem. 1984, 27(3), 292; Lee, T.-C.; Salemnick, G.; J. Org. Chem. 1975, 24, 3608.

The introduction of a bromine substituent onto the azaindole (Step G, Scheme 31) may be accomplished according to the procedures as described in Raveglia, L.F.; Giardina, G.A.M.; Grugni, M.; Rigolio, R.; Farina, C. ; J. Heterocycl. Chem. 1997, 34(2), 557-559; Talik, T.; Talik, Z.; Ban-Oganowska, H.; Synthesis 1974, 293; Abramovitch, R. A.; Saha, M.; Can. J. Chem. 1966, 44,1765.

It is well known in the art that heterocycles may be prepared from an aldehyde, carboxylic acid, carboxylic acid ester, carboxylic acid amide, carboxylic acid halide, or cyano moiety or attached to another carbon substituted by a bromide or other leaving group such as a triflate, mesylate, chloride, iodide, or phosponate. The methods for preparing such intermediates from intermediates typified by the carboxylic acid intermediate, 69, bromo intermediate, 76, or aldehyde intermediate, 70 described above are known by a typical chemist practitioner. The methods or types of heterocycles which may be constructed are described in the chemical literature. Some representative references for finding such heterocycles and their construction are included in reference 55 through 67 but should in no way be construed as limiting. However, examination of these references shows that many versatile methods are available for synthesizing diversely substituted heterocycles and it is apparent to one skilled in the art that these can be applied to prepare compounds of Formula I. Chemists well versed in the art can now easily, quickly, and routinely find numerous reactions for preparing heterocycles, amides, oximes or other substituents from the above mentioned starting materials by searching for reactions or preparations using a conventional electronic database such as Scifinder (American Chemical Society), Crossfire (Beilstein), Theilheimer, or Reaccs (MDS). The reaction conditions identified by such a search can then be employed using the substrates described in this application to produce all of the compounds envisioned and covered by this invention. In the case of amides, commercially available amines can be used in the synthesis. Alternatively, the above mentioned search programs can be used to locate literature preparations of known amines or procedures to synthesize new amines. These procedures are then carried out by one with typical skill in the art to provide the compounds of Formula I for use as antiviral agents.

As shown below in Scheme 32, step a13, suitable substituted azaindoles, such as the bromoazaindole intermediate, 76, may undergo metal mediated couplings with aryl groups, heterocycles, or vinyl stannanes to provide compounds of Formula I wherein R₅ is aryl, heteroaryl, or heteroalicyclic for example. The bromoazaindole intermediates, 76 (or azaindole triflates or iodides) may undergo Stille-type coupling with heteroarylstannanes as shown in Scheme 32, step a13. Conditions for this reaction are well known in the art and references 68-70 as well as reference 52 provide numerous conditions in addition to the specific examples provided in Scheme 14 and in the specific embodiments. It can be well recognized that an indole stannane could also couple to a heterocyclic or aryl halide or triflate to construct compounds of Formula I. Suzuki coupling (reference 71) between the bromo intermediate, 76, and a suitable boronate could also be employed and some specific examples are contained in this application.

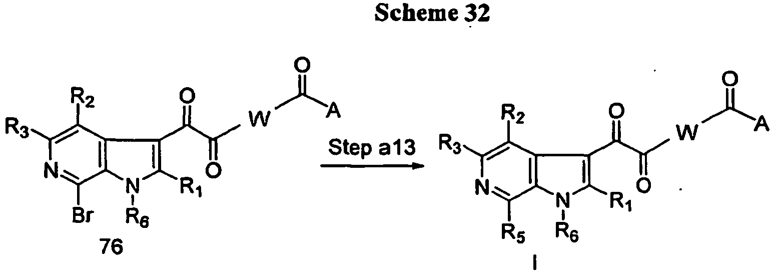

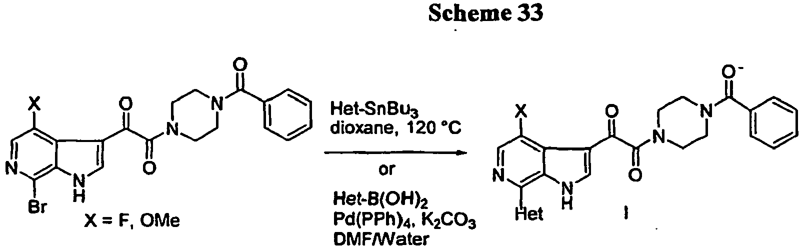

As shown in Scheme 34, step a14, aldehyde intermediates, 70, may be used to generate numerous compounds of Formula I. The aldehyde group may be a precursor for any of the substituents R₁ through R₅ but the transormation for R₅ is depicted above for simplicity. The aldehyde intermediate 70, may be reacted to become incorporated into a ring as 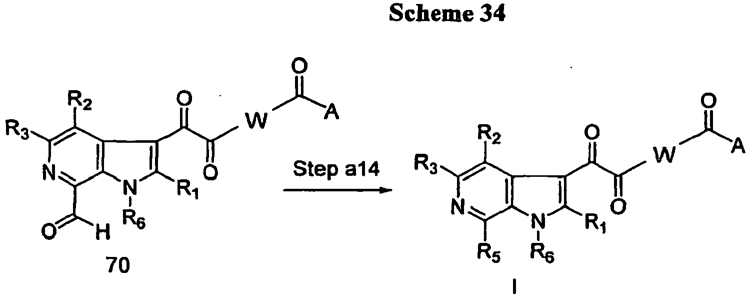 described in the claims or be converted into an acyclic group. The aldehyde, 70, may be reacted with a Tosmic based reagent to generate oxazoles (references 42 and 43 for example). The aldehyde, 70, may be reacted with a Tosmic reagent and than an amine to give imidazoles as in reference 72 or the aldehyde intermediate, 70, may be reacted with hydroxylamine to give an oxime which is a compound of Formula I as described below. Oxidation of the oxime with NBS, t-butyl hypochlorite, or the other known reagents would provide the N-oxide which react with alkynes or 3 alkoxy vinyl esters to give isoxazoles of varying substitution. Reaction of the aldehyde intermediate 70, with the known reagent, 77 (reference 70) shown below under basic conditions would provide 4-aminotrityl oxazoles.

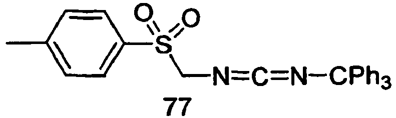

Removal of the trityl group would provide 4-amino oxazoles which could be substitutued by acylation, reductive alkylation or alkylation reactions or heterocycle forming reactions. The trityl could be replaced with an alternate protecting group such as a monomethoxy trityl, CBZ, benzyl, or appropriate silyl group if desired. Reference 73 demonstrates the preparation of oxazoles containing a triflouoromethyl moiety and the conditions described therein demonstrates the synthesis of oxazoles with fluorinated methyl groups appended to them.

The aldehyde could also be reacted with a metal or Grignard (alkyl, aryl, or heteroaryl) to generate secondary alcohols. These would be efficacious or could be oxidized to the ketone with TPAP or MnO₂ or PCC for example to provide ketones of Formula I which could be utilized for treatment or reacted with metal reagents to give tertiary alcohols or alternatively converted to oximes by reaction with hydroxylamine hydrochlorides in ethanolic solvents. Alternatively the aldehyde could be converted to benzyl amines via reductive amination. An example of oxazole formation via a Tosmic reagent is shown below in Scheme 35. The same reaction would work with aldehydes at other positions and also in the 5 and 6 aza indole series.

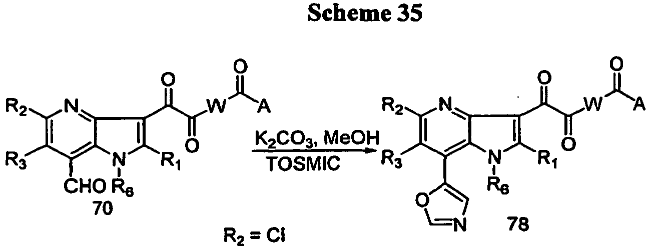

Scheme 36 shows in step a15, a cyano intermediate, such as 62, which could be directly converted to compounds of Formula I via heterocycle formation or reaction with organometallic reagents.

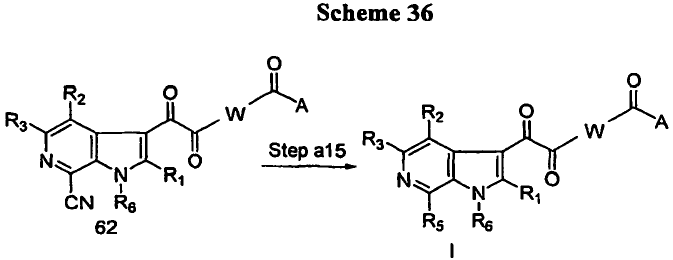

Scheme 37 shows a method for acylation of a cyanoindole intermediate of formula 65 with oxalyl chloride which would give acid chloride, 79, which could then be coupled with the appropriate amine in the presence of base to provide 80.

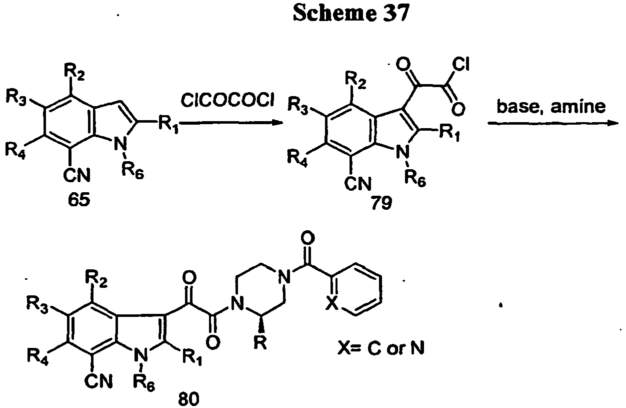

The nitrile intermediate, 80, could be converted to the tetrazole of formula 81, which could then be alkylated with trimethylsilyldiazomethane to give the compound of formula 82 (Scheme 38).

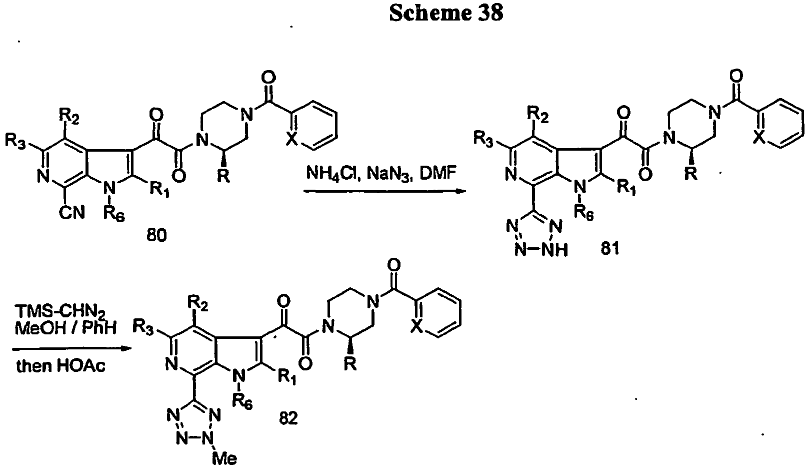

Tetrazole alkylation with alkyl halides would be carried out prior to azaindole acylation as shown in Scheme 39. Intermediate 65 could be converted to tetrazole, 83, which could be alkylated to provide 84. Intermediate 84 could then be acylated and hydrolyzed to provide 85 which could be subjected to amide formation conditions to provide 86. The group appended to the tetrazole may be quite diverse and still exhibit impressive potency.

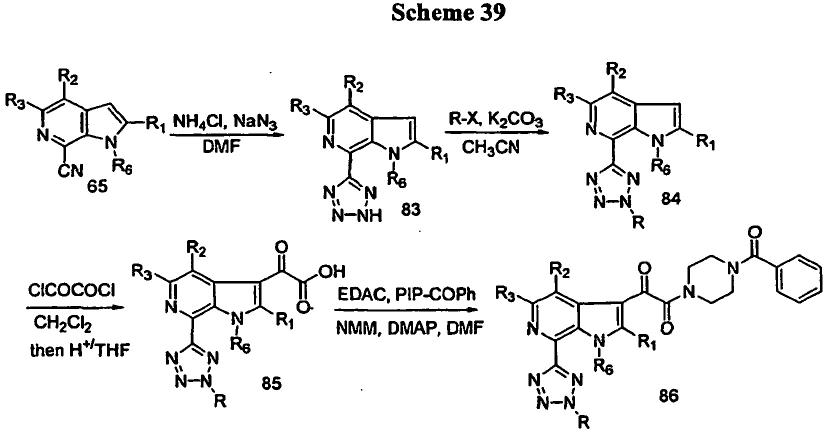

Scheme 40 shows that an oxadiazole such as , 88, may be prepared by the addition of hydroxylamine to the nitrile, 80, followed by ring closure of intermediate 87 with phosgene. Alkylation of oxadiazole, 88, with trimethylsilyldiazomethane would give the compound of formula 89.

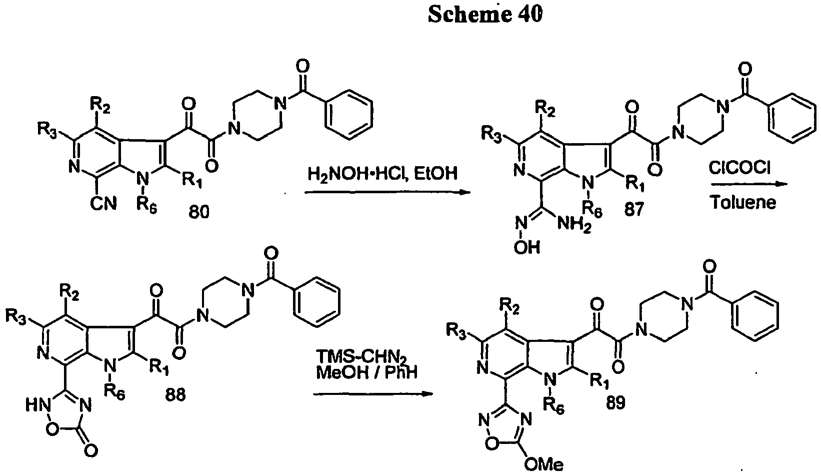

A 7-cyanoindole, such as 80, could be efficiently converted to the imidate ester under conventional Pinner conditions using 1,4-dioxane as the solvent. The imidate ester can be reacted with nitrogen, oxygen and sulfur nucleophiles to provide C7-substituted indoles, for example: imidazolines, benzimidazoles, azabenzimidazoles, oxazolines, oxadiazoles, thiazolines, triazoles, pyrimidines and amidines etc. For example the imidate may be reacted with acetyl hydrazide with heating in a nonparticipating solvent such as dioxane, THF, or benzene for example. (aqueous base or aqueous base in an alcoholic solvent may need to be added to effect final dehydrative cyclization in some cases) to form a methyl triazine. Other hydrazines can be used. Triazines can also be installed via coupling of stannyl triazines with 4,5,6,or 7-bromo or chloro azaindoles. The examples give an example of the formation of many of these heterocycles.

### References:

(1) Das, B. P.; Boykin, D. W. J. Med. Chem. 1977, 20, 531.
(2) Czarny, A.; Wilson, W. D.; Boykin, D. W. J. Heterocyclic Chem. 1996, 33, 1393.
(3) Francesconi, I.; Wilson, W. D.; Tanious, F. A.; Hall, J. E.; Bender, B. C.; Tidwell, R. R.; McCurdy, D.; Boykin, D. W. J. Med. Chem. 1999, 42, 2260.

Scheme 41 shows addition of either hydroxylamine or hydroxylamine acetic acid to aldehyde intermediate 90 may give oximes of Formula 91.

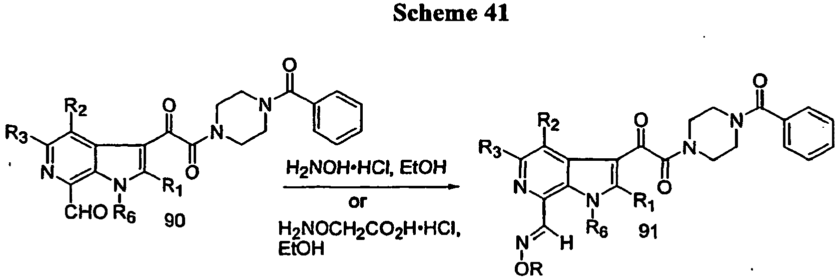

An acid may be a precursor for substituents R₁ through R₅ when it occupies the corresponding position such as R₅ as shown in Scheme 42.

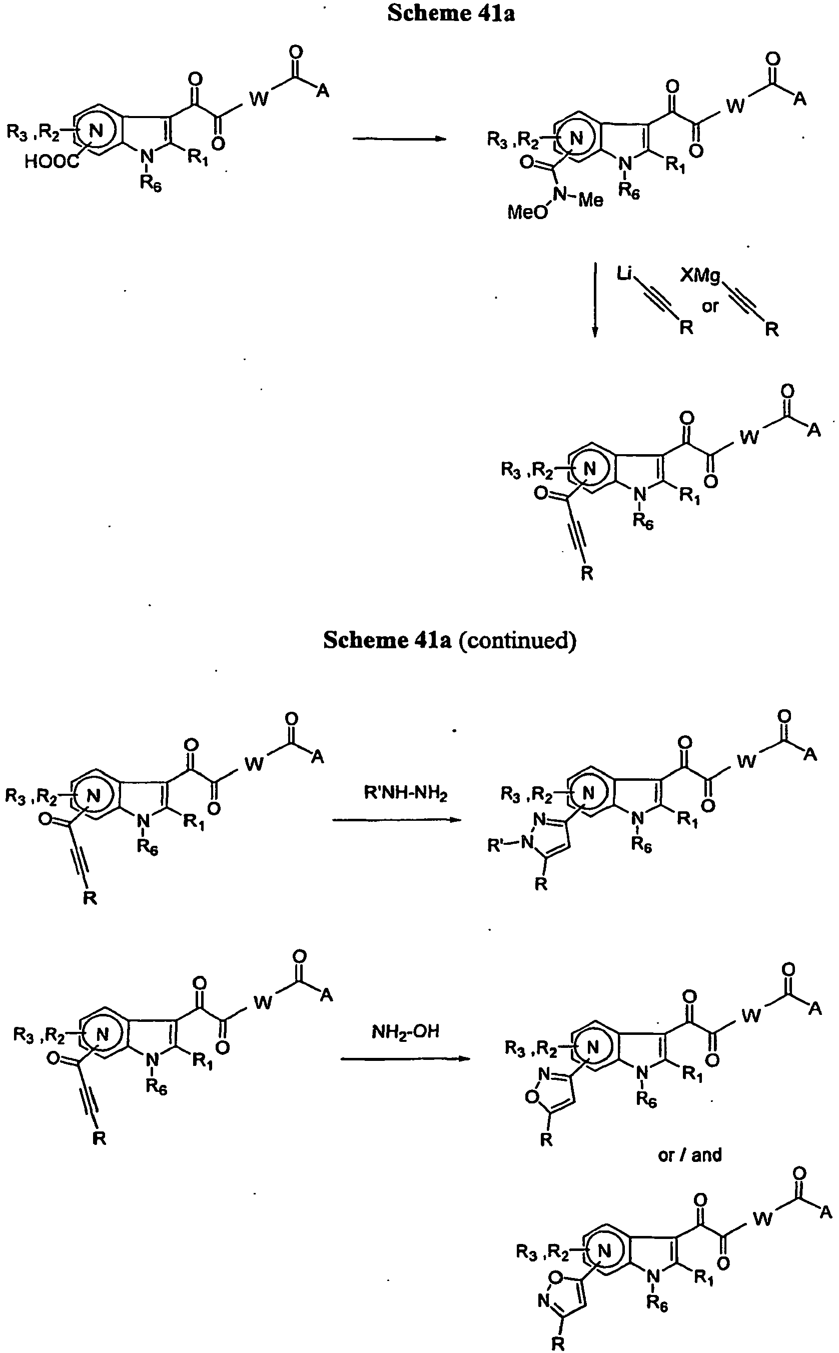

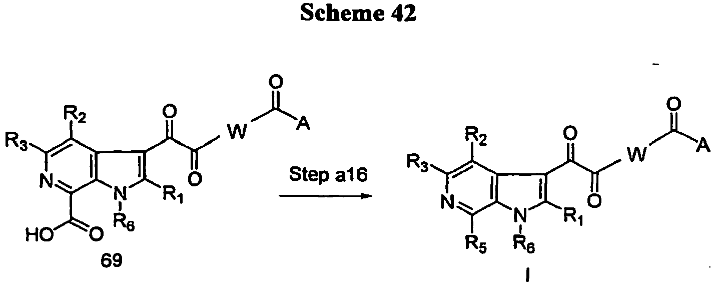

An acid intermediate, such as 69, may be used as a versatile precursor to generate numerous substituted compounds. The acid could be converted to hydrazonyl bromide and then a pyrazole via reference 74. One method for general heterocycle synthesis would be to convert the acid to an alpha bromo ketone (ref 75) by conversion to the acid chloride using standard methods, reaction with diazomethane, and finally reaction with HBr. The alpha bromo ketone could be used to prepare many different compounds of Formula I as it can be converted to many heterocycles or other compounds of Formula I. Alpha amino ketones can be prepared by displacement of the bromide with amines. Alternatively, the alpha bromo ketone could be used to prepare heterocycles not available directly from the aldeheyde or acid. For example, using the conditions of Hulton in reference 76 to react with the alpha bromo ketone would provide oxazoles. Reaction of the alpha bromoketone with urea via the methods of reference 77 would provide 2-amino oxazoles. The alpha bromoketone could also be used to generate furans using beta keto esters(ref 78-80) or other methods, pyrroles (from beta dicarbonyls as in ref 81 or by Hantsch methods (ref 82) thiazoles , isoxazoles and imidazoles (ref 83) example using literature procedures. Coupling of the aforementioned acid chloride with N-methyl-O-methyl hydroxyl amine would provide a "Weinreb Amide" which could be used to react with alkyl lithiums or Grignard reagents to generate ketones. Reaction of the Weinreb anion with a dianion of a hydroxyl amine would generate isoxazoles (ref 84). Reaction with an acetylenic lithium or other carbanion would generate alkynyl indole ketones. Reaction of this alkynyl intermediate with diazomethane or other diazo compounds would give pyrazoles (ref 85). Reaction with azide or hydroxyl amine would give heterocycles after elimination of water. Nitrile oxides would react with the alkynyl ketone to give isoxazoles (ref 86). Reaction of the initial acid to provide an acid chloride using for example oxalyl chloride or thionyl chloride or triphenyl phosphine/ carbon tetrachloride provides a useful intermediate as noted above. Reaction of the acid chloride with an alpha ester substituted isocyanide and base would give 2-substituted oxazoles (ref 87). These could be converted to amines, alcohols, or halides using standard reductions or Hoffman/Curtius type rearrangements.

Scheme 43 describes alternate chemistry for installing the oxoacetyl piperazine moiety onto the 3 position of the azaindoles. StepA"' in Scheme 43 depicts reaction with formaldehyde and dimethylamine using the conditions in Frydman, B.; Despuy, M.E.; Rapoport, H.; J. Am. Chem. Soc. 1965, 87, 3530 will provide the dimethylamino compound shown.

Step B'" shows displacement with potassium cyanide would provide the cyano derivative according to the method described in Miyashita, K.; Kondoh, K.; Tsuchiya, K.; Miyabe, H.; Imanishi, T.; Chem. Pharm. Bull. 1997, 45(5), 932-935 or in Kawase, M.; Sinhababu, A.K.; Borchardt, R.T.; Chem. Pharm. Bull. 1990, 38(11), 2939-2946. The same transformation could also be carried out using TMSCN and a tetrabutylammonium flouride source as in Iwao, M.; Motoi, O.; Tetrahedron Lett. 1995, 36(33), 5929-5932. Sodium cyanide could also be utilized.

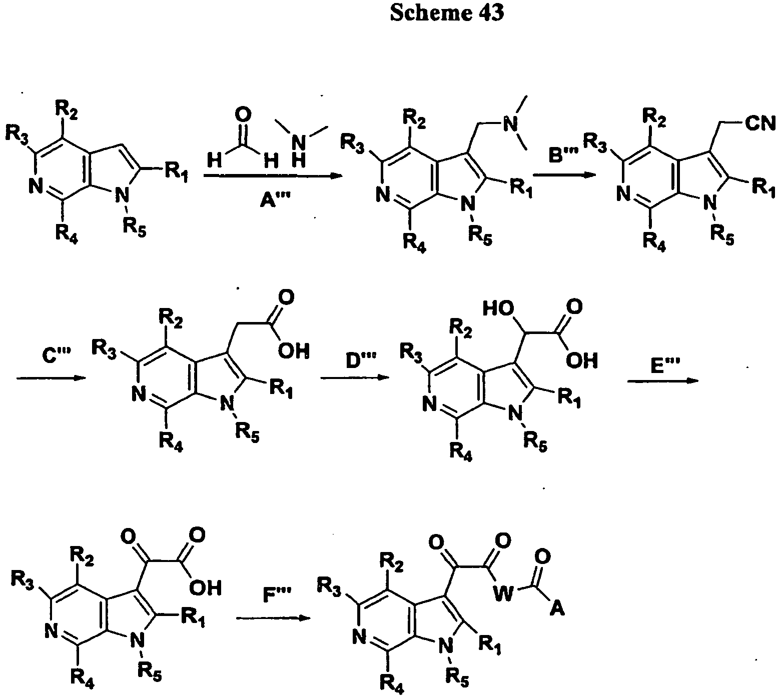

Step C'" of Scheme 43 depicts hydrolysis of the nitrile with sodium hydroxide and methanol would provide the acid via the methods described in Iwao, M.; Motoi, O.; Tetrahedron Lett. 1995, 36(33), 5929-5932 for example. Other basic hydrolysis conditions using either NaOH or KOH as described in Thesing, J.; et al.; Chem. Ber. 1955, 88, 1295 and Geissman, T.A.; Armen, A.; J. Am. Chem. Soc. 1952, 74, 3916. The use of a nitrilase enzyme to achieve the same transformation is described by Klempier N, de Raadt A, Griengl H, Heinisch G, J. Heterocycl. Chem., 1992 29, 93, and may be applicable.

Step D"' of Scheme 43 depicts an alpha hydroxylation which may be accomplished by methods as described in Hanessian, S.; Wang, W.; Gai, Y.; Tetrahedron Lett. 1996, 37(42), 7477-7480; Robinson, R. A.; Clark, J. S.; Holmes, A. B.; J. Am. Chem. Soc. 1993, 115(22), 10400-10401 (KN(TMS)₂ and then camphorsulfonyloxaziridine or another oxaziridine; andDavis, F.A.; Reddy, R.T.; Reddy, R.E.; J. Org. Chem. 1992, 57(24), 6387-6389.

Step E"' of Scheme 43 shows methods for the oxidation of the alpha hydroxy ester to the ketone which may be accomplished according to the methods described in Mohand, S.A.; Levina, A.; Muzart, J.; Synth. Comm. 1995, 25 (14), 2051-2059. A preferred method for step E'" is that of Ma, Z.; Bobbitt, J.M.; J. Org. Chem. 1991, 56(21), 6110-6114 which utilizes 4-(NH-Ac)-TEMPO in a solvent such as CH₂Cl₂ in the presence of para toluenesulfonic acid. The method described in Corson, B.B.; Dodge, R.A.; Harris, S.A.; Hazen, R.K.; Org. Synth. 1941, I, 241 for the oxidation of the alpha hydroxy ester to the ketone uses KmnO₄ as oxidant. Other methods for the oxidation of the alpha hydroxy ester to the ketone include those described in Hunaeus, ; Zincke,; Ber. Dtsch Chem. Ges. 1877, 10, 1489; Acree,; Am. Chem. 1913, 50, 391; and Claisen,; Ber. Dtsch. Chem. Ges. 1877, 10, 846.

Step F"' of Scheme 43 depicts the coupling reactions which may be carried out as described previously in the application and by a preferred method which is described in Li, H.; Jiang, X.; Ye, Y.-H.; Fan, C.; Romoff, T.; Goodman, M. Organic Lett., 1999, 1, 91-93 and employs 3-(Diethoxyphosphoryloxy)-1,2,3-benzotriazin-4(3*H*)-one (DEPBT); a new coupling reagent with remarkable resistance to racemization.

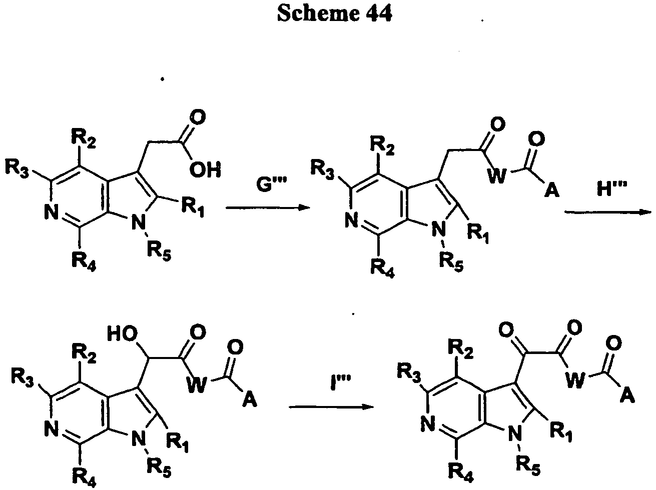

Scheme 44 depicts the preparation of Formula I compounds by coupling HWC(O)A to the acid as described in Step F"' of Scheme 43, followed by hydroxylation as in Step D"' of Scheme 43 and oxidation as described in Step E'" of Scheme 43.

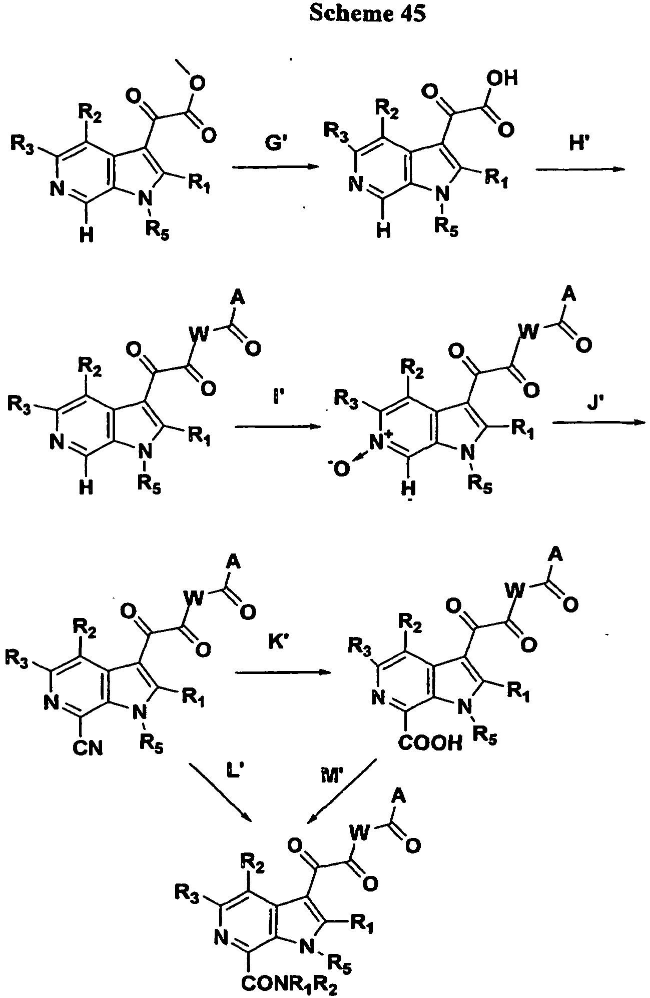

Scheme 45 depicts a method for the preparation which could be used to obtain amido compounds of Formula I. Step G' represents ester hydrolysis followed by amide formation (Step H' as described in Step F"' of Scheme 43). Step I' of Scheme 45 depicts the preparation of the N-oxide which could be accomplished according to the procedures in Suzuki, H.; Iwata, C.; Sakurai, K.; Tokumoto, K.; Takahashi, H.; Hanada, M.; Yokoyama, Y.; Murakami, Y.; Tetrahedron 1997, 53(5), 1593-1606; Suzuki, H.; Yokoyama, Y.; Miyagi, C.; Murakami, Y.; Chem. Pharm. Bull. 1991, 39(8), 2170-2172; and Ohmato, T.; Koike, K.; Sakamoto, Y.; Chem. Pharm. Bull. 1981, 29, 390. Cyanation of the N-oxide is shown in Step J' of Scheme 45 which may be accomplished according to Suzuki, H.; Iwata, C.; Sakurai, K.; Tokumoto, K.; Takahashi, H.; Hanada, M.; Yokoyama, Y.; Murakami, Y.; Tetrahedron 1997, 53(5), 1593-1606 and Suzuki, H.; Yokoyama, Y.; Miyagi, C.; Murakami, Y.; Chem. Pharm. Bull. 1991, 39(8), 2170-2172. Hydrolysis of the nitrile to the acid is depicted in Step K' of Scheme 45 according to procedures such as Shiotani, S.; Tanigucchi, K.; J. Heterocycl. Chem. 1996, 33(4), 1051-1056; Memoli, K.A.; Tetrahedron Lett. 1996, 37(21), 3617-3618; Adolfsson, H.; Waemmark, K.; Moberg, C.; J. Org. Chem. 1994, 59(8), 2004-2009; and El Hadri, A.; Leclerc, G.; J. Heterocycl. Chem. 1993, 30(3), 631-635. Step L' of Scheme 45 depicts a method which could be utilized for the preparation of amido compounds of Formula I from the cyano derivative which may be accomplished according to procedures described in Shiotani, S.; Taniguchi, K.; J. Heterocycl. Chem. 1997, 34(2), 493-499; Boogaard, A.T.; Pandit, U.K.; Koomen, G.-J.; Tetrahedron 1994, 50(8), 2551-2560; Rivalle, C.; Bisagni, E.; Heterocycles 1994, 38(2), 391-397; and Macor, J.E.; Post, R.; Ryan, K.; J. Heterocycl. Chem. 1992, 29(6), 1465-1467. Step M' of Scheme 45 shows a method which could be used for the preparation of amido compounds of Formula I from the acid derivative which may be accomplished according to procedures described in Norman, M.H.; Navas, F. III; Thompson, J.B.; Rigdon, G.C.; J. Med Chem. 1996, 39(24), 4692-4703; Hong, F.; Pang, Y.-P.; Cusack, B.; Richelson, E.; J. Chem. Soc., Perkin Trans 1 1997, 14, 2083-2088; Langry, K. C.; Org. Prep. Proced. Int. 1994, 26(4), 429-438; Romero, D.L.; Morge, R.A.; Biles, C.; Berrios-Pena, N.; May, P.D.; Palmer, J.R.; Johnson, P.D.; Smith, H.W.; Busso, M.; Tan, C.-K.; Voorman, R.L.; Reusser, F.; Althaus, I.W.; Downey, K.M.; et al.; J. Med. Chem. 1994, 37(7), 999-1014 and Bhattacharjee, A.; Mukhopadhyay, R.; Bhattacharjya, A.; Indian J. Chem., Sect B 1994, 33(7), 679-682.

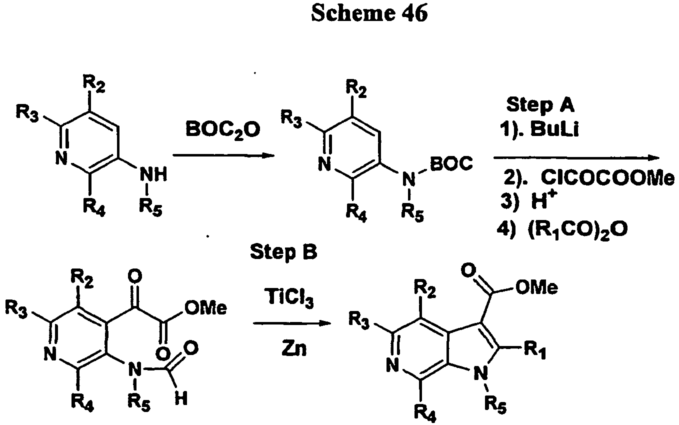

Scheme 46 shows a method which could be used for the synthesis of an azaindole acetic acid derivative. Protection of the amine group could be effected by treatment with di-tert-butyldicarbonate to introduce the t-Butoxycarbonyl (BOC) group. Introduction of the oxalate moiety may then be accomplished as shown in Step A of Scheme 46 according to the procedures described in Hewawasam, P.; Meanwell, N. A.; Tetrahedron Lett. 1994, 35(40), 7303-7306 (using t-Buli, or s-buli, THF); or Stanetty, P.; Koller, H.; Mihovilovic, M.; J. Org. Chem. 1992, 57(25), 6833-6837 (using t-Buli). The intermediate thus formed could then be cyclized to form the azaindole as shown in Step B of Scheme 46 according to the procedures described in Fuerstner, A.; Ernst, A.; Krause, H.; Ptock, A.; Tetrahedron 1996, 52(21), 7329-7344 (using. TiCl3, Zn, DME); or Fuerstner, A.; Hupperts, A.; J. Am. Chem. Soc. 1995, 117(16), 4468-4475 (using Zn, excess Tms-Cl, TiCl3 (cat.), MeCN).

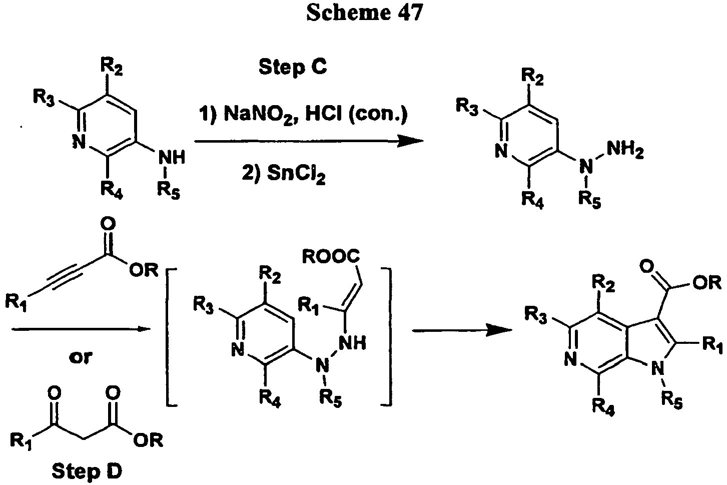

Scheme 47 describes an alternate synthesis which could be used to prepare azaindole acetic acid derivatives. Step C of Scheme 47 could be accomplished by using the procedures described in Harden, F.A.; Quinn, RJ.; Scammells, P.J.; J. Med. Chem. 1991, 34(9), 2892-2898 [use of 1. NaNO₂, conc. HCl 2. SnCl₂, conc. HCl (cat.)]. Typically, 10 equivalents of NaNO₂ and 1.0 equivalents of substrate reacted at 0 °C for 0.25 to 1h and to this reaction mixture was added 3.5 equivalents of SnCl₂. Alternatively, the procedure described in De Roos, K.B.; Salemink, C.A.; Recl. Trav. Chim. Pays-Bas 1971, 90, 1181 (use of NaNO₂, AcOH, H₂O) could be used. The intermediate thus formed could be further reacted and cyclized to provide azaindole acetic acid derivatives as shown in Step D of Scheme 47 and according to the procedures described inAtkinson, C. M.; Mattocks, A. R.; J. Chem. Soc. 1957, 3722; Ain Khan, M.; Ferreira Da Rocha, J.; Heterocycles 1978, 9, 1617; Fusco, R.; Sannicolo, F.; Tetrahedron 1980, 36, 161 [use of HCl (conc)]; Abramovitch, R. A.; Spenser, I. D.; Adv. Heterocycl. Chem. 1964, 3, 79 (use of ZnCl₂, p-Cymene); and Clemo, G. R.; Holt, R. J. W.; J. Chem. Soc. 1953, 1313; (use of ZnCl₂, EtOH, Sealed tube).

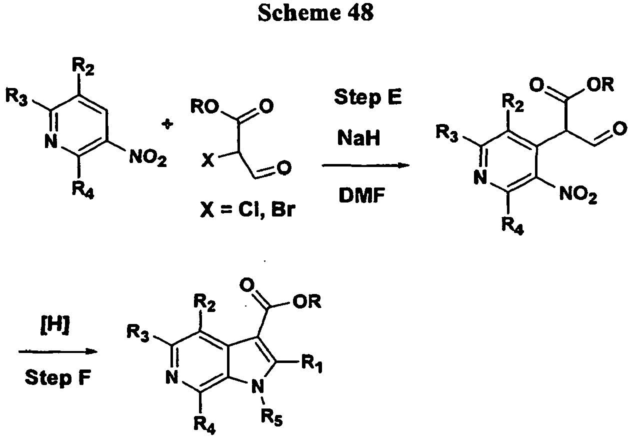

Scheme 48 depicts another possible route to azaindole acetic acid derivatives. Step E of Scheme 48 could be carried out as shown or according to procedures such as those described in Yurovskaya, M.A.; Khamlova, I.G.; Nesterov, V.N.; Shishkin, O.V.; Struchkov, T.; Khim Geterotsikl Soedin 1995, 11, 1543-1550; Grzegozek, M.; Wozniak, M.; Baranski, A.; Van Der Plas, H.C.; J. Heterocycl. Chem. 1991, 28(4), 1075-1077 (use of NaOH, DMSO); Lawrence, N.J.; Liddle, J.; Jackson, D.A.; Tetrahedron Lett. 1995, 36(46), 8477-8480 (use of. NaH, DMSO); Haglund, O.; Nilsson, M.; Synthesis 1994, 3, 242-244; (use of 2.5 equiv. CuCl, 3.5 equiv. TBu-OK, DME, Py); Makosza, M.; Sienkiewicz, K.; Wojciechowski, K.; Synthesis 1990, 9, 850-852; (use of KO-tBu, DMF); Makosza, M.; Nizamov, S.; Org. Prep. Proceed Int. 1997, 29(6), 707-710; (use of tBu-OK, THF). Step F of Scheme 48 shows the cyclization reaction which could provide the azaindole acetic acid derivatives. This reaction could be accomplished according to procedures such as those described in Frydman, B.; Baldain, G.; Repetto, J. C.; J. Org. Chem. 1973, 38, 1824 (use of H₂, Pd-C, EtOH); Bistryakova, I. D.; Smimova, N. M.; Safonova, T. S.; Khim Geterotsikl Soedin 1993, 6, 800-803 (use of H₂, Pd-C (cat.), MeOH); Taga, M.; Ohtsuka, H.; Inoue, I.; Kawaguchi, T.; Nomura, S.; Yamada, K.; Date, T.; Hiramatsu, H.; Sato, Y.; Heterocycles 1996, 42(1), 251-263 (use of SnCl₂, HCl, Et₂O); Arcari, M.; Aveta, R.; Brandt, A.; Cecchetelli, L.; Corsi, G.B.; Dirella, M.; Gazz. Chim. Ital. 1991,121 (11), 499-504 [use of Na₂S₂O₆, THF/EtOH/H₂O (2:2:1)]; Moody, C. J.; Rahimtoola, K. F.; J. Chem. Soc., Perkin Trans 1 1990, 673 (use of TiCl₃, NH₄Oac, acetone, H₂O).

Scheme 49 provides another route to azaindole intermediates which could then be further elaborated to provide compounds of Formula I, such as the amido derivatives shown. Steps G".and H" of Scheme 49 may be carried out according to the procedures described in Takahashi, K.; Shibasaki, K.; Ogura, K.; Iida, H.; Chem. Lett. 1983, 859; and Itoh, N.; Chem. Pharm. Bull. 1962, 10, 55. Elaboration of the intermediate to the amido compound of Formula I could be accomplished as previously described for Steps I'- M' of Scheme 45.

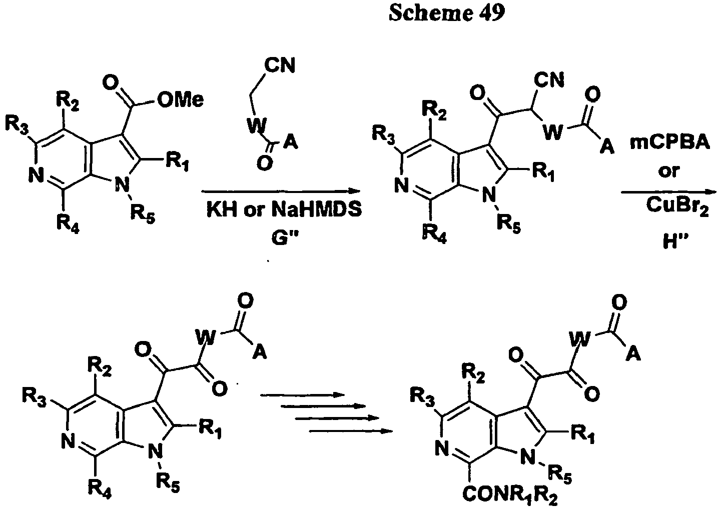

Scheme 50 shows the preparation of azaindole oxalic acid derivatives. The starting materials in Scheme 50 may be prepared according to Tetrahedron Lett. 1995, 36, 2389-2392. Steps A', B', C', and D' of Scheme 50 may be carried out according to procedures described in Jones, R.A.; Pastor, J.; Siro, J.; Voro, T.N.; Tetrahedron 1997, 53(2), 479-486; and Singh, S.K.; Dekhane, M.; Le Hyaric, M.; Potier, P.; Dodd, R.H.; Heterocycles 1997, 44(1), 379-391. Step E' of Scheme 50 could be carried out according to the procedures described in Suzuki, H.; Iwata, C.; Sakurai, K.; Tokumoto, K.; Takahashi, H.; Hanada, M.; Yokoyama, Y.; Murakami, Y.; Tetrahedron 1997, 53(5), 1593-1606; Suzuki, H.; Yokoyama, Y.; Miyagi, C.; Murakami, Y.; Chem. Pharm. Bull. 1991, 39(8), 2170-2172; Hagen, T.J.; Narayanan, K.; Names, J.; Cook, J.M.; J. Org. Chem. 1989, 54, 2170; Murakami, Y.; Yokoyama, Y.; Watanabe, T.; Aoki, C.; et al.; Heterocycles 1987, 26, 875; and Hagen, T. J.; Cook, J.M.; Tetrahedron Lett. 1988, 29(20), 2421. Step F' of Scheme 50 shows the conversion of the phenol to a fluoro, chloro or bromo derivative. Conversion of the phenol to the fluoro derivative could be carried out according to procedures described in Christe, K.O.; Pavlath, A.E.; J. Org. Chem. 1965, 30, 3170; Murakami, Y.; Aoyama, Y.; Nakanishi, S.; Chem. Lett. 1976, 857; Christe, K. O.; Pavlath, A. E.; J. Org. Chem. 1965, 30, 4104; and Christe, K.O.; Pavlath, A.E.; J. Org. Chem. 1966, 31, 559. Conversion of the phenol to the chloro derivative could be carried out according to procedures described in Wright, S.W.; Org. Prep. Proc. Int. 1997, 29(1), 128-131; Hartmann, H.; Schulze, M.; Guenther, R.; Dyes Pigm 1991, 16(2), 119-136; Bay, E.; Bak, D. A.; Timony, P. E.; Leone-Bay, A.; J. Org. Chem. 1990, 55, 3415; Hoffmann, H.; et al.; Chem. Ber. 1962, 95, 523; and Vanallan, J.A.; Reynolds, G.A.; J. Org. Chem. 1963, 28, 1022. Conversion of the phenol to the bromo derivative may be carried out according to procedures described in Katritzky, A.R.; Li, J.; Stevens, C.V.; Ager, D.J.; Org. Prep. Proc. Int. 1994, 26(4), 439-444; Judice, J.K.; Keipert, S.J.; Cram, D.J.; J. Chem. Soc., Chem. Commun. 1993, 17, 1323-1325; Schaeffer, J.P.; Higgins, J.; J. Org. Chem. 1967, 32, 1607; Wiley, G.A.; Hershkowitz, R.L.; Rein, R.M.; Chung, B.C.; J. Am. Chem. Soc. 1964, 86, 964; and Tayaka, H.; Akutagawa, S.; Noyori, R.; Org. Syn. 1988, 67, 20.

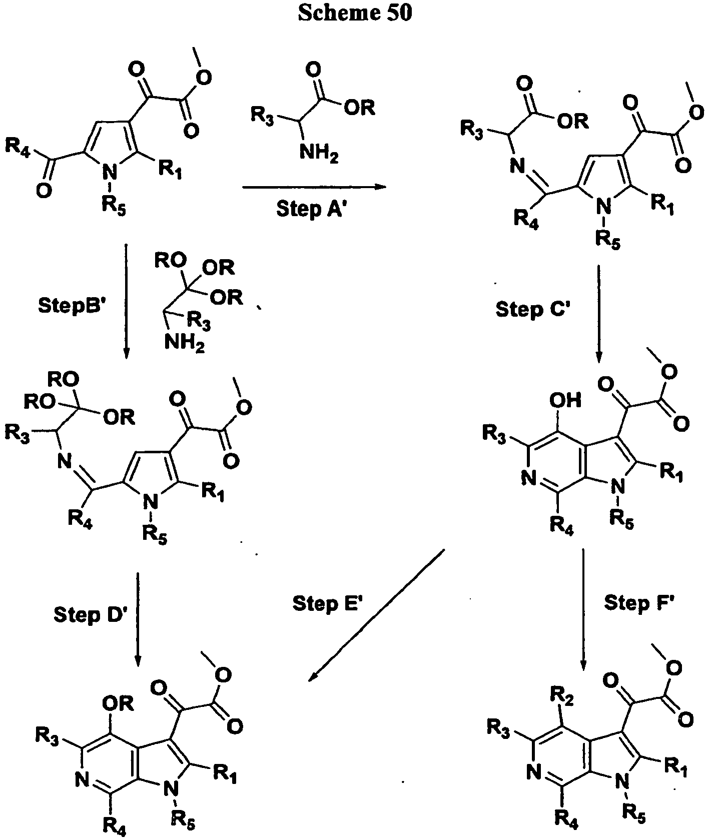

Scheme 51 describes methods for the preparation of azaindole acetic acid derivatives by the same methods employed for the preparation of azaindole oxalic acid derivatives as shown and described in Scheme 50 above. The starting material employed in Scheme 51 could be prepared according to J. Org. Chem. 1999, 64, 7788-7801. Steps A", B", C", D", and E" of Scheme 51 could be carried out in the same fashion as previously described for Steps Steps A', B', C', D', and E' of Scheme 50.

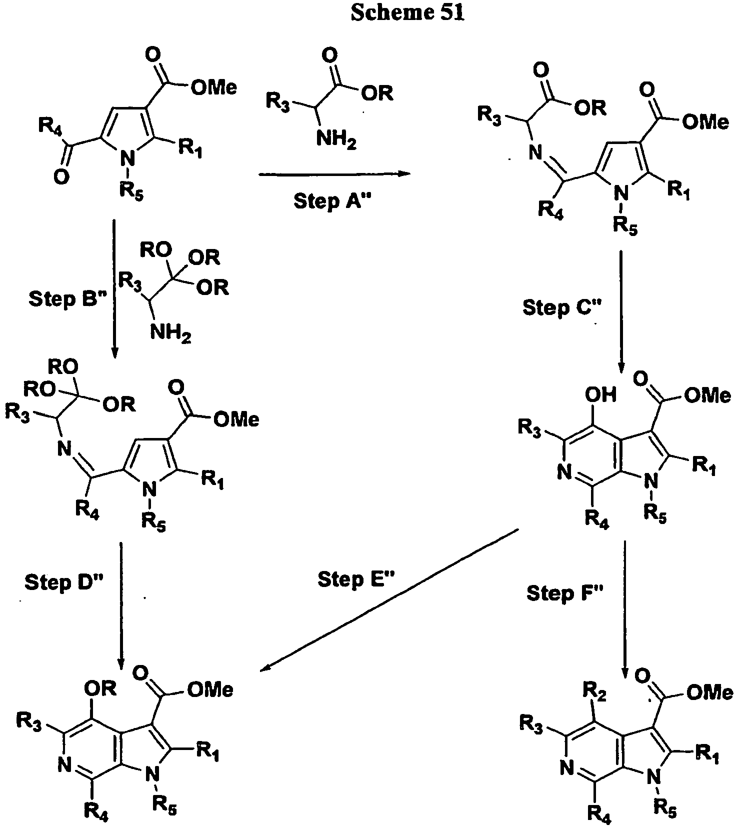

The remaining schemes provide additional background, examples, and conditions for carrying out this invention. Specific methods for preparing W and modifying A are presented. As shown in Scheme 52, the azaindoles may be treated with oxalyl chloride in either THF or ether to afford the desired glyoxyl chlorides according to literature procedures (Lingens, F.; Lange, J. Justus Liebigs Ann. Chem. 1970, 738, 46-53). The intermediate glyoxyl chlorides may be coupled with benzoyl piperazines (Desai, M.; Watthey, J.W. Org. Prep. Proc. Int. 1976, 8, 85-86) under basic conditions to afford compounds of Formula I directly.

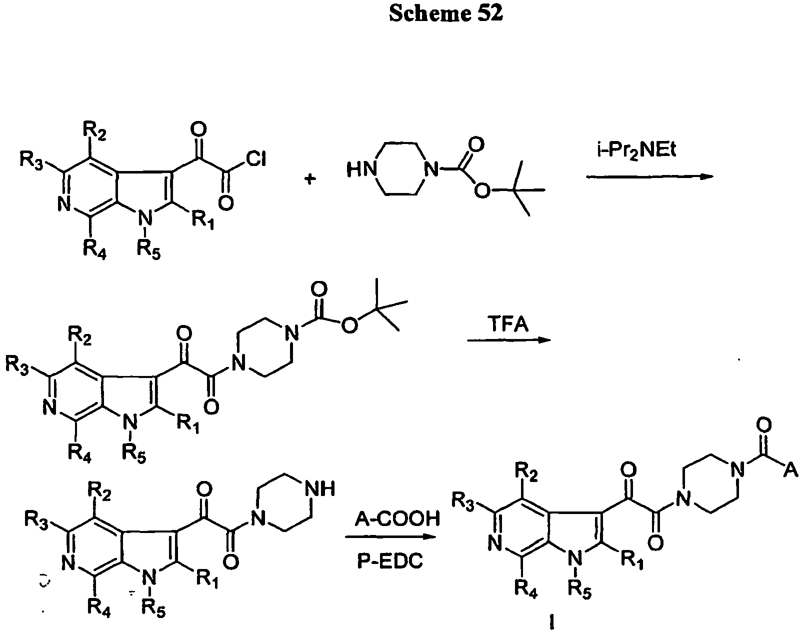

Alternatively, **Scheme 52** treatment of the azaindole-3-glyoxyl chloride, (Scheme 52) with tert-butyl 1-piperazinecarboxylate affords the piperazine coupled product. It is apparent to one skilled in the art that use of an alternative Boc protected piperazine which are synthesized as shown below would provide compounds of formula I with alternative groups of formula W. As discussed earlier, other amine protecting groups which do not require acidic deprotection conditions could be utilized if desired. Deprotection of the Boc group is effected with 20% TFA/CH₂Cl₂ to yield the free piperazine. This product is then coupled with carboxylic acid in the presence of polymer supported 1-(3-Dimethylaminopropyl)-3-ethylcarbodiimide (P-EDC) to afford products of Formula I. This sequence provides a general method for synthesizing compounds of varied A in formula I.

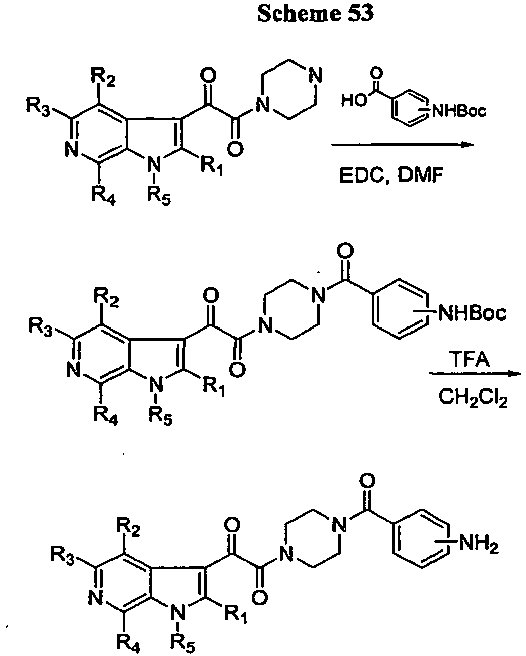

An example for preparing compounds of Formula I which possess substituents in A (or other parts of the molecule) which might interfere with the standard reaction schemes reactions is shown in Scheme 53. The piperazine derivative (Scheme 53) was treated with Boc-protected aminobenzoic acid in the presence of EDC to afford the piperazine diamide. A portion of the resulting product was separated and subjected to TFA in order to remove the Boc group, thus yielding amino derivatives.

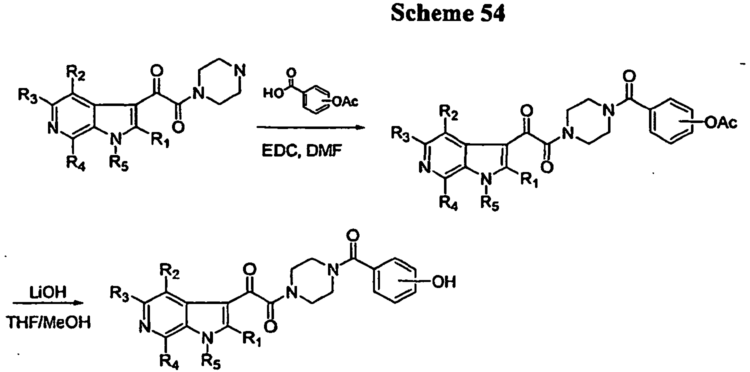

Similarly, substituents which possess a reactive alcohol can be incorporated as. below. The piperazine derivative (Scheme 54) was treated with acetoxybenzoic acid in the presence of EDC to afford the piperazine diamide derivative. A portion of the resulting product was separated and subjected to LiOH hydrolysis in order to remove the acetate group, thus yielding hydroxy derivatives.

Examples containing substituted piperazines are prepared using the general procedures outlined in Schemes 55-38. Substituted piperazines are either commercially available from Aldrich, Co. or prepared according to literature procedures (Behun et al, Ref. 88(a), Scheme 3.1, eq. 01). Hydrogenation of alkyl substituted pyrazines under 40 to 50 psi pressure in EtOH afforded substituted piperazines. When the substituent was an ester or amide, the pyrazine systems could be partially reduced to the tetrahydropyrazine (Rossen et al, Ref. 88(b), Scheme 55, eq. 02). The carbonyl substituted piperazines could be obtained under the same conditions described above by using commercially available dibenzyl piperazines (Scheme 55, eq. 03).

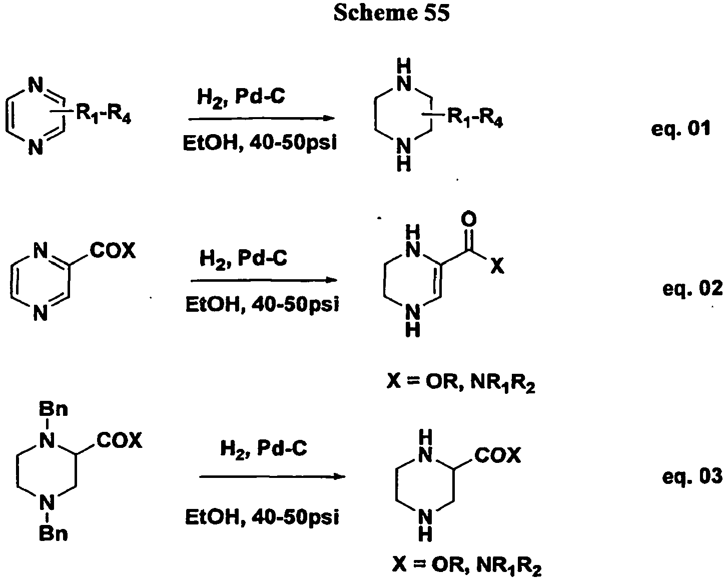

2-Trifluoromethylpiperazine (Jenneskens et al., Ref. 88c) was prepared through a four step route (Scheme 56). Using Lewis acid TiCl₄, N,N'-dibenzylethylenediamine reacted with trifluoropyruvates to afford the hemiacetal, which was reduced at room temperature by Et₃SiH in TFA to afford the lactam. LiAlH₄ treatment then reduced the lactam to 1,4-dibenzyl-2-trifluoromethylpiperazine. Finally, hydrogenation of the dibenzyl-2-trifluoromethylpiperazine in HOAc gave the desired product, 2-trifluoromethylpiperazine.

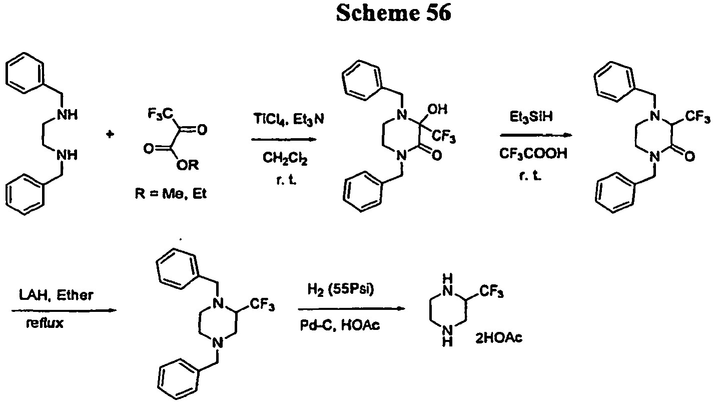

Mono-benzoylation of symmetric substituted piperazines could be achieved by using one of the following procedures (Scheme 57). (a) Treatment of a solution of piperazine in acetic acid with acetyl chloride afforded the desired mon-benzoylated piperazine (Desai et al. Ref. 27, Scheme 57, eq. 04). (b) Symmetric piperazines were treated with 2 equivalents of n-butyllithium, followed by the addition of benzoyl chloride at room temperature (Wang et al, Ref. 89, Scheme 57, eq. 05).

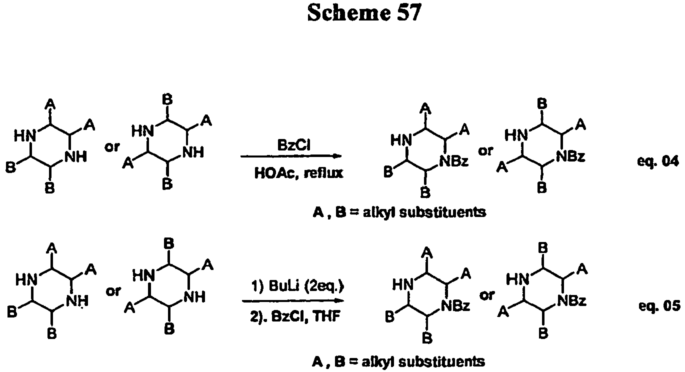

Mono-benzoylation of unsymmetric substituted piperazines could be achieved by using one of the following procedures (Scheme 57), in which all the methods were exemplified by mono-alkyl substituted piperazines. (a) Unsymmetric piperazines were treated with 2 equivalents of n-butyllithium, followed by the addition of benzoyl chloride at room temperature to afford a mixture of two regioisomers, which could be separated by chromatography (Wang et al, Ref. 89 and 90(b), Scheme 58 eq. 06); (b) Benzoic acid was converted to its pentafluorophenyl ester, and then further reaction with 2-alkylpiperazine to provide the mono-benzoylpiperazines with the benzoyl group at the less hindered nitrogen (Adamczyk et al, Ref. 90(a), Scheme 58, eq. 07); (c) A mixture of piperazine and methyl benzoate was treated with dialkylaluminum chloride in methylene chloride for 2-4 days to yield the mono-benzoylpiperazine with the benzoyl group at the less hindered nitrogen (Scheme 58 eq. 08); (d) Unsymmetric piperazines were treated with 2 equivalents of n-butyllithium, followed by subsequent addition of triethylsilyl chloride and benzoyl chloride in THF at room temperature to afford mono-benzoylpiperazines with the benzoyl group at the more hindered nitrogen (Wang et al, Ref. 90(b), Scheme 58, eq. 09). When the substituent at position 2 was a ester or amide, the mono-benzoylation with benzoyl chloride occurred at the less hindered nitrogen of the piperazine with triethylamine as base in THF (Scheme 58, eq. 10).

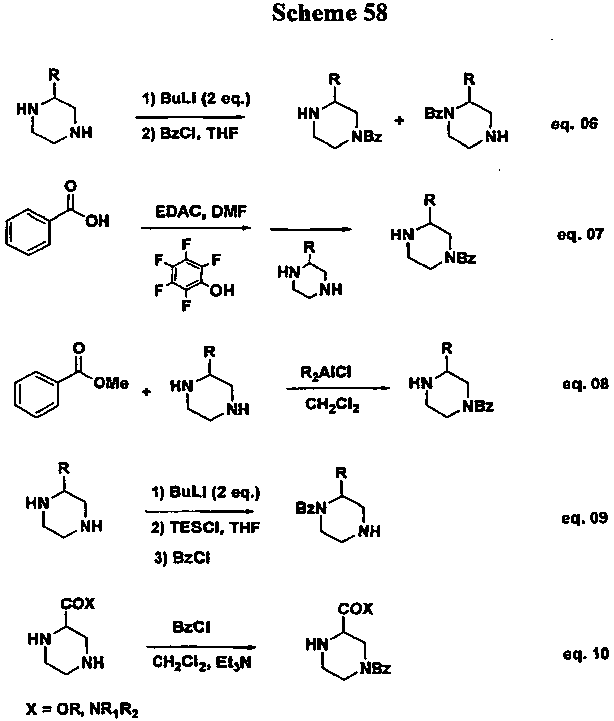

In the case of tetrahydropyrazines (Scheme 59, eq. 11), mono-benzoylation occurred at the more hindered nitrogen under the same conditions as those in equation 10 of Scheme 58, in the well precedented manner. (Rossen et al, Ref. 88(b)).

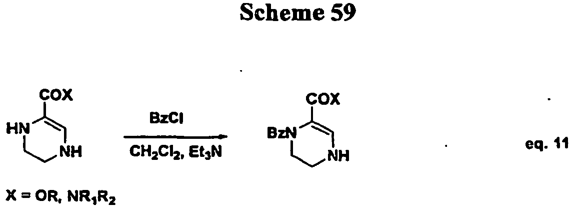

Furthermore, the ester group can be selectively reduced by NaBH₄ in the presence of the benzamide (Masuzawa et al, Ref. 91), which is shown in Scheme 60.

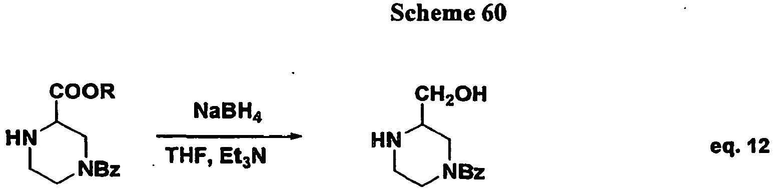

The ester groups on either the piperazine linkers or on the azaindole nucleus could be hydrolyzed to the corresponding acid under basic conditions such as K₂CO₃ (Scheme 61, eq. 13) or NaOMe (Scheme 61, eq. 14) as bases in MeOH and water.

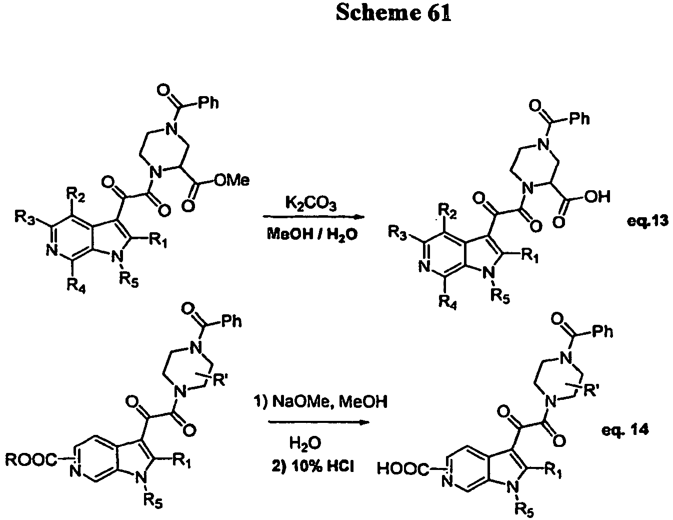

Reaction of an azaindole glyoxyl chloride with substituted benzoyl piperazines or tetrahydropyrazines in CH₂Cl₂ using I-Pr₂Net as base afforded the coupled products as shown in Scheme 62.

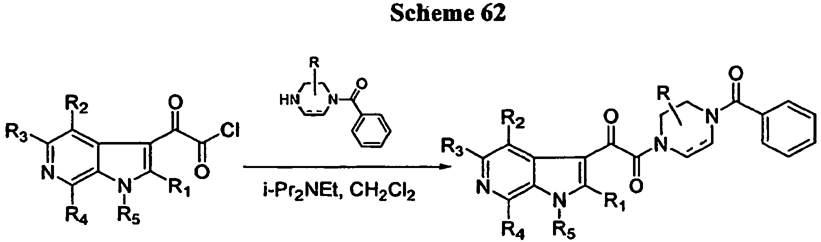

In the case of coupling reactions using 3-hydroxylmethyl-benzoylpiperazine, the hydroxyl group was temporarily protected as its TMS ether with BSTFA (N,O-bistrimethylsilyl)triffuoroacetamide) (Furber et al, Ref. 92). The unprotected nitrogen atom can then be reacted with glyoxyl chlorides to form the desired diamides. During workup, the TMS masking group was removed to give free hydroxylmethylpiperazine diamides as shown in Scheme 63.

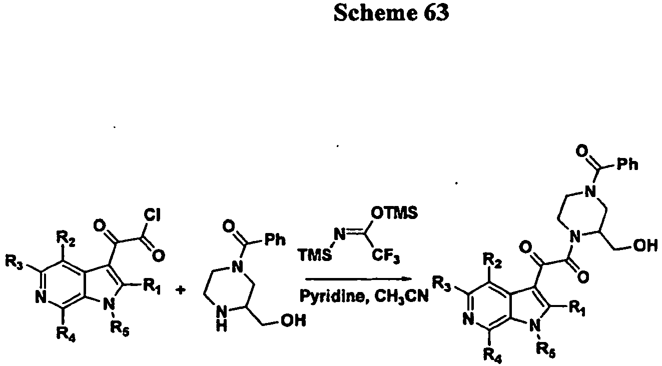

Piperazine intermediates were prepared using standard chemistry as shown in Scheme 64. 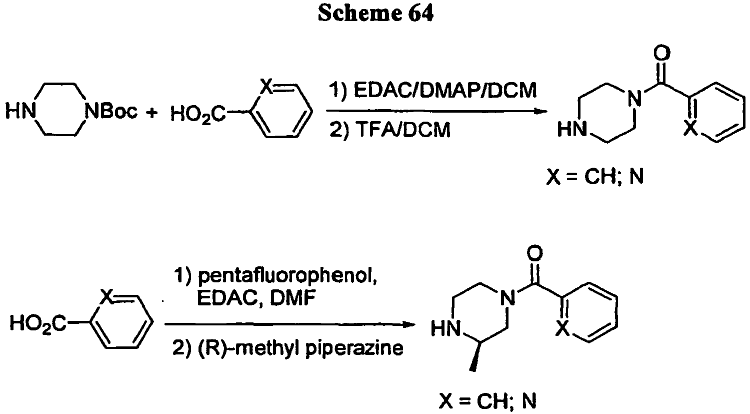 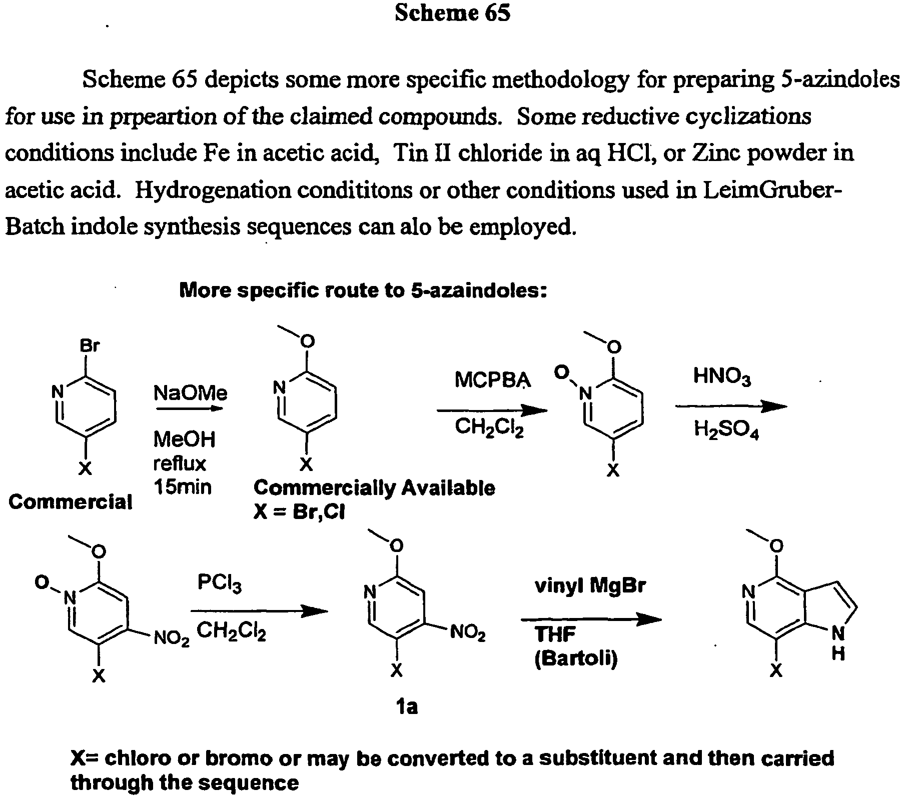 Tautomers of nitrogen containing heterocycles are covered by this patent application. For example, a hydroxy pyrazine is also known to represent its corresponding tautomer as well as shown in Scheme 66. 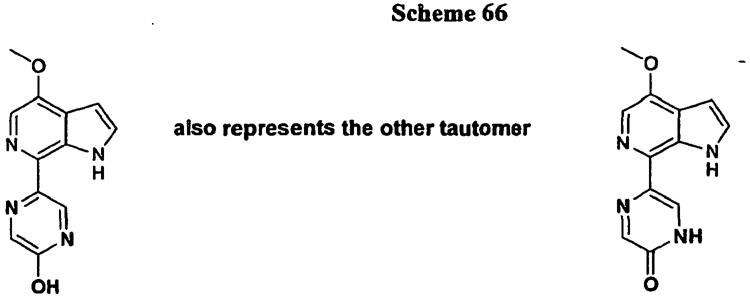 Scheme 67-74 provides some nonlimiting methodology for the preparation of substituted pyrazines which can be incorporated into substituents of compounds of claim 1, particularly as part of R⁴. It should be noted that the nomenclature in these schemes does not coincide with that of the claims but rather shows examples of methods which can be used to prepare pieces which make up the compounds of the claims. Thus R₁ and R₂ in these schemes does not refer to the R1 and R2 in the claims but for example refers to chemically compatible groups which might be envisioned by chemists skilled in the art and which can be utilized to prepare compounds of the claims.

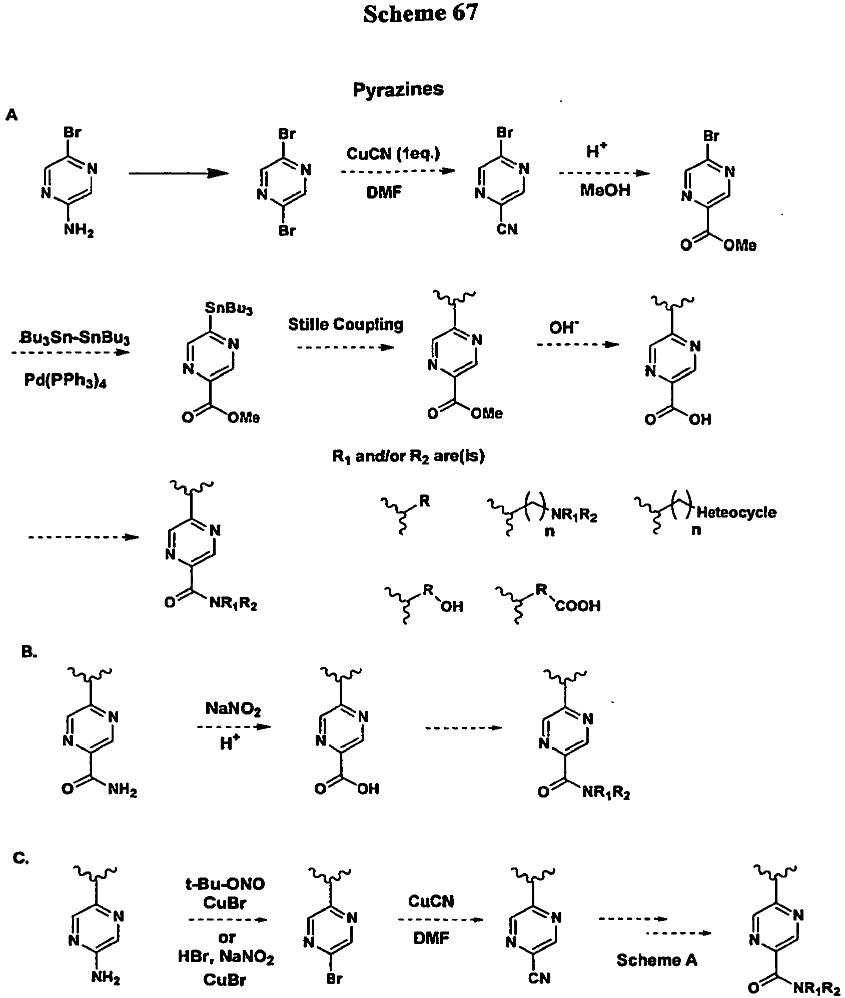

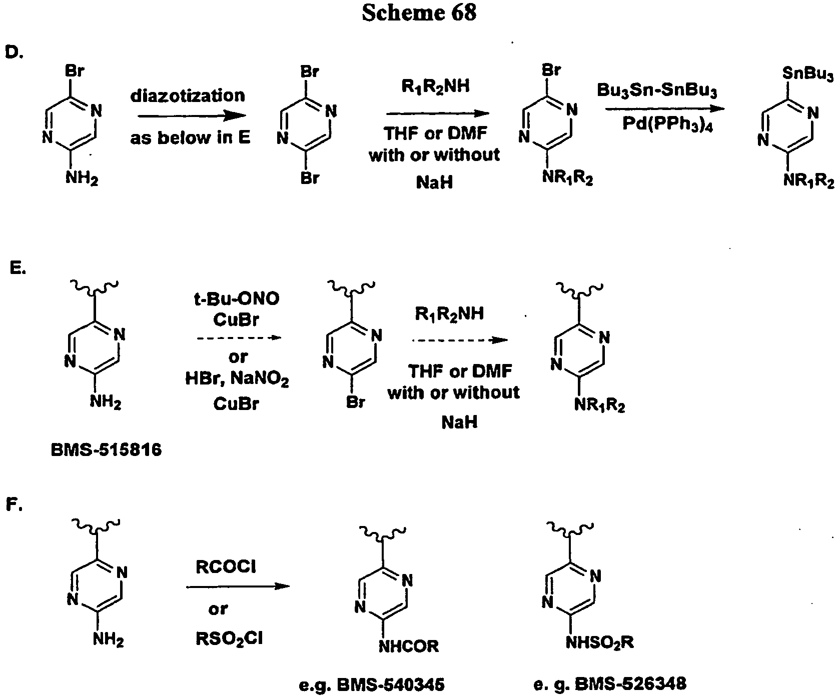

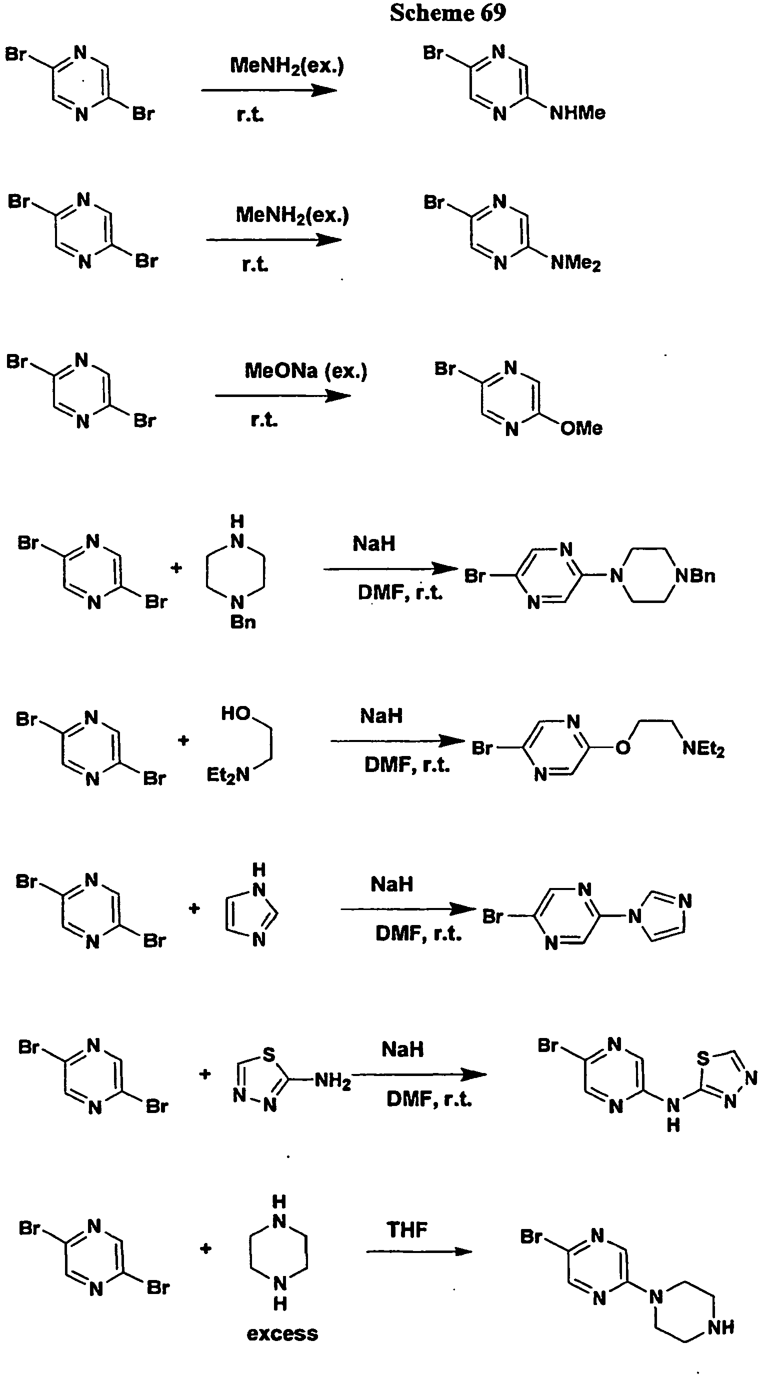

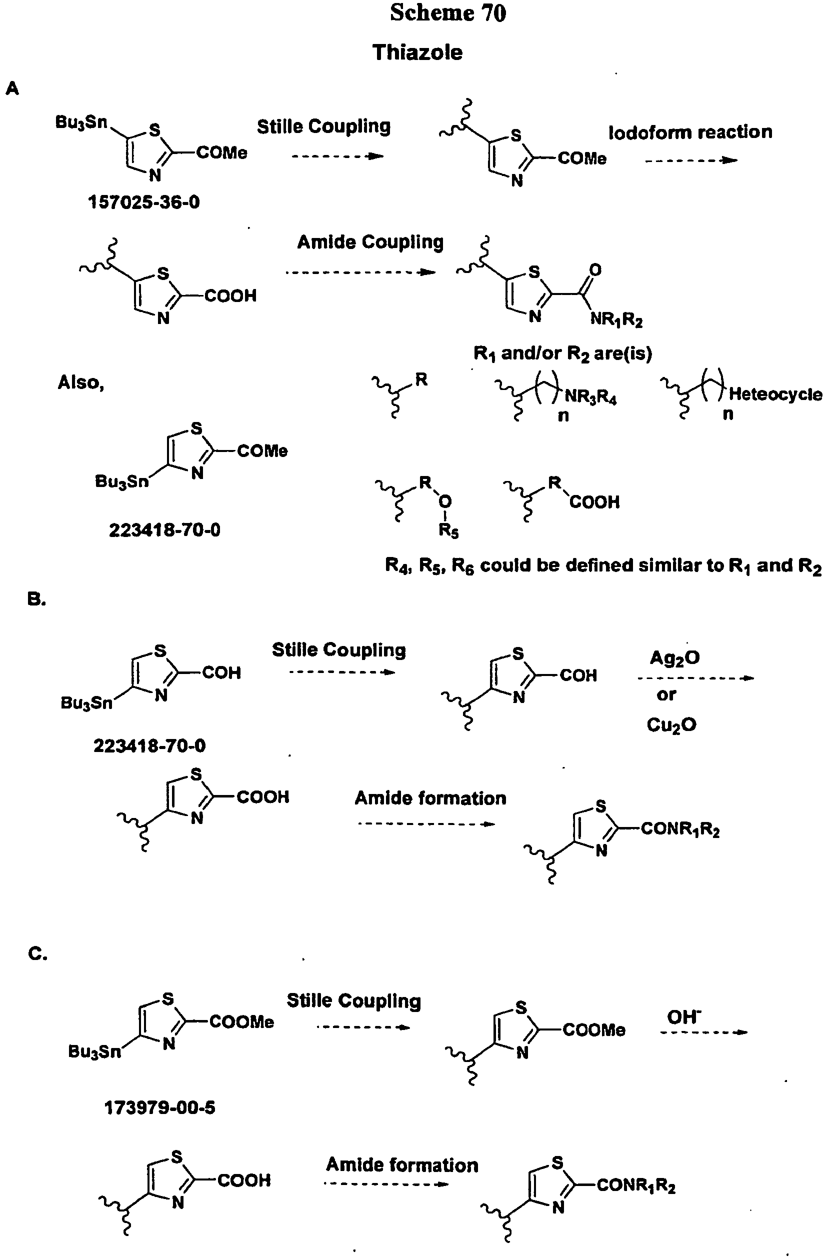

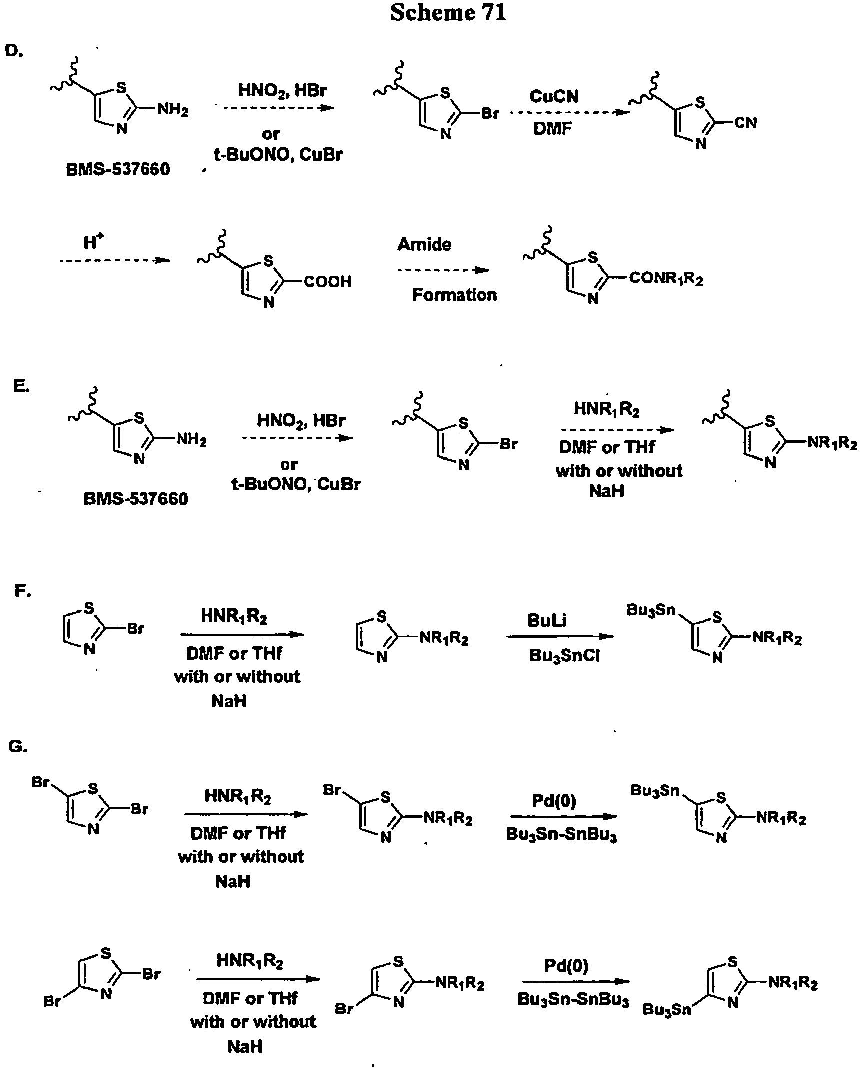

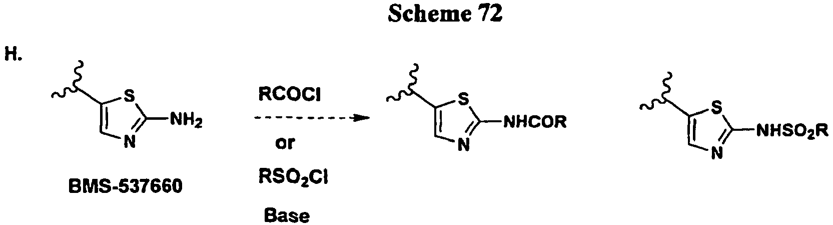

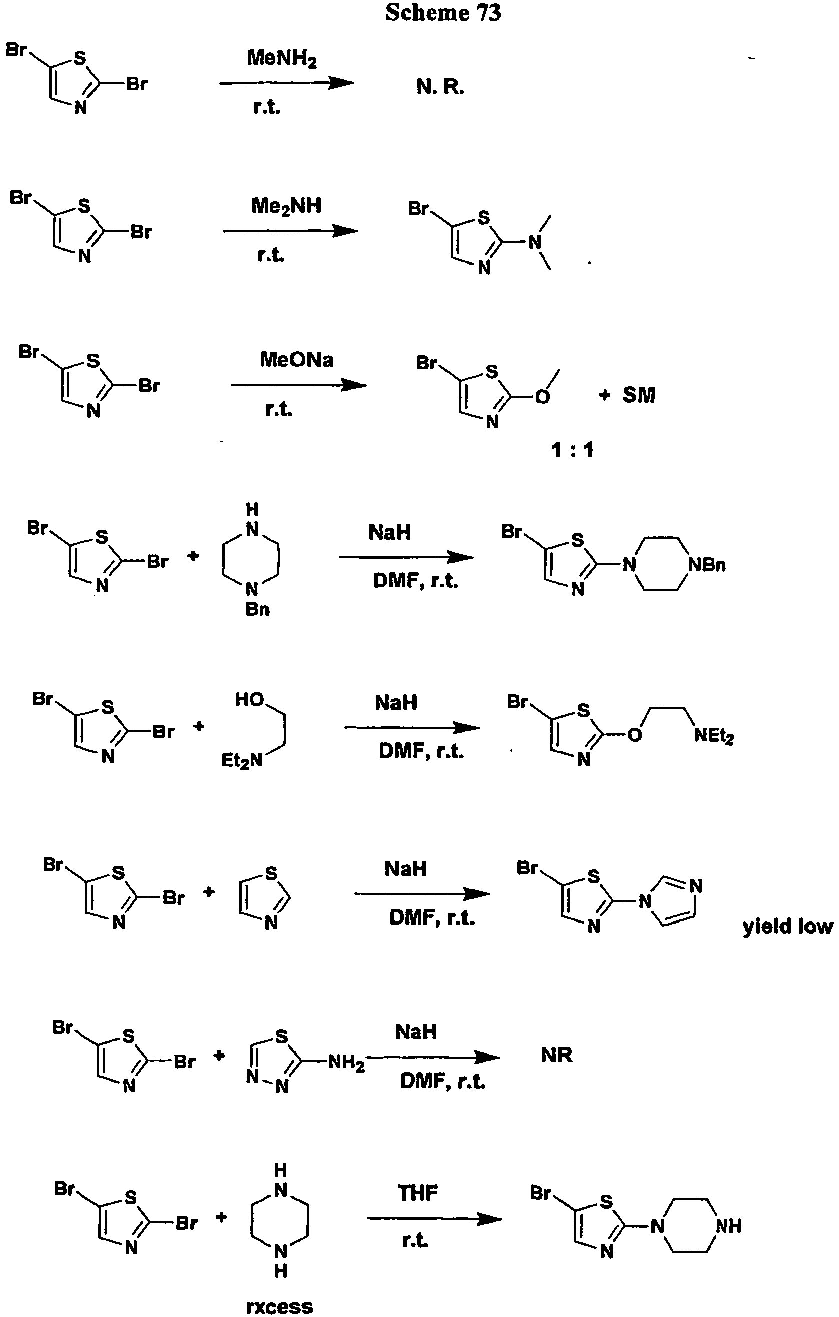

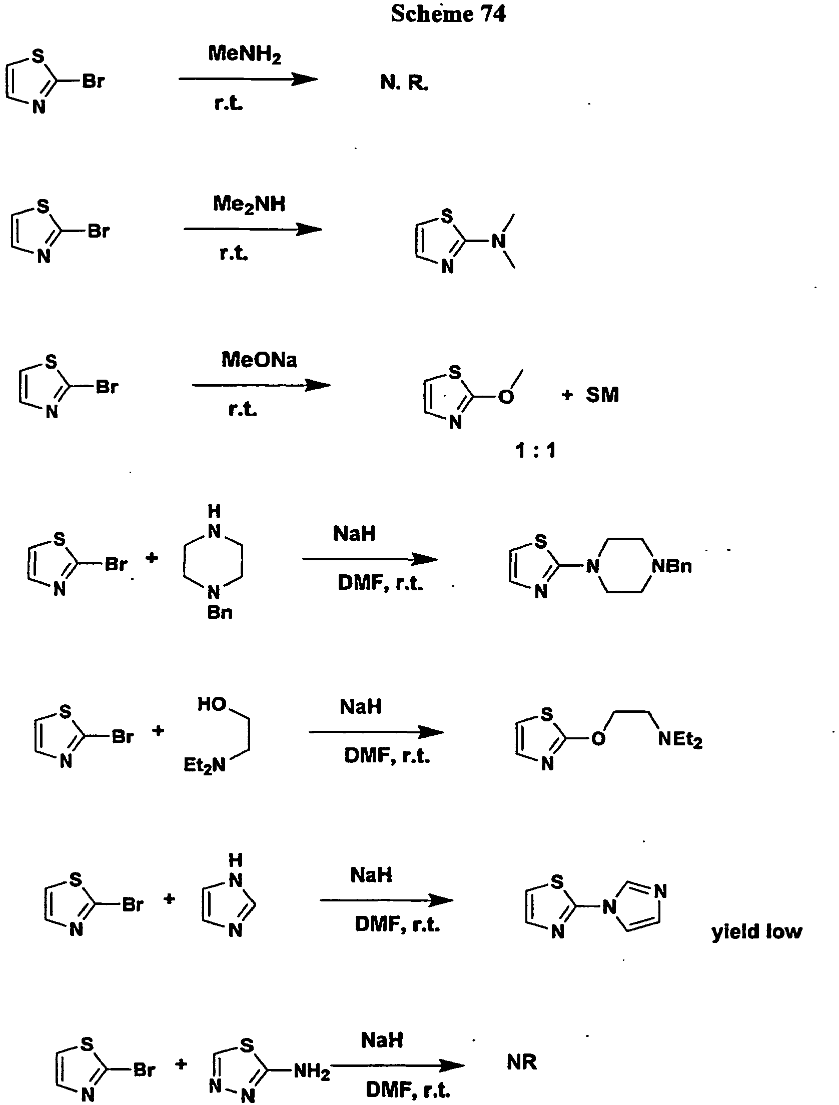

Throughout the chemistry discussion, chemical transformations which are well known in the art have been discussed. The average practioner in the art knows these transformations well and a comprehensive list of useful conditions for nearly all the transformations is available to organic chemists and this list is contained in reference 52 authored by Larock and is incorporated in its entirety for the synthesis of compounds of Formula I.

### Chemistry

### General:

Additional preparations of starting materials and intermediates are contained in Wang et. al. PCT WO 01/62255 which is incorporated by reference.

### Chemistry

All Liquid Chromatography (LC) data were recorded on a Shimadzu LC-10AS liquid chromatograph using a SPD-10AV UV-Vis detector with Mass Spectrometry (MS) data determined using a Micromass Platform for LC in electrospray mode.

### LC/MS Method (i.e., compound identification)

| | |
|---|---|
| Column A: | YMC ODS-A S7 3.0x50 mm column |
| Column B: | PHX-LLTNA C18 4.6x30 mm column |
| Column C: | TERRA ms C18 4.6x30 mm column |
| Column D: | YMC ODS-A C18 4.6x30 mm column |
| Column E: | YMC ODS-A C18 4.6x33 mm column |
| Column F: | YMC C18 S5 4.6x50 mm column |
| Column G: | XTERRA C18 S7 3.0x50 mm column |
| Gradient: | 100% Solvent A / 0% Solvent B to 0% Solvent A / 100% Solvent B |

Solvent A = 10% MeOH - 90% H₂O - 0.1 % TFA, Solvent B = 90% MeOH - 10% H₂O-0.1% TFA; and Rₜ in min.
Gradient time: 2 minutes

| | |
|---|---|
| Hold time | 1 minute |
| Flow rate: | 5 mL/min |
| Detector Wavelength: | 220 nm |
| Solvent A: | 10% MeOH / 90% H₂O / 0.1 % Trifluoroacetic Acid |
| Solvent B: | 10% H₂O / 90% MeOH / 0.1% Trifluoroacetic Acid |

Compounds purified by preparative HPLC were diluted in MeOH (1.2 mL) and purified using the following methods on a Shimadzu LC-10A automated preparative HPLC system or on a Shimadzu LC-8A automated preparative HPLC system with detector (SPD-10AV UV-VIS) wavelength and solvent systems (A and B) the same as above.

### Preparative HPLC Method (i.e., compound purification)

Purification Method: Initial gradient (40% B, 60% A) ramp to final gradient (100% B, 0% A) over 20 minutes, hold for 3 minutes (100% B, 0% A)

| | |
|---|---|
| Solvent A: | 10% MeOH / 90% H₂O / 0.1 % Trifluoroacetic Acid |
| Solvent B: | 10% H₂O / 90% MeOH / 0.1% Trifluoroacetic Acid |
| Column: | YMC C18 S5 20x100 mm column |
| Detector Wavelength: | 220 nm |

### Typical Procedures and Characterization of Selected Examples:

### Preparation of Intermediates:

### Intermediate 1

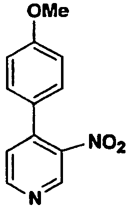

4-Methoxyphenylboronic acid (24.54 g), 4-chloro-3-nitropyridine hydrochloride (26.24 g), Pd(Ph₃P)₄ (4 g) and K₂CO₃ (111g) were combined in DME (500 mL). The reaction was heated to reflux for 10 hours. After the mixture cooled down to room temperature, it was poured into saturated aqueous NH₄OAc (500 mL )solution. The aqueous phase was extracted with EtOAc (3 x 200 mL). The combined extract was concentrated to give a residue which was purified using silica gel chromatography (10% to 30% EtOAc / PE) to afford 10.6 g of Intermediate 1, 3-Nitro-4-(4-methoxyphenyl)pyridine. MS *m*/z: (M+H)⁺ calcd for C₁₂H₁₁N₂O₃: 231.08; found 231.02. HPLC retention time: 1.07 minutes (column B).

### Intermediate 1a

### Alternate route to 5-azaindoles:

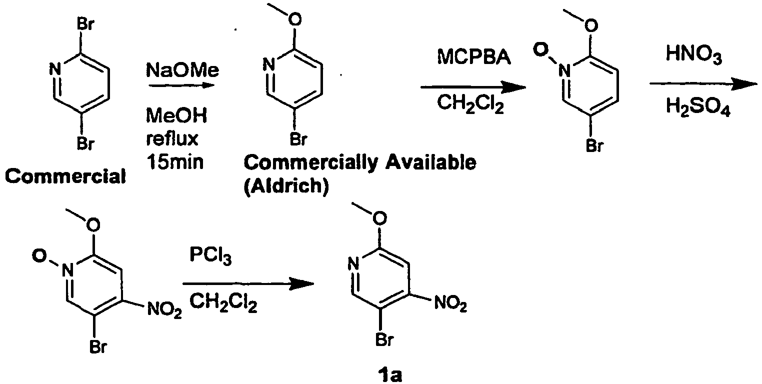

2-methoxy-5-bromo pyridine can be purchased from Aldrich (or others) or prepared. Oxidation with 1.1 eq of MCPBA in dichloromethane (20ml per 10.6 mmol bromide) in the presence of anhydrous MgSO4 (0.4g per mL dichloromethane) with stirring from 0° to ambient temperature for approximately 14 h provided the N-oxide after workup and flash chromatographic purification over silica gel using a 5% Etoac/Hexane gradient of increasing EtOAc. The N-oxide (1.6g) was dissolved in 10mL 98% sulfuric acid and cooled to 0°. 10 mL of 69% nitric acid was added and then allowed to warm to ambient temp with stirring. The reaction was then heated and stirred at 80° C for 14h and then poured over ice, extracted with dichloromethane, washed with water, and concentrated to give a yellow solid which was purified by flash chromatography over Silica gel using 1: EtOAc/hexane and then a gradient to provide a yellow crystalline solid: ). ¹H NMR (CDCl3) δ 8.50 (s,1H), 7.59 (s,1H), 4.12 (3H, s). LC MS showed desired M+H. TheN-oxide was reduced by dissolving the startingmaterial in dichloromethane (0.147M substrate) and cooling to 0°. A solution of 1.2 eq PCl₃ (0.44M) in dicloromethane was added slowly to keep the reaction at 0°. Warm to ambient temp and stir for 72h. Aqueous workup and concentration provided a yellow solid which could be used in subsequent reactions or purified by chromatography. Note: a similar sequence could be used with 2-methoxy-5-chloro-pyridine as starting material.

### Intermediate 2a

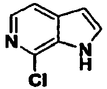

*Typical procedure for preparing azaindole from nitropyridine:* Preparation of 7-chloro-6-azaindole, Intermediate 2a, is an example of Step A of Scheme 1. 2-chloro-3-nitropyridine (5.0g, 31.5mmol)) was dissolved in dry THF (200 mL). After the solution was cooled to -78 °C, vinyl magnesium bromide (1.0M in THF, 100 mL) was added dropwise. The reaction temperature was maintained at -78°C for 1 h, and then at -20°C for another 12 h before it was quenched by addition of 20% NH₄Cl aqueous solution (150 mL). The aqueous phase was extracted with EtOAc (3 x 150 mL). The combined organic layer was dried over MgSO₄ , filtered and the filtrate was concentrated *in vacuo* to give a residue which was purified by silica gel column chromatography (EtOAc / Hexane, 1 / 10) to afford 1.5g (31%) of 7-chloro-6-azaindole, Intermediate 2a. ¹H NMR (500 MHz, CD₃OD) δ 7.84 (d,1H, *J* = 10.7 Hz), 7.55 (dd, 1H, *J* = 10.9, 5.45 Hz), 6.62 (d, 1H, *J* = 5.54 Hz), 4.89 (s, 1H). MS *m*/z: (M+H)⁺ calcd for C₇H₆ClN₂: 153.02; found 152.93. HPLC retention time: 0.43 minutes (column A).

### Intermediate 2b

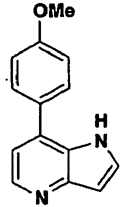

Intermediate 2b, 7-(4-Methoxyphenyl)-4-azaindole, was prepared by the same method as Intermediate 2a starting from 3-Nitro-4-(4-methoxyphenyl)pyridine, Intermediate 1. MS m/z: (M+H)⁺ calcd for C₁₄H₁₃N₂O: 225.10; found 225.02. HPLC retention time: 1.39 minutes (column B).

### Intermediate 2c

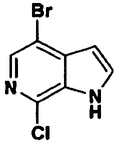

Intermediate 2c, 4-bromo-7-chloro-6-azaindole, was prepared by the same method as Intermediate 2a, starting from 2-Chloro-3-nitro-5-bromo-pyridine (available from Aldrich, Co.). MS m/z: (M+H)⁺ calcd for C₇H₅BrCIN₂: 230.93; found 231.15. HPLC retention time: 1.62 minutes (column B).

### Intermediate 2d

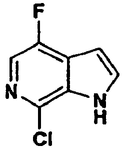

Intermediate 2d, 4-fluoro-7-chloro-6-azaindole (above), was prepared according to the following scheme:

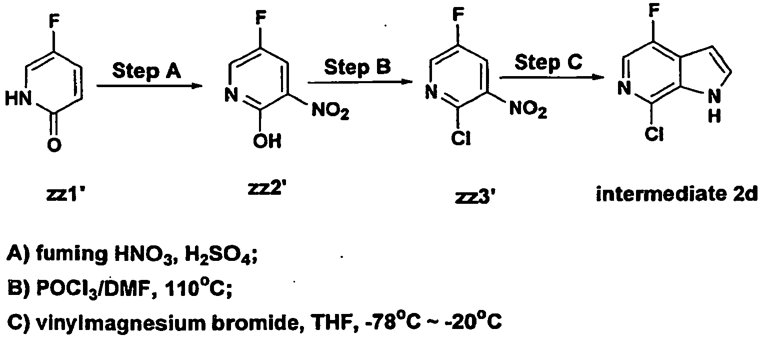

It should be noted that 2-chloro-5-fluoro-3-nitro pyridine, zz3', may be prepared by the method in example 5B of the reference Marfat, A. ; and Robinson, R. P. ; "Azaoxindole Derivatives" US. Patent 5,811,432 1998. The preparation below provides some details which enhance the yields of this route.

In Step A, compound zz1' (1.2 g, 0.01 mol) was dissolved in sulfuric acid (2.7 mL) at room temperature. Premixed fuming nitric acid (1 mL) and sulfuric acid was added dropwise at 5-10 °C to the solution of compound zz1'. The reaction mixture was then heated at 85 °C for 1 hour, then was cooled to room temperature and poured into ice (20 g). The yellow solid precipitate was collected by filtration, washed with water and air dried to provide 1.01 g of compound zz2'.

In Step B, compound zz2' (500 mg, 3.16 mmol) was dissolved in phosphorous oxychloride (1.7 mL, 18.9 mmol) and dimethoxyethane at room temperature. The reaction was heated to 110 °C for 5 hours. The excess phosphorous oxychloride was then removed by concentrating the reaction mixture in vacuo. The residue was chromatographed on silica gel, eluted with chloroform (100%) to afford 176 mg of product zz3'.

In Step C, compound zz3' (140 mg, 0.79 mmol) was dissolved in THF (5 mL) and cooled to -78 °C under a nitrogen atmosphere. To this solution was added dropwise a solution of vinyl magnesium bromide (1.2 mmol, 1.0 M in diethyl ether, 1.2 mL). The reaction mixture was then kept at -20 °C for 15 hours. The reaction mixture was then quenched with saturated ammonium chloride, and extracted with ethyl acetate. The combined organic layers were washed with brine, dried over magnesium sulfate, filtered, and the filtrate was concentrated in vacuo. The residue was chromatographed on silica to provide 130 mg of intermediate 2i. ¹H NMR (500 MHz, CD₃OD) δ 7.78 (s, 1H), 7.60 (d, 1H, *J =* 3.0 Hz), 6.71 (d, 1H, *J =* 3.05 Hz). MS *m*/*z*: (M+H)⁺ calcd for C₇H₅CIFN₂: 171.10; found 171.00. HPLC retention time: 1.22 minutes (column A).

Intermediate 2d, 4-fluoro-7-chloro-6-azaindole, was prepared by the same method as Intermediate 2a, starting from 2-Chloro-3-nitro-5-fluoro-pyridine which was prepared according to the procedure above. Experimental details for this preparation are contained in Wang et. al. PCT WO 01/62255. ¹H NMR (500 MHz, CD₃OD) δ 7.78 (s, 1H), 7.60 (d, 1H, *J* = 3.0 Hz), 6.71 (d, 1H, *J* = 3.05 Hz). MS *m*/*z*: (M+H)⁺ calcd for C₇HCIFN₂: 171.10; found 171.00. HPLC retention time: 1.22 minutes (column A).

### Intermediate 2e

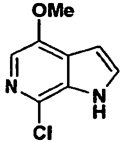

Intermediate 2e was prepared by either Method A or Method B, below:

Method A: A mixture of 4-bromo-7-chloro-6-azaindole (1 g), CuI (0.65 g) and NaOMe (4 mL, 25% in methanol) in MeOH (16 mL) was heated at 110 - 120 °C for 16 hours in a sealed tube. After cooling to room temperature, the reaction mixture was neutralized with 1N HCl to pH 7. The aqueous solution was extracted with EtOAc (3 x 30 mL). Then the combined organic layer was dried over MgSO₄, filtered and the filtrate was concentrated *in vacuo* to afford a residue, which was purified by using silica gel chromotography to give 0.3 g of 4-methoxy-7-chloro-6-azaindole, Intermediate 2e. MS *m*/*z*: (M+H)⁺ calcd for C₈H₈ClN₂O: 183.03; found 183.09. HPLC retention time: 1.02 minutes (column B).

Method B :A mixture of 4-bromo-7-chloro-6-azaindole (6 g), CuBr (3.7 g) and NaOMe (30 mL, 5% in MeOH) was heated at 110°C for 24 hours in a sealed tube. After cooling to room temperature, the reaction mixture was added to saturated aqueous NH₄Cl. The resulting aqueous solution was extracted with EtOAc (3 x 30 mL). The combined organic layer was dried over MgSO₄, filtered and the filtrate was concentrated *in vacuo* to afford a residue, which was purified by using silica gel chromotography to give 1.8 g of 4-methoxy-7-chloro-6-azaindole, Intermediate 2e.

### Intermediate 2f

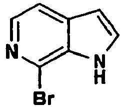

Intermediate 2f, 7-bromo-6-azaindole was prepared by the same method as Intermediate 2a, starting from 2-Bromo-3-nitro-pyridine (available from Aldrich, Co.). MS m/z: (M+H)⁺ calcd for C₇H₆BrN₂: 197.97; found 197.01. HPLC retention time: 0.50 minutes (column A).

### Intermediate 2g

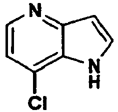

Intermediate 2g, 7-chloro-4-azaindole was prepared by the same method as Intermediate 2a, starting from 4-Chloro-3-nitro-pyridine (HCl salt, available from Austin Chemical Company, Inc.). MS *m*/*z*: (M+H)⁺ calcd for C₇H₆ClN₂: 153.02; found 152.90. HPLC retention time: 0.45 minutes (column A).

### Intermediate 2h

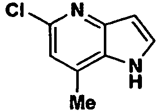

Intermediate 2h, 5-chloro-7-methyl-4-azaindole was prepared by the same method as Intermediate 2a, starting from 2-Chloro-4-methyl-5-nitro-pyridine (available from Aldrich, Co.). MS *m*/*z*: (M+H)⁺ calcd for C₈H₈ClN₂: 167.04; found 166.99. HPLC retention time: 1.22 minutes (column B).

### Example 2i

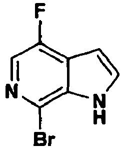

Intermediate 2j, 4-fluoro-7-bromo-6-azaindole, was prepared by the same method as Intermediate 2e, using POBr₃ in the step B instead of POCl₃. MS m/z: (M+H)⁺ calcd for C₇H₅BrFN₂: 214.96; found 214.97. HPLC retention time: 1.28 minutes (column G).

### Intermediate 2j

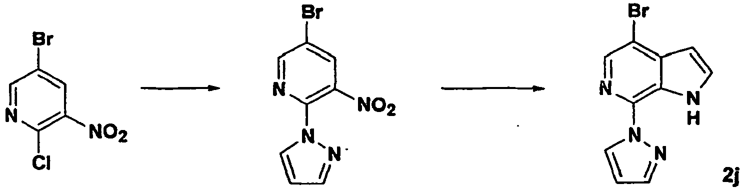

To a mixture of 5-bromo-2-chloro-3-nitropyridine (10 g, 42 mmol) in 1,4-dioxane (100 ml) was added pyrazole (5.8 g, 85 mmol). The resulting mixture was stirred at 120°C for 26.5 h., and then evaporated after cooling to r.t. The crude material was purified by flash chromatography (0 to 5% EtOAc/Hexanes) to give the desired product 5-Bromo-3-nitro-2-pyrazol-1-yl-pyridine. **¹H NMR**: (CD₃OD) δ 8.77 (s, 1H), 8.56 (s, 1H), 8.45 (s, 1H), 7.73 (s, 1H), 6.57 (s, 1H); **LC/MS**: (ES+) m/z (M+H)⁺= 269, 271, HPLC Rₜ = 1.223.

To a 250 mL round bottom flask was charged 5-Bromo-3-nitro-2-pyrazol-1-yl-pyridine (1.02 g, 3.8 mmol) and THF (30 ml). The mixture was then cooled to - 78°C, and added a THF solution of vinylmagnesium bromide (23 mL, 18.4 mmol, 0.8 *M*). After three minutes, the reaction mixture was warmed to -45°C and remained stirring for **1** h. The reaction was then quenched with ammonium chloride, and the resulting mixture extracted with EtOAc. The combined extracts were evaporated *in vacuo,* and the residue purified by flash column chromatography (5% EtOAc/Hexanes) to give compound **2** (which by HPLC contained about 50% of a side product, presumably 3-vinylamino of compound **1**) ; **¹H NMR:** (CDCl₃) δ 10.75 (b s, 1H), 8.73 (s, 1H), 8.10 (s, 1H), 7.82 (s, 1H), 7.52 (s, 1H), 6.67 (s, 1H), 6.53 (s, 1H); **LC/MS:** (ES+) m/z (M+H) = 262, 264; HPLC Rₜ = 1.670.

### Intermediate 2k

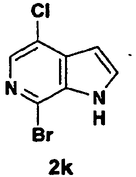

To a solution of 2-bromo-5-chloro-3-nitropyridine 5 (20 g, 84 mmol, prepared in 2 steps from 2-amino-5-chloropyridine as described in WO9622990) in THF (300 ml) at -78°C was charged a THF solution of vinylmagnesium bromide (280 ml, 252 mmol, 0.9 *M*). The resulting mixture was stirred at -78°C for one hour, followed by quenching with aqueous ammonium chloride (500 ml, *sat.)* and extracted with EtOAc (5 x 500 ml). The combined organic extracts were washed with aqueous ammonium chloride (2 x 500 ml, sat.) and water (3 x 500 ml), dried (MgSO₄) and evaporated to give a brownish residue. The crude material was triturated with CH₂Cl₂, and the solid formed filtered to give compound 6 as a yellow solid (8.0 g, 41%); **¹H NMR:** *(DMSO-d₆)* 12.30 (b s, 1H), 7.99 (s, 1H), 7.80 (d, *J* = 3.0, 1H), 6.71 (d, *J* = 3.0, 1H); **LC/MS**: (ES+) m/z (M+H)⁺ = 231, 233, 235; HPLC Rₜ = 1.833.

### Intermediate 2m

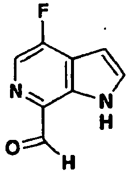

4-Fluoro-7-Bromo-6-azaindole (500 mg, 1.74 mmol) was dissolved in THF (5ml) and cooled to -78°C and *n*-BuLi (2.5 M, 2.1 ml) was added dropwise. The reaction mixture was stirred at -78°C for 15 min, then stirred at 0°C for 30 min. The reation was cooled to -78°C again, and DMF(0.7 ml, 8.7mmol) was added. After stirring for 30 min, water was added to quench the reaction. The reaction mixture was extracted with ethylacetate. The organic layer was dried over MgSO₄, filtered, concentrated and chromatographied to afford 208 mg of intermediate 2m. LC/MS: (ES⁺) m/z (M+H)⁺ = 164.98. Rt = 0.44 min.

### Intermediate 2n

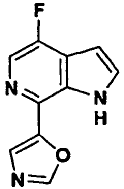

A mixture of intermediate 2m (50 mg, 0.30 mmol), potassium carbonate (42 mg, 0.30 mmol) and tosylmethyl isocyanide (60 mg,0.30 mmol) in MeOH(3ml) was heated to reflux for about 2 hr. The solvent was removed in vacuum and the residue was treated with ice water and extracted with ether. The organic layer was washed with an aqueous solution of HCl (2%), water and dried over magnesium sulfate. After filtration and evaporation of the solvent, the residue was purified on silica to afford the title compound (60mg).LC/MS: (ES⁺) m/z (M+H)⁺ = 204. Rt = 0.77 min.

### Intermediate 2o

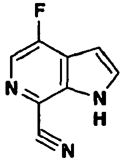

4-Fluoro-7-Bromo-6-azaindole (510 mg, 2.39 mmol) in anhydrous DMF (5 mL) was treated with copper cyanide (430 mg, 4.8 mmol) at 150°C in a seal tube for 1h. An aqueous solution of NH₄OH (10 mL) was added and the reaction was extracted with diethylether (2 x 50 mL) and ethylacetate (2 x 50 mL). The organic phases were combined and dried over sodium sulfate, filtered, concentrated in vacuum and chromatographied on silica gel (gradient elution AcOEt/Hexanes 0-30%) to afford the title compound as a brownish solid (255 mg, 66%) LC/MS: (ES⁺) m/z (M+H)⁺ = 162.

### Intermediate 2p

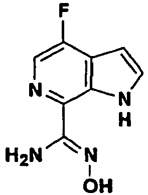

Intermediate 2o (82 mg, 0.51 mmol) was dissolved in absolute ethanol (200% proof, 5 mL) and treated with hydroxylamine hydrochloride (53 mg, 0.76 mmol) and triethylamine (140 µL, 1.0 mmol) and the reaction mixture was heated up at 80°C in a seal tube for 2h. The solvent was removed in vacuum and the pale yellow solid residue was washed with water to afford the title compound. LC/MS: (ES⁺) m/z (M+H)⁺ = 195. This compound was taken to the next step without further purification.

### Intermediate 2q

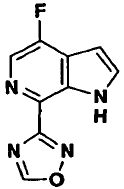

Intermediate 2p was dissolved in trimethylorthoformate (1 mL) and heated at 85°C in a seal tube for 1h, then it was cooled to rt, the solvent was removed in vacuum and the residue was chromatographied on silica gel (AcOEt/Hexanes, gradient elution 10-60%) to afford the title compound (54 mg, LC/MS: (ES⁺) m/z (M+H)⁺ =205).

### Intermediate 2r

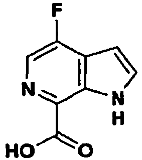

Intermediate 2q (100 mg, 0.62 mmol, crude) in ethanol (5 mL) was treated with an aqueous solution of sodium hydroxide (50%, 2 mL) and the reaction mixture was heated at 110°C overnight in a seal tube. The pH was adjusted to 2 with HCl (6N) and a brown precipitate was filtered off. The solution was concentrated to dryness to afford the title compound as a pale yellow solid LC/MS: (ES⁺) m/z (M+H)⁺ =181. This compound was used without further purification.

### Intermediate 2s

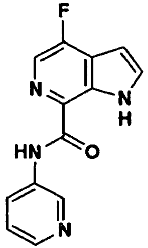

Intermediate 2r (0.62 mmol) was dissolved in DMF (1 mL) and treated with 3-aminopyridine (58.3 mg, 0.62 mmol), DEBT (185 mg, 0.62) and Hunig's base (216 µL, 1.26 mmol) and the reaction mixture was stirred at room temperature for 18h. Water was added and the reaction was extracted with AcOEt (2 x 25 mL) and CHCl₃ (2 x 25 mL), dried over sodium sulfate, concentrated and chromatographied on silica gel (AcOEt/Hexanes gradient elution 0-50%) to afford the title compound as a brownish solid LC/MS: (ES⁺) m/z (M+H)⁺ =257.

### Intermediate 2s

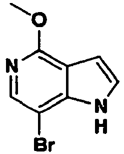

Intermediate 2h, 4-methoxy-7-bromo-5-azaindole was prepared by the same method as Intermediate 2a, starting from 2-methoxy-5-bromo-4-nitro-pyridine (intermediate 1a). ¹H NMR (CDCl3) δ 8.52 (s,1H), 7.84 (s,1H), 7.12 (t, 1H), 6.68 (d, 1H), 3.99 (s, 3H). LC MS showed desired M+H.

### Intermediate 2t

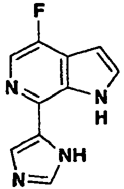

A mixture of aldehyde intermediate **2m** (150 mg, 0.91 mmol), sodium cyanide (44mg, 0.091mmol) and tosylmethyl isocyanide (177 mg, 0.91 mmol) in EtOH(3ml) was stirred at room temperature for 30min, then filtered and the crystals were washed with ether-hexane (1:1) and dried. The obtained crystals, and a saturated solution of ammonia in dry methanol (8ml)) were heated between 100-110°C for 16hr. The mixture was concentrated and chromatographed to provide 20mg of intermediate 2. LC/MS: (ES⁺) m/z(m+H)⁺ = 203. Rt = 0.64 min.

### Intermediate 3a

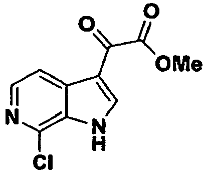

*Typical procedure for acylation of azaindole:* Preparation of Methyl (7-chloro-6-azaindol-3-yl)-oxoacetate, Intermediate 3a is an example of Step B of Scheme 1. 7-Chloro-6-azaindole, Intermediate 2a (0.5 g, 3.3 mmol) was added to a suspension of AlCl₃ (2.2 g, 16.3 mmol) in CH₂Cl₂ (100 mL). Stirring was continued at rt for 10 minutes before methyl chlorooxoacetate (2.0 g, 16.3 mmol) was added dropwise. The reaction was stirred for 8 h. The reaction was quenched with iced aqueous NH₄OAc solution (10%, 200 mL). The aqueous phase was extracted with CH₂Cl₂ (3 x 100mL). The combined organic layer was dried over MgSO₄, filtered and the filtrate was concentrated *in vacuo* to give a residue which was carried to the next step without further purification. Intermediate 2, Methyl (7-chloro-6-azaindol-3-yl)-oxoacetate: MS *m*/*z*: (M+H)⁺ calcd for C₁₀H₈ClN₂O₃: 239.02; found 238.97. HPLC retention time: 1.07 minutes (column A).

### Intermediate 3b

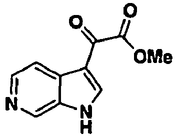

Intermediate 3b, Methyl (6-azaindol-3-yl)-oxoacetate, was prepared by the same method as Intermediate 3a, starting from 6-azaindole. MS m/z: (M+H)⁺ calcd for C₁₀H₉N₂O₃: 205.06; found 205.14. HPLC retention time: 0.49 minutes (column A).

### Intermediate 3c

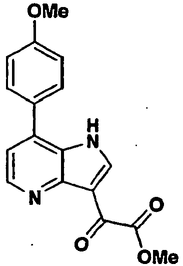

Intermediate 3c, Methyl (7-(4-methoxyphenyl)-4-azaindol-3-yl)-oxoacetate, was prepared by the same method as Intermediate 3a, starting from 7-(4-methoxyphenyl)-4-azaindole (Intermediate 2b). MS *m*/*z*: (M+H)⁺ calcd for C₁₇H₁₅N₂O₄: 311.10; found 311.04. HPLC retention time: 1.15 minutes (column A).

### Intermediate 3d

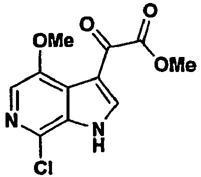

Intermediate 3d, methyl (7-chloro-4-methoxy-6-azaindol-3-yl)-oxoacetate was prepared by the same method as Intermediate 3a, starting from Intermediate 2e, 4-methoxy-7-chloro-6-azaindole. MS *m*/*z*: (M+H)⁺ calcd for C₁₂H₁₂ClN₂O₄: 283.05; found 283.22. **HPLC** retention time: 1.37 minutes (column B).

### Intermediate 3e

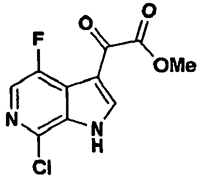

Intermediate 3e, Methyl (7-chloro-4-fluoro-6-azaindol-3-yl)-oxoacetate was prepared by the same method as Intermediate 3a starting from Intermediate 2d, 4-fluoro-7-chloro-6-azaindole.. ¹H NMR (500 MHz, CD₃OD) δ 8.63 (s, 1H), 8.00 (s, 1H), 3.95 (s, 3H). MS *m*/*z*: (M+H)⁺ calcd for C₁₀H₇ClFN₂O₃: 257.01; found 257.00. HPLC retention time: 1.26 minutes (column A).

### Intermediate 3f

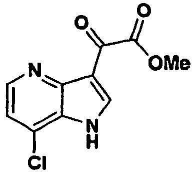

Intermediate 3f, Methyl (7-chloro-4-azaindol-3-yl)-oxoacetate was prepared by the same method as Intermediate 3a, starting from Intermediate 2g, 7-chloro-4-azaindole. MS *m*/*z*: (M+H)⁺ calcd for C₁₀HClN₂O₃: 239.02; found 238.97. HPLC retention time: 0.60 minutes (column A).

### Intermediate 3g

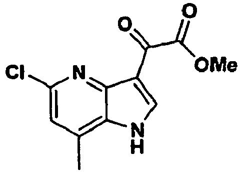

Intermediate 3g, Methyl (5-chloro-7-methyl-4-azaindol-3-yl)-oxoacetate was prepared by the same method as Intermediate 3a, starting from Intermediate 2h, 5-chloro-7-methyl-4-azaindole. MS *m*/*z*: (M+H)⁺ calcd for C₁₁H₁₀ClN₂O₃: 253.04; found 252.97. HPLC retention time: 1.48 minutes (column B).

### Intermediate 4a

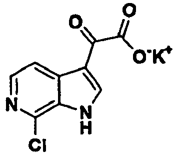

*Typical procedure of hydrolysis of ester:* Preparation of Potassium (7-chloro-6-azaindol-3-yl)-oxoacetate, Intermediate 4a, is an example of Step C of Scheme 1. Crude methyl (7-chloro-6-azaindol-3-yl)-oxoacetate, Intermediate 3a, and an excess of K₂CO₃ (2 g) were dissolved in MeOH (20 mL) and H₂O (20 mL). After 8 h, the solution was concentrated and the residue was purified by silica gel column chromatography to provide 200 mg of Potassium (7-chloro-6-azaindol-3-yl)-oxoacetate. MS *m*/*z*: (M+H)⁺ of the corresponding acid was observed. Calc'd for C₉H₆CIN₂O₃ : 225.01; found 225.05. HPLC retention time: 0.83 minutes (column A).

### Intermediate 4b

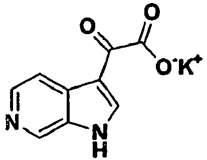

Potassium (6-azaindol-3-yl)oxoacetate, Intermediate 4b, was prepared by the same method as Intermediate 4a, starting from Methyl (6-azaindol-3-yl)oxoacetate, Intermediate 3b. MS *m*/*z:* (M+H)⁺ of the corresponding acid was observed. Calc'd for C₉H₇N₂O₃: 191.05; Found 190.99. HPLC retention time: 0.12 minutes (column A).

### Intermediate 4c

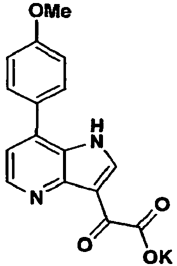

Intermediate 4c, Potassium (7-(4-methoxyphenyl)-4-azaindol-3-yl)-oxoacetate, was prepared by the same method as Intermediate 4a, starting from Methyl (7-(4-methoxyphenyl)-4-azaindol-3-yl)-oxoacetate, Intermediate 3c. MS *m*/*z*: (M-K+H)⁺ calcd for C₁₆H₁₃N₂O₄: 297.07; found 297.04. HPLC retention time: 1.00 minutes (column A).

### Intermediate 4d

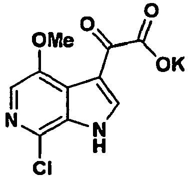

Intermediate 4d, Potassium (7-chloro-4-methoxy-6-azaindol-3-yl)-oxoacetate was prepared by the same method as Intermediate 4a starting from Methyl (7-chloro-4-methoxy-6-azaindol-3-yl)-oxoacetate, Intermediate 3d. MS *m*/*z*: (M+H)⁺ of the corresponding acid of compound 4d (M-K+H)⁺ calcd for C₁₀H₈ClN₂O₄: 255.02; found 255.07. HPLC retention time: 0.74 minutes (column A).

### Intermediate 4e

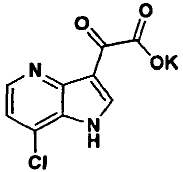

Intermediate 4e, Potassium (7-chloro-4-azaindol-3-yl)-oxoacetate was prepared by the same method as Intermediate 4a, starting from Methyl (7-chloro-4-azaindol-3-yl)-oxoacetate, Intermediate 3f . MS *m*/*z*: (M+H)⁺ of the corresponding acid of compound 4e (M-K+H)⁺ calcd for C₉H₆ClN₂O₃: 225.01; found 225.27. HPLC retention time: 0.33 minutes (column A).

### Intermediate 4f

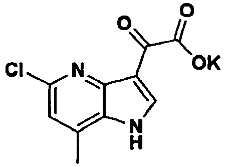

Intermediate 4f, Potassium (5-chloro-7-methyl-4-azaindol-3-yl)-oxoacetate was prepared by the same method as Intermediate 4a, starting from Methyl (5-chloro-7-methyl-4-azaindol-3-yl)-oxoacetate, Intermediate 3g. MS *m*/*z*: (M+H)⁺ of the corresponding acid of compound 4f (M-K+H)⁺ calcd for C₁₀H₈ClN₂O₃: 239.02; found 238.94. HPLC retention time: 1.24 minutes (column B).

### Intermediate 4g

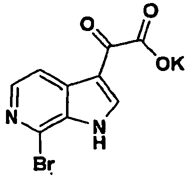

Intermediate 4g, Potassium (7-bromo-6-azaindol-3-yl)-oxoacetate was prepared by the same method as Intermediate 4a, starting from Methyl (7-bromo-6-azaindol-3-yl)-oxoacetate (prepared according to the method of Intermediate 3a from 7-Bromo-6-azaindole, Intermediate 2f). ¹H NMR (500 MHz, DMSO-d₆) δ 8.59 (s, 1H), 8.16 (d, 1H, J = 5.3 Hz), 8.08 (d, 1H, J = 5.45 Hz); ¹³C NMR (125 MHz, DMSO-d₆) □δ 180.5, 164.0, 141.6,140.4,132.4,125.3,115.5,113.0.

### Intermediate 4h

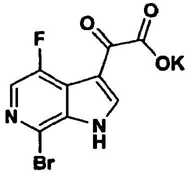

Intermediate 4h, Potassium (7-bromo-4-fluoro-6-azaindol-3-yl)-oxoacetate was prepared by the same method as Intermediate 4a, starting from Methyl (7-bromo-4-fluoro-6-azaindol-3-yl)-oxoacetate (prepared according to the method of Intermediate 3a from 7-Bromo-4-fluoro-6-azaindole, Intermediate 2i). MS *m*/*z*: (M+H)⁺ of the corresponding acid of compound 4g (M-K+H)⁺ calcd for C₉H₅BrFN₂O₃: 286.95; found 286.94. HPLC retention time: 0.94 minutes (column A).

### Intermediate 4I

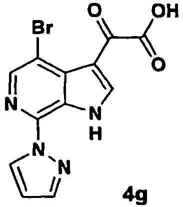

1-ethyl-3-methylimidazolium chloride (0.172 g, 1.1 mmol) was added to aluminum chloride (0.560 g, 4.2 mmol), and the mixture vigorously stirred. Upon formation of a liquid, intermediate 2j was added, followed by ethyl chlorooxoacetate (0.12 ml, 1.1 mmol). The mixture was allowed to stir at r.t. for 16 h, after which additional chlorooxoacetate was added (0.12 ml, 1.1 mmol). Following this addition, the reaction was allowed to stir at r.t. for another 24 h. The flask was cooled to 0°C and water added, upon which precipitates were formed. The solid material was filtered, washed with water and methanol, and dried under high vacuum to give compound 3; **LC/MS:** (ES+) m/z (M+H) = 334, 336; HPLC Rₜ =1.390.

### Intermediate 4j

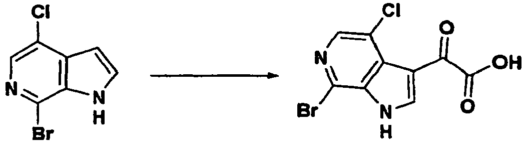

To 1-ethyl-3-methylimidazolium chloride (2.54 g, 17.3 mmol) was added aluminum chloride (6.91 g, 51.8 mmol). The mixture was stirred vigorously at ambient temperature for ten minutes. To the resulting yellow liquid was added intermediate 2k (2.0 g, 8.64 mmol) and ethyl chlorooxoacetate (2.0 ml, 17.3 mmol), and was stirred at ambient temperature for 16 h. The reaction mixture was then added ice/water (300 ml) to give precipitates, which were filtered and washed with water to give the title compound as a yellow solid (1.98 g). The aqueous solution was extracted with EtOAc (3 x 300 ml), and the extracts evaporated *in vacuo* to give a second batch of compound 8 as a yellow solid (439 mg, total yield 92%); **¹H NMR:** *(DMSO-d₆)* 14.25 (b s, 1H), 13.37 (s, 1H), 8.56 (s, 1H), 8.18 (s, 1H); **LC/MS**: (ES+) m/z (M+H)⁺ = 303, 305, 307; HPLC Rₜ = 1.360.

### Intermediate 4k

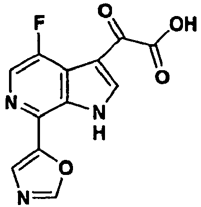

1-Ethyl-3-methylimidazolium chloride (82mg, 0.56 mmol) was added to a flask which contained intermediate 2n (56 mg, 0.28 mmol) and the mixture was cooled to 0°C. Aluminum chloride (336 mg, 2.52 mmol) was added in one portion followed by ClCOCOOEt (58 µL, 0.56 mmol) and the reaction mixture was stirred at room temperature for 2 days. Ice water was added to quench the reaction. The reaction mixture was filtered. The solid was washed with water and diethylether and dried in air to afford the title compound (58mg). LC/MS: (ES⁺) m/z (M+H)⁺ = 276. Rt = 0.85 min.

### Intermediate 4m

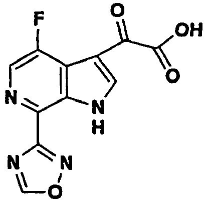

1-Ethyl-3-methylimidazolium chloride (73mg, 0.52 mmol) and aluminum chloride (198 mg, 1.56 mmol) were stirred together under nitrogen for 1h. To this solution was added intemediate 2q (54 mg, 0.26 mmol) and ethyloxalylchloride (58 µL, 0.52 mmol) and the reaction mixture was stirred at rt for 18h. The reaction was quenched with water and the mixture was stirred for 15 min. The solid was collected by filtration and washed with water and diethylether. LC/MS (ES⁺) m/z (M+H)⁺ =276. This compound was used without further purification.

### Intermediate 4n

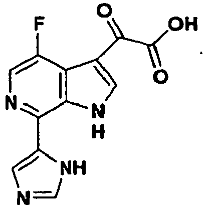

1-Ethyl-3-methylimidazolium chloride (26mg, 0.18 mmol) was added to a flask which contained intermediate 2t (18 mg, 0.09 mmol) and the mixture was cooled to 0°C. Aluminum chloride (92 mg, 0.54mmol) was added in one portion followed by ClCOCOOEt (20µL, 0.18 mmol) and the reaction mixture was stirred at room temperature for 2 days. Ice water was added to quench the reaction. The reaction mixture was filtered. The solid was washed with water and diethylether and dried in air to afford compound D (18mg). LC/MS: (ES⁺) m/z(m+H)⁺ = 275. Rt = 0.49 min.

### Intermediate 5a

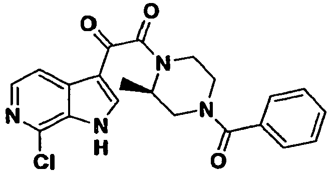

### Typical procedure for coupling piperazine derivative and azaindole acid:

Preparation of 1-benzoyl-3-(R)-methyl-4-[(7-chloro-6-azaindol-3-yl)-oxoacetyl]piperazine, Intermediate 5, is an example of Step D of Scheme 1. Potassium 7-chloro-6-azaindole 3-glyoxylate, Intermediate 4a, (100 mg, 0.44 mmol), 3-(*R*)-methyl-1-benzoylpiperazine (107 mg, 0.44 mol), 3-(diethoxyphosphoryloxy)-1,2,3-benzotriazin-4(3*H*)-one (DEPBT) (101 mg, 0.44 mol) and Hunig's Base (diisopropylethylamine, 0.5 mL) were combined in 5 mL of DMF. The mixture was stirred at rt for 8 h. DMF was removed *via* evaporation at reduced pressure and the residue was purified using a Shimadzu automated preparative HPLC System to give 1-(benzoyl)-3-(R)-methyl-4-[(7-chloro-6-azaindol-3-yl)-oxoacetyl]-piperazine (70 mg, 39%). MS *m*/*z*: (M+H)⁺ Calc'd for C₂₁H₂₀ClN₄O₃: 411.12; Found 411.06. HPLC retention time: 1.32 minutes (column A).

### Intermediate 5b

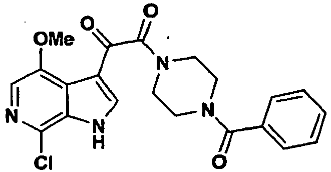

Intermediate 5b, 1-benzoyl-4-[(7-chloro-4-methoxy-6-azaindol-3-yl)-oxoacetyl]piperazine was prepared by the same method as Intermediate 5a starting from Potassium (7-chloro-4-methoxy-6-azaindol-3-yl)-oxoacetate, Intermediate 4d, and 1-benzoylpiperazine. MS *m*/*z*: (M+H)⁺ calcd for C₂₁H₂₀ClN₄O₄: 427.12; found 427.12. HPLC retention time: 1.28 minutes (column A).

### Intermediate 5c

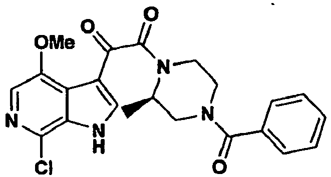

Intermediate 5c, 1-benzoyl-3-(R)-methyl-4-[(7-chloro-4-methoxy-6-azaindol-3-yl)-oxoacetyl]piperazine was prepared by the same method as Intermediate 5a starting from Potassium (7-chloro-4-methoxy-6-azaindol-3-yl)-oxoacetate, Intermediate 4d, and 1-benzoylpiperazine. ¹HNMR (500 MHz, CDCl₃) δ 8.10 (s, 1H), 7.72 (s, 1H), 7.40 (s, 5H), 3.89 (s, 3H), 3.71 - 3.40 (m, 8H). MS *m*/*z*: (M+H)⁺ calcd for C₂₂H₂₂ClN₄O₄: 441.13; found 441.17. HPLC retention time: 1.33 minutes (column A).

### Intermediate 5d

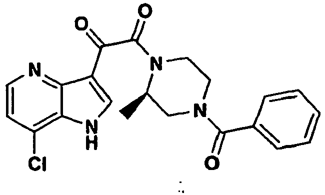

Intermediate 5d, 1-benzoyl-3-(R)-methyl-4-[(7-chloro-4-azaindol-3-yl)-oxoacetyl]piperazine was prepared by the same method as Intermediate 5a, starting from Potassium (7-chloro-4-azaindol-3-yl)-oxoacetate, Intermediate 4e, and 1-benzoyl-3-(R)-methyl piperazine. MS *m*/*z*: (M+H)⁺ calcd for C₂₁H₂₀ClN₄O₃ 411.12, found 411.04. HPLC retention time: 1.10 minutes (column A).

### Intermediate 5e

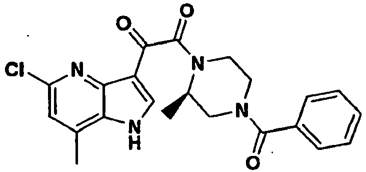

Intermediate 5e, 1-benzoyl-3-(R)-methyl-4-[(5-chloro-7-methyl-4-azaindol-3-yl)-oxoacetyl]piperazine was prepared by the same method as Intermediate 5a, starting from Potassium (5-chloro-7-methyl-4-azaindol-3-yl)-oxoacetate, Intermediate 4f, and 1-benzoyl-3-(R)-methyl piperazine. MS *mlz:* (M+H)⁺ calcd for C₂₂H₂₂ClN₄O₃ 425.24, found 425.04. HPLC retention time: 1.72 minutes (column B).

### Intermediate 5f

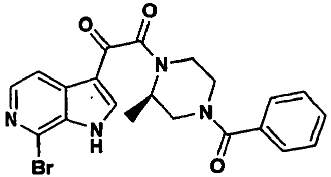

Intermediate 5f, 1-benzoyl-3-(R)-methyl-4-[(7-bromo-6-azaindol-3-yl)-oxoacetyl]piperazine was prepared by the same method as Intermediate 5a, starting from (7-bromo-6-azaindol-3-yl)-oxoacetic acid potassium salt, Intermediate 4g, and 1-benzoyl-3-(R)-methylpiperazine. MS *m*/*z*: (M+H)⁺ calcd for C₂₁H₂₀BrN₄O₃: 455.07; found 455.14. HPLC retention time: 1.45 minutes (column B).

### Intermediate 5g

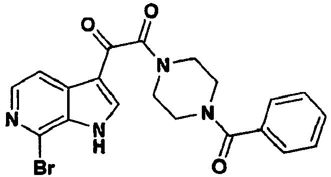

Intermediate 5g, 1-benzoyl-4-[(7-bromo-6-azaindol-3-yl)-oxoacetyl]piperazine was prepared by the same method as Intermediate 5a, starting from (7-bromo-6-azaindol-3-yl)-oxoacetic acid potassium salt, Intermediate 4g, and 1-benzoylpiperazine. MS *m*/*z*: (M+H)⁺ calcd for C₂₀H₁₈BrN₄O₃: 441.06; found 441.07. HPLC retention time: 1.43 minutes (column B).

### Intermediate 5h

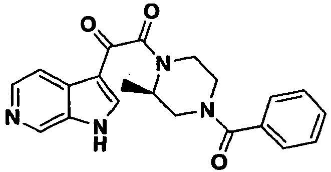

Intermediate 5h, 1-benzoyl-3-(R)-methyl-4-[(6-azaindol-3-yl)-oxoacetyl]piperazine was prepared by the same method as Intermediate 5a starting from Potassium (6-azaindol-3-yl)oxoacetate, Intermediate 4b, and 1-benzoyl-3-(R)-methylpiperazine. MS *m*/*z*: (M+H)⁺ Calc'd for C₂₁H₂₁N₄O₃: 377.16; Found 377.10. HPLC retention time: 0.88 minutes (column A).

### Intermediate 5i

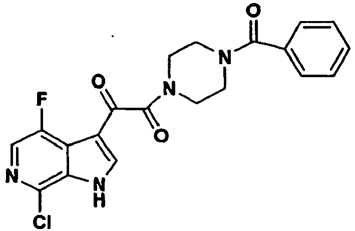

Addition of intermediate 2d to a solution of aluminum trichloride in dichloromethane stirring at ambient temperature followed 30 minutes later with chloromethyl or chloroethyl oxalate (according to the method described for intermediate 3a) provides either the methyl or ethyl ester, respectively. Hydrolysis with KOH (as in the standard hydrolysis procedure described for intermediate 4a) provided potassium (7-chloro-4-fluoro-6-azaindol-3-yl)oxoacetate. Potassium (7-chloro-4-fluoro-6-azaindol-3-yl)oxoacetate was then reacted with 1-benzoyl piperazine in the presence of DEPBT under the standard conditions (as described for intermediate 5a) to provide 1-benzoyl-4-[(4-fluoro-7-chloro-6-azaindol-3-yl) oxoacetyl]piperazine, intermediate 5i. ¹H NMR (500 MHz, CD₃OD) δ 8.40 (s, 1H), 8.04 (s, 1H), 7.46 (bs, 5H), 3.80-3.50 (m, 8H); LC/MS (ES⁺) m/z (M+H)⁺ 415 observed; retention time 1.247 minutes; LC/MS method: YMC ODS-A C18 S7 3.0 x 50 mm column; Start %B = 0, Final %B = 100, Gradient time = 2 minutes; Flow rate = 5 mL/min; detector wavelength = 220 nm.

### Intermediate 5j

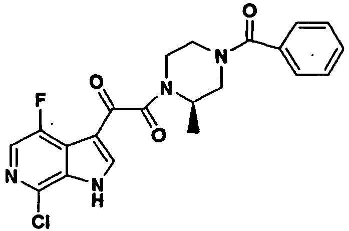

1-benzoyl-3-(R)-methyl-4-[(4-fluoro-7-chloro-6-azaindol-3-yl)-oxoacetyl]-piperazine was prepared by coupling potassium (7-chloro-4-fluoro-6-azaindol-3-yl)oxoacetate, prepared as described above for intermediate 5i, with 1-benzoyl-3-(R)-methylpiperazine in the presence of DEPBT under the standard conditions (as described for intermediate 5a) to provide 1-benzoyl-3-(R)-methyl-4-[(4-fluoro-7-chloro-6-azaindol-3-yl)-oxoacetyl] piperazine, intermediate 5j. ¹H NMR (500 MHz, CD₃OD) δ 8.42, 8.37 (s, s, 1H), 8.03 (s, 1H), 7.71-7.45 (m, 5H), 4.72-3.05 (m, 7H), 1.45-1.28 (m, 3H); LC/MS (ES⁺) m/z (M+H)⁺ 429 observed; retention time 1.297 minutes; LC/MS method: YMC ODS-A C18 S7 3.0 x 50 mm column; Start %B = 0, Final %B = 100, Gradient time = 2 minutes; Flow rate = 5 mL/min; detector wavelength = 220 nm.

### Intermediate 5k

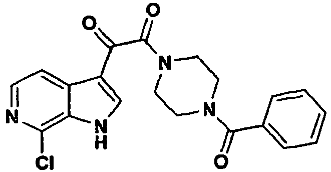

Intermediate 5k, 1-benzoyl-4-[(7-chloro-6-azaindol-3-yl)-oxoacetyl]piperazine was prepared by the same method as Intermediate 5a, starting from (7-chloro-6-azaindol-3-yl)-oxoacetic acid potassium salt, Intermediate 4a, and 1-benzoylpiperazine. MS *m*/*z*: (M+H)⁺ calcd for C₂₀H₁₈ClN₄O₃: 397.11; found 396.97. HPLC retention time: 2.37 minutes (column F, gradient time = 3 min, flow rate = 4 ml/min).

### Intermediate 51

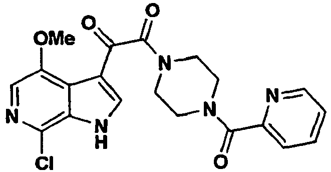

Intermediate 51, 1-picolinoyl-4-[(4-methoxy-7-chloro-6-azaindol-3-yl)-oxoacetyl]piperazine was prepared by the same method as Intermediate 5a starting from Potassium (4-methoxy-7-chloro-6-azaindol-3-yl)oxoacetate, Intermediate 4d, and picolinoyl-piperazine. ¹H NMR (500 MHz, DMSO-d₆) 88.63 - 7.45 (m, 7 H), 3.94 (s, 3H), 3.82 - 2.50 (m, 8H). MS m/z: (M+H)⁺ Calc'd for C₂₀H₁₉ClN₅O₄: 428.11; Found 428.11. HPLC retention time: 1.09 minutes (column A).

### Intermediate 5m

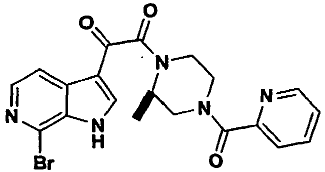

Intermediate 5m, (R)-1-picolinoyl-3- methyl-4-[(7-bromo-6-azaindol-3-yl)-oxoacetyl]piperazine was prepared by the same method as Intermediate 5a starting from Potassium (7-bromo-6-azaindol-3-yl)oxoacetate, Intermediate 4g, and (R)-3-methyl-1-picolinoyl-piperazine. MS *m*/*z*: (M+H)⁺ Calc'd for C₂₀H₁₉BrN₅O₃: 456.07; Found 456.11. HPLC retention time: 1.12 minutes (column A).

### Intermediate 5n

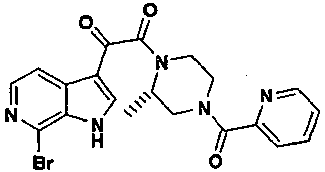

Intermediate 5n, (S)-1-picolinoyl-3- methyl-4-[(7-bromo-6-azaindol-3-yl)-oxoacetyl]piperazine was prepared by the same method as Intermediate 5a starting from Potassium (7-bromo-6-azaindol-3-yl)oxoacetate, Intermediate 4g, and (S)-3-methyl-1-picolinoyl-piperazine. ¹H NMR (500 MHz, CDCl₃) δ8.63 - 7.36 (m, 7H), 5.02 - 3.06 (m, 7H), 1.42 -1.26 (m, 3H).

### Intermediate 5o

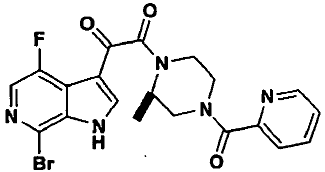

Intermediate 5o, (R)-1-picolinoyl-3- methyl-4-[(7-bromo-4-fluoro-6-azaindol-3-yl)-oxoacetyl]piperazine was prepared by the same method as Intermediate 5a starting from Potassium (7-bromo-4-fluoro-6-azaindol-3-yl)oxoacetate, Intermediate 4h, and (R)-3-methyl-1-picolinoyl-piperazine. ¹H NMR (500 MHz, CD₃OD) δ8.68 - 7.52 (m, 6H), 4.94 - 2.69 (m, 7H), 1.48 -1.24 (m, 3H). MS m/z: (M+H)⁺ Calc'd for C₂₀H₁₈BrFN₅O₃: 474.06; Found 474.23. HPLC retention time: 1.20 minutes (column A).

### Intermediate 5p

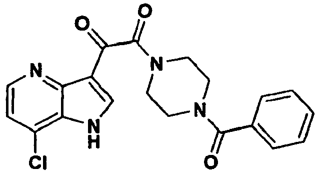

Intermediate 5p, 1-benzoyl-4-[(7-chloro-4-azaindol-3-yl)-oxoacetyl]piperazine was prepared by the same method as Intermediate 5a starting from Potassium (7-chloro-4-fluoro-4-azaindol-3-yl)oxoacetate, Intermediate **4e**, and 1-benzoylpiperazine. ¹H NMR (500 MHz, CD₃OD) δ8.83 (s, 1H), 8.63 (d, 1H, J = 5.35 Hz), 7.91 (d, 1H, J = 5.75 Hz), 7.47 (m, 5H), 3.80 - 3.30 (m, 3H). MS *m*/*z*: (M+H)⁺ Calc'd for C₂₀H₁₈ClN₄O₃: 397.11; Found 397.02. HPLC retention time: 1.20 minutes (column A).

### Intermediate 5q

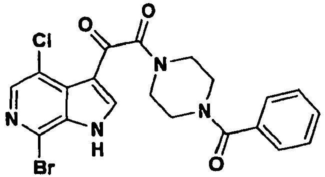

Intermediate 5q, 1-(4-Benzoyl-piperazin-1-yl)-2-(7-bromo-4-chloro-1H-pyrrolo[2,3-c]pyridin-3-yl)-ethane-1,2-dione
To a solution of acid **intermediate 4j** (2.4 g, 7.9 mmol) in DMF (40 ml) was added 3-(diethoxyphosphoryloxy)-1,2,3-benzotriazin-4(3*H*)-one (DEPBT, 5.96 g, 19.9 mmol), benzoylpiperazine hydrochloride (2.71 g, 11.9 mmol), and *N,N-*diisopropylethylamine (14 ml, 80.4 mmol). The mixture was stirred at ambient temperature for 16 h. The reaction mixture was then added water (400 ml) and extracted with EtOAc (4 x 300 ml). The combined extracts were evaporated *in vacuo* to give a brownish residue, which was triturated with MeOH to provide the title compound as a white solid (2.8 g, 74%); **¹H NMR**: (DMSO-*d₆*) 13.41 (s, 1H), 8.48 (s, 1H), 8.19 (s, 1H); 7.45 (b s, 5H), 3.80 - 3.35 (b m, 8H); **LC/MS**: (ES+) m/z (M+H)⁺= 475, 477, 479; HPLC Rₜ = 1.953.

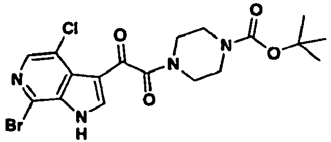

**Intermediate** 5r was prepared by procedure used for 5q using mono N-Boc piperazine . **¹H NMR**: (CDCl₃) δ 8.26 (s, 1H), 8.19 (s, 1H), 3.71 (b s, 2H), 3.53 (b m, 6H), 1.48 (s, 9H); **LC/MS**: (ES+) m/z (M+H)⁺= 471, 473, 475; HPLC Rₜ = 1.543.

### Intermediate 6

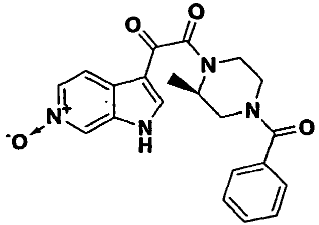

*Typical procedure for N-Oxide formation:* Preparation of 1-benzoyl-3-(R)-methyl-4-[(6-oxide-6-azaindol-3-yl)-oxoacetyl]piperazine, Intermediate 6. 20 mg of 1-benzoyl-3-(R)-methyl-4-[(6-azaindol-3-yl)-oxoacetyl]piperazine, Intermediate 5h, (0.053 mmol) was dissolved in CH₂Cl₂ (2 mL). 18 mg of mCPBA (0.11 mmol) was then added into the solution and the reaction was stirred for 12 h at rt. CH₂Cl₂ was removed via evaporation at reduced pressure and the residue was purified using a Shimadzu automated preparative HPLC System to give the compound shown above (5.4 mg, 26%). MS *m*/*z*: (M+H)⁺ Calc'd for C₂₁H₂₁N₄O₄: 393.16; Found 393.11. HPLC retention time: 0.90 minutes (column A).

### Intermediate 7

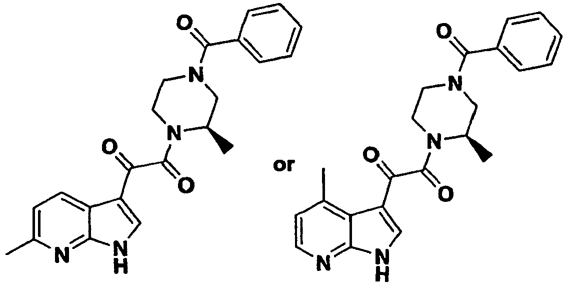

Preparation of 1-benzoyl-3-(R)-methyl-4-[(6-methyl-7-azaindol-3-yl)-oxoacetyl]-piperazine or 1-benzoyl-3-(R)-methyl-4-[(4-methyl-7-azaindol-3-yl)-oxoacetyl]-piperazine. An excess of MeMgI (3M in THF, 0.21 ml, 0.63 mmol) was added into a solution of 1-benzoyl-3-(R)-methyl-4-[(6-oxide-6-azaindol-3-yl)-oxoacetyl]piperazine, Intermediate 6, (25 mg, 0.064 mmol). The reaction mixture was stirred at rt and then quenched with MeOH. The solvents were removed under vacuum, the residue was diluted with MeOH and purified using a Shimadzu automated preparative HPLC System to give a compound shown above which was a single isomer but regiochemistry was not definitively assigned. (6.7 mg, 27%). MS *m*/*z*: (M+H)⁺ Calc'd for C₂₂H₂₃N₄O₃: 391.18; Found 391.17. HPLC retention time: 1.35 minutes (column B).

### Intermediate 8

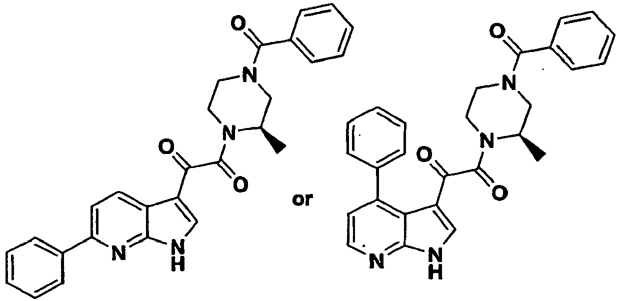

1-benzoyl-3-(R)-methyl-4-[(6-phenyl-7-azaindol-3-yl)-oxoacetyl]piperazine or 1-benzoyl-3-(R)-methyl-4-[(4-phenyl-7-azaindol-3-yl)-oxoacetyl]piperazine (regiochemistry was not definitively assigned) were prepared by the method described for Example 7 starting with 1-benzoyl-3-(R)-methyl-4-[(6-oxide-6-azaindol-3-yl)-oxoacetyl]piperazine, Intermediate 6, and phenyl magnesium bromide (phenyl Grignard reagent). MS *m*/*z*: (M+H)⁺ Calc'd for C₂₇H₂₅N₄O₃: 453.19; Found 454.20. HPLC retention time: 1.46 minutes (column B).

### Intermediate 9

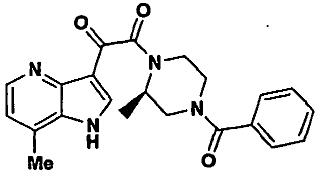

A mixture of Pd (10% on carbon, 100 mg), trifluoroacetic acid (1 mL) and 1-benzoyl-3-(R)-methyl-4-[(5-chloro-7-methyl-4-azaindol-3-yl)-oxoacetyl]piperazine, Intermediate 5e (1.5 g) in MeOH (50 mL) and EtOAc (50 mL) was shaken in a Parr reactor under a hydrogen atmosphere (45 psi) for 48 hours. After solids were removed via filtration, the filtrate was concentrated *in vacuo* to afford intermediate 9 (1 g) which was used without further purification. MS *m*/*z*: (M+H)⁺ calcd for C₂₁H₂₁N₄O₃ 391.18, found 391.15. HPLC retention time: 1.15 minutes (column A).

### Intermediates 10 and 11

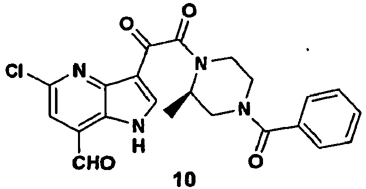

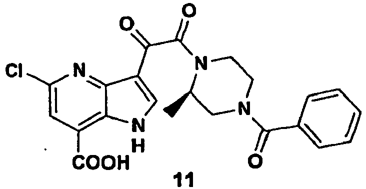

Preparation of Intermediate 10, 1-benzoyl-3-(R)-methyl-4-[(5-chloro-7-carbonyl-4-azaindol-3-yl)-oxoacetyl]-piperazine and Intermediate 11, 1-benzoyl-3-(R)-methyl-4-[(5-chloro-7-hydroxycarbonyl-4-azaindol-3-yl)-oxoacetyl]-piperazine: A mixture of 1-benzoyl-3-(R)-methyl-4-[(5-chloro-7-methyl-4-azaindol-3-yl)-oxoacetyl]piperazine (1.78 g) and SeO₂ (4.7 g) in dioxane/water (100 : 1) was refluxed for 10 hours. After cooling to room temperature, the mixture was concentrated *in vacuo* to provide a residue. The residue was purified by using silica gel chromatography with EtOAc and MeOH as eluting solvents to afford intermediate 10 (350 mg) and intermediate 11 (410 mg).
Intermediate 10, 1-benzoyl-3-(R)-methyl-4-[(5-chloro-7-carbonyl-4-azaindol-3-yl)-oxoacetyl]-piperazine: MS *m*/*z*: (M+H)⁺calcd for C₂₂H₂₀ClN₄O₄: 439.12, found 439.01. HPLC retention time: 1.37 minutes (column A); Intermediate 11, 1-benzoyl-3-(R)-methyl-4-[(5-chloro-7-hydroxycarbonyl-4-azaindol-3-yl)-oxoacetyl]-piperazine: MS *m*/*z*: (M+H)⁺ calcd for C₂₂H₂₀ClN₄O₅: 455.11, found 455.10. HPLC retention time: 1.44 minutes (column A).

### Intermediates 12 and 13

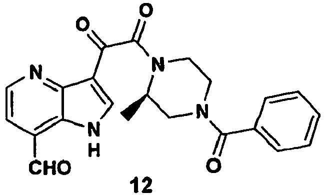

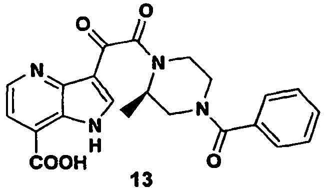

Intermediate 12, 1-benzoyl-3-(R)-methyl-4-[(7-carbonyl-4-azaindol-3-yl)-oxoacetyl]-piperazine and Intermediate 13, 1-benzoyl-3-(R)-methyl-4-[(7-hydroxycarbonyl-4-azaindol-3-yl)-oxoacetyl]-piperazine were made according to the same procedure of preparing Intermediates 10 and 11, by using Intermediate 9 as a starting material. Intermediate 12, 1-benzoyl-3-(R)-methyl-4-[(7-carbonyl-4-azaindol-3-yl)-oxoacetyl]-piperazine: MS *mlz:* (M+H)⁺ calcd for C₂₂H₂₁N₄O₄: 405.16, found 405.14. HPLC retention time: 0.91 minutes (column A); Intermediate 13, 1-benzoyl-3-(R)-methyl-4-[(7-hydroxycarbonyl-4-azaindol-3-yl)-oxoacetyl]-piperazine: MS m/z: (M+H)⁺ calcd for C₂₂H₂₁N₄O₅: 421.15, found 421.09. HPLC retention time: 1.02 minutes (column A).

### Intermediates 14a-1-14a-21

The following tin agents and boron agents can be purchased from commercial resources and used without any further treatment (Table 2).

**Table 2**

| Intermediate Number | Structure | Company |
|---|---|---|
| 14a-1 | 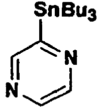 | Frontier Scientific, Inc. |
| 14a-2 | 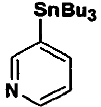 | Maybridge Chem. Co. |
| 14a-3 | 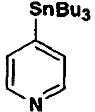 | Frontier Scientific, Inc. |
| 14a-4 | 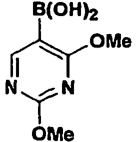 | Matnx Scientific |
| 14a-5 | 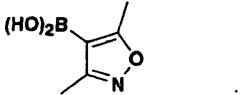 | Matrix Scientific |
| 14a-6 | 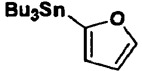 | Aldrich, Co. |
| 14a-7 | 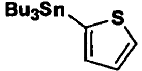 | Aldrich, Co. |
| 14a-8 | 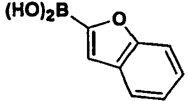 | Aldrich, Co. |
| 14a-9 | 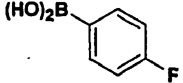 | Aldrich, Co. |
| 14a-10 | 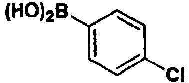 | Aldrich, Co. |
| 14a-11 | 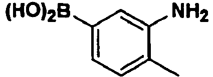 | Lancaster |
| 14a-12 | 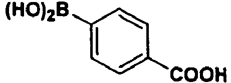 | Aldrich, Co. |
| 14a-13 | 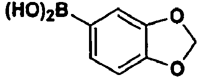 | Aldrich, Co. |
| 14a-14 | 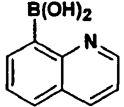 | Frontier Scientific, Inc. |
| 14a-15 | 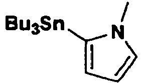 | Matrix Scientific |
| 14a-16 | 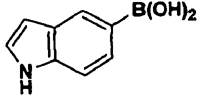 | Frontier Scientific, Inc. |
| 14a-17 | 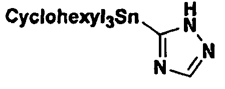 | Riedel-de Haen AG |
| 14a-18 | 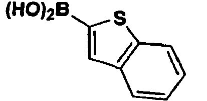 | Lancaster |
| 14a-19 | 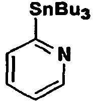 | Lancaster |
| 14a-20 | 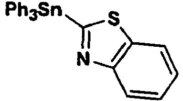 | Aldrich, Co. |
| 14a-21 | 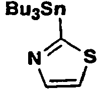 | Frontier Scientific, Inc. |

### Preparation of Tin Agents:

### Intermediates 14-1-14-65

The following known tin agents and boron agents could be prepared according to the documented procedures indicated without any modification (Table 3):

**Table 3**

| Intermediate Number | Structure | Reference |
|---|---|---|
| 14-1 | 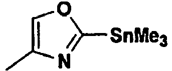 | Dondoni, A., et al Synthesis, 1987, 693 |
| 14-2 | 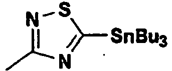 | Aldous, D. J., et al US - 5,453,433 |
| 14-3 | 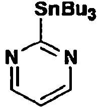 | Sandosham, J., et al Tetrahedron 1994, 50, 275. |
| 14-4 | 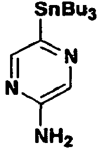 | Lehn, L. M., et al. Chem. Eur. J. 2000, 6, 4133. |
| 14-5 | 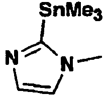 | Jutzi, P., et al J. Organometallic Chem. 1983, 246, 163. |
| 14-6 | 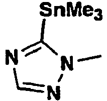 | Jutzi, P., et al J. Organometallic Chem. 1983, 246, 163. |
| 14-7 | 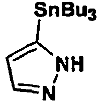 | Graybill, T. L., et al Bioorg. Med. Chem. Lett. 1995, 5 (4), 387. |
| 14-8 | 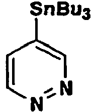 | Heldmann, D. K., et al Tetrahedron Lett. 1997, 38, 5791. |
| 14-9 | 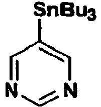 | Kennedy, G., et al Tetrahedron Lett. 1996, 37, 7611. |
| 14-10 | 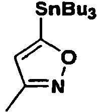 | Kondo, Y., et al Tetrahedron Lett. 1989,30, 4249 |
| 14-11 | 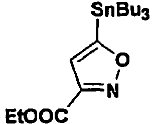 | Kondo, Y., et al Tetrahedron Lett. 1989,30, 4249 |
| 14-12 | 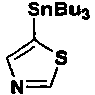 | Or, Y. S., et al US-6,054,435 |
| 14-13 | 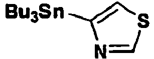 | Or, Y.S., et al US-6,054,435 |
| 14-14 | 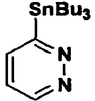 | Okada, T., et al WO-0123383 |
| 14-15 | 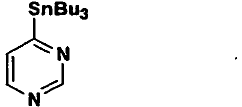 | Okada, T., et al WO-0123383 |
| 14-16 | 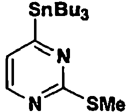 | Sandosham, J., et al Tetrahedron 1994, 50, 275 |
| 14-17 | 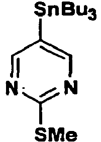 | Sandosham, J., et al Acta Chem. Scand. 1989, 43, 684. |
| 14-18 | 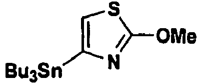 | Nicolaou, K. C., et al WO-9967252 |
| 14-19 | 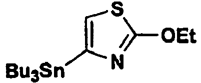 | Nicolaou, K. C., et al WO-9967252 |
| 14-20 | 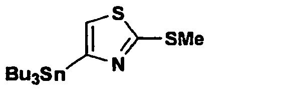 | Nicolaou, K. C., et al WO-9967252 |
| 14-21 | 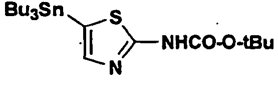 | Benheda, R., et al Tetrahedron Lett. 1999, 40, 5701. |
| 14-22 | 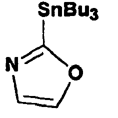 | Collins, I., et al Tetrahedron Lett. 1999, 40, 4069. |
| 14-23 | 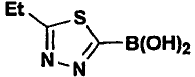 | Fuss, R. W., et al DE-19502178 |
| 14-24 | 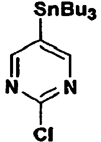 | Bunnage, M.E.et.al PCT Int. Appl. WO 0024745 A1 (2000); and Sandosham, J. et. Al Tetrahedron (1994), 50(1), 275-84. |
| 14-25 | 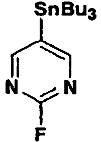 | From 5-iodo2-chloro-1,3 pyrimidine. Fluoropyrimidines are obtained by fluorination of chloropyrimidines with CsF in N-methyl-2-pyrrolidinone or DMF 2.5-63 h at 80-150 °C. The iodo is then converted to the lithium reagent with tBuLi and trapped with Bu₃SnCl. See Sandosham above. |
| 14-26 | 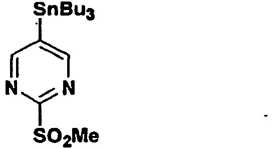 | Arukwe, J.; Benneche, T.; Undheim, K. J. Chem. Soc., Perkin Trans. 1 (1989), (2), 255-9. |
| 14-27 | 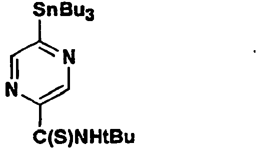 | Fruit, C.; Heterocycles (1999), 51(10), 2349-2365. |
| 14-28 | 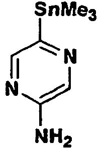 | Ziener, U.; et.al. Chem.-Eur. J. (2000), 6(22), 4132-4139. - |
| 14-29 | 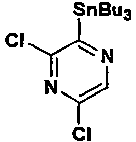 | Turck, A.; et.al Lab.. J. Organomet. Chem. (1991), 412(3), 301-10. Metallation of 2,6-dicloropyrazine and quench with Bu₃SnCl. |
| 14-30 | 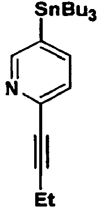 | Ueno, K.; Sasaki, A.; Kawano, K.; Okabe, T.; Kitazawa, N.; Takahashi, K.; Yamamoto, N.; Suzuki, Y.; Matsunaga, M.; Kubota, A. PCT Int. Appl. WO 9918077 A1 (1999). |
| 14-31 | 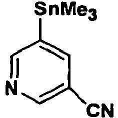 | Fensome, A.; Miller, L. L.; Ullrich, J.W.; Bender, RH.W.; Zhang, P.; Wrobel, J.E.; Zhi, L.; Jones, T.K.; Marschke, K.B.; Tegley, C.M. PCT Int. Appl. WO 0066556 A1 (2000). |
| 14-32 | 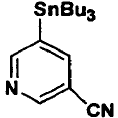 | Maw, G.N.; Middleton, D.S. Jpn. Kokai Tokkyo Koho JP 2000016984 A2 (2000). Chem. Pharm. Bull. |
| 14-33 | 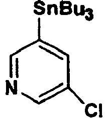 | (1998), 46(3), 400-412. |
| 14-34 | 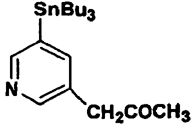 | Hayashi, K.; Kito, T.; Mitsuyama, J.; Yamakawa, T.; Kuroda, H.; Kawafuchi, H. PCT Int. Appl. WO 9951588 A1 (1999). |
| 14-35 | 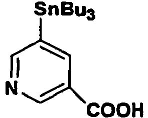 | Brown, A.D.; Dickinson, RP.; Wythes, M.J. PCT Int. Appl. WO 9321178 A1 (1993). |
| 14-36 | 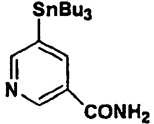 | Brown, A.D.; Dickinson, R.P.; Wythes, M.J. PCT Int. Appl. WO 9321178 A1 (1993). |
| 14-37 | 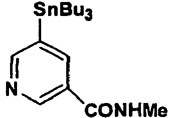 | Zalutsky, M.R. PCT Int. Appl. WHO 0032240 A2 (2000). Brown. A.D.; Dickinson, |
| 14-38 | 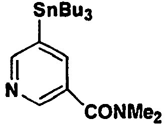 | Brown, A.D.; Dickinson, R.P.; Wythes, M. J. PCT Int. Appl. WO 9321178 A1 (1993). |
| 14-39 | 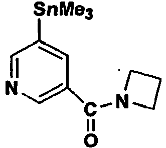 | North, P.C.; Wadman, S.N. PCT Int. Appl. WO 9408993 A1 (1994). |
| 14-40 | 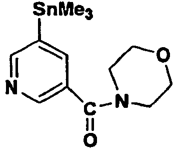 | North, P.C.; Wadman, S.N. PCT Int. Appl. WO 9408993 A1 (1994). |
| 14-41 | 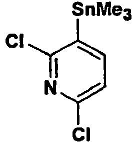 | Achab, S.; Guyot, M.; Potier, P. Tetrahedron Lett. (1993), 34(13), 2127-30. |
| 14-42 | 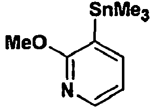 | Muratake, H.; Tonegawa, M.; Natsume, M... Chem. Pharma. Bull. (1998), 46(3), 400-412. Dehmlow, E.V.;Sleegers, A. Liebigs Ann. Chem. (1992), (9), 953-9. |
| 14-43 | 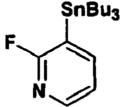 | Proudfoot, J.R.; Hargrave, K.; Kapadia, S. PCT Int. Appl. WO 9907379 A1 (1999); and Chem. Pharm. Bull. (1998), 46(3), 400-412. |
| 14-44 | 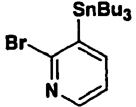 | Cruskie, M.P. Jr.; Zoltewicz, J.A.; Abboud, K.A. J. Org. Chem. (1995), 60(23), 7491-5. |
| 14-45 | 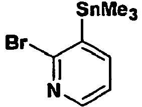 | Muratake, H.; et.al Chem. Pharm. Bull. (1998), 46(3), 400-412. |
| 14-46 | 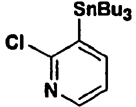 | Muratake, H.; Tonegawa, M.; Natsume, M. Chem. Pharm. Bull. (1998), 46(3), 400-412. Dolle, RE.; Graybill, T.L.; Osifo, I.K.; Harris, A.L.; Miller, M.S.; Gregory, J.S. U.S. US 5622967(1997). |
| 14-47 | 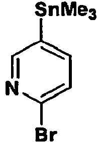 | Henze, O.; Lehmann, U.; Schlueter, A.D. Synthesis (1999), (4), 683-687. |
| 14-48 | 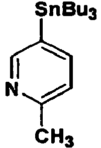 | Hayashi, K.; Kito, T.; Mitsuyama, J.; Yamakawa, T.; Kuroda, H.; Kawafuchi, H. PCT Int. Appl. WO 9951588 A1 (1999); Reuman, M.; Daum, S.J.; Singh, B.; Wentland, M.P.; Perni, R.B.; Pennock, P.; Carabateas, P.M.; Gruett, M.D.; Saindane, M.T.; et al. J. Med. Chem. (1995), 38(14), 2531-40. |
| 14-49 | 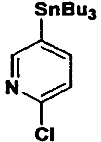 | Barros, M.T.; Maycock, C.D.; Ventura, M.R. Tetrahedron Lett. (1999), 40(3), 557-560. Sirisoma, N.S.; Johnson, C.R. Tetrahedron Lett. (1998), 39(15), 2059-2062. Trost, B.M.; Cook, G.R Tetrahedron Lett. (1996), 37(42), 7485-7488. |
| 14-50 | 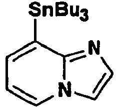 | Bunnage, M.E.; Maw, G.N.; Rawson, D.J.; Wood, A.; Mathias, J.P.; Street, S.D.A. PCT Int. Appl. WO 0024745 A1 (2000). |
| 14-51 | 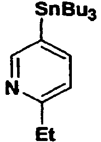 | Bunnage, M.E.; Maw, G.N.; Rawson, D.J.; Wood, A.; Mathias, J. P.; Street, S.D.A. PCT Int. Appl. WO 0024745 A1 (2000). |
| 14-52 | 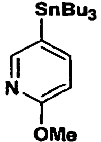 | Hayashi, K.; Kito, T.; Mitsuyama, J.; Yamakawa, T.; Kuroda, H.; Kawafuchi, H. PCT Int Appl. WO 9951588 A1 (1999); and Sirisoma, N.S.; Johnson, C.R Tetrahedron Lett. (1998), 39(15), 2059-2062. |
| 14-53 | 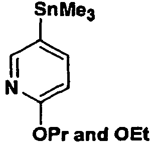 | Schnatterer, S.; Kern, M.; Sanft, U. PCT Int. Appl. WO 9965901 A1 (1999). |
| 14-54 | 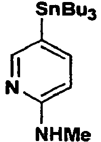 | Hayashi, K.; Kito, T.; Mitsuyama, J.; Yamakawa, T.; Kuroda, H.; Kawafuchi, H. PCT Int. Appl. WO 9951588 A1 (1999). |
| 14-55 | 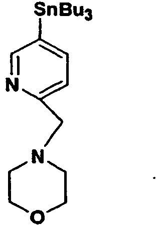 | Betagen, R.; Breitfelder, S.; Cirillo, P.F.; Gilmore, T.A.; Hickey, E.R.; Kirrane, T.M.; Moriak, M.H.; Moss, N.; Patel, U.R.; Proudfoot, J.R.; Regan, J.R.; Sharma, R.; Sun, S.; Swinamer, A.D.; Takahashi, H. PCT Int. Appl. WO 0055139 A2 (2000). |
| 14-56 | 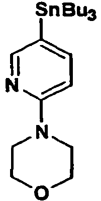 | Ueno, K.; Sasaki, A.; Kawano, K.; Okabe, T.; Kitazawa, N.; Takahashi, K.; Yamamoto, N.; Suzuki, Y.; Matsunaga, M.; Kubota, A. PCT Int. Appl. WO 9918077 A1 (1999). |
| 14-57 | 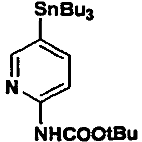 | Calderwood, D.; Arnold, L.D.; Mazdiyasni, H.; Hirst, G.; Deng, B.B. PCT Int. Appl. WO 0017202 A1 (2000). |
| 14-58 | 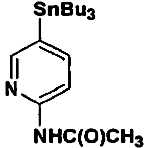 | Hayashi, K.; Kito, T.; Mitsuyama, J.; Yamakawa, T.; Kuroda, H.; Kawafuchi, H. PCT Int. Appl. WO 9951588 A1 (1999). |
| 14-59 | 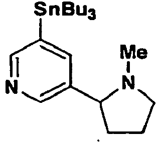 | Saji, H.; Watanabe, A.; Magata, Y.; Ohmono, Y.; Kiyono, Y.; Yamada, Y.; Iida, Y.; Yonekura, H.; Konishi, J.; Yokoyama, A. Chem. Pharm. Bull. (1997), 45(2), 284-290. |
| 14-60 | 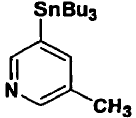 | Hayashi, K.; Kito, T.; Mitsuyama, J.; Yamakawa, T.; Kuroda, H.; Kawafuchi, H. PCT Int. Appl. WO 9951588 A1 (1999); Reuman, M.; Daum, S.J.; Singh, B.; Wentland, M.P.; Perni, R.B.; Pennock, P.; Carabateas, P.M.; Gruett, M.D.; Saindane, M.T.; et al. J. Med. Chem. (1995), 38(14), 2531-40. |
| 14-61 | 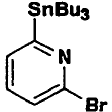 | Iino, Y.; Fujita, K.; Kodaira, A.; Hatanaka, T.; Takehana, K.; Kobayashi, T.; Konishi, A.; Yamamoto, T. PCT Int. Appl. WO 0102359 A1 (2001). |
| 14-62 | 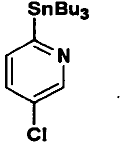 | Iino, Y.; Fujita, K.; Kodaira, A.; Hatanaka, T.; Takehana, K.; Kobayashi, T.; Konishi, A.; Yamamoto, T. PCT Int. Appl. WO 0102359 A1 (2001). |
| 14-63 | 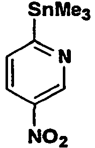 | Torrado, A.; Imperiali, B. J. Org. Chem. (1996), 61(25), 8940-8948. |
| 14-64 | 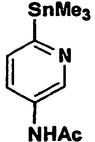 | Iino, Y.; Fujita, K.; Kodaira, A.; Hatanaka, T.; Takehana, K.; Kobayashi, T.; Konishi, A.; Yamamoto, T. PCT Int. Appl. WO 0102359 A1 (2001). |
| 14-65 | 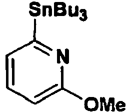 | Gros, P.; Fort, Y. Synthesis (1999), (5), 754-756 and Gros, P.; Fort, Y.; Caubere, P. J. Chem. Soc., Perkin Trans. 1 (1997), (20), 3071-3080. |

### Intermediate 14-66

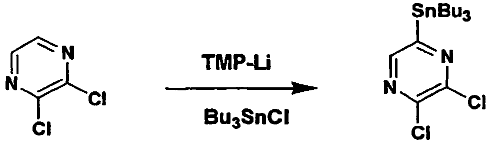

Preparation of 2,3-dicloro-5-(tri-n-butylstannyl)pyrazine (An example of general procedure Tin-01, below): TMP-Li (2,2,6,6-tetramethylpiperidinyl lithium) was prepared by addition of n-butyl lithium (1.6 M, 6.25 mL) to a solution of 2,2,4,4-tetramethylpiperidine (1.4 g) in dry THF (180 mL) at -78 °C. The solution was then allowed to warm to 0 °C, was stirred at 0 °C for 15 minutes, then was cooled to -78 °C. To the solution was added 2,3-dichloropyrazine (1.35 g), and followed by an addition of tri-n-butyltin chloride (3.25 g) in another 2 hours. The reaction was quenched with aqueous ammonium chloride solution. The organic layer was separated, and aqueous layer was extracted with ethyl acetate (3 x 100 mL). The combined organic extract was dried over magnesium sulfate, filtered and the filtrate concentrated *in vacuo.* The residue was purified by silica gel chromatography to afford 2,3-dicloro-5-(tri-n-butylstannyl)pyrazine (1 g).

### Intermediate 14-67

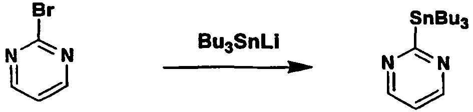

Preparation of 2-(tri-n-butylstannyl)-pyrimidine: (Example of the general procedure Tin-03, below) Tri-n-butylstannyl lithium was prepared at 0 °C in dry THF (20 mL) from tri-butyltin hydride (2.2 mL) and LDA (lithium diisopropylamide, 2M, 4.09 mL). The tri-n-butylstannyl lithium solution was then cooled to -78 °C and to it was added 2-bromopyrimidine (1 g). The reaction The mixture was then allowed to warm up to room temperature over 8 hours. reaction was then quenched with aqueous ammonium chloride solution. The organic layer was separated, and aqueous layer was extracted with ethyl acetate (3 x 20 mL). The combined organic layer was dried over magnesium sulfate, filtered and the filtrate concentrated *in vacuo.* The residue was purified by silica gel chromatography to afford 2-(tri-n-butylstannyl)-pyrimidine (190 mg).

### Intermediate 14-68

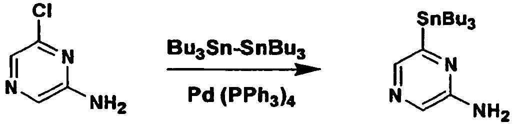

Preparation of 2-amino-6-(tri-n-butylstannyl)pyrazine (Example of the general procedure Tin-04, below): To a sealed tube, 2-amino-6-chloro-pyrazine (1 g), bis(tri-butyltin) (3.92 mL) and tetrakis-triphenylphosphine palladium, Pd(Ph₃P)₄ (100 mg) were combined in dioxane (10 mL). The reaction was heated at 110-120 °C for 10 h. After the mixture cooled down to room temperature, it was poured into 20 mL of water. The solution was extracted with EtOAc (4 x 20 mL). The combined extract was concentrated *in vacuo* to give a residue which was purified by silica gel chromatography to afford 2-amino-6-(tri-n-butylstannyl)pyrazine (0.5 g)

### Intermediate 14-69

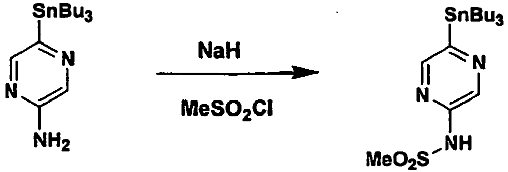

Preparation of 2-methylsulfonylamino-5-(tri-n-butylstannyl)pyrazine (Example of general procedure Thin-05, below): NaH (60%, 20 mg) was added into a solution of 2-amino-5-(tri-n-butylstannyl)pyrazine (0.2 g) in THF (30 mL) at room temperature. After the mixture stirred at room temperature for 30 minutes, to it was added methylsulfonyl chloride (63 mg). The reaction mixture was stirred at room temperature over 8 hours. The reaction was quenched with aqueous ammonium chloride solution. The organic layer was separated, and the aqueous layer was extracted with ethyl acetate (3 x 100 mL). The combined organic extract was dried over magnesium sulfate, filtered and the filtrate was concentrated in vacuo. The residue was purified by silica gel chromatography to afford 2-methylsulfonylamino-5-(tri-n-butylstannyl)pyrazine (20 mg).

### Intermediates 14-70 - 14-129

The intermediates 14-70 - 14-129 were prepared according to the following general procedures designated Tin-01 through Thin-05.

### General Procedure Tin-01:

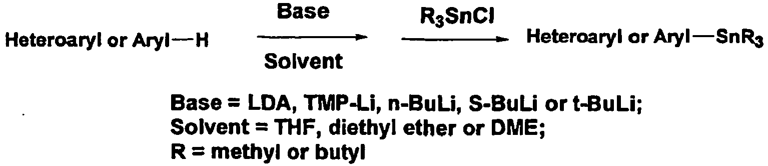

To a solution of a base (1.1 equivalents) selected from lithium diisopropylamide, 2,2,6,6-tetramethylpiperidinyl lithium, n-butyl lithium, sec-butyl lithium or tert-butyl lithium in a solvent selected from tetrahydrofuran, diethyl ether or dimethoxyethane (concentration of approximately 0.05 mmol base/mL of solvent) at -78 °C was added an appropriate aryl or heteroaryl substrate (1.0 equivalents) followed by an addition of tri-n-butyltin chloride or trimethyltin chloride (1.1 equivalents) in another 2 hours. The reaction was quenched with aqueous ammonium chloride solution. The organic layer was separated, and aqueous layer was extracted with ethyl acetate. The combined organic extract was dried over magnesium sulfate, filtered and the filtrate concentrated *in vacuo.* The residue was purified by silica gel chromatography to afford the desired stannane.

### General Procedure Tin-02:

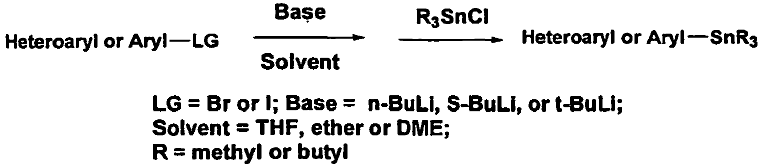

To a solution of a base (1.1 equivalents) selected from n-butyl lithium, sec-butyl lithium or tert-butyl lithium in a solvent selected from tetrahydrofuran, diethyl ether or dimethoxyethane (concentration of approximately 0.05 mmol base/mL of solvent) at -78 °C was added an appropriate aryl or heteroaryl bromide or aryl or heteroaryl iodide substrate (1.0 equivalents). The reaction mixture was stirred at -78 °C for a period suitable to generate the anion via metal-halogen exchange then to it was added tri-n-butyltin chloride or trimethyltin chloride (1.1 equivalents). The reaction was quenched with aqueous ammonium chloride solution. The organic layer was separated, and aqueous layer was extracted with ethyl acetate. The combined organic extract was dried over magnesium sulfate, filtered and the filtrate concentrated *in vacuo.* The residue was purified by silica gel chromatography to afford the desired stannane.

### General Procedure Tin-03:

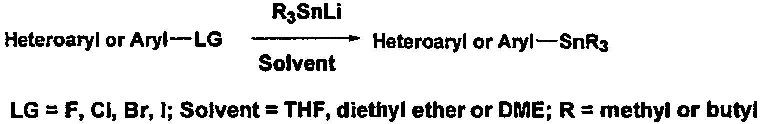

Tri-n-butylstannyl lithium or trimethylstannyl lithium (1.3 equivalents) was prepared at 0 °C in dry solvent selected from THF, diethyl ether or dimethoxyethane (20 mL) from tri-n-butyltin hydride or trimethyltin hydride, respectively (1.3 equivalents) and LDA (lithium diisopropylamide, 1.3 equivalents) at a concentration of approximately 0.4 mmol of alkylstannyl lithium/mL of solvent. The tri-n-butylstannyl lithium or trimethylstannyl lithium solution was then cooled to -78 °C and to it was added an appropriate haloaryl or haloheteroaryl substrate (1.0 equivalent). The reaction mixture was then allowed to warm up to room temperature over 8 hours. The reaction was then quenched with aqueous ammonium chloride solution. The organic layer was separated, and aqueous layer was extracted with ethyl acetate (3 x 20 mL). The combined organic layer was dried over magnesium sulfate, filtered and the filtrate concentrated *in vacuo.* The residue was purified by silica gel chromatography to afford the desired stannane intermediate.

### General Procedure Tin-04:

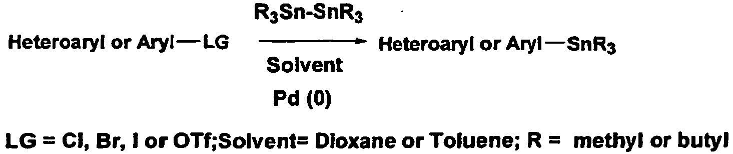

To a sealed tube, an appropriate aryl or heteroaryl substrate (1.0 equivalent), bis(tri-butyltin) or hexamethylditin (1.0 equivalent) and tetrakis-triphenylphosphine palladium, Pd(Ph₃P₄) (1.0 mol%) were combined in dioxane or toluene (10 mL). The reaction was heated at 110-120 °C for 10 h. After the mixture cooled down to room temperature, it was poured into water. The solution was extracted with ethyl acetate and the combined extracts were concentrated *in vacuo* to give a residue which was purified by silica gel chromatography to afford the desired stannane product.

### General Procedure Tin-05:

The following general reaction scheme depicts the derivatization of stannane intermediates in which the stannane has a reactive ring NH group or reactive exocyclic amino, hydroxy or thiol group. The starting stannane is treated with base in an appropriate solvent then is reacted with suitable electrophiles such as alkyl halides, acid chlorides, sulfonyl chlorides, isocyanates and the like.

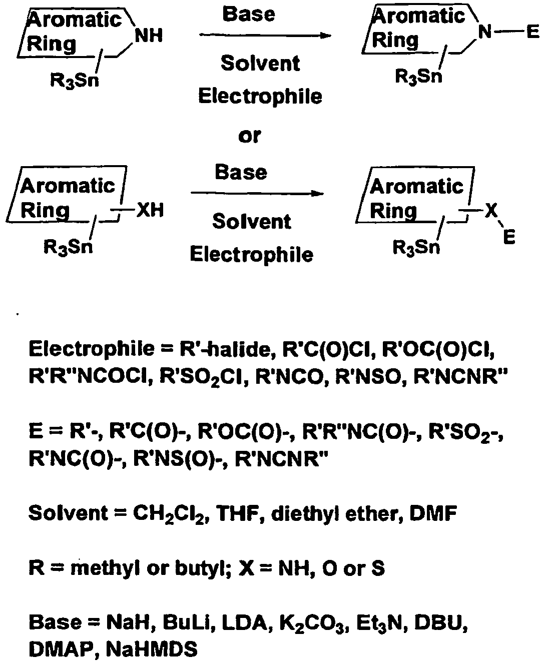

An appropriate base selected from sodium hydride, n-butyl lithium, lithium diisopropylamide, potassium carbonate, triethylamine, DBU, DMAP or sodium hexamethyldisilazide (1.0 equivalent) was added into a solution of an appropriate stannane substrate (as depicted above, 1.0 equivalent) in an appropriate solvent selected from dichloromethane, THF, diethyl ether or N,N-dimethylformamide at a temperature between -78 °C and room temperature. After the mixture stirred for a period sufficient to allow deprotonation, typically for 5 to 30 minutes, then to it was added an appropriate electrophile such as an alkyl halide, acid chloride, sulfonyl (1.0 equivalent). The reaction mixture was stirred, typically at room temperature, over a period of 2 to 8 hours. The reaction was quenched with aqueous ammonium chloride solution. The organic layer was separated, and the aqueous layer was extracted with ethyl acetate (3 x 100 mL). The combined organic extract was dried over magnesium sulfate, filtered and the filtrate was concentrated in vacuo. The residue was purified by silica gel chromatography to afford the desired stannane intermediate.

### General procedure Tin-06

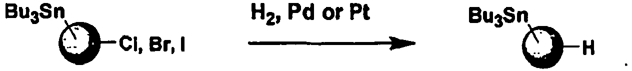

An aryl hilide stannane agent was dissolved in appropriate alcohol, either methanol or ethanol. After a cataylst (pt or pd) was added into the solvent, the reaction mixture is placed in an environment of hydrogen under normal or raised pressure. After reaction finishes, the catalyst is filtered, and, concentration of the mother solution provides a residue which is used in the further reactions without any purification.
Rf = retention time

| Intermed. Number | Structure | Starting Material | Method Applied | Identification |
|---|---|---|---|---|
| 14-70 | 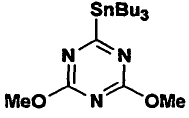 | 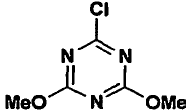 | Tin-04 | R_{f} = 2.33 min (Column A) ¹H NMR (500 MHz, CDC13) δ 4.00 (s, 6H), 1.63 - 0.85 (m, 27H) |
| 14-71 | 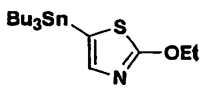 | 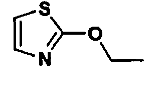 | Tin-01 | R_{f} = 2.52 mm Column A) H NMR (300 MHz, CDCl₃) δ 7.02 (s, 1H), 4.44 (q, 2H, J= 7.02 Hz), 1.63 - 0.85 (m, 30H) |
| 14-72 | 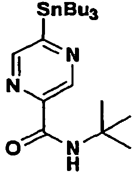 | 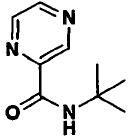 | Tin-01 | R_{f} = 2.84 min Column B) H NMR (500 MHz, CDC13) δ 9.48 (s, 1H), 8.45 (s, 1H), 2.03 - 0.88 (m, 36H) |
| 14-73 | 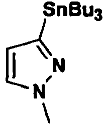 | 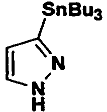 | Tin-05 | R_{f} = 2.27 min (Column A) ¹H NMR (500 MHz, CDCl₃) δ 7.53 (m, 1H), 6.29 (m, 1H), 3.94 (s, 3H), 1.56-0.87 (m, 27H) |
| 14-74 | 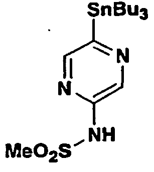 | 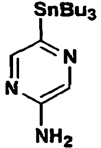 | Tin-05 | R_{f} = 2.22 min (Column A) |
| 14-75 | 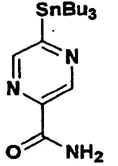 | 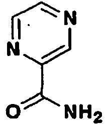 | Tin-01 | R_{f} = 2.44 min (Column B) H NMR (500 MHz, CDCl₃) δ 8.89 (s, 1H), 8.34 (s, 1H), 1.61 - 0.85 (m, 27H) |
| 14-76 | 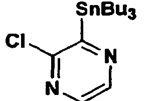 | 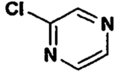 | Tin-01 | R_{f} = 3.41 mm (Column A, flow rate = 4 ml/min) ¹H NMR (300 MHz, CDCl3) δ 8.58 (d, 1H, *J* = 2.52 Hz), 8.13 (d, 1H, *J*= 2.52 Hz), 1.63 - 0.85 (m, 27H) |
| 14-77 | 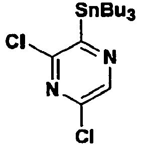 | 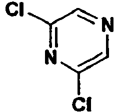 | Tin-01' | R_{f} = 3.89 min (Column A, flow rate = 4 ml/min) ¹H NMR (300 MHz, CDCl3) δ 8.63 (s, 1H), 1.61-0.85 (m, 27H) |
| 14-78 | 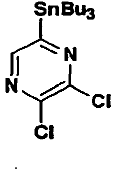 | 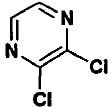 | Tin-01 | R_{f} = 3.86 min (Column A, flow rate = 4 ml/min) ¹H NMR (300 MHz, CDCl3) δ 8.24 (s, 1H), 1.61-0.85 (m, 27H) |
| 14-79 | 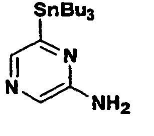 | 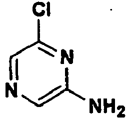 | Tin-04 | R_{f} = 2.10 min (Column B) ¹H NMR (500 MHz, CDCl3) δ 7.90 (s, 1H), 7.26 (s, 1H), 1.58-0.87 (m, 27H) |
| 14-80 | 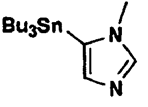 | 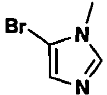 | Tin-04 | R_{f} = 1.83 min (Column A) |
| 14-81 | 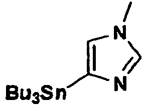 | 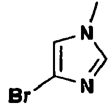 | Tin-04 | R_{f} = 1.84 min (Column A) |
| 14-82 | 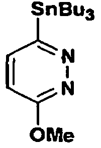 | 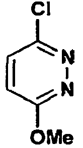 | Tin-04 | R_{f} = 1.84 min (Column A) |
| 14-83 | 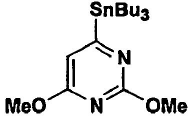 | 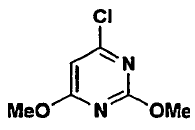 | Tin-04 | R_{f} = 1.90 min (Column A) |
| 14-84 | 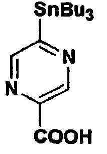 | 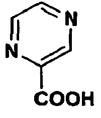 | Tin-01 | R_{f} = 2.23 min (Column A) |
| 14-85 | 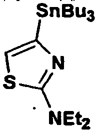 | 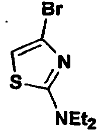 | Tin-04 | R_{f} = 1.92 min (Column A) |
| 14-86 | 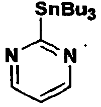 | 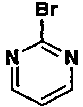 | Tin-03 | R_{f} = 2.01 min (Column A) |
| 14-87 | 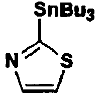 | 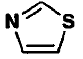 | Tin-01 | R_{f} = 2.45 min (Column A) |
| 14-88 | 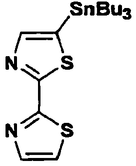 | 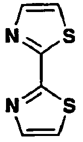 | Tin-01 | R_{f} = 2.61 min (Column C) |
| 14-89 | 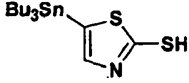 | 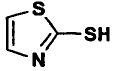 | Tin-01 | R_{f} = 2.31 min (Column C) |
| 14-90 | 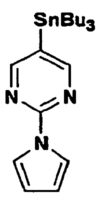 | 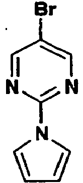 | Tin-04 | R_{f} = 2.71 min (Column D) |
| 14-91 | 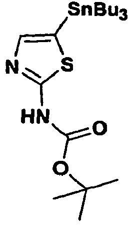 | 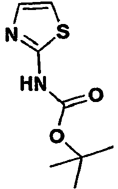 | Tin-01 | R_{f} = 2.49 min (Column C) |
| 14-92 | 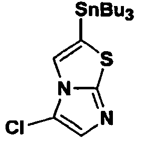 | 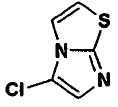 | Tin-01 | R_{f} = 2.42 min (Column C) |
| 14-93 | 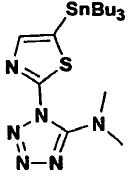 | 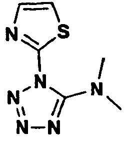 | Tin-01 | R_{f} = 3.49 min (Column C) Flow Rate = 4 ml/min |
| 14-94 | 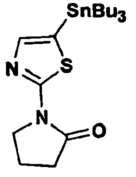 | 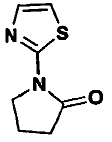 | Tin-01 | R_{f} = 2.46 min (Column C) |
| 14-95 | 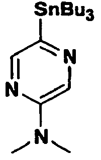 | 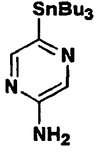 | Tin-05 | R_{f} = 2.15 min (Column A) |
| 14-96 | 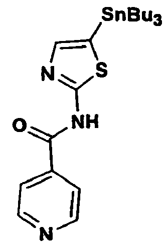 | 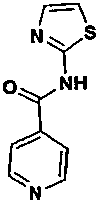 | Tin-01 | R_{f} = 2.28 min (Column C) |
| 14:97 | 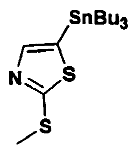 | 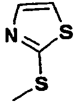 | Tin-01 | R_{f} = 2.60 min (Column C) |
| 14-98 | 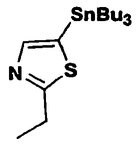 | 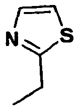 | Tin-01 | R_{f} = 2.37 min (Column A) |
| 14-99 | 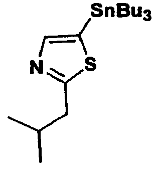 | 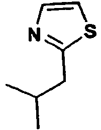 | Tin-01 | R_{f} = 2.59 min (Column A) |
| 14-100 | 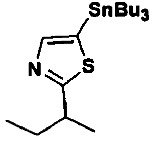 | 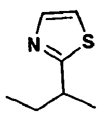 | Tin-01 | R_{f} = 2.49 min (Column C) |
| 14-101 | 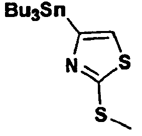 | 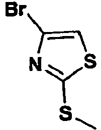 | Tin-04 | R_{f} = 2.41 min (Column A) |
| 14-102 | 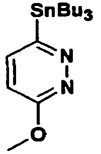 | 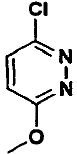 | Tin-04 | R_{f} = 1.18 min (Column E) |
| 14-103 | 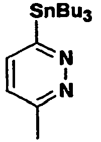 | 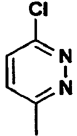 | Tin-04 | R_{f} = 1.92 min (Column E) |
| 14-104 | 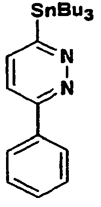 | 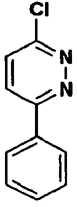 | Tin-04 | R_{f} = 2.01 min (Column E) |
| 14-105 | 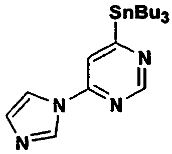 | 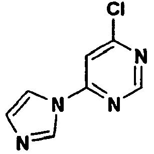 | Tin-04 | R_{f} = 2.15 min (Column E) |
| 14-106 | 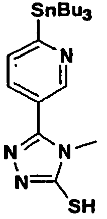 | 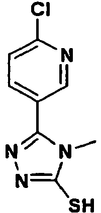 | Tin-04 | R_{f} = 1.91 min (Column E) |
| 14-107 | 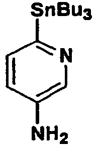 | 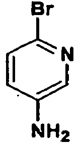 | Tin-04 | Rf = 1.95 min (Column A) |
| 14-108 | 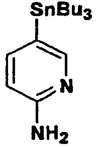 | 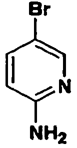 | Tin-04 | Rf = 1.93 min (Column A) |
| 12-109 | 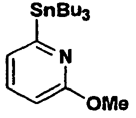 | 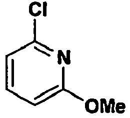 | Tin-01 | Rf = 1.95 min (Column A) |
| 14-110 | 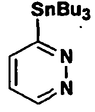 | 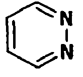 | Tin-01 | Rf = 1.83 min (Column A) H NMR (500 MHz, CDCl3) δ 9.03 (d, 1H, J = 5.15 Hz), 7.49 (d, 1H, J = 7.95 Hz), 7.26 (m, 1H), 1.61 - 0.86 (m, 27H); ¹³C NMR (125 MHz, CDCl3) δ 175.3, 149.8, 133.2, 123.7, 29.0, 27.3, 13.6, 10.1. |
| 14-111 | 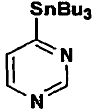 | 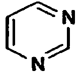 | Tin-01 | Rf = 2.18 min Column E) ¹H NMR (500 MHz, CDCl3) δ 9.22 (s, 1H), 8.46 (d, 1H, J = 4.80 Hz), 7.42 (d, 1H, J = 4.75 Hz), 1.56 - 0.86 (m, 27H); ¹³C NMR (125 MHz, CDC13) δ 185.4, 158.0, 153.2, 130.6, 28.9, 27.2, 13.5, 9.9. |
| 14-112 | 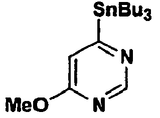 | 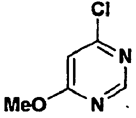 | Tin-04 | Rf = 1,96 min (Column A) |
| 14-113 | 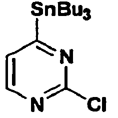 | 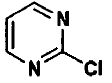 | Tin-01 | Rf = 2.61 min (Column A) |
| 14-114 | 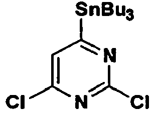 | 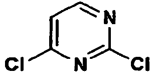 | Tin-01 | Rf = 2.85 min (Column A) |
| 14-115 | 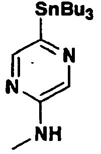 | 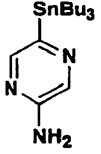 | Tin-05 | Rf = 2.09 min (Column A) ¹H NMR (500 MHz, CDCl3) δ 8.12 (s, 1H), 7.95 (s, 1H), 4.11 (s, 1H), 2.95 (s, 3H), 2.03 - 0.85 (m, 27H) |
| 14-116 | 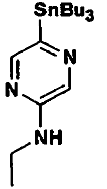 | 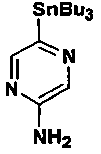 | Tin-05 | Rf = 2.16 min Column A) H NMR (500 MHz, CDCl3) δ 8.08 (s, 1H), 7.92 (s, 1H), 4.49 (s, 1H), 3.35 (m, 2H), 1.63-0.85 (m, 30H) |
| 14-117 | 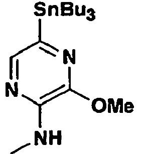 | 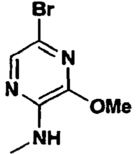 | Tin-04 | Rf = 2.19 min (Column A) |
| 14-118 | 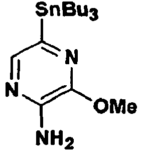 | 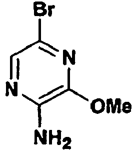 | Tin-04 | Rf = 2.18 min (Column A) |
| 14-119 | 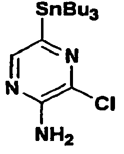 | 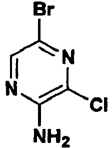 | Tin-04 | Rf = 2.47 min (Column A) ¹H NMR (500 MHz, CDCl3) δ 7.85 (s, 1H), 4.91 (s, 2H), 2.16 - 0.87 (m, 27H) |
| 14-120 | 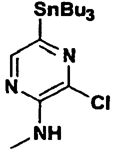 | 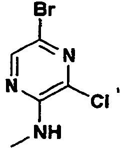 | Tin-04 | Rf = 2.61 min (Column A) |
| 14-121 | 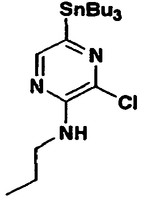 | 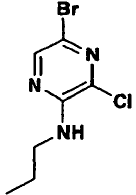 | Tin-04 | Rf = 2.92 min (Column A) |
| 14-122 | 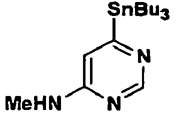 | 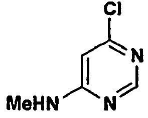 | Tin-04 | Rf = 1.93 min (Column A) |
| 14-123 | 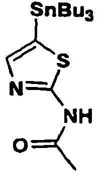 | 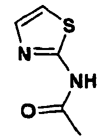 | Tin-01 | Rf = 2.20 min (Column A) |
| 14-124 | 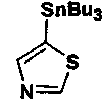 | 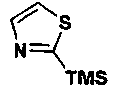 | Tin-01 | Rf = 2.50 min (Column A) ¹H NMR (500 MHz, CDCl3) δ 9.07 (s, 1H), 7.87 (s, 1H), 1.59 - 0.85 (m, 27H) |
| 12-125 | 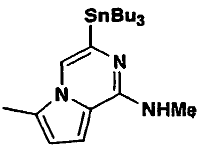 | 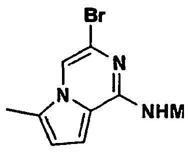 | Tin-04 | Rf = 1.97 min (Column A) |
| 14-126 | 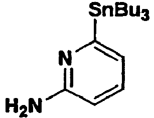 | 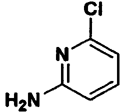 | Tin-04 | Rf = 1.9 min (Column A) |
| 14-127 | 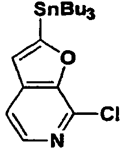 | 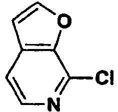 | Tin-01 | Rf = 2.70 min (Column E) ¹H NMR (500 MHz, CDCl3) δ 8.11 (d, 1H, J = 5.2 Hz), 7.41 (d, 1H, J = 5.2 Hz), 6.94 (s, 1H), 1.62 - 0.89 (m, 27H) |
| 14-128 | 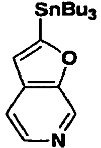 | 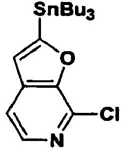 | Tin-06 | ¹H NMR (500 MHz, CDCl3) δ 8.12 (d, 1H, J = 5.2 Hz), 7.78 (s, 1H), 7.46 (d, 1H, J = 5.2 Hz), 6.84 (s, 1H), 1.98 - 0.85 (m, 27H) |
| 14-129 | 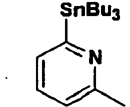 | 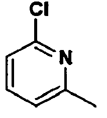 | Tin-01 | Rf = 1.86 min (Column A) |
| | | | | |

The following table contains novel stannane reagents which can be prepared by the methodology described above and then could be used to prepare compounds of formula I

**Table 3**

| Intermediate Number | Structure | Reference |
|---|---|---|
| | 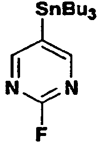 | From 5-iodo2-chloro-1,3 pyrimidine. Fluoropyrimidines are obtained by fluorination of chloropyrimidines with CsF in N-methyl-2-pyrrolidinone or DMF 2.5-63 h at 80-150□. The iodo is then converted to the lithium reagent with tBuLi and trapped with Bu3SnCl. See Sandosham above. |
| | 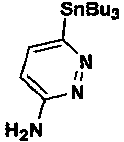 | |
| | 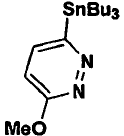 | |
| | 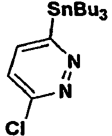 | |
| | 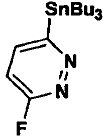 | |
| | 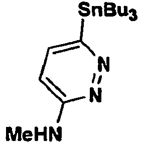 | |
| | 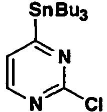 | |
| | 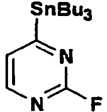 | |
| | 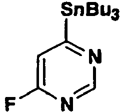 | |
| | 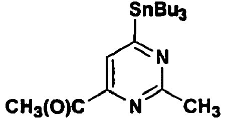 | |
| | 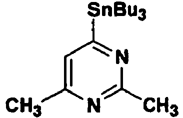 | |
| | 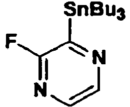 | |
| | 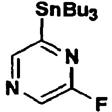 | |
| | 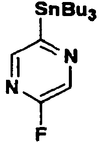 | |
| | 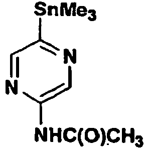 | |
| | 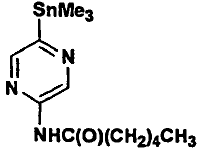 | |
| | 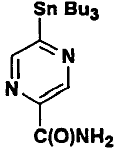 | |
| | 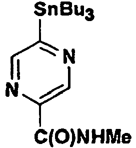 | |
| | 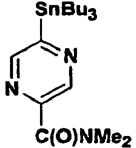 | |
| | 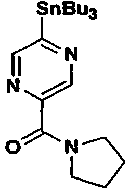 | |
| | 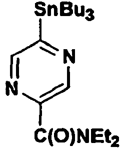 | |
| | 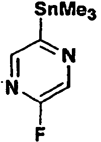 | |
| | 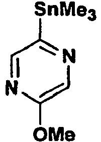 | |
| | 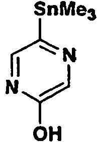 | |
| | 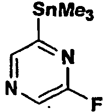 | |
| | 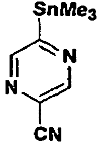 | |
| | 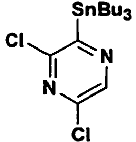 | Turck, A.; et.al Lab.. J. Organomet. Chem. (1991), 412(3), 301-10. Metallation of 2,6-dicloropyrazine and quench with Bu3SnCl |
| | 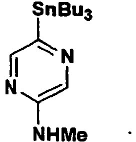 | Analogous to Lehn, L. M., et al. Chem. Eur. J. 2000, 6, 4133. |
| | 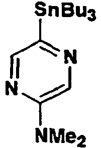 | |
| | 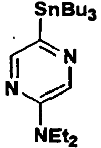 | |
| | 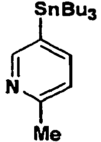 | |
| | 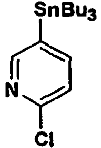 | |
| | 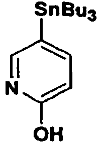 | |
| | 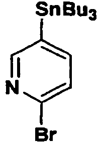 | |
| | 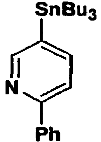 | |
| | 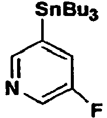 | |
| | 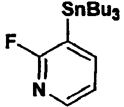 | |
| | 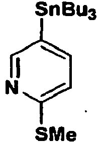 | |
| | 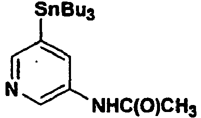 | |
| | 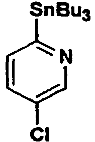 | |
| | 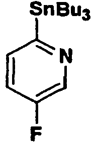 | |
| | 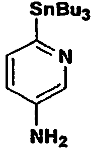 | |
| | 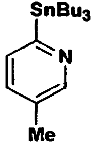 | |
| | 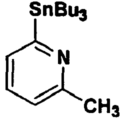 | |
| | 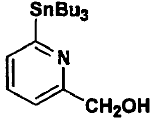 | |
| | 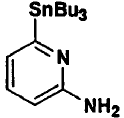 | |
| | 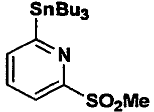 | |
| | 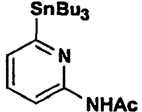 | |
| | 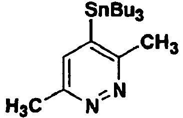 | |
| | 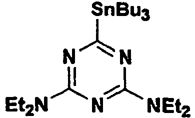 | |
| | 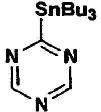 | |
| | 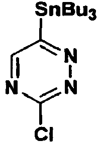 | |
| | 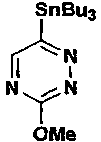 | |
| | 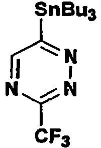 | |
| | 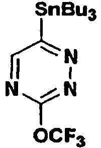 | |
| | 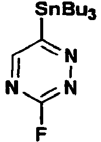 | |
| | 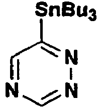 | |
| | 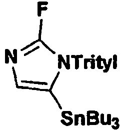 | Metallation ot 1-trityl-4-iodo imidazole (prepared in Takahashi, Kazuyuki; Kirk, Kenneth L.; Cohen, Louis A. Lab. Chem., Natl. Inst. Arthritis Diabetes Dig. Kidney Dis., Bethesda, MD, USA. J. Labelled Compd. Radiopharm. (1986), 23(1), 1-8) using tBuLi in THF at -78 and quenching with Bu₃SnCl. Detritylate with TFA or aq HCl after coupling to azaindole core. |
| | 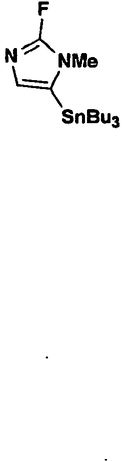 | Metallation of 1-methyl-4-iodo imidazole (prepared in Takahashi, Kazuyuki; Kirk, Kenneth L.; Cohen, Louis A. Lab. Chem., Natl. Inst. Arthritis Diabetes Dig. Kidney Dis., Bethesda, MD, USA. J. Labelled Compd. Radiopharm. (1986), 23(1), 1-8) using tBuLi in THF at -78 and quenching with Bu₃SnCl. El Borai, M.; Moustafa, A. H.; Anwar, M.; Abdel Hay, F.I. The bromo derivative is described in Pol. J. Chem. (1981), 55(7-8), 1659-65 and can be used to generate the tin reagent via transmetallation. |
| | 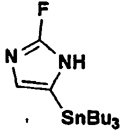 | |
| | 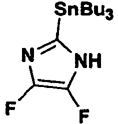 | 4,5 difluoromidazole prepared as in Dolensky, Bohumil;et.al, USA. J. Fluorine Chem. (2001), 107(1), 147-148. |
| | 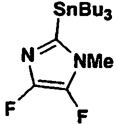 | Dolensky, Bohumil;et.al, USA. J. Fluorine Chem. (2001), 107(1), 147-148. |
| | 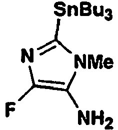 | |

### Select general procedures, via S_{N}Ar reactions, for the preparation of starting materials for Tin agents

### a. Preparation of 2-bromo-5-substituted-pyrazine, 5-bromo-2-subsituted-thiazole, 2-substituted-thiazaole, 4-chloro-6-substituted-pyrimidine and 5-bromo-2-substituted-pyrimide

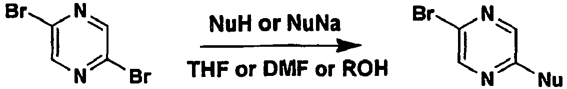

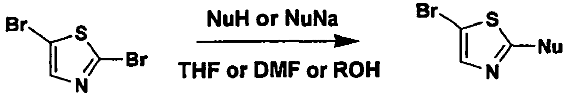

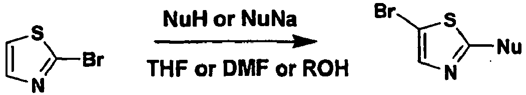

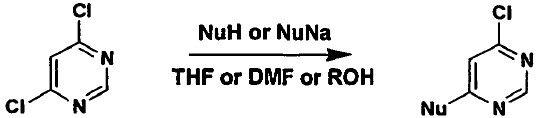

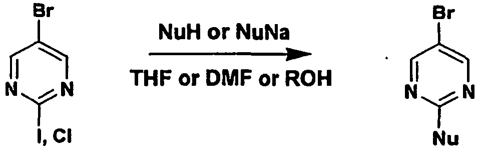

To a flask, an appropriate pyrazine, pyrimidine or thiazole (1.0 equivalent) and a nucleophile, such as amine, alcohol or thio-derivatives in one equivanlence or an exess amount were combined in a solvent such as THF, DMF or alcohol, with or without an addition of NaH. The reaction was either stirred at room temperature or under heating for one to three days. After all the solvents were removed, the residue was partitioned between saturated NaHCO₃ and EtOAc. The aqueous layer was extracted with ethyl acetate and the combined extracts were concentrated *in vacuo* to give a residue, which was purified by silica gel chromatography to afford the desired product.

| **Starting Material** | **Product** | **Reaction Condition** | **Rf (minutes)** | **MS (M+H)+ Cald.** | **MS (M+H)+ Obsv.** |
|---|---|---|---|---|---|
| 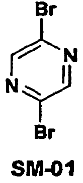 | 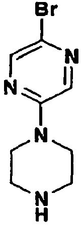 | SM-01 (2g) Piperazine (10 g), THF (50 ml), r.t. | 0.5b (column G) | 243.02 | 243.03 |
| 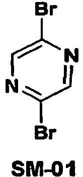 | 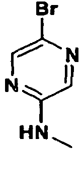 | SM-01 (1 g), MeNH₂ (2M in THF, 100 ml), r.t. | 0.89 (column E) | 187.93 | 187.98 |
| 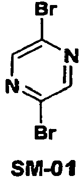 | 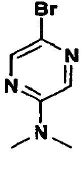 | SM-01 (1g ), Me₂NH (2M in THF, 100 ml), r.t. | 1.19 (column E) | 201.92 | 202.00 |
| 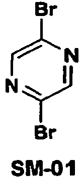 | 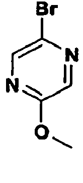 | SM-01 (1 g), MeONa (0.5M in MeOH, 100 ml), r.t. | 1.05 (column E) | 189.91 | 188.97 |
| 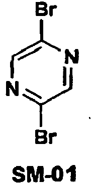 | 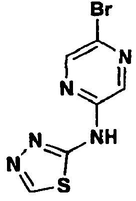 | SM-01 (50 mg), NaH (17 mg), 2-amino-1,3,4-thiadiazole (25 mg), DMF 5 ml) r.t. | 1.21 (column E) | 257.94 | 257.89 |
| 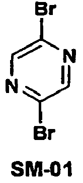 | 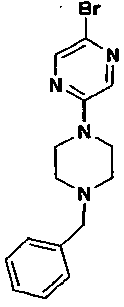 | SM-01 (50 mg), NaH (17 mg), N-benzylpiper azine (25 mg), DMF 5 ml) r.t. | 1.04 (column E) | 333.07 | 332.99 |
| 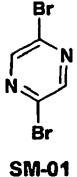 | 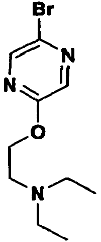 | SM-01 (50 mg), NaH (17 mg), N,N-diethylami no-ethanol (0.033 ml), DMF 5 ml) r.t. | 0.72 (column E) | 214.06 | 273.97 |
| 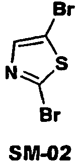 | 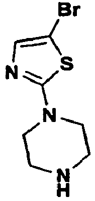 | SM-02 (2 g) Piperazine (10 g), THF (50 ml), r.t. | 0.89 (column E) | 247.99 | 247.97 |
| 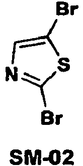 | 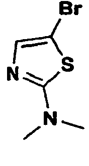 | SM-05 (1 g), Me₂NH (2M in THF, 100 ml), r.t. | 0.65 (column E) | 206.89 | 206.96 |
| 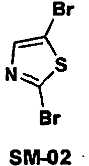 | 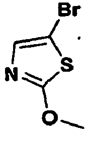 | SM-02 (1 g), MeONa (0.5M in MeOH, 100 ml), r.t. | 1.35 (column E) | 193.93 | 193.84 |
| 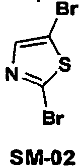 | 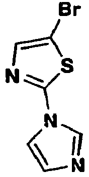 | SM-03 (50 mg), NaH (16 mg), imidazole (77 mg), DMF 5 ml) r.t. | 0.89 (column E) | 229.94 | 229.83 |
| 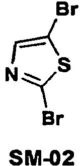 | 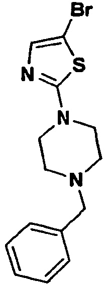 | SM-02 (50 mg), NaH (16 mg), N-benzylpiper azine (30 mg), DMF 5 ml) r.t. | 1.02 (column E) | 338.03 | 337.98 |
| 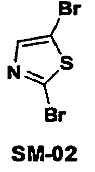 | 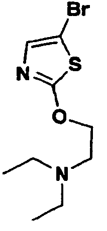 | SM-02 (50 mg), NaH (16 mg), N,N-diethylami no-ethanol (0.033 ml), DMF 5 ml) r.t. SM-03 (50 | 0.83 (column E) | 279.02 | 278.95 |
| 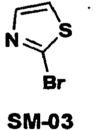 | 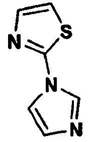 | mg), NaH (25 mg), imidazole (25 mg), DMF 5 ml) r.t. | 0.31 (column E) | 151.91 | 152.03 |
| 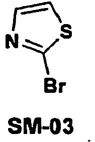 | 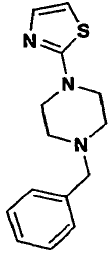 | SM-03 (50 mg), NaH (25 mg), N-benzylpiper azine (37 mg), DMF 5 ml) r.t. | 0.66 (column E) | 260.07 | 260.12 |
| 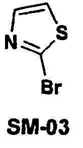 | 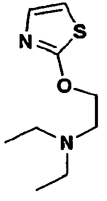 | SM-03 (50 mg), NaH (25 mg), N,N-diethylami no-ethanol (0.05 ml), DMF 5 ml) r.t. | 0.46 (column E) | 201.11 | 201.02 |
| 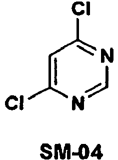 | 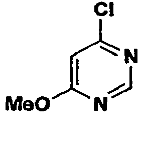 | SM-04 (1g), MeONa (0.5M in MeOH, 13.52 ml), r.t. | 0.86 (column E) | 145.02 | 144.99 |
| 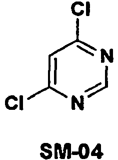 | 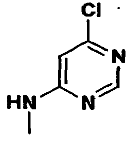 | SM-04 (1g), MeNH₂ (2M in THF, 100ml), r.t. | 0.46 (column E), | 144.03 | 143.96 |
| 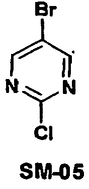 | 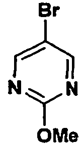 | SM-05 (1g), MeONa (0.5M in MeOH, 100ml), 1 day, r.t. | 0.91 (column E) | 188.97 | 188.91 |
| 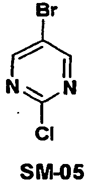 | 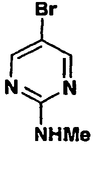 | SM-05 (1g), MeNH₂ (2M in THF, 100 ml), r.t. | 0.84 (column E) | 187.99 | 187.94 |
| 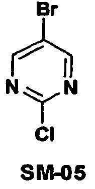 | 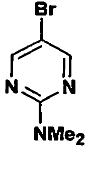 | SM-05 (1g), Me₂NH (2M in THF, 100 ml), r.t. | 1.24 (column E) | 202.00 | 201.98 |

### b. Preparation of 2-bromo-5,6-disubstituted-pyrazine

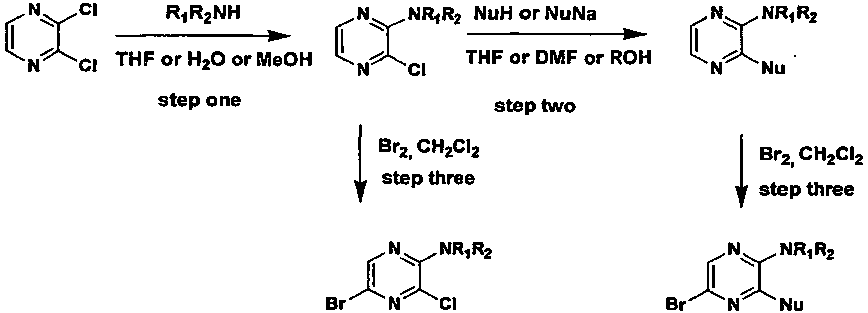

### Step one

To a flask, an appropriate pyrazine (1.0 equivalent) and a nucleophile, such as amine or sodium alkoxide in an exess amount were combined in a solvent such as water or THF or without solvent. The reaction was either stirred at room temperature or under heating for one to three days. After all the solvents were removed, a residue was collected and used in the further steps without any purification.

| **Starting Material** | **Product** | **Redaction Condition** | **Rf (minutes)** | **MS (M+H)+ Cald.** | **MS (M+H)+ Obsv.** |
|---|---|---|---|---|---|
| 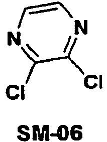 | 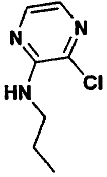 | SM-06 (100 mg), propylamine (2 ml), r.t. | 1.28 (column C) | 172.06 | 172.09 |
| 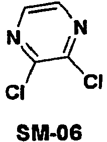 | 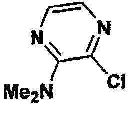 | SM-06 (100 mg), Me₂NH (2M in THF, 10 ml) or Me₂NH (40% in water, 10 ml), r.t. | 1.21 (column C) | 158.05 | 158.07 |
| 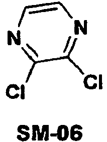 | 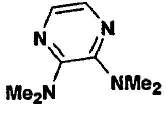 | SM-06 (100 mg), Me2NH (40% in water, 10 ml), 100°C | 0.49 (column C) | 167.13 | 167.19 |
| 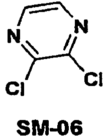 | 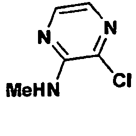 | SM-V6 (100 mg), MeNH₂ (2M in THF, 10 ml), r.t. | 0.72 (column C) | 144.03 | 144.07 |
| 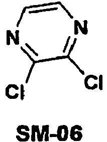 | 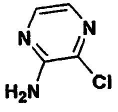 | SM-06 (100 mg), NH₄0H (10 ml), 100°C | 0.41 (column C) | 162.04 (M+Me OH+H)⁺ | 162.06 (M+MeO H+H)⁺ |

### Step two

To a flask, the crude pyrazine derivative obtained from the step one (1.0 equivalent) and a nucleophile, such as amine or sodium alkoxide in an exess amount were combined in a solvent such as water or THF or without solvent. The reaction was either stirred at room temperature or under heating for one to three days. After all the solvents were removed, a residue was collected and used in the further steps without any purification.

| **Starting Material** | **Product** | **Reaction Condition** | **Rf (minutes)** | **MS (M+H)+ Cald.** | **MS (M+H)+ Obsv.** |
|---|---|---|---|---|---|
| 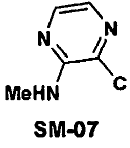 | 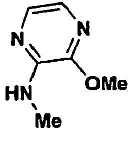 | SM-07 (2 g), MeONa (12.5 wt%, 100ml, 100°C | 0.28 (column C) | 140.08 | 140.14 |
| 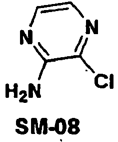 | 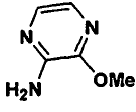 | SM-08 (2 g), MeONa (12.5 wt%, 20ml), 100°C | 0.28 (column C) | 158.13 | 158.09 |
| 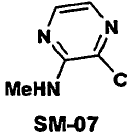 | 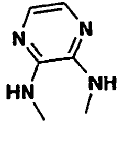 | SM-07 (2 g), MeNH₂ (40% in water, 100 ml), 110°C | 0.34 (column C) | 139.10 | 139.13 |

### Step three

To a flask, the crude pyrazine derivative obtained from the step two (1.0 equivalent) was dissolved in methylene chloride. A slightly excess of bromine was then added into the mixed solution. The reaction was stirred at room temperature for ten hours. After all the solvents were removed, a residue was collected and purified by silica gel chromatography to afford the desired product.

| **Starting Material** | **Product** | **Reaction Condition** | **Rf (minutes)** | **MS (M+H)+ Cald.** | **MS (M+H)+ Obsv.** |
|---|---|---|---|---|---|
| 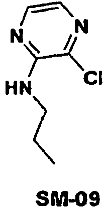 | 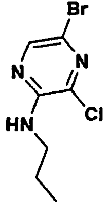 | SM-09 (5 g), bromine (1.34 ml), CH₂Cl₂ (100 ml) | 1.77 (column C) | 249.97 | 250.02 |
| 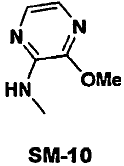 | 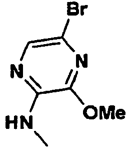 | SM-10 (2 g), bromine (0.72 ml), CH₂Cl₂ (20 ml) | 1.13 (column C) | 217.99 | 217.98 |
| 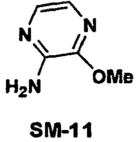 | 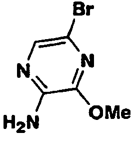 | SM-11 (2 g), bromine (0.72 ml), CH₂Cl₂ (20 ml) | 0.98 (column C) | 203.98 | 203.99 |

### General procedure of the preparation of 2-alkyl-5-bromo-pyrimide:

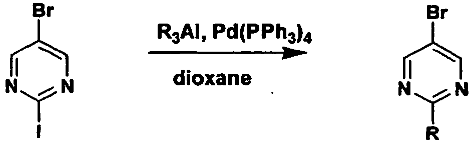

To a sealed tube, 5-bromo-2-iodopyrimidine (1.0 equivalent), tri-alkylalumimun (1.5 equivalent) and tetrakis-triphenylphosphine palladium, Pd(Ph₃P)₄ (1.0 mol%) were combined in dioxane(10 mL). The reaction was heated at 110-120 °C for 10 h. After the mixture cooled down to room temperature, it was poured into water. The solution was extracted with ethyl acetate and the combined extracts were concentrated *in vacuo* to give a residue which was purified by silica gel chromatography to afford the desired 2-alkyl-5-bromopyrimidine product.

| **R3Al** | **Product** | **Rf (minutes)** | **MS (M+H)+ Cald.** | **MS (M+H)+ Obsv.** |
|---|---|---|---|---|
| Me₃Al | 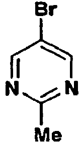 | 0.90 (column E) | 172.94 | 172.97 |
| (1-Hu)₃Al | 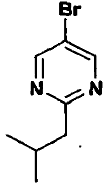 | 1.45 (column E) | 215.02 | 214.99 |

Prep of triazine stannane for Stille coupling to prepare examples of claim 1. (the sulfur can thenbe removed with Raney Nickel to give additional desulfurized triazines) 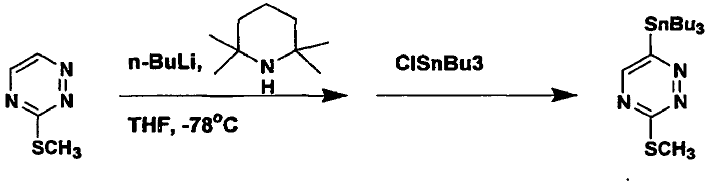 2,2,6,6-tetramethylpiperidine (2.0ml, 11.81mmol) in 30ml of THF was cooled to - 78oC and treated with n-butyllithium (4.7ml), 11.81mmol, 2.5M in hexane). After stirring 30min at 0oC, the reaction was cooled to -78oC again and 3-methylthio-1,2,4-triazine (1.0g, 7.87mmol) was added. The resulting solution was stirred at - 78oC for 30min before tributyltin chloride (2.1ml, 7.87mmol) was added. The reaction was kept at -78oC for 1hr, then quenched with water. The THF solvent was removed on rotarory evaporator and the remaining solution was extracted with ethylacetate. The organic layer was dried over MgSO4, filtered and the filtrate was concentrated. The residue was chromatographed to afford 96mg of 3-methylthio-6-tributyltin-1,2,4-triazine.
1H NMR (300Hz, CHCl3): 8.83 (s, 1H); 2.62 ( s, 3H); 2.04 - 0.79 (m, 27H). LC/MS: (ES+) M/Z (M+H)+ = 418, RT = 2.29min.

### Intermediate 15

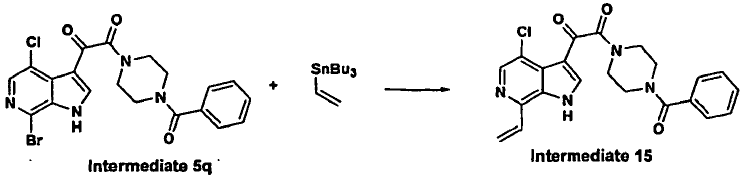

To a mixture of **5q** (50 mg, 105 µmol) and Pd(PPh₃)₄ (25 mg, 21 µmol) was added 1,4-dioxane (1 ml) and **vinyl tributylstannane** (50 mg, 158 µmol). The reaction mixture was heated in a sealed tube at 145°C for 3 hours. After cooling to ambient temperature, the reaction mixture was added MeOH (4 ml) and then filtered. The filtrate was purified by preparative reverse phase HPLC to give the TFA salt of **Intermediate 13** using the method: Start %B = 30, Final %B = 75, Gradient time = 20 min, Flow Rate = 25 ml/min, Column : YMC C18 5um 20 x 100mm, Fraction Collection: 7.92 - 8.58 min. **¹H NMR:** (CD₃OD) δ 8.61 (s, 1H), 8.37 (s, 1H), 7.47 (b s, 5H), 7.31 (dd, *J* = 17.3, 11.3, 1H), 6.50 (d, *J* = 17.3, 1H), 5.97 (d, *J* = 11.3, 1H), 3.97 - 3.38 (b m, 8H); **LC/MS:** (ES+) m/z (M+H)⁺= 423, 425; HPLC Rₜ = 1.887.

### Intermediate 14

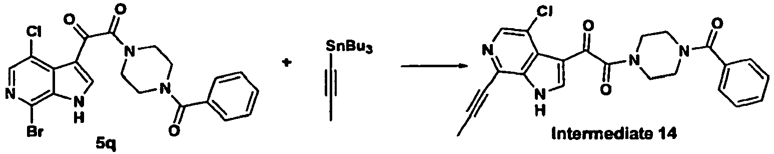

To a mixture of **intermediate 5q** (30 mg, 63 µmol) and Pd(PPh₃)₄ (20 mg, 17 µmol) was added 1,4-dioxane (1 ml) and 1-tributylstannyl propyne (40 mg, 122 µmol). The reaction mixture was heated in a sealed tube at 145°C for 2 hours. After cooling to ambient temperature, the reaction mixture was added MeOH (4 ml) and then filtered. The filtrate was purified by preparative reverse phase HPLC to give the TFA salt of **intermediate 14** (1-(4-Benzoyl-pipemin-1-yl)-2-(4-chloro-7-prop-1-ynyl-1H-pyrrolo[2,3-c]pyridin-3-yl)-ethane-1,2-dione) using the method: Start %B = 20, Final %B = 80, Gradient time = 20 min, Flow Rate = 25 ml/min, Column : YMC C 18 5um 20 x 100mm, Fraction Collection: 8.74 - 9.00 min. **¹H NMR:** (CD₃OD) δ 8.47 (s, 1H), 8.27 (s, 1H), 7.46 (b s, 5H), 3.82 - 3.34 (b m, 8H), 2.26 (s, 3H); **LC/MS:** (ES+) m/z(M+H)⁺=435,437; HPLC Rₜ = 2.123.

### Intermediate 15

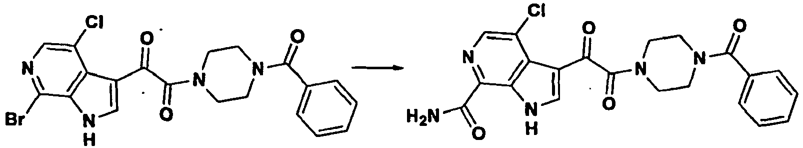

To a solution of intermediate 5q (50 mg, 0.11 mmol) in DMF (1 ml) was added CuCN (30 mg, 0.335 mmol). The reaction mixture was heated at 170°C for 30 min. After cooling to ambient temperature, the reaction mixture was diluted with MeOH (15 ml), filtered under gravity, and the filtrate evaporated *in vacuo* to afforded a brownish residue. To the residue in EtOH (3 ml) at ambient temperature was bubbled hydrogen chloride gas for 10 minutes to give a yellow solution, which was purified by preparative reverse phase HPLC using the method: Start %B = 15, Final %B = 85, Gradient time =15 min, Flow Rate = 40 ml/min, Column : TERRA C18 5 um 30 x 100 mm, Fraction Collection: 10.40 - 10.85 min; **¹H NMR:** (CD₃OD) 8.35 (s, 1H), 8.33 (s, 1H), 7.42 (b s, 5H), 3.95 - 3.41 (b m, 8H); **LC/MS:** (ES+) m/z (M+H)⁺= 440, 442; HPLC Rₜ = 1.820.

### Intermediate 16

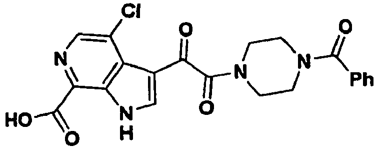

### Preparation of intermediate 16:

To a suspension of **intermediate 15** (6 mg, 13 µmol) in a mixture of AcOH (0.5 ml) and Ac₂O (1.0 ml) at 0°C was charged with sodium nitrite (17 mg, 246 µmol). The reaction mixture was stirred at 0°C for 30 min. and then at ambient temperature for 1 hour. After addition of MeOH (4 ml), the reaction mixture was purified by preparative reverse phase HPLC to give the TFA solvate of the title compound using the method: Start %B = 15, Final %B = 80, Gradient time = 15 min, Flow Rate = 25 ml/min, Column : YMC C18 5um 20 x 100mm, Fraction Collection: 9.48 - 10.03 min. **¹H NMR:** (DMSO-*d₆*) □ 12.76 (s, 1H), 8.48 (s, 1H), 8.32 (d, *J* = 3.0, 1H), 7.44 (b s, 5H), 3.97 - 3.47 (b m, overlapping with water peak, 8H); **LC/MS:** (ES+) m/z (M+H)⁺= 441, 443; HPLC Rₜ = 1.530.
**Ref:** Amide hydrolysis: Evans, D. A.; Carter, P. H.; Dinsmore, C. J.; Barrow, J. C.; Katz, J. L.; Kung, D. W. Tetrahedron Lett. 1997, 38, 4535 and references cited therein.

### Preparation of Compounds of Formula I

### EXAMPLE 1

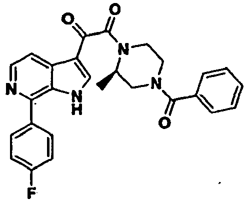

*Typical procedure for coupling azaindole with aromatic boron reagent (An example of the general procedure described below for examples 2-14):* Preparation of 1-benzoyl-3-(R)-methyl-4-[(7-(4-fluorophenyl)-6-azaindol-3-yl)-oxoacetyl]-piperazine is an example of Step E as described in Scheme 15. To a sealed tube, 1-(benzoyl)-3-(R)-methyl-4-[(7-chloro-6-azaindol-3-yl)-oxoacetyl]piperazine, Intermediate 5a, (20 mg, 0.049 mmol), 4-fluorophenylboronic acid, Intermediate 14a-9, (8.2 mg, 0.059 mmol), Pd(Ph₃P)₄ (5 mg) and K₂CO₃ (20 mg, 0.14 mmol) were combined in 1.5 mL of DMF and 1.5 mL of water. The reaction was heated at 110-120 °C for 10 h. After the mixture cooled down to rt, it was poured into 20 mL of water. The solution was extracted with EtOAc (4 x 20 mL). The combined extract was concentrated to give a residue which was purified using a Shimadzu automated preparative HPLC System to give compound 1-benzoyl-3-(R)-methyl-4-[(7-(4-fluorophenyl)-6-azaindol-3-yl)-oxoacetyl]piperazine (1.8 mg, 7.9%). MS *m*/*z*: (M+H)⁺ Calc'd forC₂₇H₂₄FN₄O₃: 471.18; found 471.08. HPLC retention time: 1.12 minutes (column A).

### EXAMPLES 2-14

Examples 2-14 were prepared according to the following general method in a manner analogous to the preparation of Example 1.

*Typical procedure for coupling azaindole with aromatic boron reagent:* To a sealed tube, an appropriately substituted azaindole intermediate (0.049 mmol), an appropriate boronic acid derivative (0.059 mmol), Pd(Ph₃P)₄ (5 mg) and K₂CO₃ (20 mg, 0.14 mmol) were combined in 1.5 mL of DMF and 1.5 mL of water. The reaction was heated at 110-120 °C for 10 h. After the mixture cooled down to rt, it was poured into 20 mL of water. The solution was extracted with EtOAc (4 x 20 mL). The combined extract was concentrated *in vacuo* to give a residue which was purified using a Shimadzu automated preparative HPLC System to provide the desired compound.

### EXAMPLE 2

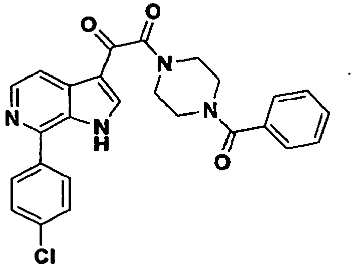

Example 2, was prepared according to the general method described above starting from Intermediate 5g and 4-chlorophenyl boronic acid, Intermediate 14a-10, to provide 1-benzoyl-4-[(7-(4-chlorophenyl)-6-azaindol-3-yl)-oxoacetyl]piperazine. MS *m*/*z*: (M+H)⁺ Calc'd forC₂₇H₂₄FN₄O₃: 473.14; found 473.13. HPLC retention time: 1.43 minutes (column B).

### EXAMPLE 3

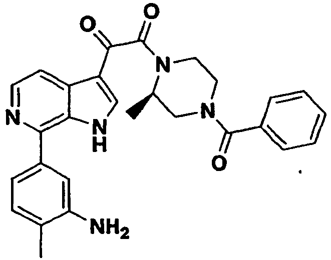

Example 3, was prepared according to the general method described above starting from Intermediate 5a and 3-amino-4-methylphenyl boronic acid, Intermediate 14a-11, to provide 1-benzoyl-3-®-methyl-4-[(7-(3-amino-4-methylphenyl)-6-azaindol-3-yl)-oxoacetyl] piperazine. MS *m*/z: (M+H)⁺ Calc'd forC₂₇H₂₄ClN₄O₃: 482.22; found 482.25. HPLC retention time: 1.35 minutes (column B).

### EXAMPLE 4

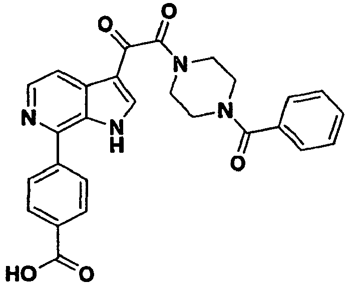

Example 4, was prepared according to the general method described above starting from Intermediate 5g and 4-hydroxycarbonylphenyl boronic acid, Intermediate 14a-12, to provide 1-benzoyl-4-[(7-(4-carboxy-phenyl)-6-azaindol-3-yl)-oxoacetyl]piperazine. MS *m*/z: (M+H)⁺ Calc'd forC₂₇H₂₄ClN₄O₃: 483.17; found 483.10. HPLC retention time: 1.00 minutes (column A).

### EXAMPLE 5

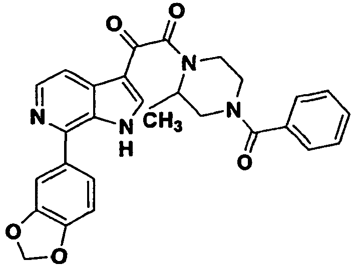

Example 5, was prepared according to the general method described above from 1-benzoyl-3-methyl-4-[(7-chloro-6-azaindol-3-yl)-oxoacetyl]piperazine and 3,4-methylenedioxyphenyl boronic acid, Intermediate 14a-13, to provide 1-benzoyl-3-methyl-4-[(7-(3,4-methylenedioxyphenyl)-6-azaindol-3-yl)-oxoacetyl]piperazine. MS *m*/z: (M+H)⁺ Calc'd forC₂₈H₂₅N₄O₅: 497.18; found 497.03. HPLC retention time: 1.41 minutes (column B).

### EXAMPLE 6

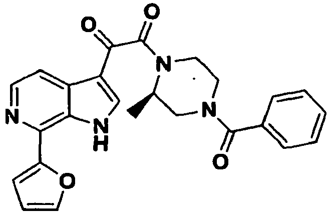

Example 6, was prepared according to the general method described above starting from Intermediate 5a and furan-2-yl boronic acid to provide 1-benzoyl-3-®-methyl-4-[(7-(furan-2-yl)-6-azaindol-3-yl)-oxoacetyl]piperazine; MS *m*/z: (M+H)⁺ Calc'd forC₂₅H₂₃N₄O₄: 443.17; found 443.12. HPLC retention time: 1.20 minutes (column A).

### EXAMPLE 7

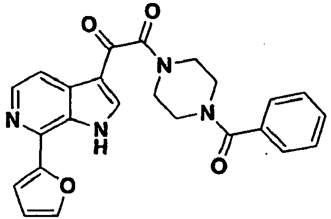

Example 7, was prepared according to the general method described above starting from Intermediate 5g and furan-2-yl boronic acid to provide 1-benzoyl-4-[(7-(furan-2-yl)-6-azaindol-3-yl)-oxoacetyl] piperazine MS *m*/z: (M+H)⁺ Calc'd forC₂₄H₂₁N₄O₄: 429.16; found 428.98. HPLC retention time: 1.36 minutes (column A).

### EXAMPLE 8

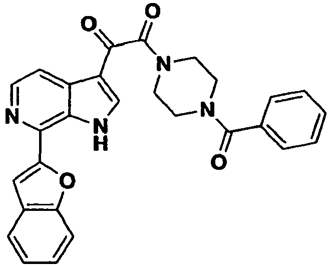

Example 8, was prepared according to the general method described above starting from Intermediate 5g and benzofuran-2-yl boronic acid to provide 1-benzoyl-4-[(7-(benzofuran-2-yl)-6-azaindol-3-yl)-oxoacetyl]piperazine MS *m*/z: (M+H)⁺ Calc'd forC₂₈H₂₃N₄O₄: 479.17; found 479.09. HPLC retention time: 1.67 minutes (column B).

### EXAMPLE 9

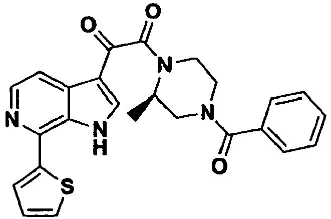

Example 9, was prepared according to the general method described above starting from Intermediate 5a and thien-2-yl boronic acid to provide 1-(benzoyl)-3-®-methyl-4-[(7-(thien-2-yl)-6-azaindol-3-yl)-oxoacetyl)piperazine MS *m*/z: (M+H)⁺ Calc'd forC₂₅H₂₃N₄O₃S: 459.15; found 459.10. HPLC retention time: 1.20 minutes (column A).

### EXAMPLE 10

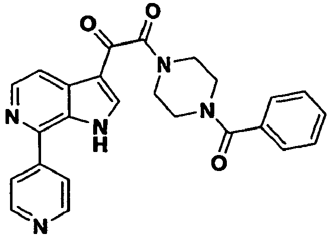

Example 10, was prepared according to the general method described above starting from Intermediate 5g and pyridin-4-yl boronic acid to provide 1-(benzoyl)-4-[(7-(pyridin-4-yl)-6-azaindol-3-ylroxoacetyl]piperazine MS *m*/z: (M+H)⁺ Calc'd forC₂₅H₂₂N₅O₃: 440.17; found 440.10. HPLC retention time: 0.97 minutes (column A).

### EXAMPLE 11

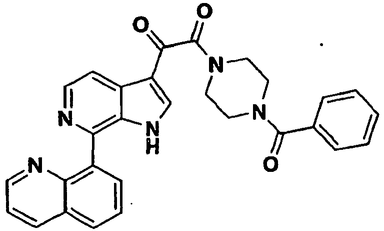

Example 11, was prepared according to the general method described above starting from Intermediate 5g and quinolin-8-yl boronic acid, Intermediate 14a-14, to provide 1-benzoyl-4-[(7-(quinolin-8-yl)-6-azaindol-3-yl)-oxoacetyl]piperazine MS *m*/z: (M+H)⁺ Calc'd forC₂₅H₂₂N₅O₃: 490.19; found 490.09. HPLC retention time: 1.34 minutes (column B).

### EXAMPLE 12

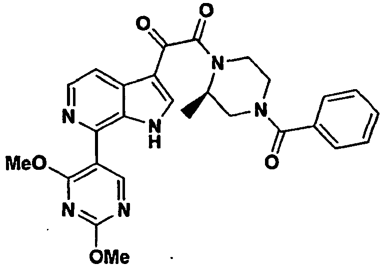

Example 12, was prepared according to the general method described above starting from Intermediate 5a and 2,4-dimethoxypyrimidin-5-yl boronic acid, Intermediate 14a-4, to provide 1-benzoyl-3-®-methyl-4-[(7-(2,4-dimethoxy-pyrimidin-5-yl)-6-azaindol-3-yl)-oxoacetyl]piperazine MS *m*/z: (M+H)⁺ Calc'd for C₂₇H₂₇N₆O₅: 515.20; found 515.28. HPLC retention time: 1.17 minutes (column B).

### EXAMPLE 13

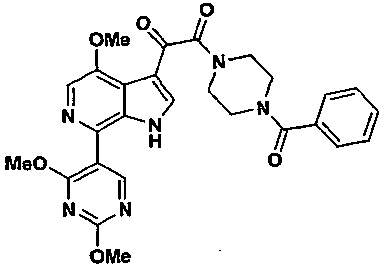

Example 13, was prepared according to the general method described above starting from Intermediate 5b and 2,4-dimethoxypyrimidin-5-yl boronic acid, Intermediate 14a-4, to provide 1-benzoyl-4-[(4-methoxy-7-(2,4-dimethoxy-pyrimidin-5-yl)-6-azaindol-3-yl)-oxoacetyl]piperazine ¹H NMR (500 MHz, CD₃OD) δ 8.71 (s, 1H), 8.64 (s, 1H), 8.21 (s, 1H), 7.48 (s, 5H), 4.15 (s, 3H), 4.13 (s, 3H), 3.84 (s, 3H), 3.64 - 3.34 (m, 8H). MS *m*/z: (M+H)⁺ Calc'd for C₂₉H₃₅N₆O₆: 531.20; found 531.26. HPLC retention time: 1.09 minutes (column A).

### EXAMPLE 14

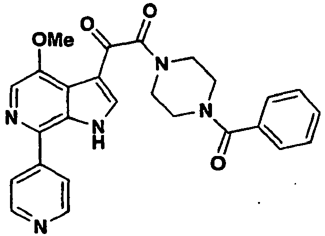

Example 14, was prepared according to the general method described above starting from Intermediate 5b and pyridin-4-yl boronic acid to provide 1-benzoyl-4-[(4-methoxy-7-(pyridin-4-yl)-6-azaindol-3-yl)-oxoacetyl]piperazine MS *m*/z: (M+H)⁺ Calc'd for C₂₆H₂₄N₅O₄: 470.18; found 470.32. HPLC retention time: 1.02 minutes (column A).

### EXAMPLE 15

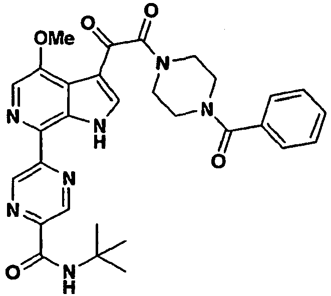

*Typical procedure for coupling azaindole with aromatic tin reagent (An example of the general procedure described below for examples 16-53):* Preparation of Example 15, 1-benzoyl-4-[(4-methoxy-7-(2-(1,1-dimethylethylaminocarbonyl)-pyrazin-5-yl)-6-azaindol-3-yl)-oxoacetyl]piperazine is an example of Step E as described in Scheme 15. To a sealed tube, 1-benzoyl-4-[(7-chloro-4-methoxy-6-azaindol-3-yl)-oxoacetyl]piperazine, Intermediate 5b, (20 mg), 2-(1,1-dimethylethylaminocarbonyl)-5-tributylstannyl-pyrazine (1.2 equivalents, 27 mg.) and Pd(Ph₃P)₄ (1 mg) were combined in 1.5 mL of dioxane. The reaction was heated at 110-120 °C for 10 h. After the mixture cooled down to room temperature, it was poured into 5 mL of water. The solution was extracted with EtOAc (4 x 5 mL). The combined extract was concentrated *in vacuo* to give a residue which was purified using a Shimadzu automated preparative HPLC System to give compound 1-benzoyl-4-[(4-methoxy-7-(2-(1,1-dimethylethylaminocarbonyl)-pyrazin-5-yl)-6-azaindol-3-yl)-oxoacetyl]piperazine (1 mg); MS *m*/z: (M+H)⁺ Calc'd for C₃₀H₃₂N₇O₅: 570.25; found 570.43. HPLC retention time: 1.83 minutes (column B).

### EXAMPLES 16-54

Examples 16-54 were prepared according to the following general procedure by a method analogous to the method described for the preparation of Example 15.

*Typical procedure for coupling azaindole with aromatic tin reagent:* To a sealed tube, an appropriate azaindole (0.049 mmol), an appropriate stannane (0.059 mmol) and Pd(Ph₃P)₄ (1 mg) were combined in 1.5 mL of dioxane. The reaction was heated at 110-120 °C for 10 h. After the mixture cooled down to rt, it was poured into 5 mL of water. The solution was extracted with EtOAc (4 x 5 mL). The combined extract was concentrated to give a residue which was purified using a Shimadzu automated preparative HPLC System to provide the desired compound.

### EXAMPLE 16

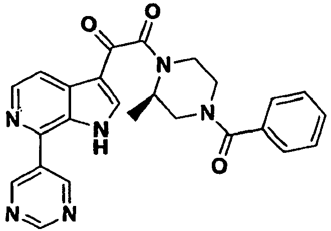

Example 16, was prepared according to the general method described above starting from Intermediate 5a and pyrimidin-5-yl tributyltin, Intermediate 14-9, to provide 1-benzoyl-3-(R)-methyl-4-[(7-(pyrimidin-5-yl)-6-azaindol-3-yl)-oxoacetyl]piperazine; MS *m*/z: (M+H)⁺ Calc'd forC₂₅H₂₃N₆O₃: 455.18; found 455.17. HPLC retention time: 1.33 minutes (column B).

### EXAMPLE 17

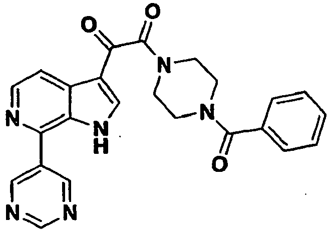

Example 17, was prepared according to the general method described above starting from Intermediate 5g and pyrimidin-5-yl tributyltin, Intermediate 14-9, to provide 1-benzoyl-4-[(7-(pyrimidin-5-yl)-6-azaindol-3-yl)-oxoacetyl]piperazine MS *m*/*z*: (M+H)⁺ Calc'd forC₂₅H₂₃N₆O₃: 441.17; found 441.07. HPLC retention time: 1.30 minutes (column B).

### EXAMPLE 18

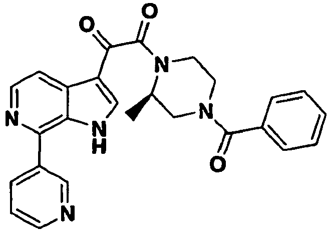

Example 18, was prepared according to the general method described above starting from Intermediate 5a and pyridin-3-yl tributyltin, Intermediate 14a-2, to provide 1-benzoyl-3-(R)-methyl-4-[(7-(pyridin-3-yl)-6-azaindol-3-yl)-oxoacetyl]piperazine MS *m*/z: (M+H)⁺ Calc'd forC₂₆H₂₄N₅O₃: 454.19; found 454.17. HPLC retention time: 1.11 minutes (column A).

### EXAMPLE 19

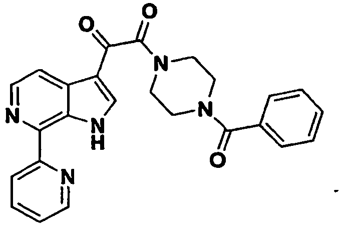

Example 19, was prepared according to the general method described above starting from Intermediate 5g and pyridin-2-yl tributyltin, Intermediate 14a-19, to provide 1-benzoyl-4-[(7-(pyridin-2-yl)-6-azaindol-3-yl)-oxoacetyl]piperazine MS *m*/z: (M+H)⁺ Calc'd forC₂₅H₂₂N₅O₃: 440.17; found 440.07. HPLC retention time: 1.40 minutes (column B).

### EXAMPLE 20

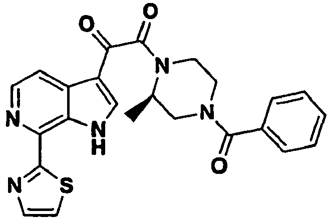

Example 20, was prepared according to the general method described above starting from Intermediate 5a and thiazol-2-yl tributyltin, Intermediate 14a-21, to provide 1-benzoyl-3-(R)-methyl-4-[(7-(thiazol-2-yl)-6-azaindol-3-yl)oxoacetyl]piperazine; MS *m*/z: (M+H)⁺ Calc'd forC₂₄H₂₂N₅O₃S: 460.14; found 460.15. HPLC retention time: 1.48 minutes (column B).

### EXAMPLE 21

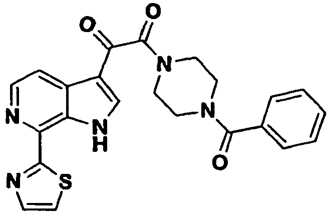

Example 21, was prepared according to the general method described above starting from Intermediate 5g and thiazol-2-yl tributyltin, Intermediate 14a-21, to provide 1-benzoyl-4-[(7-(thiazol-2-yl)-6-azaindol-3-yl)-oxoacetyl]piperazine; MS *m*/z: (M+H)⁺ Calc'd forC₂₃H₂₀N₅O₃S: 446.13; found 446.03. HPLC retention time: 1.44 minutes (column B).

### EXAMPLE 22

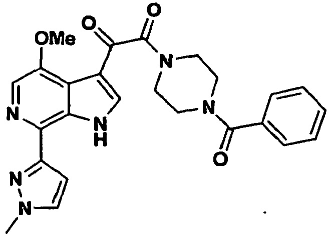

Example 22, was prepared according to the general method described above starting from Intermediate 5b and 1-methylpyrazol-3-yl tributyltin, to provide 1-benzoyl-4-[(4-methoxy-7-(1-methyl-pyrazol-3-yl)-6-azaindol-3-yl)-oxoacetyl]piperazine; MS *m*/z: (M+H)⁺ Calc'd for C₂₅H₂₅N₆O₄: 473.19; found 473.28. HPLC retention time: 1.18 minutes (column B).

### EXAMPLE 23

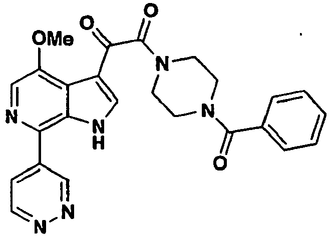

Example 23, was prepared according to the general method described above starting from Intermediate 5b and Intermeidiate 14-9 to provide 1-benzoyl-4-[(4-methoxy-7-(pyridazin-4-yl)-6-azaindol-3-yl)-oxoacetyl]piperazine; MS *m*/z: (M+H)⁺ Calc'd for C₂₅H₂₃N₆O₄: 471.18; found 471.26. HPLC retention time: 1.20 minutes (column B).

### EXAMPLE 24

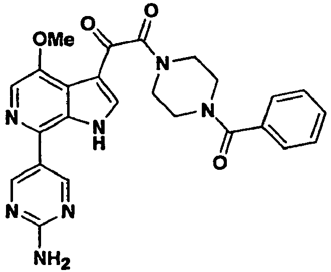

Example 24, was prepared according to the general method described above starting from Intermediate 5b and 2-aminopyrimidin-5-yl tributyltin, to provide 1-benzoyl-4-[(4-methoxy-7-(2-amino-pyrimidin-5-yl))-6-azaindol-3-yl)-oxoacetyl]piperazine MS *m*/z: (M+H)⁺ Calc'd for for C₂₃H₂₄N₇O₄: 486.19: found 486.24. HPLC retention time: 1.19 minutes (column A).

### EXAMPLE 25

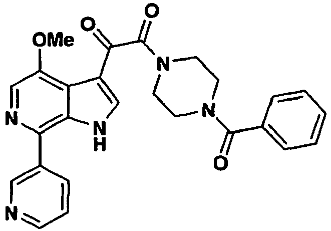

Example 25, was prepared according to the general method described above starting from Intermediate 5b and pyridin-3-yl tributyltin, Intermediate 14a-2, to provide 1-benzoyl-4-[(4-methoxy-7-(pyridin-3-yl)-6-azaindol-3-yl)-oxoacetyl]piperazine; MS *m*/z: (M+H)⁺ Calc'd for C₂₆H₂₄N₅O₄: 470.18; found 470.19. HPLC retention time: 1.04 minutes (column A).

### EXAMPLE 26

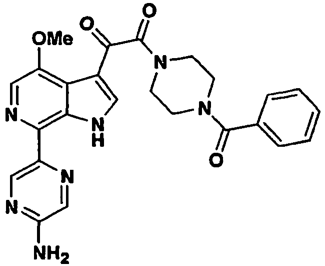

Example 26, was prepared according to the general method described above starting from Intermediate 5b and 2-aminopyrazin-5-yl trimethyltin, Intermediate 14-28, to provide 1-benzoyl-4-[(4-methoxy-7-(2-amino-pyrazin-5-yl))-6-azaindol-3-yl)-oxoacetyl]piperazine; MS *m*/z: (M+H)⁺ Calc'd for C₂₅H₂₄N₇O₄: 486.19; found 470.19. HPLC retention time: 1.13 minutes (column B).

### EXAMPLE 27

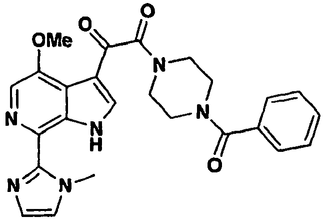

Example 27, was prepared according to the general method described above starting from Intermediate 5b and 1-methylimidazol-2-yl trimethyltin, Intermediate 14-5, to provide 1-benzoyl-4-[(4-methoxy-7-(1-methyl-imidazol-2-yl)-6-azaindol-3-yl)-oxoacetyl]piperazine; MS *m*/z: (M+H)⁺ Calc'd for C₂₅H₂₅N₆O₄: 473.18; found 473.27. HPLC retention time: 1.07 minutes (column B).

### EXAMPLE 28

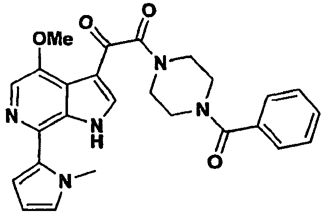

Example 28, was prepared according to the general method described above starting from Intermediate 5b and 1-methylpyrrol-2-yl tributyltin, Intermediate 14a-15, to provide 1-benzoyl-4-[(4-methoxy-7-(1-methyl-pyrrol-2-yl)-6-azaindol-3-yl)-oxoacetyl]piperazine; MS *m*/z: (M+H)⁺ Calc'd for C₂₆H₂₆N₅O₄: 472.20; found 470.26. HPLC retention time: 1.11 minutes (column A).

### EXAMPLE 29

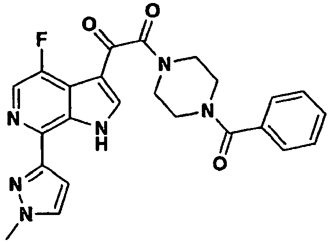

Example 29, was prepared according to the general method described above starting from Intermediate 5i and 1-methylpyrazol-3-yl tributyltin, to provide 1-benzoyl-4-[(4-fluoro-7-(1-methyl-pyrazol-3-yl)-6-azaindol-3-yl)-oxoacetyl]piperazine; MS *m*/z: (M+H)⁺ Calc'd for C₂₄H₂₂FN₆O₃: 461.17; found 461.24. HPLC retention time: 1.36 minutes (column A).

### EXAMPLE 30

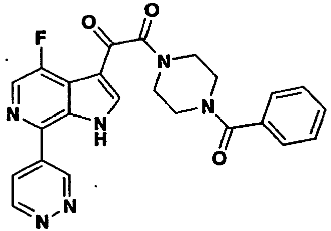

Example 30, was prepared according to the general method described above starting from Intermediate 5i and pyridazin-4-yl tributyltin, Intermediate 14-8, to provide 1-benzoyl-4-[(4-fluoro-7-(pyridazin-4-yl)-6-azaindol-3-yl)-oxoacetyl]piperazine ¹H NMR (500 MHz, CD₃OD) δ 9.72 (s, 1H), 9.39 (s, 1H), 8.42 (m, 2H), 8.22 (s, 1H), 7.47 (s, 5H), 3.84 - 3.38 (m, 8H). MS *m*/z: (M+H)⁺ Calc'd for C₂₄H₂₀FN₆O₃: 459.16; found 459.25. HPLC retention time: 1.26 minutes (column A).

### EXAMPLE 32

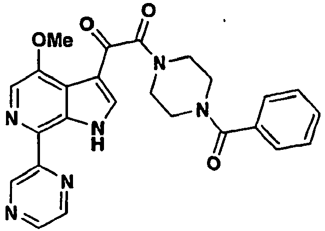

Example 32, was prepared according to the general method described above starting from Intermediate 5b and pyrazin-2-yl tributyltin, Intermediate 14a-1, to provide 1-benzoyl-4-[(4-methoxy-7-(pyrazin-2-yl)-6-azaindol-3-yl)-oxoacetyl]piperazine; MS *m*/z: (M+H)⁺ Calc'd for C₂₅H₂₃N₆O₃: 471.18; found 471.17. HPLC retention time: 1.35 minutes (column A).

### EXAMPLE 33

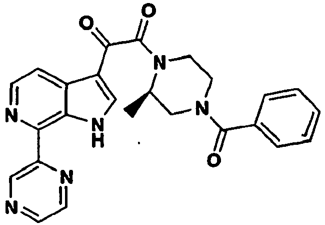

Example 33, was prepared according to the general method described above starting from Intermediate 5a and pyrazin-2-yl tributyltin, Intermediate 14a-1, to provide 1-benzoyl-3-(R)-methyl-4-[(7-(pyrazin-2-yl)-6-azaindol-3-yl)-oxoacetyl]piperazine; MS *m*/z: (M+H)⁺ Calc'd for C₂₅H₂₃N₆O₃: 455.18; found 455.26. HPLC retention time: 1.46 minutes (column A).

### EXAMPLE 34

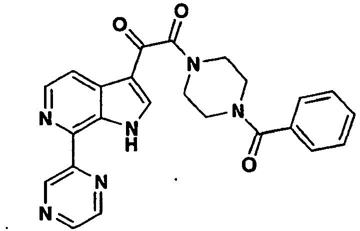

Example 34, was prepared according to the general method described above starting from Intermediate 5g and pyrazin-2-yl tributyltin, Intermediate 14a-1, to provide 1-benzoyl-4-[(7-(pyrazin-2-yl)-6-azaindol-3-yl)-oxoacetyl]piperazine; MS *m*/z: (M+H)⁺ Calc'd for C₂₄H₂₁N₆O₃: 441.17; found 441.22. HPLC retention time: 1.22 minutes (column A).

### EXAMPLE 35

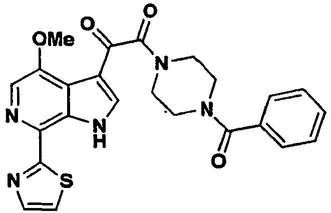

Example 35, was prepared according to the general method described above starting from Intermediate 5b and thiazol-2-yl tributyltin, Intermediate 14a-21, to provide 1-(benzoyl)-4-[(4-methoxy-7-(thiazol-2-yl)-6-azaindol-3-yl)-oxoacetyl]piperazine; MS *m*/z: (M+H)⁺ Calc'd for C₂₄H₂₂N₅O₃S: 476.14; found 476.20. HPLC retention time: 1.25 minutes (column B).

### EXAMPLE 36

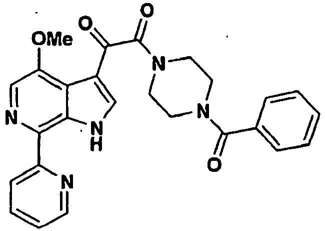

Example 36, was prepared according to the general method described above starting from Intermediate 5b and pyridin-2-yl tributyltin, Intermediate 14a-19, to provide 1-benzoyl-4-[(4-methoxy-7-(pyridin-2-yl)-6-azaindol-3-yl)-oxoacetyl]piperazine; MS *m*/z: (M+H)⁺ Calc'd for C₂₆H₂₄N₅O₄: 470.18; found 470.17. HPLC retention time: 1.04 minutes (column A).

### EXAMPLE 37

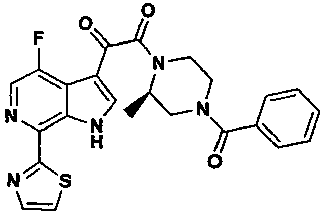

Example 37, was prepared according to the general method described above starting from Intermediate 5j and thiazol-2-yl tributyltin, Intermediate 14a-21, to provide 1-benzoyl-3-(R)-methyl-4-[(4-fluoro-7-(thiazol-2-yl)-6-azaindol-3-yl)-oxoacetyl]piperazine; MS *m*/z: (M+H)⁺ Calc'd for C₂₄H₂₁FN₅O₃S, 478.13; found 478.13. HPLC retention time: 1.34 minutes (column A).

### EXAMPLE 38

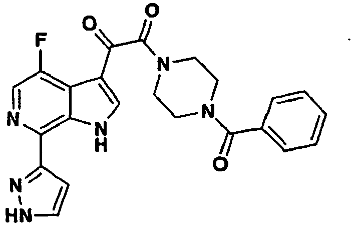

Example 38, was prepared according to the general method described above starting from Intermediate 5i and pyrazol-3-yl tributyltin, to provide 1-benzoyl-4-[(4-fluoro-7-(pyrazol-3 yl)-6-azaindol-3-yl)-oxoacetyl]piperazine MS *m*/z: (M+H)⁺ Calc'd for C₂₃H₂₀FN₆O₃: 447.16; found 447.15. HPLC retention time: 1.26 minutes (column A).

### EXAMPLE 39

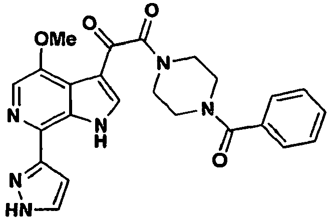

Example 39, was prepared according to the general method described above starting from Intermediate 5b and pyrazol-3-yl tributyltin, to provide 1-benzoyl-4-[(4-methoxy-7-(pyrazol-3-yl)-6-azaindol-3-yl)-oxoacetyl]piperazine; MS *m*/z: (M+H)⁺ Calc'd for C₂₄H₂₃N₆O₄: 459.18; found 459.21. HPLC retention time: 1.11 minutes (column A).

### EXAMPLE 40

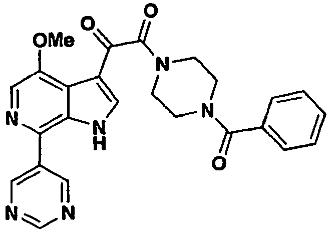

Example 40, was prepared according to the general method described above starting from Intermediate 5b and pyrimidin-5-yl tributyltin, Intermediate 14-9, to provide 1-benzoyl-4-[(4-methoxy-7-(pyrimidin-5-yl)-6-azaindol-3-yl)-oxoacetyl]piperazine MS *m*/z: (M+H)⁺ Calc'd for C₂₅H₂₃N₆O₄: 471.18; found 471.20. HPLC retention time: 1.61 minutes (column A).

### EXAMPLE 41

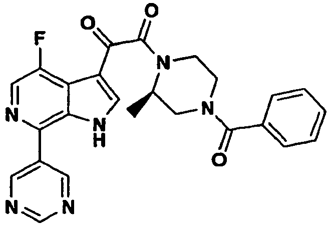

Example 41, was prepared according to the general method described above starting from Intermediate 5j and pyrimidin-5-yl tributyltin, Intermediate 14-9, to provide 1-benzoyl-3-(R)-methyl-4-[(4-fluoro-7-(pyrimidin-5-yl)-6-azaindol-3-yl)-oxoacetyl]piperazine; H NMR (500 MHz, CD₃OD) δ 9.26 (m, 3H), 8.39 (m, 2H), 7.56 (m, 5H), 4.72 - 3.12 (m, 7H), 1.40 - 0.91 (m, 3H). MS *m*/z: (M+H)⁺ Calc'd for C₂₅H₂₂FN₆O₃: 473.17; found 473.17. HPLC retention time: 1.34 minutes (column A).

### EXAMPLE 42

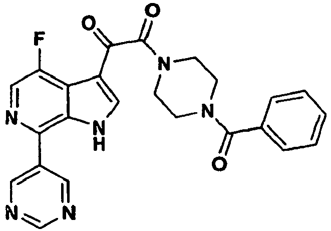

Example 42, was prepared according to the general method described above starting from Intermediate 5i and pyrimidin-5-yl tributyltin, Intermediate 14-9, to provide 1-benzoyl-4-[(4-fluoro-7-(pyrimidin-5-yl)-6-azaindol-3-yl)-oxoacetyl]piperazine; MS *m*/z: (M+H)⁺ Calc'd for C₂₄H₂₀FN₆O₃: 459.16; found 459.14. HPLC retention time: 1.28 minutes (column A).

### Example 43

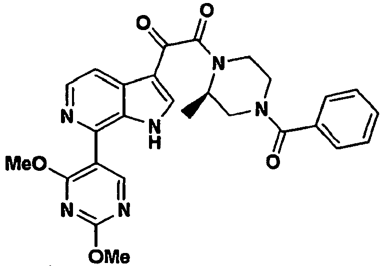

Example 43, (R)-1-(benzoyl)-3-methyl-4-[(7-(2,4-dimethoxy-pyrimidin-5-yl)-6-azaindol-3-yl)-oxoacetyl]piperazine MS *m*/z: (M+H)⁺ Calc'd for C₂₇H₂₇N₆O₅: 515.20; found 515.28. HPLC retention time: 1.17 minutes (column B).

### EXAMPLE 44

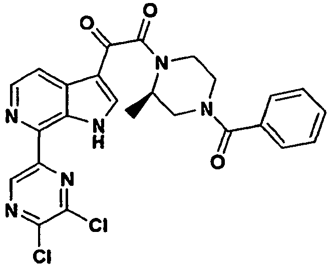

Example 44, was prepared according to the general method described above starting from Intermediate 5a and 2,3-dichloropyrazin-5-yl tributyltin, Intermediate 14-66, to provide 1-benzoyl-3-(R)-methyl-4-[(7-(2,3-dichloro-pyrazin-5-yl) -6-azaindol-3-yl)-oxoacetyl]piperazine; MS *m*/z: (M+Na)⁺ Calc'd for C₂₅H₂₀Cl₂NaN₆O₃: 545.09; found 545.29. PLC retention time: 1.87 minutes (column B).

### EXAMPLE 45

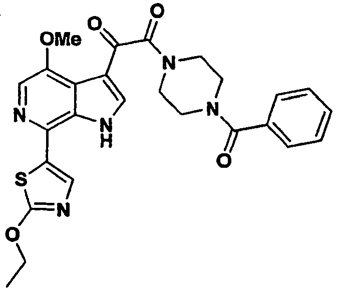

Example 45, was prepared according to the general method described above starting from Intermediate 5b and 2-ethoxythiazol-5-yl tributyltin, Intermediate 14-71, to provide 1-benzoyl-4-[(4-methoxy-7-(2-ethoxy-thiazol-5-yl)-6-azaindol-3-yl)-oxoacetyl]piperazine; MS *m*/z: (M+H)⁺ Calc'd for C₂₆H₂₆N₅O₅S: 520.17; found 520.24. HPLC retention time: 1.32 minutes (column A).

### EXAMPLE 46

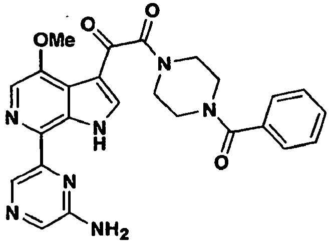

Example 46, was prepared according to the general method described above starting from Intermediate 5b and the 2-amino-pyrazin-6-yl stannane, Intermediate 14-68, to provide 1-benzoyl-4-[(4-methoxy-7-(2-amino-pyrazin-6-yl)-6-azaindol-3-yl)-oxoacetyl]piperazine; MS *m*/z: (M+H)⁺ Calc'd for C₂₅H₂₄N₇O₄: 486.19; found 486.31. HPLC retention time: 1.22 minutes (column B).

### EXAMPLE 47

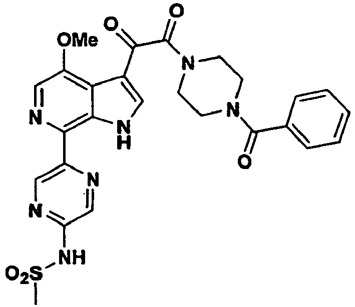

Example 47, was prepared according to the general method described above starting from Intermediate 5b and 2-methylsulfonylamino-5-(tri-n-butylstannyl)pyrazine, Intermediate 14-69, to provide 1-benzoyl-4-[(7-(2-methylsulfonylamino-pyrazin-5-yl)-6-azaindol-3-yl)-oxoacetyl)piperazine MS *m*/z: (M+H)⁺ Calc'd for C₂₆H₂₆N₇O₆S: 564.17; found 564.21. HPLC retention time: 1.24 minutes (column A).

### EXAMPLE 48

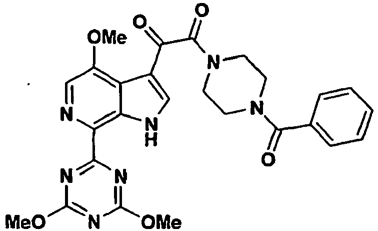

Example 48, was prepared according to the general method described above starting from Intermediate 5b and 2,4-dimethoxy-1,3,5-triazin-6-yl tributyltin, Intermediate 14-70, to provide 1-benzoyl-4-[(7-(2,4-dimethoxy-1,3,5-triazin-6-yl)-6-azaindol-3-yl)-oxoacetyl]piperazine; MS *m*/z: (M+H)⁺ Calc'd for C₂₆H₂₆N₇O₆: 532.19; found 532.12. HPLC retention time: 1.28 minutes (column A).

### EXAMPLE 49

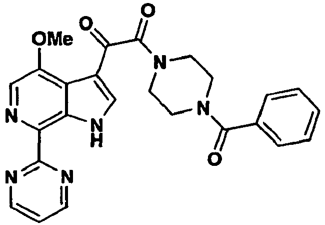

Example 49, was prepared according to the general method described above starting from Intermediate 5b and pyrimidin-2-yl tributyltin, Intermediate 14-67, to provide 1-benzoyl-4-[(4-methoxy-7-(pyrimidin-2-yl)-6-azaindol-3-yl)-oxoacetyl]piperazine; MS *m*/z: (M+H)⁺ Calc'd for C₂₅H₂₃N₆O₄: 471.18; found 471.29. HPLC retention time: 1.21 minutes (column A).

### EXAMPLE 50

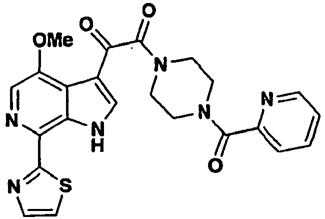

Example 50, was prepared from1-(pyridin-2-yl)-4-[(4-methoxy-7-chloro-6-azaindol-3-yl)-oxoacetyl]piperazine and thiazol-2-yl tributyltin, Intermediate 14a-21, according to the general method above to provide 1-(pyridin-2-yl)-4-[(4-methoxy-7-(thiazol-2-yl)-6-azaindol-3-yl)-oxoacetyl]piperazine MS *m*/z: (M+H)⁺ Calc'd for C₂₄H₂₅N₆O₄S: 477.13; found 477.22. HPLC retention time: 0.98 minutes (column A).

### EXAMPLE 51

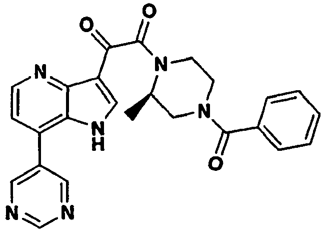

Example 51, was prepared according to the general method described above starting from Intermediate 5d and pyrimidin-5-yl tributyltin, Intermediate 14-9, to provide 1-benzoyl-3-(R)-methyl-4-[(7-(pyrimidin-5-yl)-4-azaindol-3-yl)-oxoacetyl]piperazine; MS *m*/z: (M+H)⁺ Calc'd for C₂₅H₂₃N₆O₃: 455.18; found 455.16. HPLC retention time: 0.98 minutes (column A).

### EXAMPLE 52

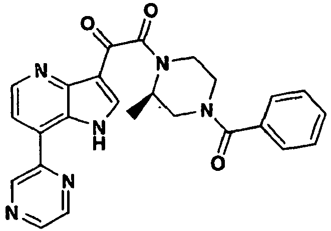

Example 52, was prepared according to the general method described above starting from Intermediate 5d and pyrimidin-2-yl tributyltin, Intermediate 14a-1, to provide 1-benzoyl-3-(R)-methyl-4-[(7-(pyrazin-2-yl)-4-azaindol-3-yl)-oxoacetyl]piperazine; MS m/z: (M+H)⁺ Calc'd for C₂₅H₂₃N₆O₃: 455.18; found 455.16. HPLC retention time: 1.09 minutes (column A).

### EXAMPLE 53

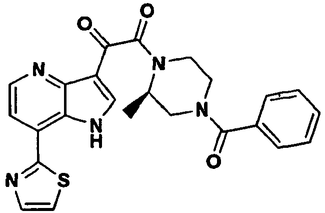

Example 53, was prepared according to the general method described above starting from Intermediate 5d and thiazol-2-yl tributyltin, Intermediate 14a-21, to provide 1-benzoyl-3-(R)-methyl-4-[(7-(thiazol-2-yl)-4-azaindol-3-yl)-oxoacetyl]piperazine; MS *m*/*z:* (M+H)⁺ Calc'd for C₂₄H₂₂N₅O₃S: 460.14; found 460.26. HPLC retention time: 1.02 minutes (column A).

### EXAMPLE 54

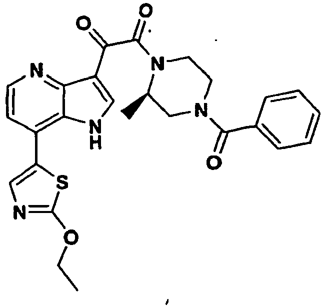

Example 54, was prepared according to the general method described above starting from Intermediate 5d and 2-ethoxythiazol-5-yl tributyltin, Intermediate 14-71, to provide 1-benzoyl-3-(R)-methyl-4-[(7-(2-ethoxy-thiazol-5-yl)-4-azaindol-3-yl)-oxoacetyl]piperazine; MS *m*/*z*: (M+H)⁺ Calc'd for C₂₆H₂₆NO₄S: 504.17; found 4504.18. HPLC retention time: 1.26 minutes (column A).

### EXAMPLE 55

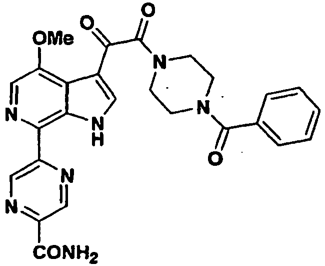

The compound of Example 15, 1-benzoyl-4-[(4-methoxy-7-(2-(1,1-dimethylethylaminocarbonyl)-pyrazin-5-yl)-6-azaindol-3-yl)-oxoacetyl]piperazine (20 mg) was dissolved in 1 drop of concentrated sulfuric acid. After 30 minutes, the mixture was diluted with 2 mL of methanol. The resulting solution was injected into a Shimadzu automated preparative HPLC System and the HPLC purification afforded the compound of Example 55, 1-benzoyl-4-[(4-methoxy-7-(2-aminocarbonyl-pyrazin-5-yl)-6-azaindol-3-yl)-oxoacetyl]piperazine (1 mg); MS *m*/*z*: (M+H)⁺ Calc'd for C₂₆H₂₄N₇O₅: 514.78; found 514.22. HPLC retention time: 1.44 minutes (column B).

### EXAMPLE 56

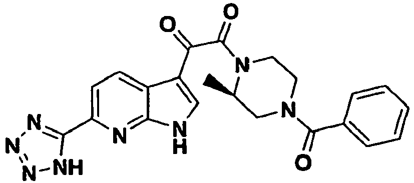

An excess of NH₄Cl (27mg) was added into a solution of 1-(benzoyl)-3-(R)-methyl-4-[(6-cyano-7-azaindol-3-yl)-oxoacetyl]piperazine (20 mg) and NaN₃ (16 mg) in DMF. The reaction was heated to reflux for 12 h. After cooling down, the mixture was concentrated under reduced pressure and the residue was purified using Shimadzu automated preparative HPLC System to give 1-benzoyl-3-(R)-methyl-4-[(6-(tetrazol-l-yl)-7-azaindol-3-yl)-oxoacetyl]piperazine (6.3mg). MS *m*/*z*: (M+H)⁺ Calc'd for C₂₂H₂₁,N₈O₃: 445.17; Found 3445.16. HPLC retention time: 1.42 minutes (column B); Column B: PHX-LUNA C18 4.6 x 30 mm.

### EXAMPLE 57

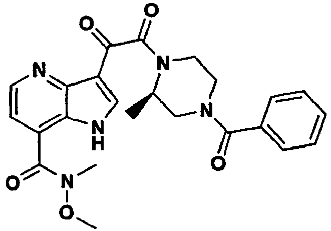

Preparation of 1-benzoyl-3-(R)-methyl-4-[(7-(methoxymethylamino)carbonyl)-4-azaindol-3-yl)-oxoacetyl]piperazine: A mixture of Intermediate 13 (267 mg), N,O-dimethylhydroxylamine hydrogen chloride (248 mg), carbon tetrabromide (844 mg), pyridine (202 mg) and triphenylphosphine (668 mg) in dichloromethane (10 mL) was stirred at room temperature for 10 hours. After solvent was removed under vaccum, the residue was purified by using silica gel chromatography to afford 1-(benzoyl)-3-(R)-methyl-4-[(7-(methoxymethylamino)carbonyl)-4-azaindol-3-yl)-oxoacetyl]piperazine (56 mg); MS m/z: (M+H)⁺ Calc'd for C₂₄H₂₆N₅O₅: 464.19; found 464.25. HPLC retention time: 1.02 minutes (column A).

### EXAMPLE 58

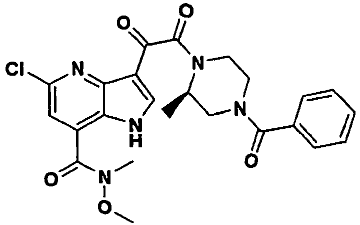

Example 58 was prepared according to the same procedure used in preparing Example 57 with the exception of using Intermediate 11 as a starting material instead of Intermediate 13. The procedure provided 1-benzoyl-3-(R)-methyl-4-[(5-chloro-(7-(methoxymethylamino)carbonyl)-4-azaindol-3-yl)-oxoacetyl]piperazine; MS *m*/*z:* (M+H)⁺ Calc'd for C₂₄H₂₅ClNO₅: 498.15; found 498.12. HPLC retention time: 1.39 minutes (column A).

### General procedure A to prepare CO-NR1R2 fromCOOH

### EXAMPLE 59

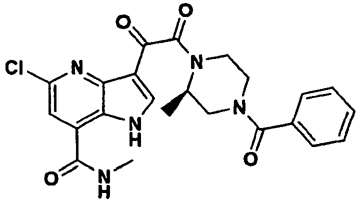

Preparation of 1-benzoyl-3-(R)-methyl-4-[(5-chloro-7-(methylamino)carbonyl)-4-azaindol-3-yl)-oxoacetyl]piperazine: A mixture of Intermediate 11 (25 mg), methylamine (2M in THF, 0.08 mL), EDC (26 mg), HOBT (11.2 mg) and diisopropylethylamine (43 mg) in tetrahydrofuran (5 mL) was stirred at room temperature for 10 hours. After the solvent was removed under vaccum, the residue was purified by using silica gel chromatography to afford 1-benzoyl-3-(R)-methyl-4-[(5-chloro-7-(methylamino)carbonyl)-4-azaindol-3-yl)-oxoacetyl]piperazine (13.6 mg); MS *m*/*z*: (M+H)⁺ Calc'd for C₂₃H₂₃ClN₅O₄: 468.14; found 468.03. HPLC retention time: 1.33 minutes (column A).

This general produre A is applied to prepare examples 94 and 135:

### Example 94

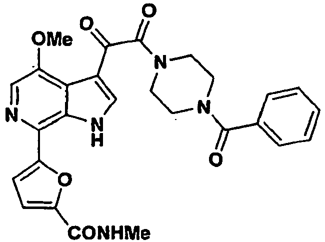

Example 94,1-benzoyl-4-[(4-methoxy-7-(2-methylaminocarbonyl-furan-5-yl)-6-azaindol-3-yl)-oxoacetyl]piperazine. ¹H NMR (500 MHz, CD₃OD) δ8.37 (s, 1H), 8.06 (s, 1H), 7.48 - 7.26 (m, 7H), 4.08 (s, 3H), 3.83 - 3.44 (m, 8H), 2.96 (s, 3H). MS m/z: (M+H)⁺ Calc'd for C₂₉H₂₆N₅O₆: 516.19; found 516.14. HPLC retention time: 1.35 minutes (column A).

### Example 135

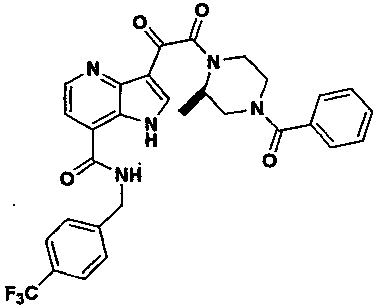

Example 135, (R)-1-benzoyl-3-methyl-4-[(7-(4-trifluoromethylbenzylamino) carbonyl-4-azaindol-3-yl)-oxoacetyl]piperazine. MS *m*/*z*: (N4+H)⁺ Calc'd for C₃₀H₂₇F₃N₅O₄: 578.20; found 578.39. HPLC retention time: 1.47 minutes (column G).

### General procedure B to prepare CO-NR1R2 fromCOOH

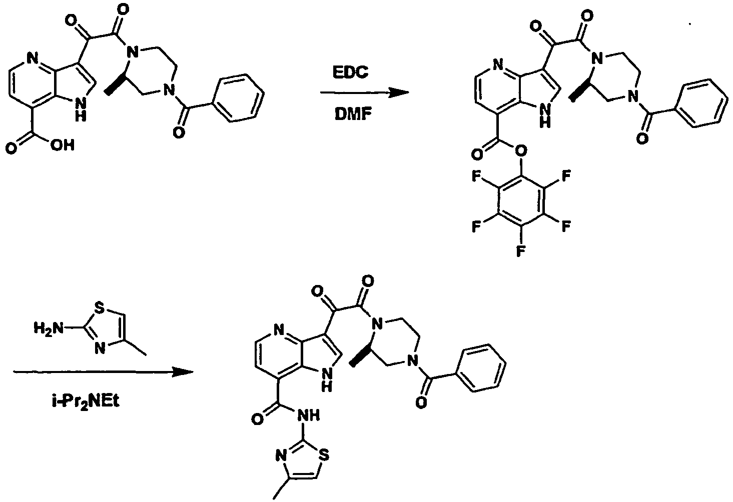

### Preparation of Example 136, (R)-1-benzoyl-3-methyl-4-[(7-(4-methylthiazol-2-yl)aminocarbonyl-4-azaindol-3-yl)-oxoacetyl]piperazine:

To a solution of (R)-1-benzoyl-3-methyl-4-[(7-hydroxylcarbonyl-4-azaindol-3-yl)-oxoacetyl]piperazine (146mg) in DMF (5ml) at room temperature was added pentafluorophenyl (70.3mg) followed by EDC (73.23mg). The reaction mixture was stirred at room temperature for 8 hours. The crude product was diluted with methylene chloride and was washed with water, 0.1N HCl and brine. The organic phase was dried over MgSO4, filtered and concentrated. The pentafluorophenyl ester was used in the following reaction without further purification.

To a stirred solution of 4-methyl-2-amino-thiazole (39.6mg) and Hunig's base (49.4mg) in DMF (5ml) at room temperature was added a solution of pentafluorophenyl ester (1/3 of the product obtained in the previous step described above) in DMF (2 ml). The reaction mixture was stirred at room temperature for 16 hours. The crude product was diluted with methylene chloride and was washed with Na2C03 (sat.) and brine. The organic phase was dried over MgSO4, filtered and concentrated. The residue was purified using. Shimadzu automated preparative HPLC System to give (R)-1-benzoyl-3-methyl-4-[(7-(4-methylthiazol-2-yl)aminocarbonyl-4-azaindol-3-yl)-oxoacetyl]piperazine (3.6mg). MS *m*/*z*: (M+H)⁺ Calc'd for C₂₆H₂₅N₆O₄S: 517.17; found 517.41. HPLC retention time: 1.25 minutes (column A).
This general produre B is applied to prepare example 137:

### Example 137

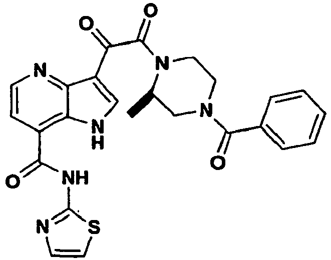

Example 137, (R)-1-benzoyl-3-methyl-4-[(7-(thiazol-2-yl)aminocarbonyl-4-azaindol-3-yl)-oxoacetyl]piperazine. MS *m*/*z*: (M+H)⁺ Calc'd for C₂₅H₂₃N₆O₄S: 503.15; found 503.29. HPLC retention time: 1.33 minutes (column A).

### EXAMPLE 60

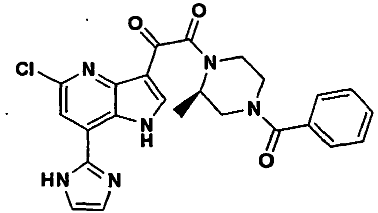

Preparation of 1-benzoyl-3-(R)-methyl-4-[(5-chloro-7-(imidazol-2-yl)-4-azaindol-3-yl)-oxoacetyl]piperazine: A mixture of Intermediate 10 (34 mg), glyoxal (40% in water, 0.2 mL) and ammonia acetate (139 mg) in methanol was heated up to reflux for 10 hours. After cooling down, the mixture was concentrated under reduced pressure and the residue was purified using Shimadzu automated preparative HPLC System to provide 1-benzoyl-3-(R)-methyl-4-[(5-chloro-7-(imidazol-2-yl)-4-azaindol-3-yl)-oxoacetyl]piperazine (1.8 mg); MS *m*/*z*: (M+H)⁺ Calc'd for C₂₄H₂₂ClN₆O₃: 477.14; found 477.13. HPLC retention time: 1.17 minutes (column A).

### EXAMPLE 61

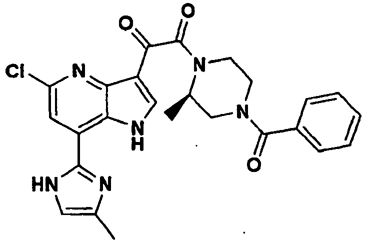

Example 61 was prepared according to the same procedure used for preparing Example 60 with the exception of using methylglyoxal as a starting material instead of glyoxal to provide1 -benzoyl-3-(R)-methyl-4-[(5-chloro-7-(4-methyl-imidazol-2-yl)-4-azaindol-3-yl)-oxoacetyl]piperazine MS *m*/*z*: (M+H)⁺ Calc'd for C₂₅H₂₄ClN₆O₃: 491.16; found 491.13. HPLC retention time: 1.26 minutes (column A).

### EXAMPLE 62

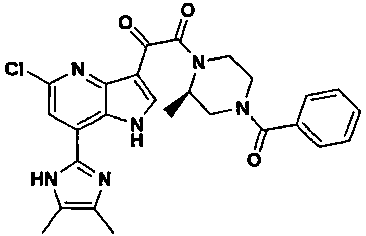

Example 62 was prepared according to the same procedure used for preparing Example 60 with the exception of using dimethylglyoxal as a starting material instead of glyoxal to provide 1-benzoyl-3-(R)-methyl-4-[(5-chloro-7-(4,5-dimethyl-imidazol-2-yl)-4-azaindol-3-yl)-oxoacetyl]piperazine; MS *m*/*z*: (M+H)⁺ Calc'd for C₂₆H₂₆ClN₆O₃: 505.18; found 505.10. HPLC retention time: 1.24 minutes (column A).

### EXAMPLE 63

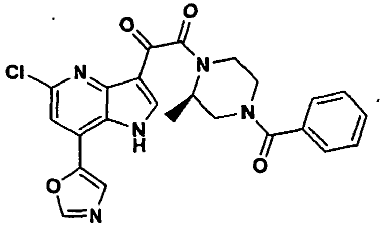

Preparation of 1-benzoyl-3-(R)-methyl-4-[(5-chloro-7-(oxazol-5-yl)-4-azaindol-3-yl)-oxoacetyl]piperazine: A mixture of Intermediate 10 (27.6 mg), tosylmethyl isocyanide (12.3 mg) and K₂CO₃ (8.7 mg) in MeOH was heated up to reflux for 10 hours. After cooling down, the mixture was concentrated under reduced pressure and the residue was purified using Shimadzu automated preparative HPLC System to provide 1-(benzoyl)-3-(R)-methyl-4-[(5-chloro-7-(oxazol-5-yl)-4-azaindol-3-yl)-oxoacetyl]piperazine (17.7 mg); MS *m*/*z*: (M+H)⁺ Calc'd for C₂₄H₂₁ ClN₅O₄: 478.13; found 478.03. HPLC retention time: 1.48 minutes (column A).

### EXAMPLE 64

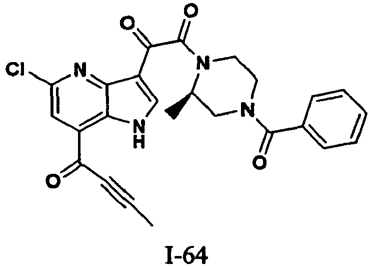

Step 1: Preparation of I-64, 1-benzoyl-3-(R)-methyl-4-[(7-(2-propynyl)carbonyl-4-azaindol-3-yl)-oxoacetyl]piperazine: Propynyllithium (21 mg) was added to a solution of Example 52 (41 mg) in tetrahydrofuran (5 ml) at -78°C. The reaction was quenched with methanol at -25°C in 2 hours. After solvents were removed under vaccum, the residue was carried to the further reactions without any purification. I-64, 1-benzoyl-3-(R)-methyl-4-[(7-(2-propynyl)carbonyl-4-azaindol-3-yl)-oxoacetyl]piperazine MS *m*/*z*: (M+H)⁺ Calc'd for C₂₅H₂₂ClN₄O₄: 477.13; found 477.17. HPLC retention time: 1.46 minutes (column A).

### Step 2: Preparation of Example 64:

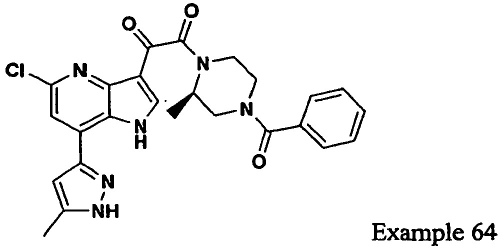

Preparation of Example 64, 1-benzoyl-3-(R)-methyl-4-[(5-chloro-7-(3-methyl-pyrazol-5-yl)-4-azaindol-3-yl)-oxoacetyl]piperazine: A mixture of I-64 (crude product from Step 1) and hydrazine (0.22 mL) in EtOAc (2 mL) and water (2 mL) was stirred at room temperature for 24 hours. Then solvents were removed under vaccum, and the residue was purified using Shimadzu automated preparative HPLC System to give 1-benzoyl-3-(R)-methyl-4-[(5-chloro-7-(3-methyl-pyrazol-5-yl)-4-azaindol-3-yl)-oxoacetyl]piperazine (9 mg); MS *m*/*z*: (M+H)⁺ Calc'd for C₂₅H₂₄ClN₆O₃: 491.16; found 491.19. HPLC retention time: 1.42 minutes (column A).

### EXAMPLES 65-67

The procedure for the preparation of Examples 65-67 is the same as that described previously for the preparation of Intermediate 5a and is as follows: Potassium 7-(4-methoxyphenyl)-4-azaindole-3-glyoxylate, Intermediate 4c (147 mg, 0.44 mmol), an appropriate 1-benzoylpiperazine derivative (0.44 mmol), 3-(diethoxyphosphoryloxy)-1,2,3-benzotriazin-4(3*H*)-one (DEPBT) (101 mg, 0.44 mol) and Hunig's Base (0.5 mL) were combined in 5 mL of DMF. The mixture was stirred at rt for 8 h. DMF was removed via evaporation at reduced pressure and the residue was purified using a Shimadzu automated preparative HPLC System to give the corresponding 1-benzoyl-4-[(7-(4-methoxyphenyl)-4-azaindol-3-yl)-oxoacetyl]-piperazine derivative.

### EXAMPLE 65

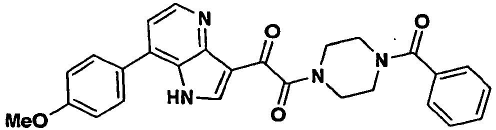

Example 19, 1-(benzoyl)-4-[(7-(4-methoxy)-4-azaindol-3-yl)-oxoacetyl]piperazine was prepared from potassium 7-(4-methoxyphenyl)-4-azaindole-3-glyoxylate and 1-(benzoyl)piperazine according to the above general procedure. MS *m*/*z*: (M+H)⁺ Calc'd for C₂₇H₂₅N₄O₄: 469.19; found 469.16. HPLC retention time: 1.26 minutes (column A).

### EXAMPLE 66

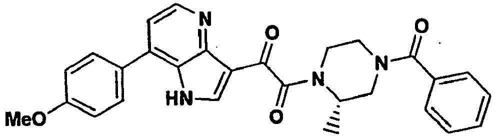

Example 66, 1-benzoyl-3-(S)-methyl-4-[(7-(4-methoxy)-4-azaindol-3-yl)-oxoacetyl]piperazine was prepared from potassium 7-(4-methoxyphenyl)-4-azaindole-3-glyoxylate and the corresponding 1-(benzoyl)-3-methylpiperazine according to the above general procedure. MS *m*/*z*: (M+H)⁺ Calc'd for C₂₈H₂₇N₄O₄: 483.20; found 483.17. HPLC retention time: 1.30 minutes (column A).

### EXAMPLE 67

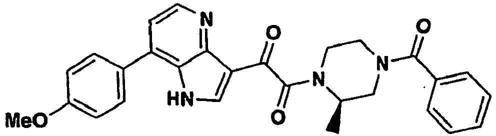

Example 67, 1-benzoyl-3-(R)-methyl-4-[(7-(4-methoxyphenyl)-4-azaindol-3-yl)oxoacetyl]piperazine was prepared from potassium 7-(4-methoxyphenyl)-4-azaindole-3-glyoxylate and the corresponding 1-benzoyl-3-methylpiperazine according to the above general procedure. MS *m*/*z*: (M+H)⁺ Calc'd for C₂₈H₂₇N₄O₄: 483.20; found 483.16. HPLC retention time: 1.28 minutes (column A).

### EXAMPLES 68-79 and 81

Examples 68-79 and 81 were prepared according to the same general method as previously described for Examples 16-54.

### EXAMPLE 68

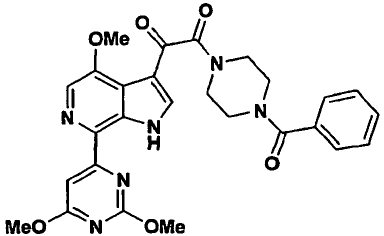

Example 68, was prepared from Intermediate 5b and the 2,4-dimethoxypyrimidin-6-yl stannane to provide 1-benzoyl-4-[(4-methoxy-7-(2,6-dimethoxy-pyrimidin-4-yl)-6-azaindol-3-yl)-oxoacetyl]piperazine. ¹H NMR (500 MHz, CDCl₃) δ 8.20 (s, 1H), 8.13 (s, 1H), 7.52 (s, 1H), 7.42 (m, 5H), 4.11 (s, 3H), 4.06 (s, 3H), 4.00 - 3.40 (m, 8H). MS *m*/*z*: (M+H)⁺ Calc'd for C₂₇H₂₇N₆O₆: 531.20; found 531.24. HPLC retention time: 1.54 minutes (column A).

### EXAMPLE 69

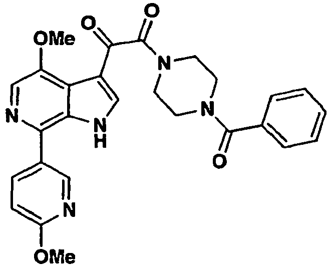

Example 69, was prepared from Intermediate 5b and the 6-methoxypyridin-3-yl stannane to provide 1-benzoyl-4-[(4-.methoxy-7-(6-methoxy-pyridin-3-yl)-6-azaindol-3-yl)-oxoacetyl]piperazine. ¹H NMR (500 MHz, CD₃OD) δ 8.69 (s, 1H), 8.63 (s, 1H), 8.11 (m, 2H), 7.49 (m, 5H), 7.10 (d, 1H, J = 8.65 Hz), 4.16 (s, 3H), 4.06 (s, 3H), 4.00 - 3.40 (m, 8H). MS *m*/*z*: (M+H)⁺ Calc'd for C₂₇H₂₆N₅O₅: 500.09; found 500.20. HPLC retention time: 1.11 minutes (column A).

### EXAMPLE 70

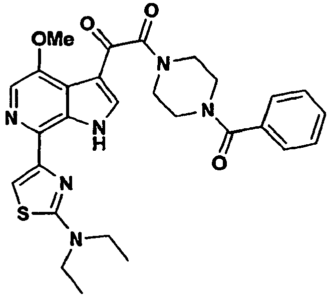

Example 70, was prepared from Intermediate 5b and the 2-diethylamino-thiazol-4-yl stannane to provide 1-benzoyl-4-[(4-methoxy-7-(2-diethylamino-thiazol-4-yl)-6-azaindol-3-yl)-oxoacetyl]piperazine. ¹H NMR (500 MHz, CD₃OD) δ 8.47 (s, 1H), 7.97 (m, 2H), 7.49 (m, 5H), 4.08 (s, 3H), 3.64 (m, 12H), 1.35 (m, 6H). MS *m*/*z*: (M+H)⁺ Calc'd for C₂₈H₃₁N₆O₄S: 547.21; found 547.22. HPLC retention time: 1.35 minutes (column A).

### EXAMPLE 71

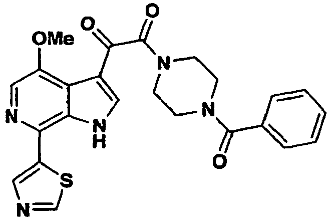

Example 71, was prepared from Intermediate 5b and the thiazol-5-yl stannane to provide 1-benzoyl-4-[(4-methoxy-7-(thioazol-5-yl)-6-azaindol-3-yl)-oxoacetyl]piperazine. ¹H NMR (500 MHz, DMSO-d₆) δ 9.19 (s, 1H), 8.64 (s, 1H), 8.34 (s, 1H), 8.11 (s, 1H), 7.46 (m, 5H), 4.00 (s, 3H), 3.55 (m, 8H). MS *m*/*z*: (M+H)⁺ Calc'd for C₂₄H₂₂N₅O₄S: 476.14; found 476.17. HPLC retention time: 1.13 minutes (column A).

### EXAMPLE 72

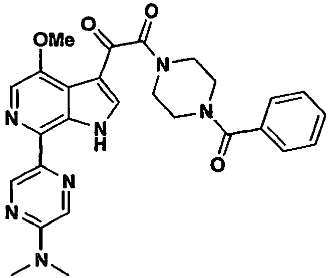

Example 72, was prepared from Intermediate 5b and the 2-dimethylamino-pyrazin-5-yl stannane to provide 1-(benzoyl)-4-[(4-methoxy-7-(2-dimethylamino-pyrazin-5-yl)-6-azaindol-3-yl)-oxoacetyl]piperazine. MS *m*/*z*: (M+H)⁺ Calc'd for C₂₇H₂₈N₇O4: 514.22; found 514.29. HPLC retention time: 1.27 minutes (column A).

### EXAMPLE 73

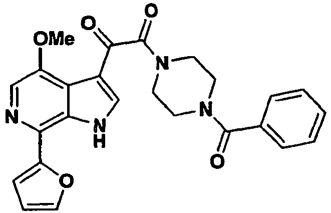

Example 73, was prepared from Intermediate 5b and the furan-2-yl stannane to provide 1-(benzoyl)-4-[(4-methoxy-7-(furan-2-yl)-6-azaindol-3-yl)-oxoacetyl]piperazine. MS *m*/*z*: (M+H)⁺ Calc'd for C₂₅H₂₃N₄O₅,: 459.17; found 459.25. HPLC retention time: 1.15 minutes (column A).

### EXAMPLE 74

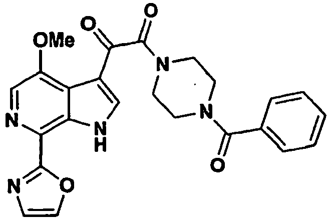

Example 74, was prepared from Intermediate 5b and the oxazol-2-yl stannane to provide 1-benzoyl-4-[(4-methoxy-7-(oxazol-2-yl)-6-azaindol-3-yl)-oxoacetyl]piperazine. ¹H NMR (500 MHz, DMSO-d₆) δ 9.19 (s, 1H), 8.64 (s, 1H), 8.34 (s, 1H), 8.11 (s, 1H), 7.46 (m, 5H), 4.00 (s, 3H), 3.55 (m, 8H). MS *m*/*z*: (M+H)⁺ Calc'd for C₂₄H₂₂N₅O_{5:} 460.16; found 460.23. HPLC retention time: 1.22 minutes (column A).

### EXAMPLE 75

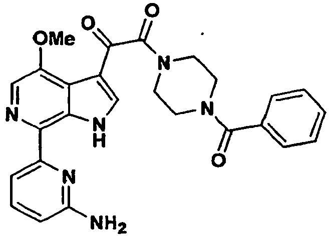

Example 75, was prepared from Intermediate 5b and the 6-aminopyridin-2-yl stannane to provide 1-benzoyl-4-[(4-methoxy-7-(2-aminopyridin-6-yl)-6-azaindol-3-yl)-oxoacetyl]piperazine. MS *m*/*z*: (M+H)⁺ Calc'd for C₂₆H₂₅N₆O₄: 485.19; found 485.24. HPLC retention time: 1.15 minutes (column A).

### EXAMPLE 76

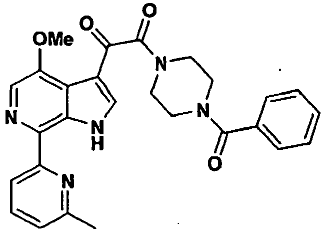

Example 76, was prepared from Intermediate 5b and the 6-methylpyridin-2-yl stannane to provide 1-benzoyl-4-[(4-methoxy-7-(2-methyl-pyridin-6yl)-6-azaindol-3-yl)-oxoacetyl]piperazine. MS *m*/*z*: (M+H)⁺ Calc'd for C₂₇H₂₆N₅O₄: 484.20; found 484.22. HPLC retention time: 1.24 minutes (column A).

### EXAMPLE 77

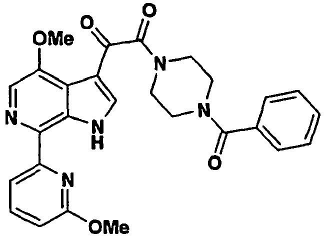

Example 77, was prepared from Intermediate 5b and the 6-methoxypyridin-2-yl stannane to provide 1-benzoyl-4-[(4-methoxy-7-(2-methoxy-pyridin-6-yl)-6-azaindol-3-yl)-oxoacetyl]piperazine. MS *m*/*z*: (M+H)⁺ Calc'd for C₂₇H₂₆N₅O₅: 500.19; found 500.23. HPLC retention time: 1.26 minutes (column A).

### EXAMPLE 78

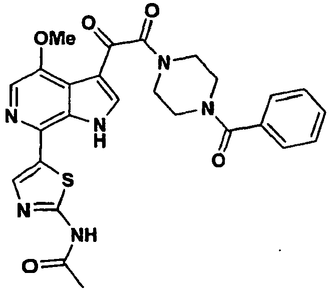

Example 78, was prepared from Intermediate 5b and the 2-acetylamino-thiazol-5-yl stannane to provide 1-benzoyl-4-[(4-methoxy-7-(2-acetylamino-thiazol-5-yl)-6-azaindol-3-yl)-oxoacetyl]piperazine. MS *m*/*z*: (M+H)⁺ Calc'd for C₂₆H₂₅N₆O₅S: 533.16; found 533.18. HPLC retention time: 1.21 minutes (column A).

### EXAMPLE 79

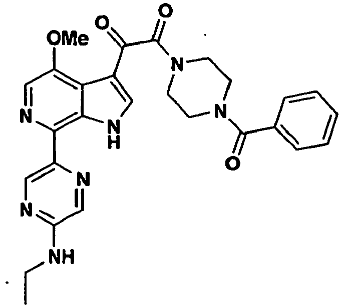

Example 79, was prepared from Intermediate 5b and the 2-ethylamino-pyrazin-5-yl stannane to provide 1-benzoyl-4-[(4-methoxy-7-(2-ethylamino-pyrazin-5-yl)-6-azaindol-3-yl)-oxoacetyl]piperazine. MS *m*/*z*: (M+H)⁺ Calc'd for C₂₇H₂₈N₇O₄: 514.22; found 514.18. HPLC retention time: 1.31 minutes (column A).

### EXAMPLE 88

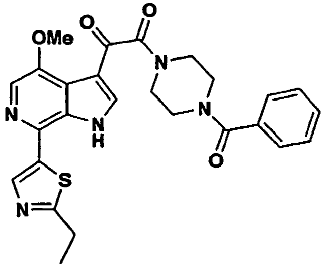

Example 88, was prepared from Intermediate 5b and the 2-ethyl-thiazol-5-yl stannane to provide 1-benzoyl-4-[(4-methoxy-7-(2-ethyl-thiazol-5-yl)-6-azaindol-3-yl)-oxoacetyl]piperazine. MS *m*/*z*: (M+H)⁺ Calc'd for C₂₆H₂₆N₅O₄S: 504.17; found 514.32. HPLC retention time: 1.50 minutes (column A).

### EXAMPLE 89

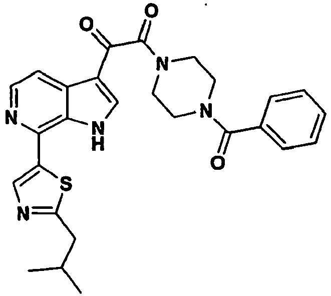

Example 89, was prepared from Intermediate 5k and the 2-isobutyl-thiazol-5-yl stannane to provide 1-benzoyl-4-[(7-(2-isobutyl-thiazol-5-yl)-6-azaindol-3-yl)-oxoacetyl]piperazine. MS *m*/*z*: (M+H)⁺ Calc'd for C₂₇H₂₈N₅O₃S: 502.19; found 502.26. HPLC retention time: 1.56 minutes (column E).

### Example 90

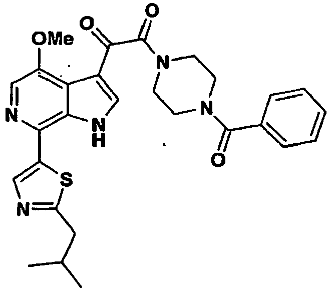

Example 90, was prepared from Intermediate 5b and the 2-isobutyl-thiazol-5-yl stannane to provide 1-benzoyl-4-[(4-methoxy-7-(2-isobutyl-thiazol-5-yl)-6-azaindol-3-yl)-oxoacetyl]piperazine. MS *m*/*z*: (M+H)⁺ Calc'd for C₂₈H₃₀N₅O₄S: 532.20; found 532.27. HPLC retention time: 1.57 minutes (column E).

### Example 91

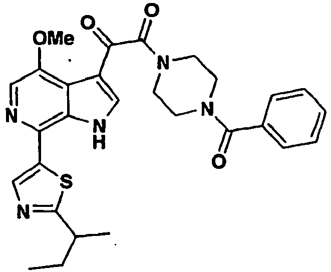

Example 91, was prepared from Intermediate 5b and the 2-(2-butyl)-thiazol-5-yl stannane to provide 1-benzoyl-4-[(4-methoxy-7-(2-(2-butyl)-thiazol-5-yl)-6-azaindol-3-yl)-oxoacetyl]piperazine. MS *m*/*z*: (M+H)⁺ Calc'd for C₂₈H₃₀N₅O₄S: 532.20; found 532.27. HPLC retention time: 1.57 minutes (column E).

### Example 92

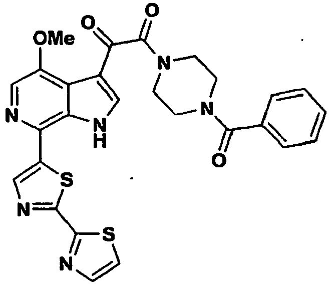

Example 92, was prepared from Intermediate 5b and the 2-(thiazol-2-yl)-thiazol-5-yl stannane to provide 1-benzoyl-4-[(4-methoxy-7-(2-(thiazol-2-yl)-thiazol-5-yl)-6-azaindol-3-yl)-oxoacetyl]piperazine. MS *m*/*z*: (M+H)⁺ Calc'd for C₂₇H₂₃N₆O₄S₂: 559.12; found 559.18. HPLC retention time: 1.55 minutes (column E).

### Example 93

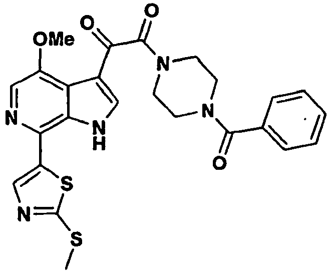

Example 93, was prepared from Intermediate 5b and the 2-methylthio-thiazol-5-yl stannane to provide 1-benzoyl-4-[(4-methoxy-7-(2-methylthio-thiazol-5-yl)-6-azaindol-3-yl)-oxoacetyl]piperazine. MS *m*/*z*: (M+H)⁺ Calc'd for C₂₅H₂₄N₅O₄S₂: 522.13; found 522.17. HPLC retention time: 1.45 minutes (column E).

### Example 95

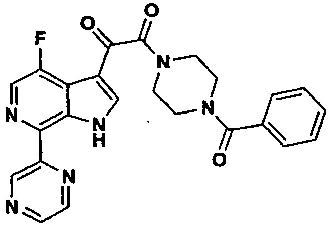

Example 95, was prepared from Intermediate 5i and the pyrazin-2-yl stannane to provide 1-benzoyl-4-[(4-fluoro-7-(pyrazin-2-yl)-6-azaindol-3-yl)-oxoacetyl]piperazine. ¹H NMR (500 MHz, CDCl₃) δ9.89 (s, 1H), 8.70 - 8.34 (m, 4H), 7.46 (m, 5H), 3.80 - 3.50 (m, 8H). MS *m*/*z*: (M+H)⁺ Calc'd for C₂₄H₂₀FN₆O₃: 459.16; found 459.33. HPLC retention time: 1.46 minutes (column G).

### Example 100

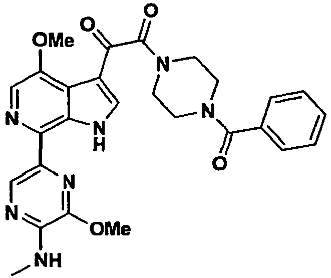

Example 100, was prepared from Intermediate 5b and the 2-methylamino-3-methoxy-pyrazin-5-yl stannane to provide 1-benzoyl-4-[(4-methoxy-7-(2-methylamino-3-methoxy-pyrazin-5-yl)-6-azaindol-3-yl)-oxoacetyl]piperazine. ¹H NMR (500 MHz, CD₃OD) δ8.65 (s, 1H), 8.43 (s, 1H), 7.95 (s, 1H), 7.45 (m, 5H), 4.21 (s, 3H), 4.12 (s, 3H), 3.89 - 3.32 (m, 8H), 3.06 (s, 3H). MS *m*/*z*: (M+H)⁺ Calc'd for C₂₇H₂₈N₇O₅: 530.22; found 530.19. HPLC retention time: 1.31 minutes (column A).

### Example 101

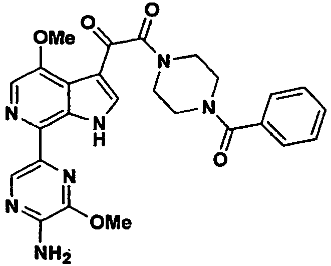

Example 101, was prepared from Intermediate 5b and the 2-amino-3-methoxy-pyrazin-5-yl stannane to provide 1-benzoyl-4-[(4-methoxy-7-(2-amino-3-methoxy-pyrazin-5-yl)-6-azaindol-3-yl)-oxoacetyl]piperazine. ¹H NMR (500 MHz, CD₃OD) δ8.67 (s, 1H), 8.34 (s, 1H), 7.96 (s, 1H), 7.48 (m, 5H), 4.22 (s, 3H), 4.12 (s, 3H), 3.92 - 3.32 (m, 8H). MS *m*/*z*: (M+H)⁺ Calc'd for C₂₆H₂₆N₇O₅: 516.20; found 516.23. HPLC retention time: 1.27 minutes (column A).

### Example 102

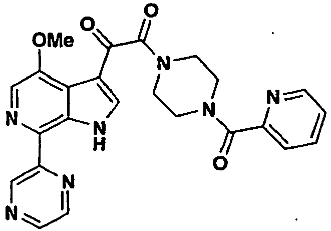

Example 102, was prepared from Intermediate 51 and the pyrazin-2-yl stannane to provide 1-picolinoyl-4-[(4-methoxy-7-(pyrazin-2-yl)-6-azaindol-3-yl)-oxoacetyl]piperazine. ¹H NMR (500 MHz, CD₃OD) δ9.59 (s, 1H), 8.79 - 7.51 (m, 8H), 4.13 (s, 3H), 3.95 -3.34 (m, 8H). MS *m*/*z*: (M+H)⁺ Calc'd for C₂₄H₂₂N₇O₄: 472.17; found 472.25. HPLC retention time: 1.15 minutes (column A).

### Example 103

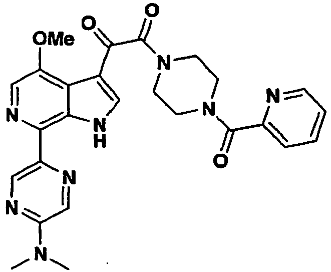

Example 103, was prepared from Intermediate 51 and the 2-dimethylamino-pyrazin-5-yl stannane to provide 1-picolinoyl-4-[(4-methoxy-7-(2-dimethylamino-pyrazin-5-yl)-6-azaindol-3-yl)-oxoacetyl]piperazine. MS *m*/*z*: (M+H)⁺ Calc'd for C₂₆H₂₇N₈O₄: 515.22; found 515.16. HPLC retention time: 1.29 minutes (column A).

### Example 104

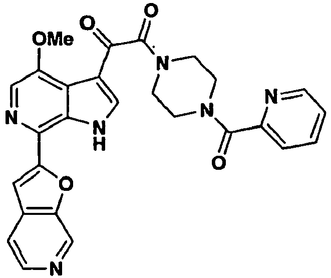

Example 104, was prepared from Intermediate 5b and the 6-aza-benzofuran-2-yl stannane to provide 1-benzoyl-4-[(4-methoxy-7-(6-aza-benzofuran-2-yl)-6-azaindol-3-yl)-oxoacetyl]piperazine. ¹H NMR (500 MHz, CDCl₃) δ8.48 (d, 1H, *J* = 8.5 Hz), 8.36 (s, 1H), 8.30 (s, 1H), 8.02 (s, 1H), 7.64 (d, 1H, *J* = 8.55 Hz), 7.41 (m, 4H), 6.92 (s, 1H), 4.12 (s, 3H), 3.87 - 3.38 (m, 8H). MS *m*/*z*: (M+H)⁺ Calc'd for C₂₈H₂₄N₅O₅: 510.18; found 510.33. HPLC retention time: 1.33 minutes (column A).

### Example 105

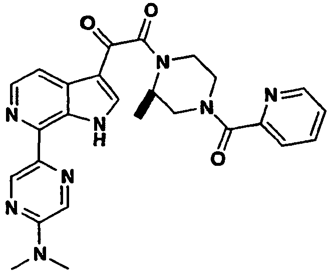

Example 105, was prepared from Intermediate 5m and the 2-dimethylamino-pyrazin-5-yl stannane to provide (R)-1-picolinoyl-3-methyl-4-[(7-(2-dimethylamino-pyrazin-5-yl)-6-azaindol-3-yl)-oxoacetyl]piperazine. MS *m*/*z*: (M+H)⁺ Calc'd for C₂₆H₂₇N₈O₃: 499.22; found 499.27. HPLC retention time: 1.17 minutes (column A).

### Example 106

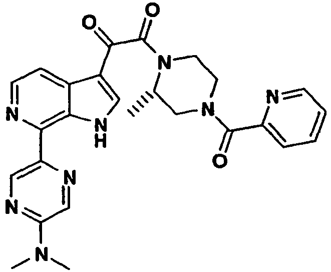

Example 106, was prepared from Intermediate 5n and the 2-dimethylamino-pyrazin-5-yl stannane to provide (S)-1-picolinoyl-3-methyl-4-[(7-(2-dimethylamino-pyrazin-5-yl)-6-azaindol-3-yl)-oxoacetyl]piperazine. ¹H NMR (500 MHz, CD₃OD) δ9.08 - 7.49 (m, 9H), 5.00 - 3.15 (m, 13H), 1.44 -1.27 (m, 3H). MS *m*/*z*: (M+H)⁺ Calc'd for C₂₆H₂₇N₈O₃: 499.22; found 499.27. HPLC retention time: 1.19 minutes (column A).

### Example 109

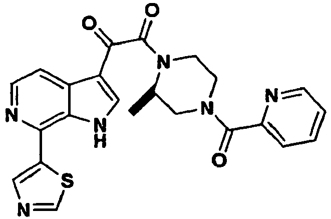

Example 109, was prepared from Intermediate 5m and the thiazol-5-yl stannane to provide (R)-1-picolinoyl-3-methyl-4-[(7-(thiazol-5-yl)-6-azaindol-3-yl)-oxoacetyl]piperazine. ¹H NMR (500 MHz, CD₃OD) δ9.42 -7.49 (m, 9H), 4.98 - 3.14 (m, 7H), 1.43 -1.26 (m, 3H). MS *m*/*z*: (M+H)⁺ Calc'd for C₂₃H₂₁N₆O₃S: 461.14; found 461.28. HPLC retention time: 1.11 minutes (column A).

### Example 110

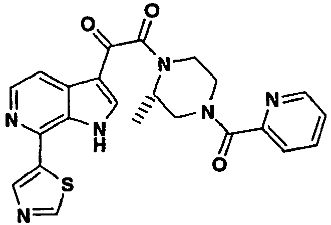

Example 110, was prepared from Intermediate 5n and the thiazol-5-yl stannane to provide (S)-1-picolinoyl-3-methyl-4-[(7-(thiazol-5-yl)-6-azaindol-3-yl)-oxoacetyl]piperazine.. ¹H NMR (500 MHz, CD₃OD) δ9.44 - 7.48 (m, 9H), 4.98 - 3.15 (m, 7H), 1.43 -1.26 (m, 3H). MS *m*/*z*: (M+H)⁺ Calc'd for C₂₃H₂₁N₆O₃S: 461.14; found 461.27. HPLC retention time: 1.13 minutes (column A).

### Example 111

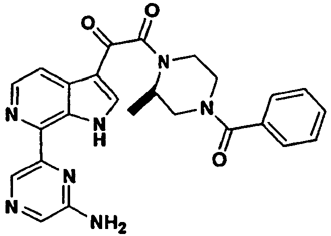

Example 111, was prepared from Intermediate 5f and the 2-amino-pyrazin-6-yl stannane to provide (R)-1-benzoyl-3-methyl-4-[(7-(2-amino-pyrazin-6-yl)-6-azaindol-3-yl)-oxoacetyl]piperazine. ¹H NMR (500 MHz, CD₃OD) δ8.68 - 7.45 (m, 10H), 4.89 - 3.13 (m, 7H), 1.39 - 0.99 (m, 3H). MS *m*/*z*: (M+H)⁺ Calc'd for C₂₅H₂₄N₇O₃: 470.19; found 470.31. HPLC retention time: 1.30 minutes (column A).

### Example 112

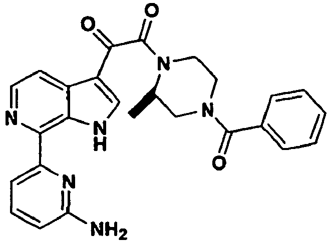

Example 112, was prepared from Intermediate 5f and the 2-amino-pyridin-6-yl stannane to provide (R)-1-benzoyl-3-methyl-4-[(7-(2-amino-pyridin-6-yl)-6-azaindol-3-yl)-oxoacetyl]piperazine. ¹H NMR (500 MHz, CD₃OD) δ8.65 - 6.89 (m, 11H), 4.90 - 3.12 (m, 7H), 1.39 - 0.99 (m, 3H). MS *m*/*z*: (M+H)⁺ Calc'd for C₂₆H₂₅N₆O₃: 469.20; found 469.32. HPLC retention time: 1.26 minutes (column A).

### Example 113

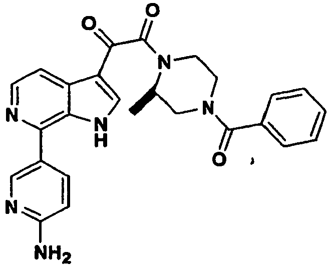

Example 113, was prepared from Intermediate 5f and the 2-amino-pyridin-5-yl stannane to provide (R)-1-benzoyl-3-methyl-4-[(7-(2-amino-pyridin-5-yl)-6-azaindol-3-yl)-oxoacetyl]piperazine. ¹H NMR (500 MHz, CD₃OD) 88.75 - 7.19 (m, 11H), 4.91 - 3.12 (m, 7H), 1.38 -1.25 (m, 3H). MS *m*/*z*: (M+R)⁺ Calc'd for C₂₆H₂₅N₆O₃: 469.20; found 469.34. HPLC retention time: 1.05 minutes (column A).

### Example 114

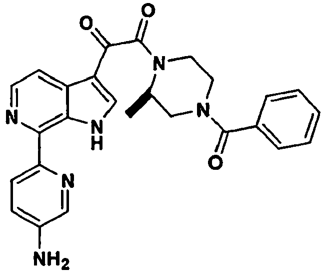

Example 114, was prepared from Intermediate 5f and the 5-amino-pyridin-2-yl stannane to provide (R)-1-benzoyl-3-methyl-4-[(7-(5-amino-pyridin-2-yl)-6-azaindol-3-yl)-oxoacetyl]piperazine. ¹H NMR (500 MHz, CD₃OD) δ8.67 - 7.20 (m, 11H), 4.88 - 3.13 (m, 7H), 1.39 -1.25 (m, 3H). MS *m*/*z*: (M+H)⁺ Calc'd for C₂₆H₂₅N₆O₃: 469.20; found 469.33. HPLC retention time: 1.22 minutes (column A).

### Example 115

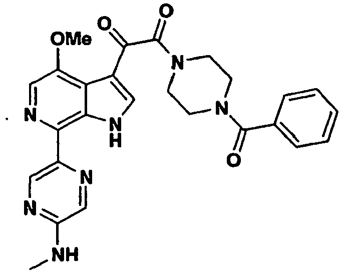

Example 115, was prepared from Intermediate 5b and the 2-methylamino-pyrazin-5-yl stannane to provide 1-benzoyl-4-[(4-methoxy-7-(2-methylamino-pyrazin-5-yl)-6-azaindol-3-yl)-oxoacetyl]piperazine. ¹H NMR (500 MHz, CD₃OD) δ8.90 (s, 1H), 8.61 (s, 1H), 8.18 (s, 1H), 7.92 (s, 1H), 7.46 (m, 5H), 4.12 (s, 3H), 3.85 - 3.40 (m, 8H), 3.02 (s, 3H). MS *m*/*z*: (M+H)⁺ Calc'd for C₂₆H₂₆N₇O₄: 500.20; found 500.23. HPLC retention time: 1.24 minutes (column A).

### Example 116

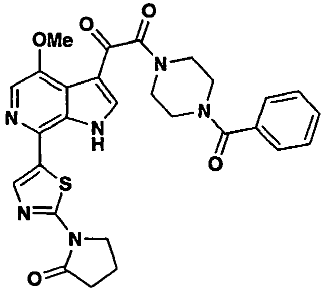

Example 116, was prepared from Intermediate 5b and the 2-(2-pyrrolidinon-1-yl)-thiazol-5-yl stannane to provide 1-benzoyl-4-[(4-methoxy-7-((2-pyrrolidinon-1-yl)-thiazol-5-yl)-6-azaindol-3-yl)-oxoacetyl]piperazine. MS *m*/*z*: (M+H)⁺ Calc'd for C₂₈H₂₇N₆O₅S₂: 559.18; found 559.11. HPLC retention time: 1.39 minutes (column E).

### Example 117

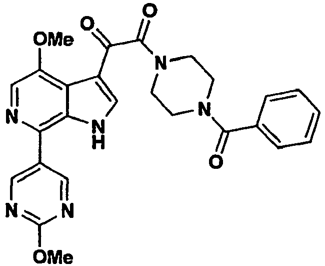

Example 117, was prepared from Intermediate 5b and the 2-methoxy-pyrimidin-5-yl stannane to provide 1-benzoyl-4-[(4-methoxy-7-(2-methoxy-pyrimidin-5-yl)-6-azaindol-3-yl)-oxoacetyl]piperazine. MS *m*/*z*: (M+H)⁺ Calc'd for C₂₆H₂₅N₆O₅: 501.19; found 501.12. HPLC retention time: 1.21 minutes (column E).

### Example 118

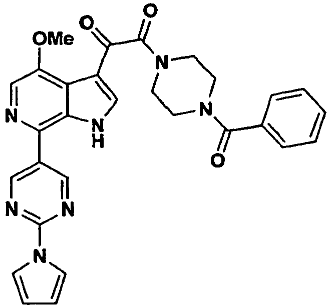

Example 118, was prepared from Intermediate 5b and the 2-(pyrrol-1-yl)-pyrimidin-5-yl stannane to provide 1-benzoyl-4-[(4-methoxy-7-(2-(pyrrol-1-yl)-pyrimidin-5-yl)-6-azaindol-3-yl)-oxoacetyl]piperazine. MS *m*/*z*: (M+H)⁺ Calc'd for C₂₉H₂₆N₇O_{4:} 536.20; found 536.33. HPLC retention time: 1.44 minutes (column C).

### Example 119

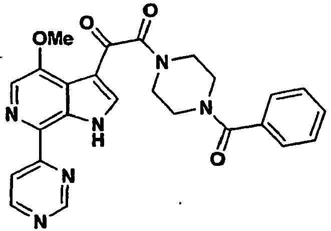

Example 119, was prepared from Intermediate 5b and the pyrimidin-4-yl stannane to provide 1-benzoyl-4-[(4-methoxy-7-(pyrimidin-5-yl)-6-azaindol-3-yl)-oxoacetyl)]piperazine. ¹H NMR (500 MHz, CD₃OD) δ9.29 (s, 1H), δ:88 (d, 1H, *J* = 5.4 Hz), 8.48 (d, 1H, *J* = 5.25 Hz), 8.26 (s, 1H), 8.18 (s, 1H), 7.43 (m, 5H), 4.13 (s, 3H), 3.85 - 3.47 (m, 8H). MS *m*/*z*: (M+H)⁺ Calc'd for C₂₅H₂₃N₆O₄: 471.18; found 471.32. HPLC retention time: 1.35 minutes (column G).

### Example 120

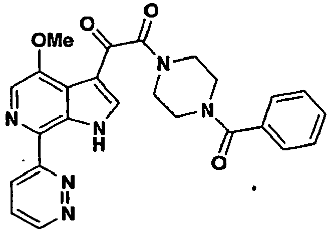

Example 119, was prepared from Intermediate 5b and the pyridazin-3-yl stannane to provide 1-benzoyl-4-[(4-methoxy-7-(pyridazin-3-yl)-6-azaindol-3-yl)-oxoacetyl]piperazine. ¹H NMR (500 MHz, CD₃OD) δ9.16 (s, 1H), 8.77 (d, 1H, *J* = 8.5 Hz), 8.26 (d, 1H, *J* = 3.05 Hz), 8.18 (s, 1H), 7.68 (m, 1H), 7.43 (m, 5H), 4.13 (s, 3H), 3.85 - 3.47 (m, 8H). MS *m*/*z*: (M+H)⁺ Calc'd for C₂₅H₂₃N₆O₄: 471.18; found 471.16. HPLC retention time: 1.35 minutes (column G).

### Example 125

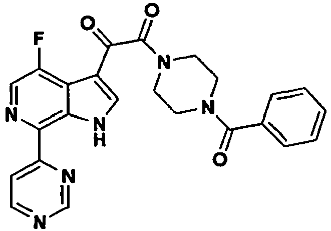

Example 125, was prepared from Intermediate 5i and the pyrimidin-4-yl stannane to provide 1-benzoyl-4-[(4-fluoro-7-(pyrimidin-S-yl)-6-azaindol-3-yl)-oxoacetyl]piperazine. ¹H NMR (500 MHz, CD₃OD) δ9.36 (s, 1H), 8.96 (d, 1H, *J* = 5.35 Hz), 8.58 (d, 1H, *J* = 5.10 Hz), 8.43 (s, 1H), 8.38 (s, 1H), 7.43 (m, 5H), 3.85 - 3.47 (m, 8H). MS *m*/*z*: (M+H)⁺ Calc'd for C₂₄H₂₀FN₆O₂: 459.16; found 459.15. HPLC retention time: 1.48 minutes (column A).

### Example 126

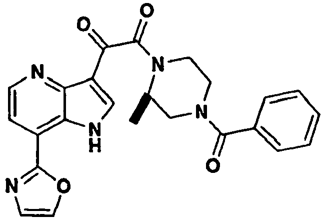

Example 126, was prepared from Intermediate 5i and the oxazol-2-yl stannane to provide (R)-1-benzoyl-3-Methyl-4-[7-(oxazol-2-yl)-4-azaindol-3-yl)-oxoacetyl]piperazine. MS *m*/*z*: (M+H)⁺ Calc'd for C₂₄H₂₂N₅O₄: 444.17; found 444.25. HPLC retention time: 1.13 minutes (column A).

### Example 131

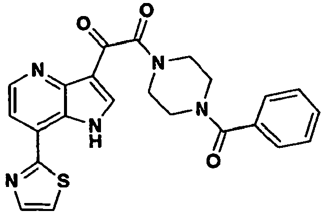

Example 131, was prepared from Intermediate 5p and the thiazol-2-yl stannane to provide 1-benzoyl-4-[7-(thiazol-2-yl)-4-azaindol-3-yl)-oxoacetyl]piperazine. MS *m*/*z*: (M+H)⁺ Calc'd for C₂₃H₂₀N₅O₃S: 446.13; found 446.04. HPLC retention time: 1.12 minutes (column A).

### EXAMPLE 80

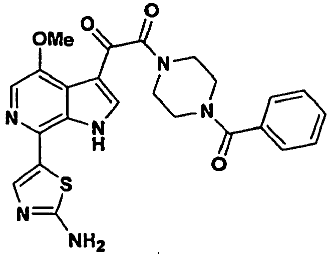

Preparation of **Example 80**, 1-benzoyl-4-[(4-methoxy-7-(2-amino-thioazol-5-yl)-6-azaindol-3-yl)-oxoacetyl]piperazine: A mixture of **Example 78** (9 mg), TFA (3 mL) and water (1 mL) was stirred at 80 °C for 10 hours. After solvent was removed under vaccum, the residue was purified by using silica gel chromatography to afford 1-benzoyl-4-[(4-methoxy-7-(2-amino-thioazol-5-yl)-6-azaindol-3-yl)-oxoacetyl]piperazine (3 mg); MS *m*/*z*: (M+H)⁺ Calc'd for C₂₄H₂₃N₆O₅S: 491.15; found 491.21. HPLC retention time: 1.20 minutes (column A).

### EXAMPLE 81

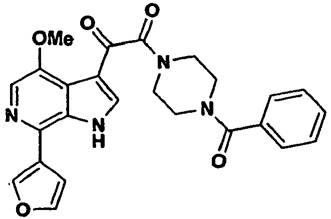

Example 81, ways prepared from Intermediate 5b and the furan-3-yl stannane to provide 1-benzoyl-4-[(4-methoxy-7-(furan-3-yl)-6-azaindol-3-yl)-oxoacetyl]piperazine. MS *m*/*z*: (M+H)⁺ Calc'd for C₂₅H₂₃N₄O₅: 459.17; found 459.24. HPLC retention time: 1.13 minutes (column A).

### Example 150

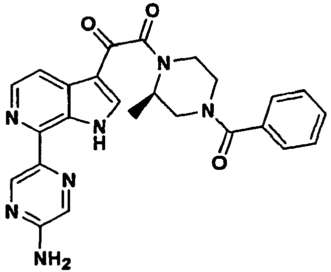

Example 150, was prepared from Intermediate 5f and the 5-amino-pyrazin-2-yl stannane to provide (R)-1-benzoyl-3-methyl-4-[(7-(5-amino-pyrazin-2-yl)-6-azaindol-3-yl)-oxoacetyl]piperazine. MS *m*/*z*: (M+H)⁺ Calc'd for C₂₅H₂₄N₇O₃: 470.19; found 470.19. HPLC retention time: 1.14 minutes (column G).

### Example 153

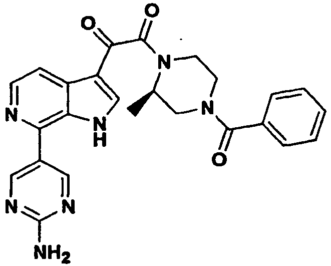

Example 153, was prepared from Intermediate 5f and the 2-amino-pyrimidin-5-yl stannane to provide (R)-1-benzoyl-3-methyl-4-[(7-(2-amino-pyrimidin-5-yl)-6-azaindol-3-yl)-oxoacetyl]piperazine. MS *m*/*z*: (M+H)⁺ Calc'd for C₂₅H₂₄N₇O₃: 470.19; found 470.22. HPLC retention time: 1.07 minutes (column G).

### Example 147

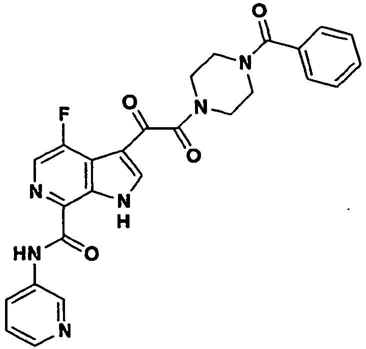

Intermediate 5i (16.5 mg, 0.05 mmol) in DMF (1 mL) was treated with *N-*benzoylpiperazine hydrochloride, DEBPT (15 mg, 0.05 mmol) and Hunig's base (34 µL, 0.2 mmol) at rt for 18h. The solvent was removed in vacuum and the residue was purified by reverse phase preparative HPLC. The fractions showing the right LC/MS(ES⁺) m/z (M+H)⁺ = 501 were collected, concentrated and purified again using a preparative TLC (5% MeOH/CH₂Cl₂) to afford the title compound as a white solid. ¹H-NMR (500 MHz, CDCl₃) δ 11.2 (s, 1H), 10.0 (s, 1H), 9.21 (s, 1 H), 8.51 (s, 1H), 8.41 (s, 1H), 8.40 (m, 1H), 8.32 (s, 1H), 7.62 (m, 1H), 7.45 (m, soh), 3.90-3.50 (bm, 8H).

### Example 156

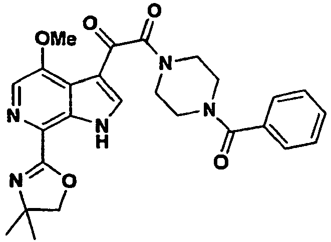

Example 156, was prepared from Intermediate 5b and the 4,4-dimethyloxazolin-2-yl stannane to provide 1-benzoyl-4-[(7-(4,4-dimethyloxazolin-2-yl)-6-azaindol-3-yl)-oxoacetyl]piperazine. MS *m*/*z*: (M+H)⁺ Calc'd for C₂₇H₂₈N₅O₅: 490.21; found 490.22. HPLC retention time: 1.20 minutes (column C).

### Example 169

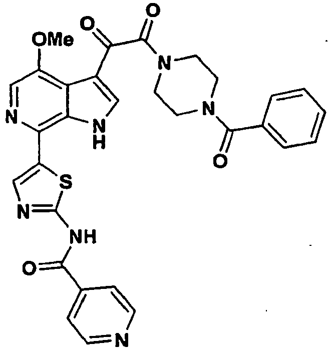

Example 169, was prepared from Intermediate 5b and the 2-(4-pyridinecarboxamido)-thiazol-5-yl stannane to provide 1-benzoyl-4-[(7-(2-(4-pyridinecarboxanudo)-thiazol-5-yl)-6-azaindol-3-yl)-oxoacetyl]piperazine. MS m/z: (M+H)⁺ Calc'd for C₃₀H₂₆N₇O₅S: 596.17; found 596.14. HPLC retention time: 1.32 minutes (column C).

### EXAMPLES 82-86, 98, 107, 108, 129, 130, 132, 133, 134

Examples 82-86, 98, 107,108, 127, 128, 129, 130, 132, 133 and 134 were prepared according to the general procedure as previously described for Examples 2-14.

### EXAMPLE 82

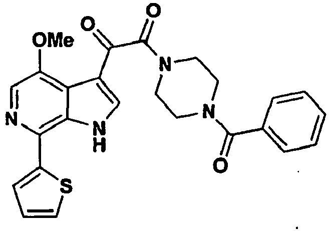

Example 82, was prepared from Intermediate 5b and thien-2-yl boronic acid to provide 1-benzoyt-4-[(4-methoxy-7-(thiophen-2-yl)-6-azaindol-3-yl)-oxoacetyl]piperazine. MS *m*/*z*: (M+H)⁺ Calc'd for C₂₅H₂₃N₄O₄S: 475.14; found 475.31. HPLC retention time: 1.14 minutes (column A).

### EXAMPLE 83

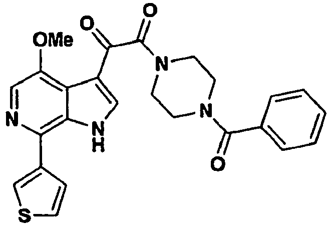

Example 83, was prepared from Intermediate 5b and thien-2-yl boronic acid to provide 1-benzoyl-4-[(4-methoxy-7-(thiophen-3-yl)-6-azaindol-3-yl)-oxoacetyl]piperazine. MS *m*/*z*: (M+H)⁺ Calc'd for C₂₅H₂₃N₄O₄S: 475.14; found 475.33. HPLC retention time: 1.16 minutes (column A).

### EXAMPLE 84

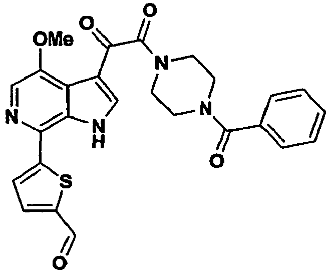

Example 84, was prepared from Intermediate 5b and 5-carbonylthien-2-yl boronic acid to provide 1-benzoyl-4-[(4-methoxy-7-(5-carbonyl-thiophen-2-yl)-6-azaindol-3-yl)-oxoacetyl]piperazine. MS *m*/*z*: (M+H)⁺ Calc'd for C₂₆H₂₃N₄O₅S: 503.14; found 503.23. HPLC retention time: 1.31 minutes (column A).

### EXAMPLE 85

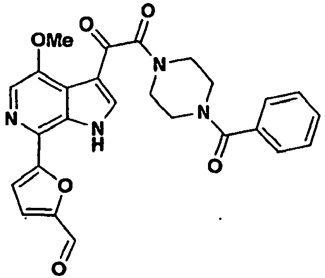

Example 76, was prepared from Intermediate 5b and 5-carbonylfuran-2-yl boronic acid to provide 1-(benzoyl)-4-[(4-methoxy-7-(5-carbonyl-furan-2-yl)-6-azaindol-3-yl)-oxoacetyl]piperazine. MS *m*/*z*: (M+H)⁺ Calc'd for C₂₆H₂₃N₄O₆: 487.16; found 487.28. HPLC retention time: 1.44 minutes (column A).

### EXAMPLE 86

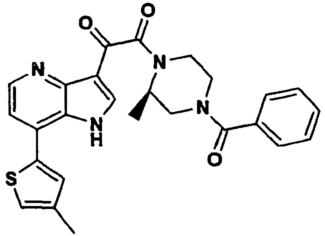

Example 86, was prepared from Intermediate 5d and 4-methylthien-2-yl boronic acid to provide 1-benzoyl-3-(R)-methyl-4-[(7-(4-methyl-thiophen-2-yl)-4-azaindol-3-yl)-oxoacetyl]piperazine. MS *m*/*z*: (M+H)⁺ Calc'd for C₆H₂₅N₄O₃S: 473.16; found 473.26. HPLC retention time: 1.28 minutes (column A).

### Example 98

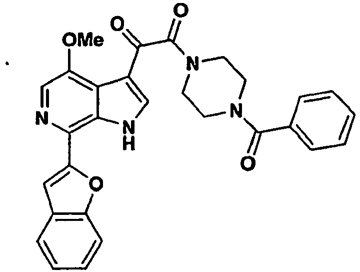

Example 98, was prepared from Intermediate 5d and 2-benzofuranyl boronic acid to provide 1-benzoyl-3-(R)-methyl-4-[(7-(benzofuran-2-yl)-4-azaindol-3-yl)-oxoacetyl]piperazine. ¹H NMR (500 MHz, CDCl₃) δ 8.24 (s, 1H), 8.09 (s, 1H), 7.70-7.26 (m, 10H), 4.03 (s, 3H), 3.97 - 3.49 (m, 8H). MS *m*/*z*: (M+H)⁺ Calc'd for C₂₉H₂₅N₄O₅: 509.18; found 509.18. HPLC retention time: 1.50 minutes (column A).

### Example 107

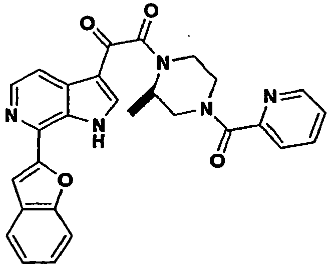

Example 107, was prepared from Intermediate 5m and 2-benzofuranyl boronic acid to provide (R)-1-picolinoyl-3-methyl-4-[(7-(benzofuran-2-yl)-6-azaindol-3 -yl)-oxoacetyl]piperazine. ¹H NMR (500 MHz, CD₃OD) δ8.77 - 7.38 (m, 12H), 4.99 - 3.16 (m, 7H), 1.44 -1.27 (m, 3H). MS *m*/*z*: (M+H)⁺ Calc'd for C₂₈H₂₄N₅O₄: 494.18; found 494.24. HPLC retention time: 1.35 minutes (column A).

### Example 108

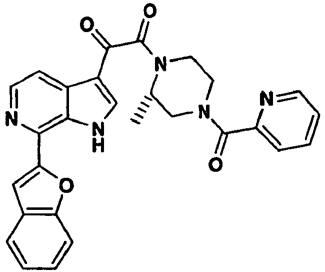

Example 108, was prepared from Intermediate 5n and 2-benzofuranyl boronic acid to provide (S)-1-picolinoyl-3-methyl-4-[(7-(benzofuran-2-yl)-6-azaindol-3-yl)-oxoacetyl]piperazine. MS *m*/*z*: (M+H)⁺ Calc'd for C₂₈H₂₄N₅O₄: 494.18; found 494.23. HPLC retention time: 1.37 minutes (column A).

### Example 127

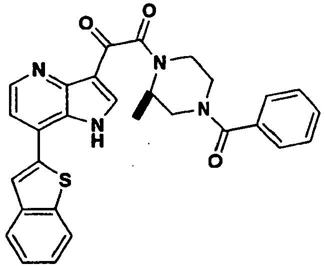

Example 127, was prepared from Intermediate 5i and the benzothiophen-2-yl boronic acid to provide (R)-1-benzoyl-3-Methyl-4-U-(benzothiophen-2-yl)-4-azaindol-3-yl)-oxoacetyl]piperazine. MS *m*/*z*: (M+H)⁺ Calc'd for C₂₉H₂₅N₄O₃S: 509.16; found 509.21. HPLC retention time: 1.42 minutes (column A).

### Example 128

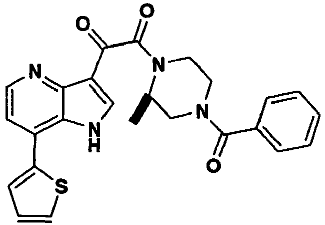

Example 128, was prepared from Intermediate 5i and the thiophen-2-yl boronic acid to provide (R)-1-benzoyl-3-Methyl-4-[7-(thiophen-2-yl)-4-azaindol-3-yl)-oxoacetyl]piperazine. MS *m*/*z*: (M+H)⁺ Calc'd for C₂₅H₂₃N₄O₃S: 459.15; found 459.27. HPLC retention time: 1.22 minutes (column A).

### Example 129

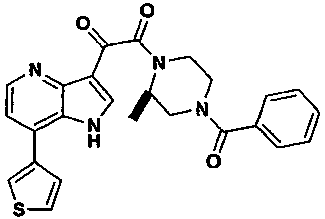

Example 129, was prepared from Intermediate 5i and the thiophen-3-yl boronic acid to provide (R)-1-benzoyl-3-Methyl-4-[7-(thiophen-3-yl)-4-azaindol-3-yl)-oxoacetyl]piperazine. MS *m*/*z*: (M+H)⁺ Calc'd for C₂₅H₂₃N₄O₃S: 459.15; found 459.34. HPLC retention time: 1.31 minutes (column A).

### Example 130

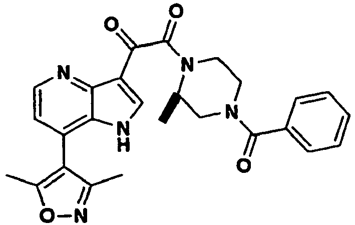

Example 130, was prepared from Intermediate 5i and the 2,5-dimethyl-isoxazol-4-yl boronic acid to provide (R)-1-benzoyl-3-Methyl-4-[7-(2,5-dimethyl-isoxazol-4-yl)-4-azainciol-3-yl)-oxoacetyl]piperazine. MS *m*/*z*: (M+H)⁺ Calc'd for C₂₆H₂₆N₅O₄: 472.20; found 472.28. HPLC retention time: 1.14 minutes (column A).

### Example 132

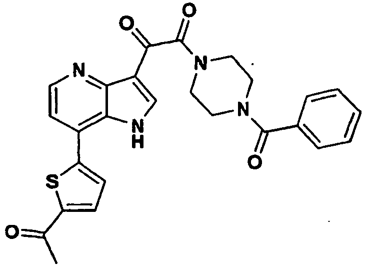

Example 132, was prepared from Intermediate 5p and the 2-methylcarbonyl-thiophen-5-yl boronic acid to provide 1-benzoyl-4-[7-(2-methylcarbonyl-thiophen-5-yl)-4-azaindol-3-yl)-oxoacetyl]piperazine. MS *m*/*z*: (M+H)⁺ Calc'd for C₂₆H₂₃N₄O₄S: 487.14; found 487.20. HPLC retention time: 1.14 minutes (column A).

### Example 133

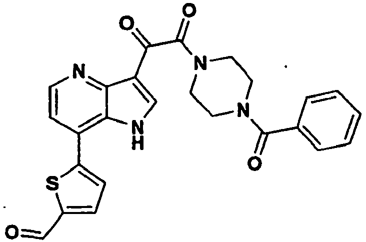

Example 133, was prepared from Intermediate 5p and the 2-carbonyl-thiophen-5-yl boronic acid to provide 1-benzoyl-4-[7-(2-carbonyl-thiophen-5-yl)-4-azaindol-3-yl)-oxoacetyl]piperazine. MS *m*/*z*: (M+H)⁺ Calc'd for C₂₅H₂₁N₄O₄S: 473.13; found 473.11. HPLC retention time: 1.14 minutes (column A).

### Example 134

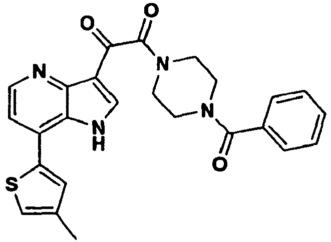

Example 134, was prepared from Intermediate 5p and the 4-methyl-thiophen-2-yl boronic acid to provide 1-benzoyl-4-[7-(4-methyl-thiophen-2-yl)-4-azaindol-3-yl)-oxoacetyl]piperazine. MS *m*/*z*: (M+H)⁺ Calc'd for C₂₅H₂₃N₄O₃S: 459.15; found 459.08. HPLC retention time: 1.26 minutes (column G).

### Example 152

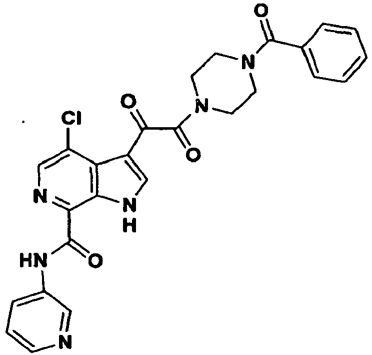

### Preparation of Example 152:

To a mixture of acid **intermediate 16** (30 mg, 68 µmol), 3-aminopyridine (26 mg, 0.27 mmol) and DMAP (50 mg, 0.41 mmol) was added THF (2 ml), and then EDC (60 mg, 0.31 mmol). The reaction mixture was stirred at ambient temperature for 16 hours. The LC/MS analysis indicated that the major product was the activated ester. The reaction mixture was then added into a DMF (2 ml) solution of 3-aminopyridine (400 mg, 4.25 mmol) and stirred at ambient temperature for 16 hours. After addition of MeOH (4 ml), the reaction mixture was purified by preparative reverse phase HPLC to give the TFA salt of the title compound using the method: Start %B = 30, Final %B = 75, Gradient time = 25 min, Flow Rate = 25 ml/min, Column : YMC C18 5um 20 x 100mm, Fraction Collection: 10.41 - 11.08 min. **¹H NMR:** (DMSO-*d₆*) δ 13.04 (s, 1H), 11.17 (s, 1H), 9.17 (s, 1H), 8.53 (s, 1H), 8.35 (m, 3H), 7.44 (b s, 6H), 3.75 - 3.37 (b m, 8H); **LC/MS:** (ES+) m/z (M+H)⁺ = 517, 519; HPLC Rₜ = 1.653.

### Example 143

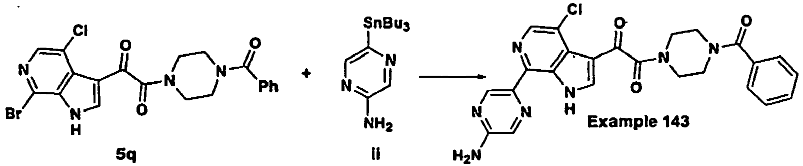

### Prep of Example 143:

To a mixture of intermediate 5q (31 mg, 65 µmol) and Pd(PPh₃)₄ (20 mg, 17 µmol) was added 1,4-dioxane (1 ml) and ii (30 mg, 78 µmol). The reaction mixture was heated in a sealed tube at 145°C for 4 hours. After cooling to ambient temperature, the reaction mixture was added MeOH (4 ml) and then filtered. The filtrate was purified by preparative reverse phase HPLC to give the TFA salt of the title compound using the method: Start %B = 25, Final %B = 90, Gradient time = 20 min, Flow Rate = 25 ml/min, Column : YMC C18 5um 20 x 100mm, Fraction Collection: 11.14 - 11.92 min. **¹H NMR:** (DMSO-*d₆*) δ 12.71 (s, 1H), 9.01 (s, 1H), 8.36 (s, 1H), 8.27 (s, 1H), 8.08 (s, 1H), 7.44 (b s, 5H), 7.44 (b s, 2H), 3.75 - 3.37 (b m, 8H); **LC/MS:** (ES+) m/z (M+H)⁺= 490, 492; HPLC Rₜ = 2.250.

### Example 149

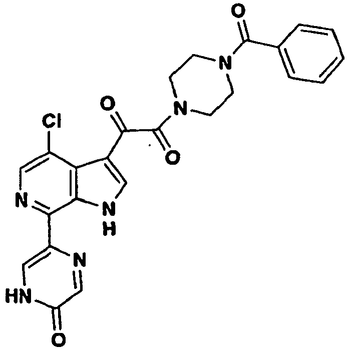

### Preparation of Example 49:

To a suspension of compound of Example 143 (12 mg, 24 µmol) in sulfuric acid (5%, 2 ml), was charged sodium nitrite (22 mg, 0.32 mol) at 0°C. The reaction mixture was stirred at 0°C for 30 minutes and then at ambient temperature for 1 hour. After addition of MeOH (4 ml), the reaction mixture was purified by preparative reverse phase HPLC to give a TEA solvate of title compound using the method: Start %B = 20, Final %B = 85, Gradient time = 15 min, Flow Rate = 25 ml/min, Column : YMC C18 5um 20 x 100mm, Fraction Collection: 10.67- 11.36 min. **¹H NMR:** (DMSO-*d₆*) δ 12.62 (s, 1H), 8.45 (s, 1H), 8.35 (s, 1H), 8.29 (s, 1H), 8.18 (s, 1H), 7.44 (b s, 5H), 3.80 - 3.30 (b m, 8H); **LC/MS:** (ES+) m/z (M+H)⁺ = 491, 493; HPLC Rₜ = 2.193.

### Example 144

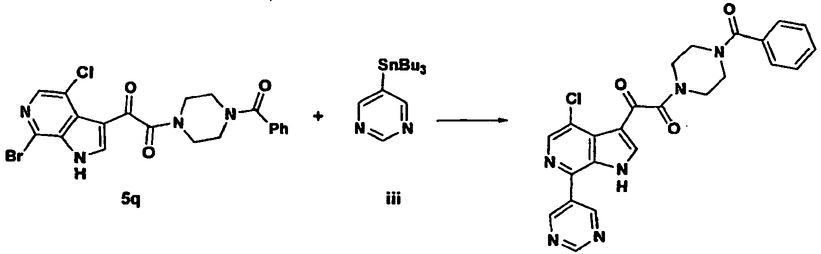

### Preparation of Example 144:

To a mixture of intermediate 5q (50 mg, 105 µmol) and Pd(PPh₃)₄ (50 mg, 43 µmol) was added 1,4-dioxane (1 ml) and iii (77 mg, 210 µmol). The reaction mixture was heated in a sealed tube at 145°C for 16 hours. After cooling to ambient temperature, the reaction mixture was added MeOH (4 ml) and then filtered. The filtrate was purified by reverse phase HPLC to give the TFA salt of the title compound of using the method: Start %B = 15, Final %B = 100, Gradient time = 20 min, Flow Rate = 25 ml/min, Column : YMC C18 5um 20 x 100mm, Fraction Collection: 11.80 - 12.31 min. **¹H NMR:** (CD₃OD) δ 9.32 (s, 1H), 9.25 (s, 2H), 8.50 (s, 1H), 8.44 (s, 1H), 7.47 (b s, 5H), 4.00 - 3.44 (b m, 8H); **LC/MS:** (ES+) m/z (M+H)⁺ = 475, 477; HPLC Rₜ = 1.833.

### EXAMPLE 87

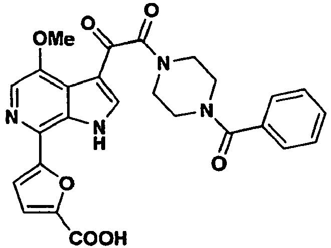

Preparation of **Example 87,** 1-benzoyl-4-[(4-methoxy-7-(2-hydroxycarbonyl-furan-5-yl)-6-azaindol-3-yl)-oxoacetyl]piperazine: A mixture of the compound of **Example 85** (19 mg), NaClO₂ (9.2 mg) in a mixed solution of CH₃CN (3 mL) and water (0.5 mL) was stirred at room temperature for 24 hours. After the reaction was quenched by 1N NaOH solution (1 ml), the mixture was extracted with diethyl ether (3 x 10 mL). The aqueous phase was acidified with 1N HCl to give a yellow solid precipitate (5mg) which was the product shown. MS *m*/*z*: (M+H)⁺ Calc'd for C₂₆H₂₃N₆O₇: 503.16; found 503.19. HPLC retention time: 1.37 minutes (column A).

### General Procedure of Converting -NH₂ Group to -NHCOR Group

Preparation of Example 99, 1-(benzoyl)-4-[(4-methoxy-7-(2-acetylamino-pyrazin-5-yl)-6-azaindol-3-yl)-oxoacetyl]piperazine: 1-(benzoyl)-4-[(4-methoxy-7-(2-amino-pyrazin-5-yl)-6-azaindol-3-yl)-oxoacetyl]piperazine (4mg) andacetic anhydride (20mg) were dissolved in pyridine (0.5ml). The reaction was stirred for three hours at room temperature. After reaction was quenched with Meoh (1ml), solvents were concentrated to give a residue which was purified using a Shimadzu automated preparative HPLC System to provide 3.0 mg of the desired compound, 1-(benzoyl)-4-[(4-methoxy-7-(2-acetylamino-pyrazin-5-yl)-6-azaindol-3-yl)-oxoacetyl]piperazine. ¹H NMR (500 MHz, CD₃OD) δ9.58 (s, 1H), 9.25 (s, 1H), 8.45 (s, 1H), 8.10 (s, 1H), 7.49 (m, 5H), 4.12 (s, 3H), 3.84 - 3.35 (m, 8H), 2.27 (s, 3H). MS *m*/*z*: (M+H)⁺ Calc'd for C₂₇H₂₆N₇O₅: 528.20; found 528.22. HPLC retention time: 1.33 minutes (column A).

### General Procedure of Converting -NH₂ Group to -OH Group

Preparation of Example 97, 1-(benzoyl)-4-[(4-methoxy-7-(2-hydroxyl-pyrazin-5-yl)-6-azaindol-3-yl)-oxoacetyl]piperazine: 1-(benzoyl)-4-[(4-methoxy-7-(2-amino-pyrazin-5-yl)-6-azaindol-3-yl)-oxoacetyl]piperazine (15 mg) and NaNO₂ (10 mg) was added into a H₂SO₄ solution (0.1ml of concentrated H₂SO₄ diluted with 0.3 ml of water). The reaction was stirred at room temperature for one hour. Then, the reaction mixture was neutralized with a saturated Na₂CO₃ solution (10 ml). The solvents were concentrated to give a residue which was purified using a Shimadzu automated preparative HPLC System to provide 4.2mg of the desired compound, 1-(benzoyl)-4-[(4-methoxy-7-(2-hydroxyl-pyrazin-5-yl)-6-azaindol-3-yl)-oxoacetyl]piperazine. ¹H NMR (500 MHz, CD₃OD) δ8.55 (s, 1H), 8.44 (s, 1H), 8.31 (s, 1H), 8.01 (s, 1H), 7.49 (m, 5H), 4.12 (s, 3H), 3.84 - 3.64 (m, 8H). MS *m*/*z*: (M+H)⁺ Calc'd for C₂₅H₂₃N₆O₅: 487.17; found 487.22. HPLC retention time: 1.13 minutes (column A). 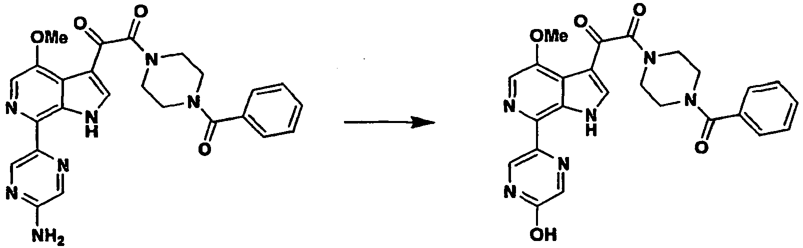 This general procedure is applied to prepare examples 121, 122,123,124,155, 157, and 162.

### Example 121

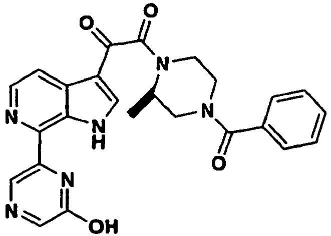

Example 121, (R)-1-(benzoyl)-3-methyl-4-[(4-methoxy-7-(2-hydroxyl-pyrazin-6-yl)-6-azaindol-3-yl)-oxoacetyl]piperazine. MS *m*/*z*: (M+H)⁺ Calc'd for C₂₅H₂₃N₆O₄: 471.18; found 471.17. HPLC retention time: 1.39 minutes (column G).

### Example 121-2

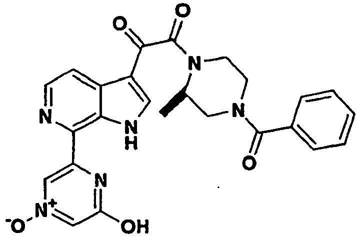

Example 121-2, (R)-1-(benzoyl)-3-methyl-4-[(4-methoxy-7-(2-hydroxyl-4-oxo-pyrazin-6-yl)-6-azaindol-3-yl)-oxoacetyl]piperazine was isolated during the preparation of Example 121. MS *m*/*z*: (M+H)⁺ Calc'd for C₂₅H₂₃N₆O₅: 487.17; found 487.17. HPLC retention time: 1.08 minutes (column G).

### Example 122

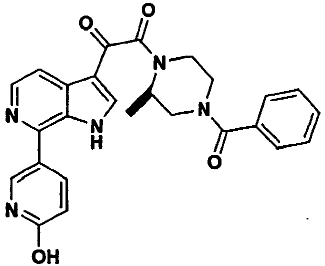

Example 122, (R)-1-(benzoyl)-3-methyl-4-[(4-methoxy-7-(2-hydroxyl-pyridin-5-yl)-6-azaindol-3-yl)-oxoacetyl]piperazine. MS *m*/*z*: (M+H)⁺ Calc'd for C₂₆H₂₄N₅O₄: 470.18; found 470.17. HPLC retention time: 1.03 minutes (column G).

### Example 123

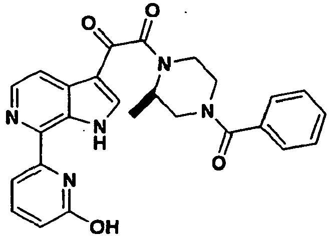

Example 123, (R)-1-(benzoyl)-3-methyl-4-[(4-methoxy-7-(2-hydroxyl-pyridin-6-yl)-6-azaindol-3-yl)-oxoacetyl]piperazine. MS *m*/*z:* (M+H)⁺ Calc'd for C₂₆H₂₄N₅O₄: 470.18; found 470.14. HPLC retention time: 1.28 minutes (column G).

### Example 124

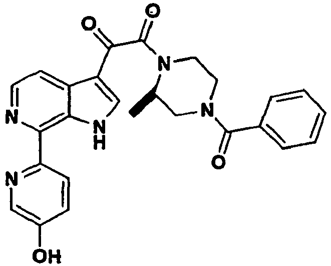

Example 124, (R)-1-(benzoyl)-3-methyl-4-[(4-methoxy-7-(5-hydroxyl-pyridin-2-yl)-6-azaindol-3-yl)-oxoacetyl]piperazine. MS *m*/*z:* (M+H)⁺ Calc'd for C₂₆H₂₄N₅O₄: 470.18; found 470.13. HPLC retention time: 1.21 minutes (column G).

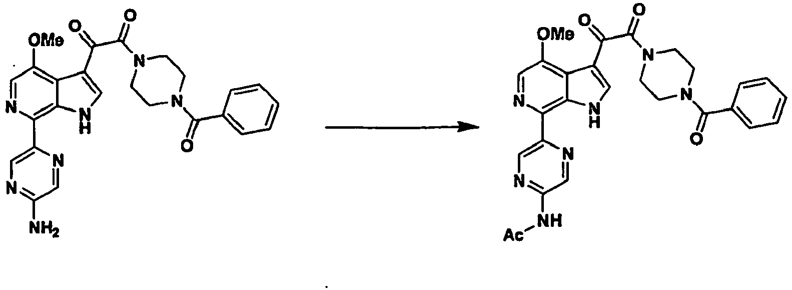

### Preparation of Example 138

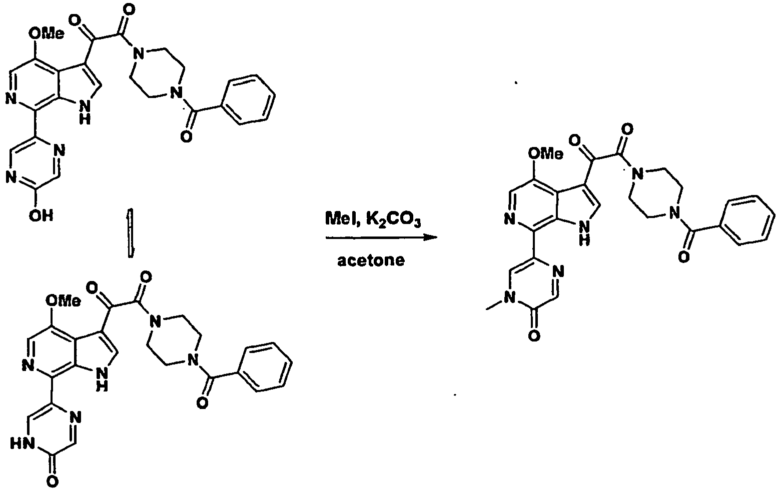

Preparation of Example 138, 1-(benzoyl)-4-[(4-methoxy-7-(1-methylpyrazin-2-on-5-yl)-6-azaindol-3-yl)-oxoacetyl]piperazine: 1-(benzoyl)-4-[(4-methoxy-7-(2-hydroxyl-pyrazin-5-yl)-6-azaindol-3-yl)-oxoacetyl]piperazine (6mg), MeI (5mg) and K₂CO₃ (4 mg)were dissolved in acetone (5 ml). The reaction was stirred for four hours at room temperature. After solid was filtered away, the mother liquid was concentrated to give a residue which was purified using a Shimadzu automated preparative HPLC System to provide 3.0 mg of the desired compound, 1-(benzoyl)-4-[(4-methoxy-7-(1-methylpyrazin-2-on-5-yl)-6-azaindol-3-yl)-oxoacetyl]piperazine. MS *m*/*z:* (M+H)⁺ Calc'd for C₂₆H₂₅N₆O₅: 501.19; found 501.14. HPLC retention time: 1.08 minutes (column G).

### Example 139

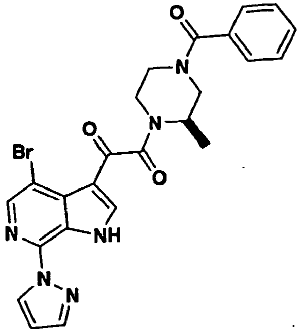

Intermediate 4i was dissolved in DMF (2 ml), and to which *N*-benzoyl-(*R*)-methylpiperazine hydrochloride (0.092 g, 0.45 mmol) and 3-(diethoxyphosphoryloxy)-1,2,3-benzotriazin-4(3*H*)-one (DEPBT, 0.180 g, 0.60 mmol) were added, followed by *N,N*-diisopropylethylamine (0.15 ml, 0.87 mmol). The reaction mixture was stirred for 2 h at r.t., and then the volatile evaporated under high vacuum. Water was added to the mixture to induce precipitation, and the solids were filtered and dried *in vacuo.* Purification of the crude solid by preparative thin layer chromatography (5% MeOH/ CH₂Cl₂), and subsequent washing with ether gave **the title compound; ¹H NMR:** (CDCl₃) δ 8.78 (s, 1H), 8.32 (s, 1H), 8.28 (s, 1H) 7.84 (s, 1H), 7.44 (b s, 5H), 6.56 (s, 1H), 5.00-3.00 (b m, 7H), 1.45-1.20 (b s, 3H); **LC/MS:** (ES+) m/z (M+H)⁺ = 521, 523; HPLC Rₜ = 1.677

### Example 140

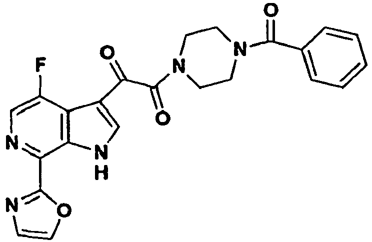

The title compound was prepared according to general procedures described before (Sn-coupling). H NMR: 8.41(m, 1H); 8.33(m, 1H); 8.16(m, 1H); 7.53(m, 1H); 7.47(bs, 5H); 3.97-3.54(m, 8H). LC/MS: (ES⁺) m/z(m+H)⁺ = 448, Rt = 1.28min.

### Example 141

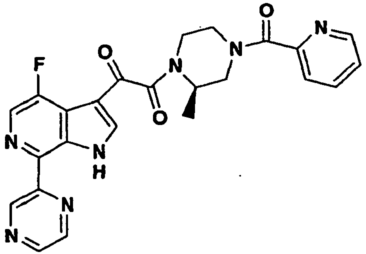

The title compound was prepared according to general procedures described before (Sn-coupling). ¹H-NMR: 9.71-9.70(m, 1H); 8.80-8.79(m, 1H); 8.66-8.42(m, 2H); 8.41-8.35(m, 2H); 7.99-7.92(m,1H), 7.69-7.53(m, 1H); 7.48-7.44(m, 1H); 5.05-3.15(m, 8H). LC/MS: (ES⁺) m/z (m+H)⁺ = 474. Rt = 1.26min.

### Example 144

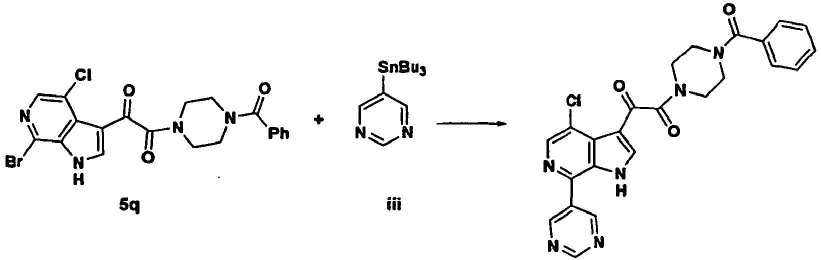

### Preparation of Example 144:

To a mixture of intermediate 5q (50 mg, 105 µmol) and Pd(PPh₃)₄ (50 mg, 43 µmol) was added 1,4-dioxane (1 ml) and iii (77 mg, 210 µmol). The reaction mixture was heated in a sealed tube at 145°C for 16 hours. After cooling to ambient temperature, the reaction mixture was added MeOH (4 ml) and then filtered. The filtrate was purified by reverse phase HPLC to give the TFA salt of the title compound of using the method: Start %B = 15, Final %B =100, Gradient time = 20 min, Flow Rate = 25 ml/min, Column : YMC C18 5um 20 x 100mm, Fraction Collection: 11.80 - 12.31 min. **¹H NMR:** (CD₃OD) δ 9.32 (s, 1H), 9.25 (s, 2H), 8.50 (s, 1H), 8.44 (s, 1H), 7.47 (b s, 5H), 4.00 - 3.44 (b m, 8H); **LC/MS:** (ES+) m/z (M+H)⁺ = 475, 477; HPLC Rₜ = 1.833.

### Example 145

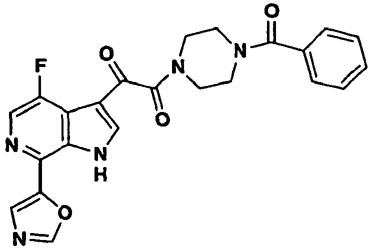

The title compound was prepared following the procedure described before for example 146 and intermediate 4k. ¹H NMR: 8.35-8.33(m, 2H); 8.11(s, 1H); 7.89(s, 1H); 7.43(bs, 5H); 3.89-3.49(m, 8H). LC/MS: (ES⁺) m/z (M+H)⁺ = 448. Rt = 1.18min.

### Example 146

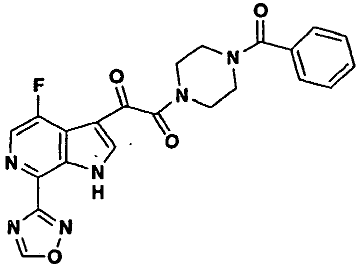

Intermediate 4m (0.26 mmol) was dissolved in DMF (1 mL) and treated with *N-*benzoylpiperazine hydrochloride (59 mg, 0.26 mmol), DEBPT (79 mg, 0.26 mmol) and Hunig's base (90 µL, 0.52 mmol) and the reaction mixture was stirred at rt for 18h. The solvent was removed in vacuum and the residue was purified by reverse phase preparative HPLC. The fractions showing the right LC/MS:(ES⁺) m/z (M+H)⁺ = 449 were collected, concentrated and purified again using a preparative TLC (5% MeOH/CH₂Cl₂) to afford the title compound as a white solid. ¹H-NMR (500 MHz, CDCl₃) δ 10.7 (s, 1H), 9.00 (s, 1H), 8.54 (s, 1H), 8.39 (s, 1H), 7.45 (m, 5H), 3.9-3.5 (bm, 8H).

### Example 148

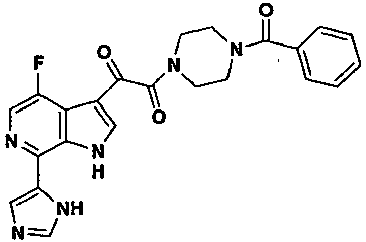

The title compound was prepared from intermediate 4n using the same coupling conditions described for the last step of the preparation of intermediate 5i. ¹H NMR: 8.82(m, 1H); 8.48-8.45(m, 1H); 8.37-8.33(m, 1H); 8.26-8.23(m, 1H); 7.47(bs, 5H); 3.97-3.54(m, 8H). LC/MS: (ES⁺ m/z(m+H)⁺ = 447 Rt = 0.94min.

### Example 151

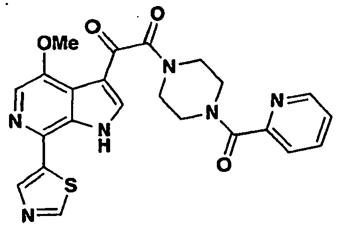

Example 151, was prepared from Intermediate 51 and the thiazol-5-yl stannane to provide 1-picolinoyl-4-[(4-methoxy-7-(thiazol-5-yl)-6-azaindol-3-yl)-oxoacetyl]piperazine. MS *m*/*z*: (M+H)⁺ Calc'd for C₂₃H₂₁N₆O₄S: 477.13; found 477.13. HPLC retention time: 0.94 minutes (column G).

### Example 154

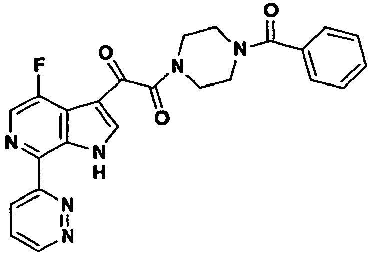

The title compound was prepared according to general procedures described before (Sn-coupling). ¹H-NMR: 9.23-9.22 (m, 1H); 8.83-8.81(m, 1H); 8.43 (m, 1H); 8.36 (m, 1H); 7.75-7.73 (m,1H), 7.44 (bs, 5H); 3.85-3.49 (m, 8H). LC/MS: (ES⁺) m/z (M+H)⁺ = 459. Rt = 1.39min.

### Example 155

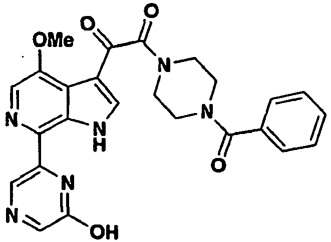

Example 155, 1-(benzoyl)-4-[(4-methoxy-7-(2-hydroxyl-pyrazin-5-yl)-6-azaindol-3-yl)-oxoacetyl]piperazine. MS *m*/*z*: (M+H)⁺ Calc'd for C₂₅H₂₃N₆O₅: 487.17; found 487.14. HPLC retention time: 1.30 minutes (column G).

### Example 157

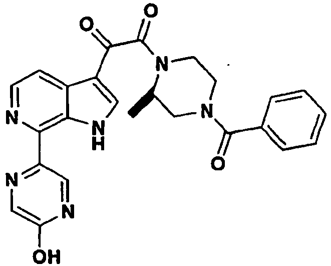

Example 157, (R)-1-(benzoyl)-3-methyl-4-[(4-methoxy-7-(5-hydroxyl-pyrazin-2-yl)-6-azaindol-3-yl)-oxoacetyl]piperazine. MS *m*/*z*: (M+H)⁺ Calc'd for C₂₅H₂₃N₆O₄: 471.18; found 471.16. HPLC retention time: 1.09 minutes (column G).

### Example 161

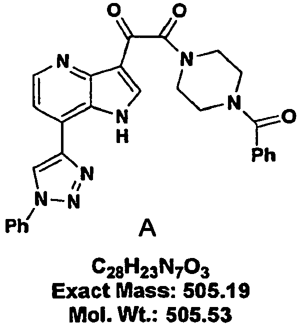

**Procedure as usual to yield A:** ¹H NMR (500 MHz, DMSO) δ 9.67 (s, 1H), 8.81 (s, 1H), 8.72 (d, *J* = 5.4 Hz, 1H), 8.25 (d, *J* = 6.1 Hz, 1H), 8.00 (dd, *J* = 8.2, 1.8 Hz, 1H), 7.68 (dd, *J*= 8.2, 7.4 Hz, 2H), 7.60 (tt, *J*= 7.4, 1.8 Hz, 2H), 7.48 (br s, 5H), 4.04-3.46 (m, 8H). MS *m*/*z*: (M+H)⁺ calcd for C₂₈H₂₄N₇O₃: 506.19; found 506.15. HPLC retention time: 1.21 minutes (XTERRA C18 S7 3.0 x 50 mm)).

### Example 162

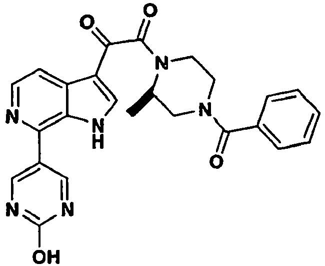

Example 162, (R)-1-(benzoyl)-3-methyl-4-[(4-methoxy-7-(2-hydroxyl-pyrimidin-5-yl)-6-azaindol-3-yl)-oxoacetyl]piperazine. MS *m*/*z*: (M+H)⁺ Calc'd for C₂₅H₂₃N₆O₄: 471.18; found 471.13. HPLC retention time: 0.95 minutes (column G).

### Example 163

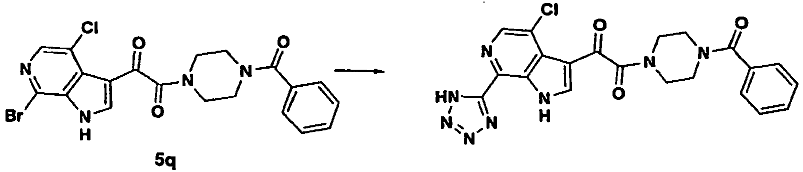

To a solution of intermediate 5q (50 mg, 0.11 mmol) in DMF (1 ml) was added CuCN (30 mg, 0.335 mmol). The reaction mixture was heated at 170°C for 30 min. After cooling to ambient temperature, the reaction mixture was diluted with MeOH (15 ml), filtered under gravity, and the filtrate evaporated *in vacuo* to afforded a brownish residue which is a cyanointermediate. To the residue in DMF (1 ml) was added sodium azide (61 mg, 0.95 mmol) and ammonium chloride (50 mg, 0.95 mmol). The mixture was heated at 90°C for one hour. The reaction mixture was then diluted with MeOH (4 ml), filtered, and the filtrate purified by preparative reverse phase HPLC using the method: Start %B = 20, Final %B = 80, Gradient time = 15 min, Flow Rate = 40 ml/min, Column : XTERRA C18 5 um 30 x 100 mm, Fraction Collection: 11.26 - 11.71 min. The material was homogenous by ¹H NMR and HPLC, although the mass spectrum indicated an extra peak at (M+H)⁺ = 431; **¹H NMR:** (CD₃OD) 8.41 (s, 1H), 8.12 (s, 1H), 7.47 (b s, 5H), 3.97 - 3.47 (b m, 8H); **LC/MS:** (ES+) m/z (M+H)⁺= 465, 467; HPLC Rₜ = 1.937

### Example 164

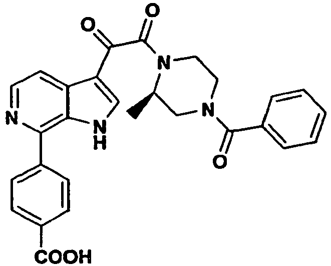

Example 164, was prepared from Intermediate 5a and the 4-hydroxycarbonylphenyl boronic acid to provide 1-benzoyl-4-[7-(4-hydroxycarbonylphenyl)-4-azaindol-3-yl)-oxoacetyl]piperazine. MS *m*/*z*: (M+H)⁺ Calc'd for C₂₈H₂₅N₄O₅: 497.18; found 497.22. HPLC retention time: 1.20 minutes (column C).

### Example 165

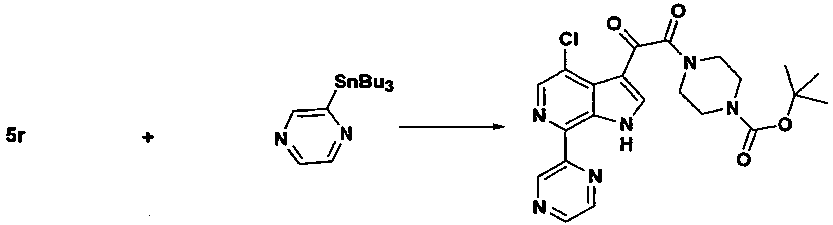

Compound of Example **165** was prepared in a similar manner to compound of Example **143** starting with intermediate **5r**, but at 125°C for 22 hours and purification by preparative thin layer chromatography (4% MeOH/CH₂Cl₂). **¹H NMR:** (CDCl₃) δ 11.85 (s, 1H), 9.91 (d, *J*= 1.6 Hz, 1H), 8.70 (d, *J*= 2.6 Hz, 1H), 8.65 (dd, *J* = 1.6, 2.6 Hz, 1H), 8.52 (s, 1H), 8.35 (d, *J* = 3.1 Hz, 1H), 3.73 (b m, 2H), 3.56 (b m, 4H), 3.53 (b m, 2H), 1.48 (s, 9H); **LC/MS**: (ES+) m/z (M+H)⁺= 471, 473; HPLC Rₜ = 1.690.

### Example 167

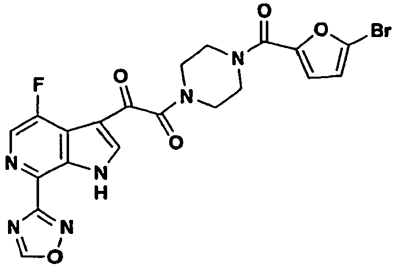

Intermediate 4m (0.098 mmol) was dissolved in DMF (1 mL) and treated with *N*-[5-(2-Bromofuroyl)]piperazine hydrochloride (30 mg, 0.098 mmol), DEBPT (60 mg, 0.19 mmol) and Hunig's base (70 µL, 0.19 mmol) and the reaction mixture was stirred at rt for 18h. The solvent was removed in vacuum and the residue was purified by reverse phase preparative HPLC. The fractions showing the right LC/MS:(ES⁺) m/z (M+H)⁺ = 518,520 were collected, concentrated and purified again using a preparative TLC (5% MeOH/CH₂Cl₂) to afford the title compound as a white solid. ¹H-NMR (500 MHz, CDCl₃) δ 10.7 (s, 1H), 9.00 (s, 1H), 8.54 (s, 1H), 8.40 (s, 1H), 7.06 (d, *J*=3.4 Hz, 1H), 6.46 06 (d, *J*=3.4 Hz, 1H), 3.90-3.66 (bm, 8H).

### Example 168

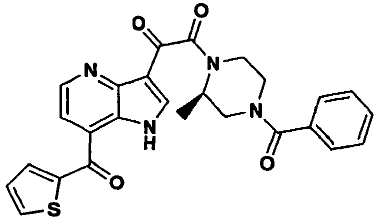

Example 168, 1-benzoyl-3-(R)-methyl-4-[(7-(2-thienylcarbonyl)-4-azaindol-3-yl)-oxoacetyl]piperazine, was prepared from a reaction 1-benzoyl-3-(R)-methyl-4-[(7-(methoxymethylamino)carbonyl)-4-azaindol-3-yl)-oxoacetyl]piperazine and 2-thienyl lithium by using the same procedure for the preapartion of I-64, 1-benzoyl-3-(R)-methyl-4-[(7-(2-propynyl)carbonyl-4-azaindol-3-yl)-oxoacetyl]piperazine. MS *m*/*z*: (M+H)⁺ Calc'd for C₂₆H₂₃N₄O₄S: 487.14; found 487.11. HPLC retention time: 1.31 minutes (column A).
6 and hereafter, the following definitions apply.

### Biology

- "µM" means micromolar;
- "mL" means milliliter;
- "µl" means microliter;
- "mg" means milligram;

The materials and experimental procedures used to obtain the results reported in Tables 1-5 are described below.

### Cells:

- Virus production-Human embryonic Kidney cell line, 293, propagated in Dulbecco's Modified Eagle Medium (Life Technologies, Gaithersburg, MD) containing 10% fetal Bovine serum (FBS, Sigma, St. Louis, MO).
- Virus infection- Human epithelial cell line, HeLa, expressing the HIV-1 receptors CD4 and CCR5 was propagated in Dulbecco's Modified Eagle Medium (Life Technologies, Gaithersburg, MD) containing 10% fetal Bovine serum (FBS, Sigma, St. Louis , MO) and supplemented with 0.2 mg/mL Geneticin (Life Technologies, Gaithersburg, MD) and 0.4 mg/mL Zeocin (Invitrogen, Carlsbad, CA).

**Virus**-Single-round infectious reporter virus was produced by co-transfecting human embryonic Kidney 293 cells with an HIV-1 envelope DNA expression vector and a proviral cDNA containing an envelope deletion mutation and the luciferase reporter gene inserted in place of HIV-1 nef sequences (Chen et al, Ref. 41). Transfections were performed using lipofectAMINE PLUS reagent as described by the manufacturer (Life Technologies, Gaithersburg, MD).

### Experiment

1. Compound was added to HeLa CD4 CCR5 cells plated in 96 well plates at a cell density of 5 X 10⁴ cells per well in 100 µl Dulbecco's Modified Eagle Medium containing 10 % fetal Bovine serum at a concentration of <20 µM.
2. 100 µl of single-round infectious reporter virus in Dulbecco's Modified Eagle Medium was then added to the plated cells and compound at an approximate multiplicity of infection (MOI) of 0.01, resulting in a final volume of 200 µl per well and a final compound concentration of <10 µM.
3. Samples were harvested 72 h after infection.
4. Viral infection was monitored by measuring luciferase expression from viral DNA in the infected cells using a luciferase reporter gene assay kit (Roche Molecular Biochemicals, Indianapolis, IN). Infected cell supernatants were removed and 50 µl of Dulbecco's Modified Eagle Medium (without phenol red) and 50 µl of luciferase assay reagent reconstituted as described by the manufacturer (Roche Molecular Biochemicals, Indianapolis, IN) was added per well. Luciferase activity was then quantified by measuring luminescence using a Wallac microbeta scintillation counter.
5. The percent inhibition for each compound was calculated by quantifying the level of luciferase expression in cells infected in the presence of each compound as a percentage of that observed for cells infected in the absence of compound and subtracting such a determined value from 100.
6. An EC₅₀ provides a method for comparing the antiviral potency of the compounds of this invention. The effective concentration for fifty percent inhibition (EC₅₀) was calculated with the Microsoft Excel Xlfit curve fitting software. For each compound, curves were generated from percent inhibition calculated at 10 different concentrations by using a four paramenter logistic model (model 205). The EC₅₀ data for the compounds is shown in Tables 5-6. Table 1 is the key for the data in Tables 5-6.

### Results

**Table 4. Biological Data Key for EC₅₀s**

| **Compounds*** with EC₅₀a >5µM | **Compounds** with EC₅₀a >1 µM but <5µM | **Compounds with** EC50 >50nM but not yet tested at higher concentrations | **Compounds with** EC50 < 1 µM |
|---|---|---|---|
| **Group C** | **Group B** | **Group A'** | **Group A** |

| | | | |
|---|---|---|---|
| *Some of these compounds may have been tested at a concentration lower than their EC₅₀ but showed some ability to cause inhibition and thus should be evaluated at a higher concentration to determine the exact EC₅₀. | | | |

In Tables 5-9, X₂, X₄ etc. indicates the point of attachment.

**Table 5**

| | | | | | | |
|---|---|---|---|---|---|---|
| 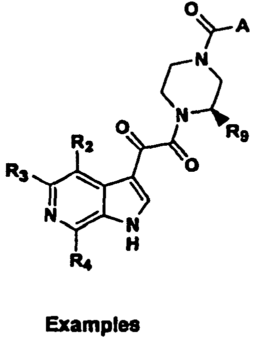 | | | | | | |

| | | | | | | |
|---|---|---|---|---|---|---|
| Table Entry (Example Number.) | R2 | R3 | R4 | R9 | A | EC₅₀ Group from Table 1 |
| 1 (Example 1) | H | H | 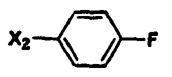 | CH₃ | 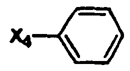 | A |
| 2 (Example 2) | H | H | 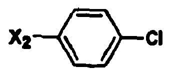 | CH₃ | 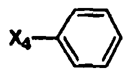 | A |
| 4 (Example 4) | H | H | 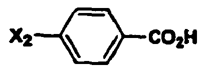 | H | 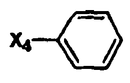 | A |
| 5 (Example 5) | H | H | 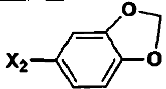 | CH₃ | 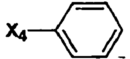 | A |
| 6 (Example 6) | H | H | 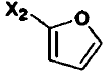 | CH₃ | 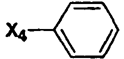 | A |
| 7 (Example 7) | H | H | 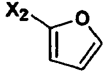 | H | 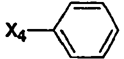 | A |
| 8 (Example 8) | H | H | 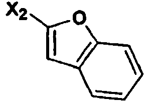 | H | 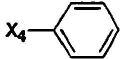 | A |
| 9 (Example 9) | H | H | 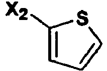 | CH₃ | 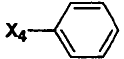 | A |
| 10 (Example 16) | H | H | 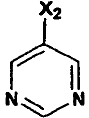 | CH₃ | 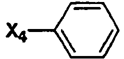 | A |
| 11 (Example 17) | H | H | 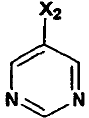 | H | 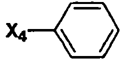 | A |
| 12 (Example 18) | H | H | 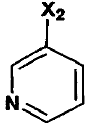 | CH₃ | 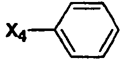 | A |
| 13 (Example 10) | H | H | 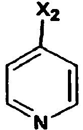 | H | 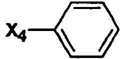 | A |
| 14 (Example 19) | H | H | 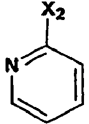 | H | 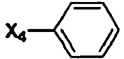 | A |
| 15 (Example 11) | H | H | 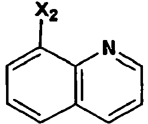 | H | 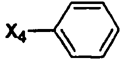 | A' |
| 16 (Example 20) | H | H | 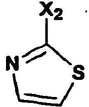 | CH₃ | 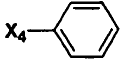 | A |
| 17 (Example 21) | H | H | 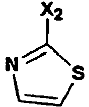 | H | 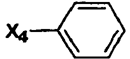 | A |
| 18 (Example 22) | OMe | H | 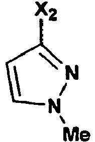 | H | 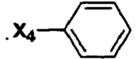 | A |
| 19 (Example 23) | OMe | H | 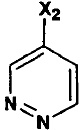 | H | 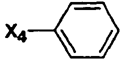 | A |
| 20 (Example 24) | OMe | H | 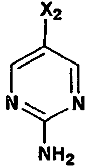 | H | 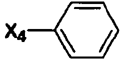 | A |
| 21 (Example 25) | OMe | H | 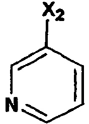 | H | 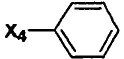 | A |
| 22 (Example 26) | OMe | H | 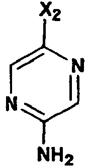 | H | 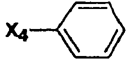 | A |
| 23 (Example 27) | OMe | H | 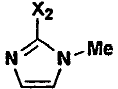 | H | 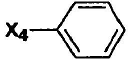 | A |
| 24 (Example 28) | OMe | H | 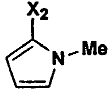 | H | 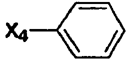 | A |
| 25 (Example 29) | F | H | 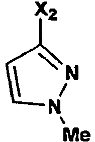 | H | 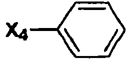 | A |
| 26 (Example 30) | F | H | 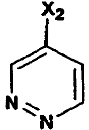 | H | 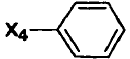 | A |
| 27 (Example 15) | OMe | H | 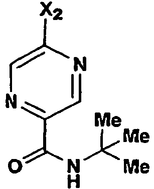 | H | 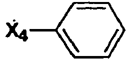 | A |
| 28 (Example 32) | OMe | H | 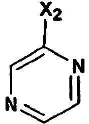 | H | 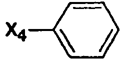 | A |
| 29 (Example 33) | H | H | 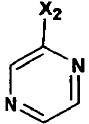 | Me | 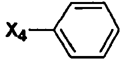 | A |
| 30 (Example 34) | H | H | 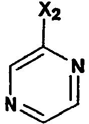 | H | 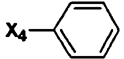 | A |
| 31 (Example 35) | OMe | H | 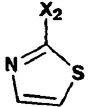 | H | 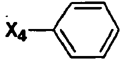 | A |
| 32 (Example 36) | OMe | H | 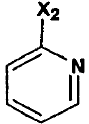 | H | 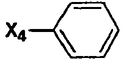 | A |
| 33 (Example 37) | F | H | 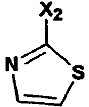 | Me | 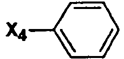 | A |
| 34 (Example 38) | F | H | 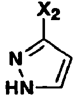 | H | 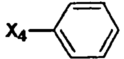 | A |
| 35 (Example 39) | OMe | H | 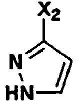 | H | 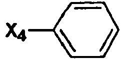 | A |
| 36 (Example 40) | OMe | H | 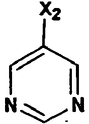 | H | 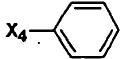 | A |
| 37 (Example 41) | F | H | 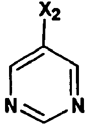 | Me | 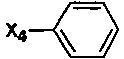 | A |
| 38 (Example 42) | F | H | 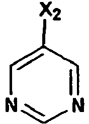 | H | 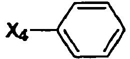 | A |
| 41 (Example 45) | OMe | H | 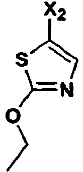 | H | 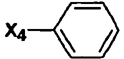 | A |
| 42 (Example 46) | OMe | H | 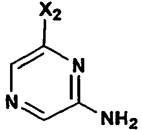 | H | 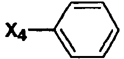 | A |
| 43 (Example 47) | OMe | H | 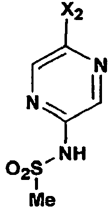 | H | 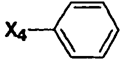 | A |
| 45 (Example 49) | OMe | H | 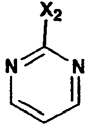 | H | 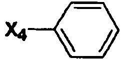 | A |
| 46 (Example 13) | OMe | H | 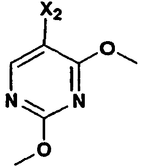 | H | 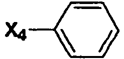 | A |
| 47 (Example 55) | OMe | H | 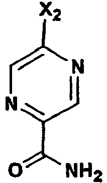 | H | 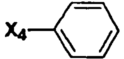 | A |
| 48 (Example 50) | OMe | H | 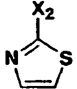 | H | 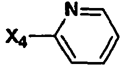 | A |
| 49 (Example 14) | OMe | H | 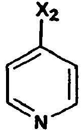 | H | 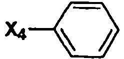 | A |
| 50 (Example 68) | OMe | H | 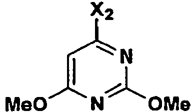 | H | 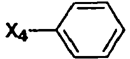 | A |
| 51 (Example 69) | OMe | H | 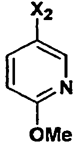 | H | 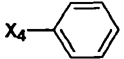 | A |
| 52 (Example 70) | OMe | H | 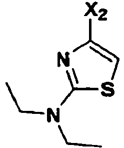 | H | 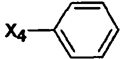 | A |
| 53 (Example 71) | OMe | H | 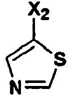 | H | 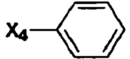 | A |
| 54 (Example 72) | OMe | H | 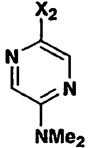 | H | 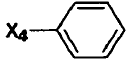 | A |
| 55 (Example 82) | OMe | H | 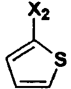 | H | 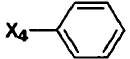 | A |
| 56 (Example 73) | OMe | H | 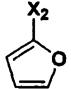 | H | 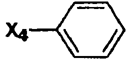 | A |
| 57 (Example 83) | OMe | H | 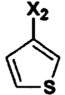 | H | 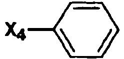 | A |
| 58 (Example 84) | OMe | H | 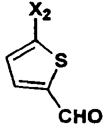 | H | 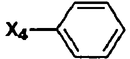 | A |
| 59 (Example 85) | OMe | H | 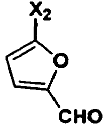 | H | 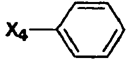 | A |
| 60 (Example 74) | OMe | H | 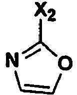 | H | 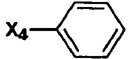 | A |
| 61 (Example 75) | OMe | H | 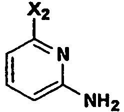 | H | 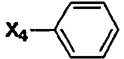 | A |
| 62 (Example 76) | OMe | H | 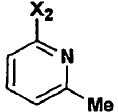 | H | 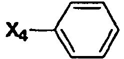 | A |
| 63 (Example 77) | OMe | H | 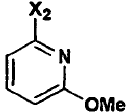 | H | 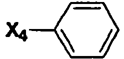 | A |
| 64 (Example 78) | OMe | H | 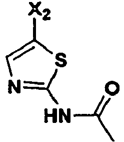 | H | 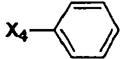 | A |
| 65 (Example 80) | OMe | H | 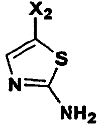 | H | 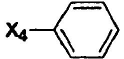 | A |
| 66 (Example 79) | OMe | H | 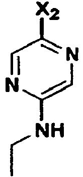 | H | 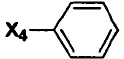 | A |
| 67 (Example 87) | OMe | H | 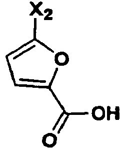 | H | 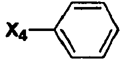 | A |
| 68 (Example 81) | OMe | H | 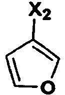 | H | 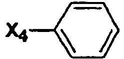 | A |
| 69 (Example 88) | OMe | H | 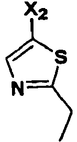 | H | 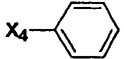 | A |
| 70 (Example 89) | H | H | 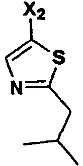 | H | 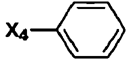 | A |
| 71 (Example 90) | OMe | H | 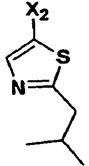 | H | 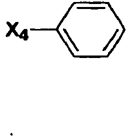 | A |
| 72 (Example 91) | OMe | H | 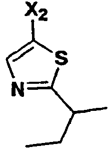 | H | 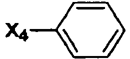 | A |
| 72 (Example 92) | OMe | H | 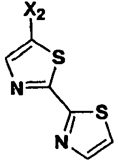 | H | 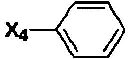 | A |
| 73 (Example 93) | OMe | H | 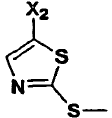 | H | 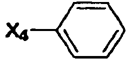 | A |
| 74 (Example 94) | OMe | H | 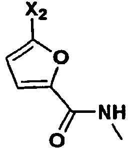 | H | 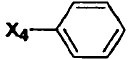 | A |
| 75 (Example 95) | F | H | 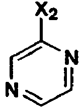 | H | 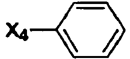 | A |
| 76 (Example 96) | Cl | H | 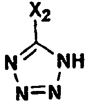 | H | 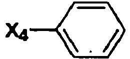 | A |
| 77 (Example 97) | OMe | H | 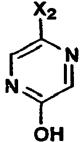 | H | 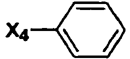 | A |
| 78 (Example 98) | OMe | H | 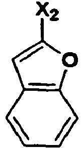 | H | 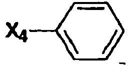 | A |
| 79 (Example 99) | OMe | H | 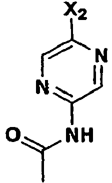 | H | 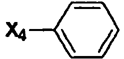 | A |
| 80 (Example 100) | OMe | H | 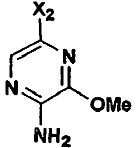 | H | 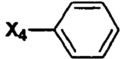 | A |
| 81 (Example 101) | OMe | H | 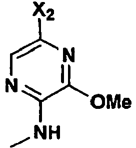 | H | 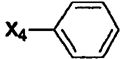 | A |
| 82 (Example 102) | OMe | H | 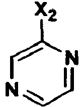 | H | 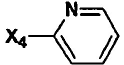 | A |
| 83 (Example 103) | OMe | H | 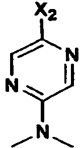 | H | 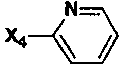 | A |
| 84 (Example 104) | OMe | H | 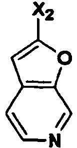 | H | 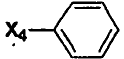 | A |
| 85 (Example 105) | H | H | 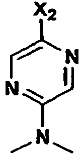 | (R)-Me | 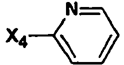 | A |
| 86 (Example 106) | H | H | 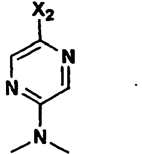 | (S)-Me | 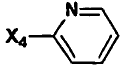 | A |
| 87 (Example 107) | H | H | 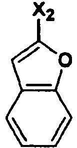 | (R)-Me | 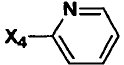 | A |
| 88 (Example 108) | H | H | 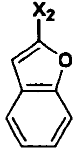 | (S)-Me | 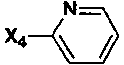 | A |
| 89 (Example 109) | H | H | 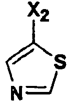 | (R)-Me | 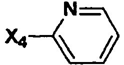 | A |
| 90 (Example 110) | H | H | 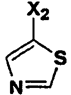 | (S)-Me | 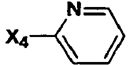 | A |
| 91 (Example 111) | H | H | 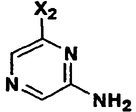 | (R)-Me | 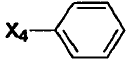 | A |
| 92 (Example 112) | H | H | 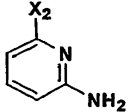 | (R)-Me | 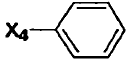 | A |
| 93 (Example 113) | H | H | 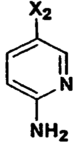 | (R)-Me | 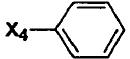 | A |
| 94 (Example 114) | H | H | 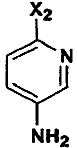 | (R)-Me | 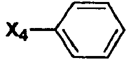 | A |
| 95 (Example 115) | OMe | H | 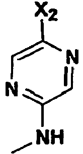 | H | 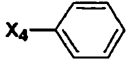 | A |
| 96 (Example 116) | OMe | H | 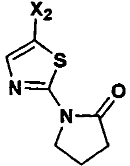 | H | 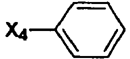 | A |
| 97 (Example 117) | OMe | H | 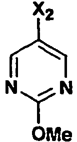 | H | 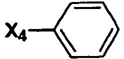 | A |
| 98 (Example 118) | OMe | H | 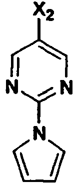 | H | 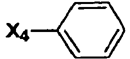 | A |
| 99 (Example 119) | OMe | H | 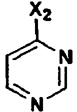 | H | 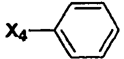 | |
| 100 (Example 120) | OMe | H | 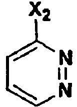 | H | 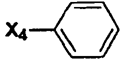 | |
| 101 (Example 121) | H | H | 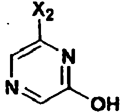 | (R)-Me | 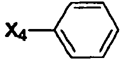 | |
| 102 (Example 121-2) | H | H | 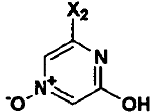 | (R)-Me | 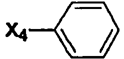 | |
| 103 (Example 122) | H | H | 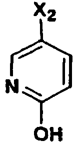 | (R)-Me | 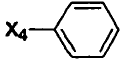 | |
| 104 (Example 123) | H | H | 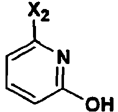 | (R)-Me | 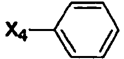 | |
| 105 (Example 124) | H | H | 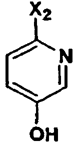 | (R)-Me | 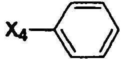 | |
| 106 (Example 125) | F | H | 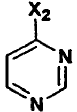 | H | 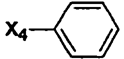 | |
| 107 (Example 138) | OMe | H | 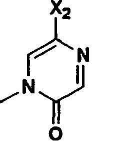 | H | 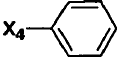 | |
| 108 (Example 139) | Br | H | 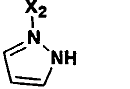 | (R)-Me | 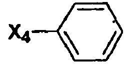 | |
| 109 (Example 140) | F | H | 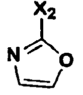 | H | 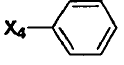 | |
| 110 (Example 141) | F | H | 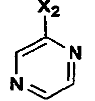 | (R)-Me | 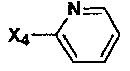 | |
| 111 | | | | | | |
| 112 (Example 143) | Cl | H | 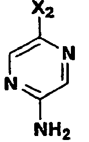 | H | 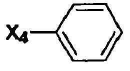 | |
| 113 (Example 144) | Cl | H | 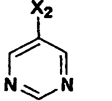 | H | 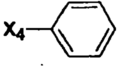 | |
| 114 (Example 145) | F | H | 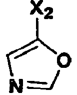 | H | 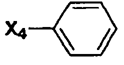 | |
| 115 (Example 146) | F | H | 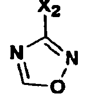 | H | 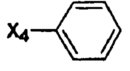 | |
| 116 (Example 147) | F | H | 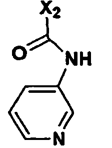 | H | 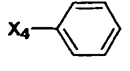 | |
| 117 (Example 148) | F | H | 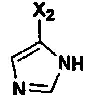 | H | 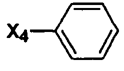 | |
| 118 (Example 149) | Cl | H | 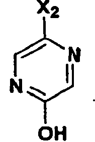 | H | 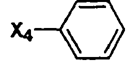 | |
| 119 (Example 150) | H | H | 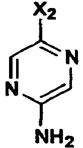 | (R)-Me | 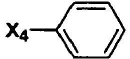 | |
| 120 (Example 151) | OMe | H | 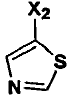 | H | 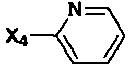 | |
| 121 (Example 152) | Cl | H | 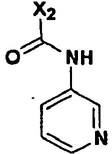 | H | 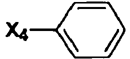 | |
| 122 (Example 153) | H | H | 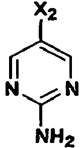 | (R)-Me | 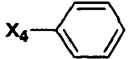 | |
| 123 (Example 154) | F | H | 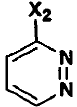 | H | 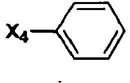 | |
| 124 (Example 155) | OMe | H | 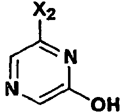 | H | 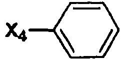 | |
| 125 (Example 156) | OMe | H | 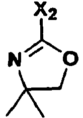 | H | 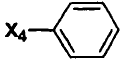 | |
| 126 (Example 157) | H | H | 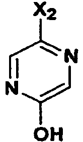 | (R)-Me | 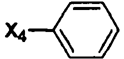 | |
| 127 (Example 165) | Cl | H | 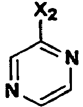 | H | 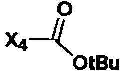 | |
| 128 (Example 166) | F | H | 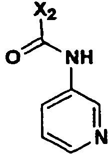 | H | 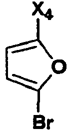 | |
| 129 (Example 167) | F | H | 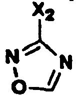 | H | 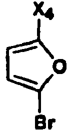 | |
| 130 (Example 162) | H | H | 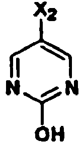 | (R)-Me | 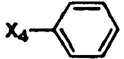 | |
| 131 (Example 163) | Cl | H | 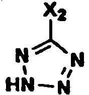 | H | 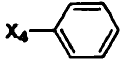 | |
| 132 (Example 164) | H | H | 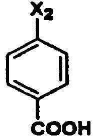 | (R)Me | 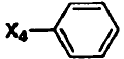 | |
| 133 (Example 169) | OMe | H | 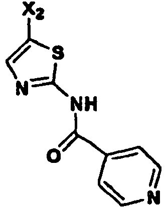 | H | 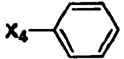 | |

**Table 6**

| | | | | | | |
|---|---|---|---|---|---|---|
| 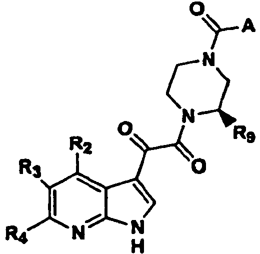 | | | | | | |

| Example 56 | | | | | | |
|---|---|---|---|---|---|---|
| Table Entry (Example number) | R 2 | R3 | R4 | R9 | A | EC₅₀ Group from Table 1 |
| 1(Example 56) | H | H | 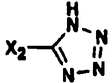 | CH₃ | 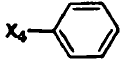 | B |

**Table 7**

| | | | | | | |
|---|---|---|---|---|---|---|
| 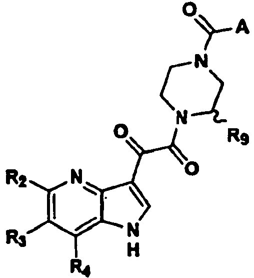 | | | | | | |

| Table Entry (Example No.) | R2 | R3 | R4 | R9 | A | EC₅₀ Group from Table 1 |
|---|---|---|---|---|---|---|
| 1 (Example 65) | H | H | 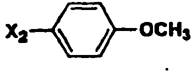 | H | 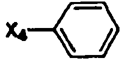 | A |
| 2 (Example 66) | H | H | 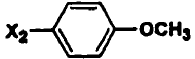 | (S)-CH₃ | 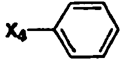 | A |
| 3 (Example 67) | H | H | 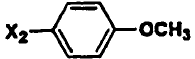 | (R)-Me | 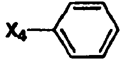 | A |
| 4 Example (57) | H | H | 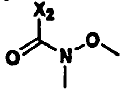 | (R)-Me | 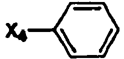 | A |
| 6 (Example 64) | Cl | H | 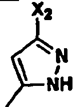 | (R)-Me | 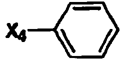 | A |
| 7 (Example 58) | Cl | H | 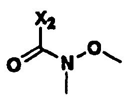 | (R)-Me | 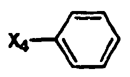 | A |
| 8 (Example 60) | Cl | H | 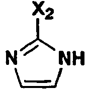 | (R)-Me | 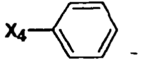 | A |
| 9 (Example 61) | Cl | H | 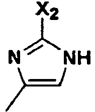 | (R)-Me | 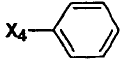 | A |
| 10 (Example 62) | Cl | H | 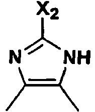 | (R)-Me | 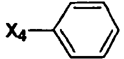 | A |
| 11 (Example 63) | Cl | H | 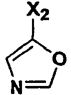 | (R)-Me | 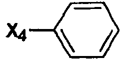 | A |
| 12 (Example 59) | Cl | H | 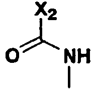 | (R)-Me | 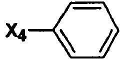 | A |
| 13 (Example 51) | H | H | 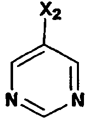 | (R)-Me | 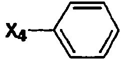 | A |
| 14 (Example 52) | H | H | 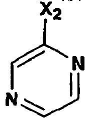 | (R)-Me | 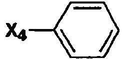 | A |
| 15 (Example 53) | H | H | 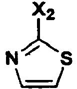 | (R)-Me | 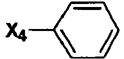 | A |
| 16 (Example 54) | H | H | 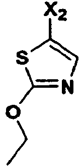 | (R)-Me | 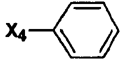 | A |
| 17 (Example 86) | H | H | 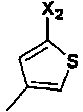 | (R)-Me | 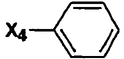 | A |
| 18 (Example 126) | H | H | 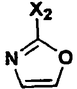 | (R)-Me | 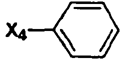 | A |
| 19 (Example 127) | H | H | 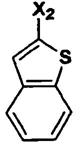 | (R)-Me | 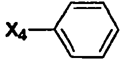 | A |
| 20 (Example 128) | H | H | 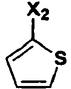 | (R)-Me | 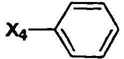 | A |
| 21 (Example 129) | H | H | 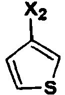 | (R)-Me | 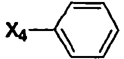 | A |
| 22 (Example 130) | H | H | 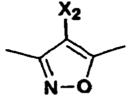 | (R)-Me | 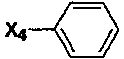 | A |
| 23 (Example 131) | H | H | 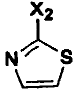 | H | 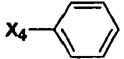 | A |
| 24 (Example 132) | H | H | 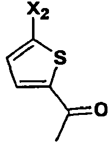 | H | 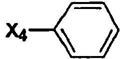 | A |
| 25 (Example 133) | H | H | 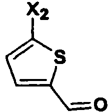 | H | 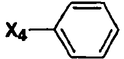 | A |
| 26 (Example 134) | H | H | 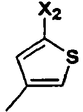 | H | 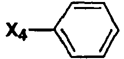 | A |
| 27 (Example 135) | H | H | 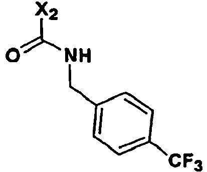 | (R)-Me | 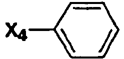 | A |
| 28 (Example 136) | H | H | 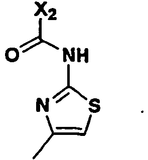 | (R)-Me | 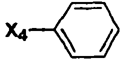 | A |
| 29 (Example 137) | H | H | 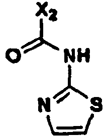 | (R)-Me | 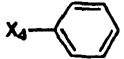 | A |
| 30 (Example 158) | H | H | 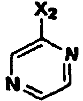 | H | 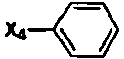 | A |
| 31 (Example 159) | H | H | 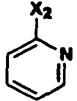 | H | 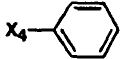 | A |
| 32 (Example 160) | H | H | 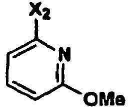 H | H | 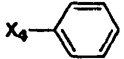 | A |
| 33 (Example 161) | H | H | 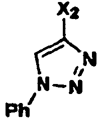 | H | 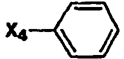 | A |
| 37 (Example 168) | H | H | 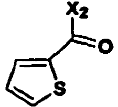 | H | 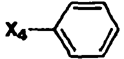 | A |

The 5-aza inhibitors shown in Table 8 can be prepared from intermediates 1a or 2s or the corresponding 7-desbromo-7-chloro intermediates which are prepared analogously and the methods herein or by using other methods described herein.

**Table 8**

| | | | | | |
|---|---|---|---|---|---|
| 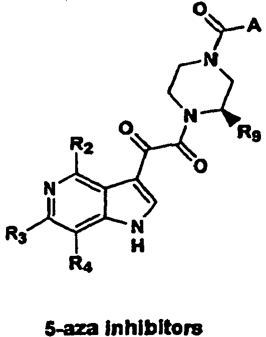 | | | | | |

| Table Entry (Example Number.) | R2 | R3 | R4 | R9 | A |
|---|---|---|---|---|---|
| 1 | MeO | H | 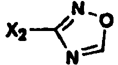 | H | 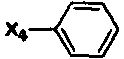 |
| 2 | MeO | H | 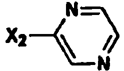 | H | 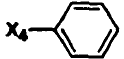 |
| 3 | MeO | H | 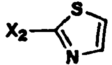 | H | 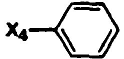 |
| 4 | MeO | H | 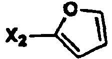 | H | 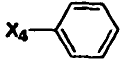 |

The compounds in Table 9 exemplify some of the many additional inhibitors which could be prepared by using methodology contained herein or exemplified in thepreparation of the compounds in Table 5.

**Table 9**

| | | | | | |
|---|---|---|---|---|---|
| 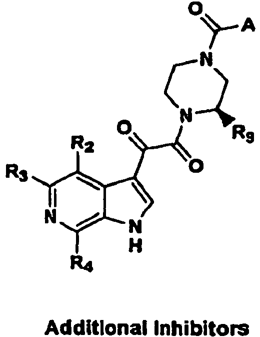 | | | | | |

| | R2 | R3 | R4 | R9 | A |
|---|---|---|---|---|---|
| | OMe | H | 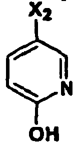 | H | 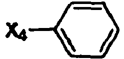 |
| | F | H | 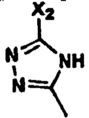 | H | 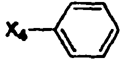 |
| | Cl | H | 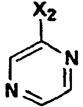 | H | 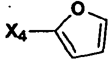 |
| | F | H | 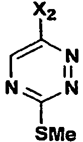 | H | 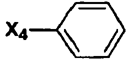 |
| | OMe | H | 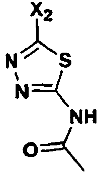 | H | 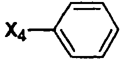 |
| | OMe | H | 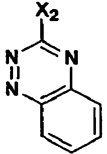 | H | 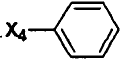 |
| | F | H | 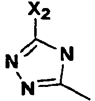 | H | 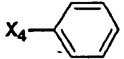 |
| | F | H | 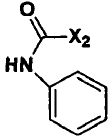 | H | 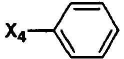 |
| | F | H | 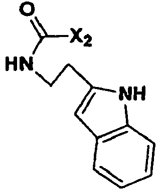 | H | 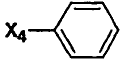 |
| | OMe | H | 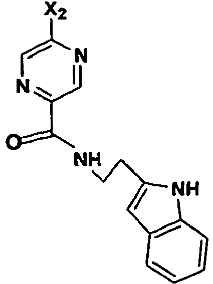 | H | 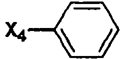 |
| | F | H | 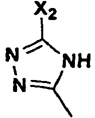 | H | 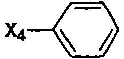 |
| | Cl | H | 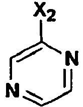 | H | 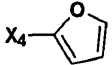 |
| | Cl | H | 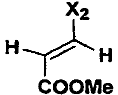 | (R)-Me | 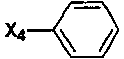 |
| | F | H | 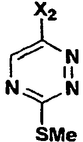 | H | 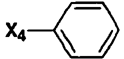 |
| | OMe | H | 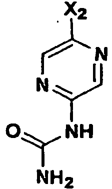 | H | 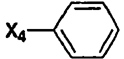 |
| | OMe | H | 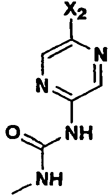 | H | 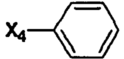 |
| | OMe | H | 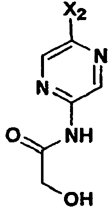 | H | 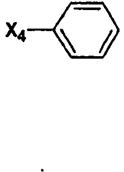 |
| | OMe | H | 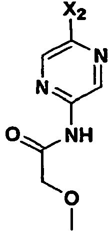 | H | 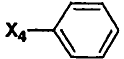 |
| | OMe | H | 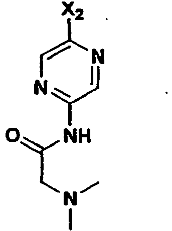 | H | 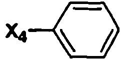 |
| | UMe | H | 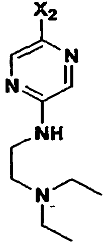 | H | 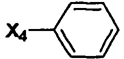 |
| | UMe | H | 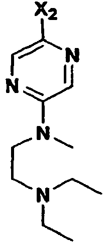 | H | 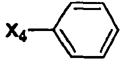 |
| | OMe | H | 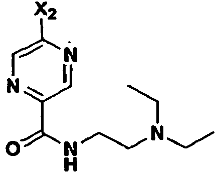 | H | 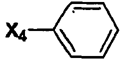 |
| | OMe | H | 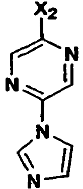 | H | 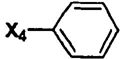 |
| | OMe | H | 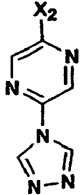 | H | 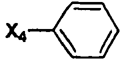 |
| | UMe | H | 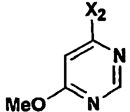 | H | 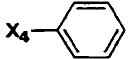 |
| | OMe | H | 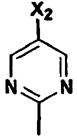 | H | 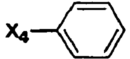 |
| | OMe | H | 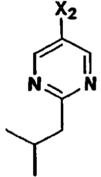 | H | 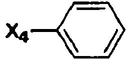 |
| | H | H | 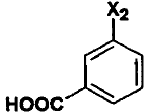 | (R)-Me | 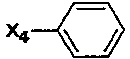 |
| | OMe | H | 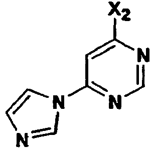 | H | 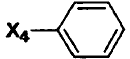 |
| | OMe | H | 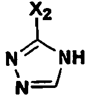 | H | 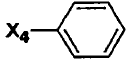 |
| | UMe | H | 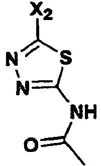 | H | 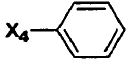 |
| | OMe | H | 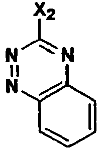 | H | 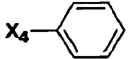 |
| | OMe | H | 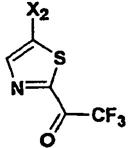 | H | 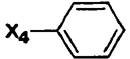 |
| | OMe | H | 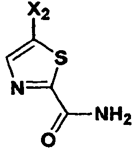 | H | 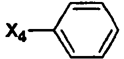 |
| | OMe | H | 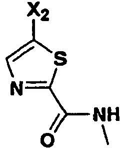 | H | 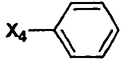 |
| | OMe | H | 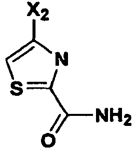 | H | 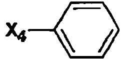 |
| | OMe | H | 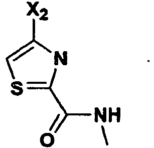 | H | 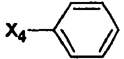 |
| | OMe | H | 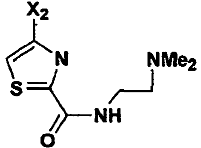 | H | 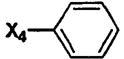 |
| | OMe | H | 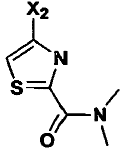 | H | 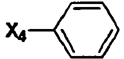 |
| | OMe | H | 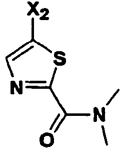 | H | 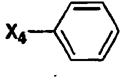 |
| | OMe | H | 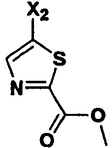 | H | 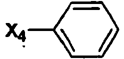 |
| | OMe | H | 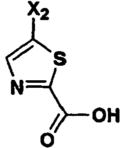 | H | 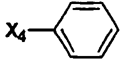 |
| | OMe | H | 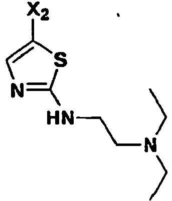 | H | 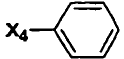 |
| | OMe | H | 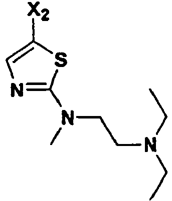 | H | 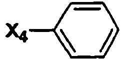 |
| | OMe | H | 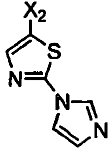 | H | 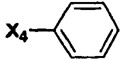 |
| | OMe | H | 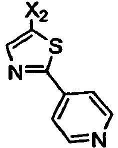 | H | 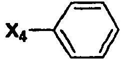 |
| | OMe | H | 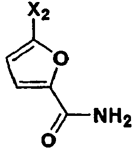 | H | 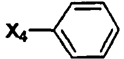 |
| | F | H | 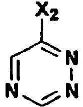 | H | 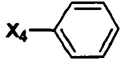 |
| | F | H | 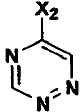 | H | 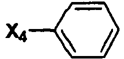 |
| | F | H | 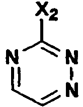 | H | 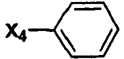 |
| | OMe | H | 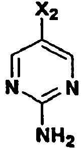 | H | 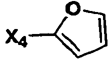 |
| | OMe | H | 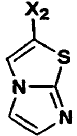 | H | 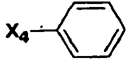 |
| | OMe | H | 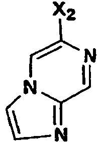 | H | 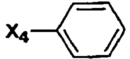 |
| | OMe | H | 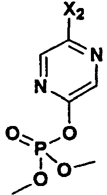 | H | 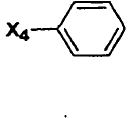 |
| | OMe | H | 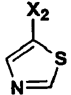 | H | 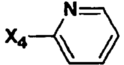 |
| | OMe | H | 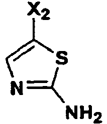 | H | 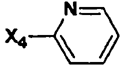 |
| | OMe | H | 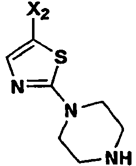 | H | 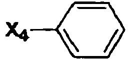 |
| | OMe | H | 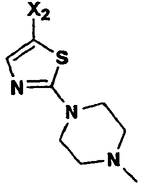 | H | 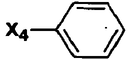 |
| | OMe | H | 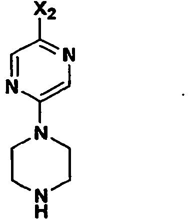 | H | 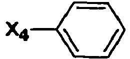 |
| | OMe | H | 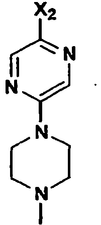 | H | 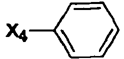 |
| | OMe | H | 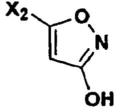 | H | 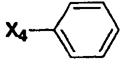 |
| | OMe | H | 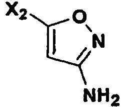 | H | 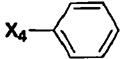 |
| | OMe | H | 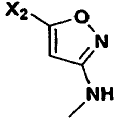 | H | 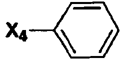 |
| | OMe | H | 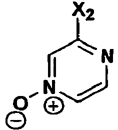 | H | 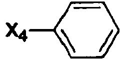 |
| | OMe | H | 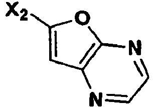 | H | 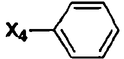 |
| | OMe | H | 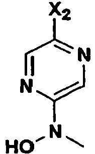 | H | 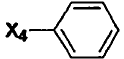 |
| | OMe | H | 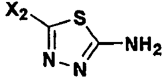 | H | 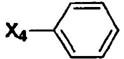 |
| | UMe | H | 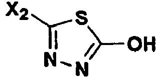 | H | 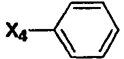 |
| | OMe | H | 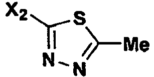 | H | 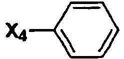 |
| | UMe | H | 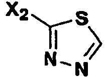 | H | 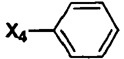 |
| | OMe | H | 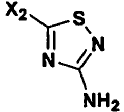 | H | 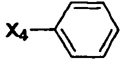 |
| | OMe | H | 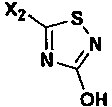 | H | 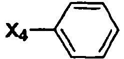 |
| | OMe | H | 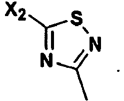 | H | 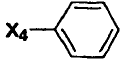 |
| | OMe | H | 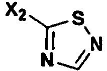 | H | 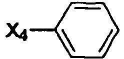 |
| | OMe | H | 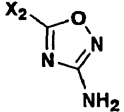 | H | 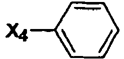 |
| | OMe | H | 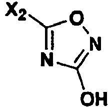 | H | 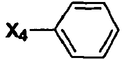 |
| | OMe | H | 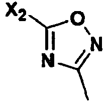 | H | 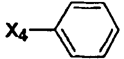 |
| | OMe | H | 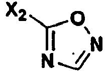 | H | 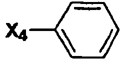 |
| | UMe | H | 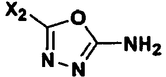 | H | 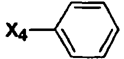 |
| | OMe | H | 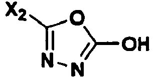 | H | 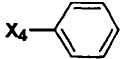 |
| | OMe | H | 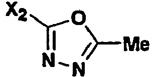 | H | 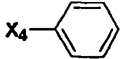 |
| | OMe | H | 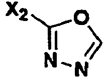 | H | 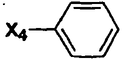 |
| | OMe | H | 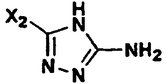 | H | 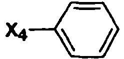 |
| | OMe | H | 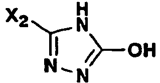 | H | 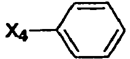 |
| | OMe | H | 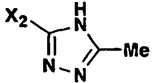 | H | 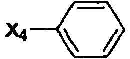 |
| | OMe | H | 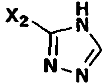 | H | 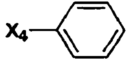 |
| | OMe | H | 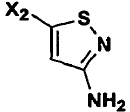 | H | 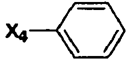 |
| | OMe | H | 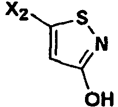 | H | 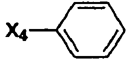 |
| | OMe | H | 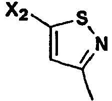 | H | 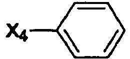 |
| | OMe | H | 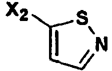 | H | 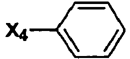 |
| | OMe | H | 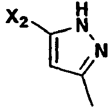 | H | 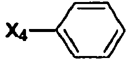 |
| | OMe | H | 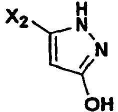 | H | 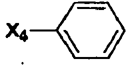 |
| | OMe | H | 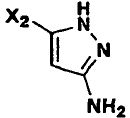 | H | 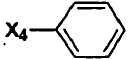 |
| | OMe | H | 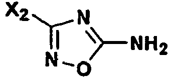 | H | 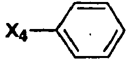 |
| | OMe | H | 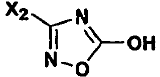 | H | 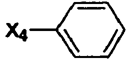 |
| | OMe | H | 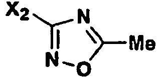 | H | 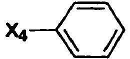 |
| | OMe | H | 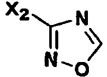 | H | 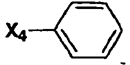 |
| | OMe | H | 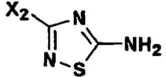 | H | 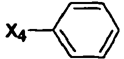 |
| | OMe | H | 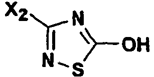 | H | 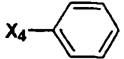 |
| | OMe | H | 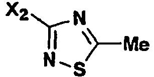 | H | 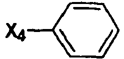 |
| | OMe | H | 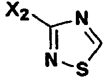 | H | 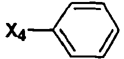 |
| | OMe | H | 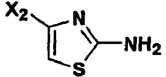 | H | 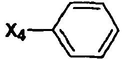 |
| | OMe | H | 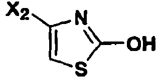 | H | 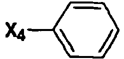 |
| | OMe | H | 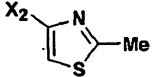 | H | 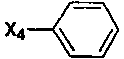 |
| | OMe | H | 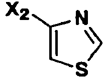 | H | 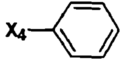 |
| | OMe | H | 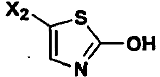 | H | 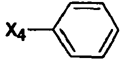 |
| | OMe | H | 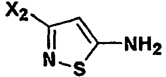 | H | 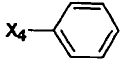 |
| | OMe | H | 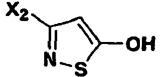 | H | 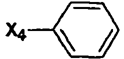 |
| | OMe | H | 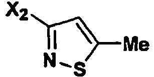 | H | 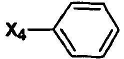 |
| | OMe | H | 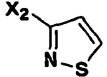 | H | 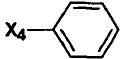 |
| | OMe | H | 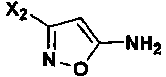 | H | 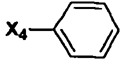 |
| | OMe | H | 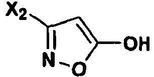 | H | 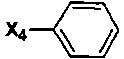 |
| | OMe | H | 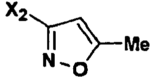 | H | 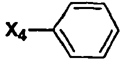 |
| | OMe | H | 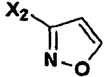 | H | 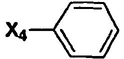 |
| | OMe | H | 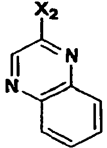 | H | 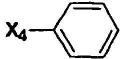 |
| | OMe | H | 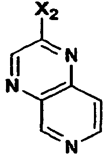 | H | 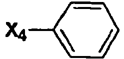 |
| | OMe | H | 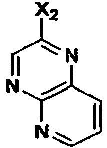 | H | 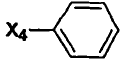 |
| | OMe | H | 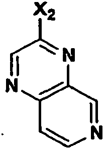 | H | 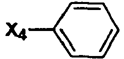 |
| | OMe | H | 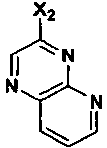 | H | 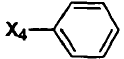 |
| | OMe | H | 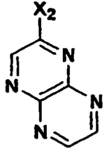 | H | 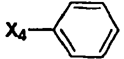 |
| | OMe | H | 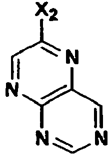 | H | 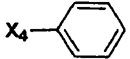 |
| | OMe | H | 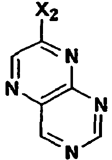 | H | 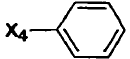 |
| | OMe | H | 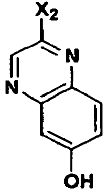 | H | 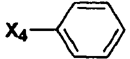 |
| | OMe | H | 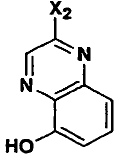 | H | 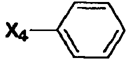 |
| | OMe | H | 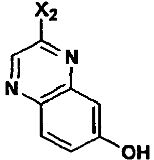 | H | 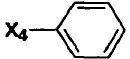 |
| | OMe | H | 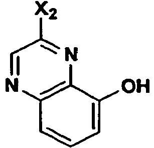 | H | 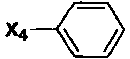 |
| | OMe | H | 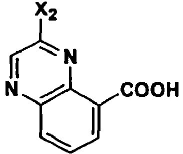 | H | 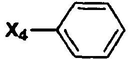 |
| | OMe | H | 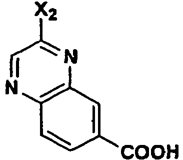 | H | 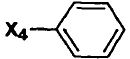 |
| | OMe | H | 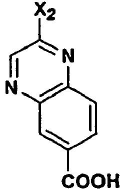 | H | 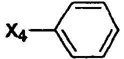 |
| | OMe | H | 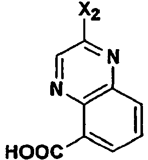 | H | 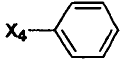 |
| | OMe | H | 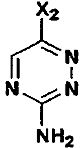 | H | 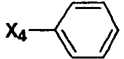 |
| | OMe | H | 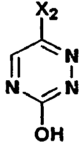 | H | 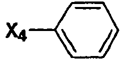 |
| | OMe | H | 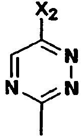 | H | 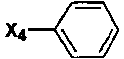 |
| | OMe | H | 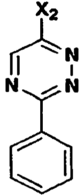 | H | 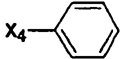 |
| | OMe | H | 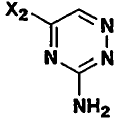 | H | 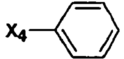 |
| | OMe | H | 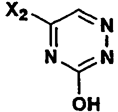 | H | 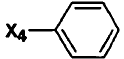 |
| | OMe | H | 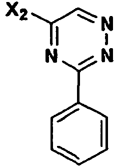 | H | 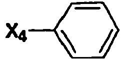 |
| | OMe | H | 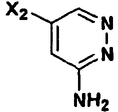 | H | 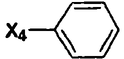 |
| | OMe | H | 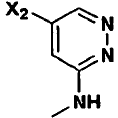 | H | 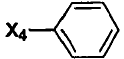 |
| | OMe | H | 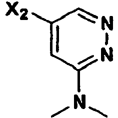 | H | 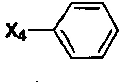 |
| | OMe | H | 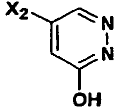 | H | 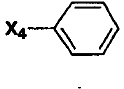 |
| | OMe | H | 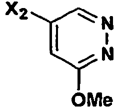 | H | 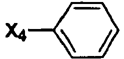 |
| | OMe | H | 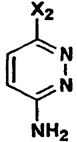 | H | 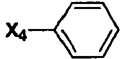 |
| | OMe | H | 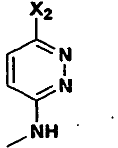 | H | 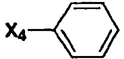 |
| | OMe | H | 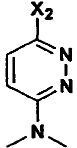 | H | 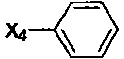 |
| | OMe | H | 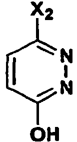 | H | 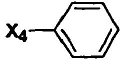 |
| | OMe | H | 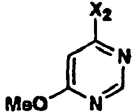 | H | 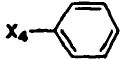 |
| | OMe | H | 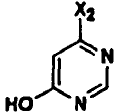 | H | 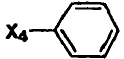 |
| | OMe | H | 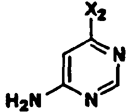 | H | 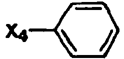 |
| | OMe | H | 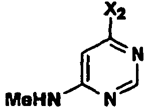 | H | 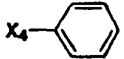 |
| | OMe | H | 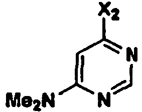 | H | 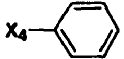 |
| | OMe | H | 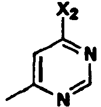 | H | 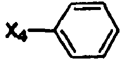 |

The inhibitors in Table 10 could be prepared using analogous procedures which were demonstrated to prepare the examples in Table 7.

**Table 10**

| | | | | | |
|---|---|---|---|---|---|
| 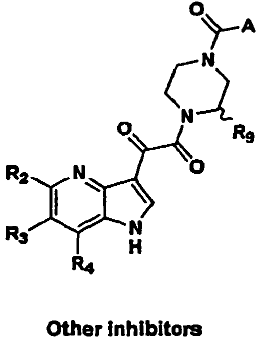 | | | | | |

| Table Entry | R2 | R3 | R4 | R9 | A |
|---|---|---|---|---|---|
| 1 | H | H | 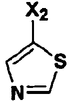 | H₃ | 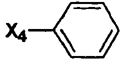 |
| 2 | H | H | 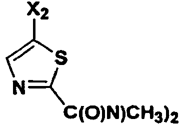 | H | 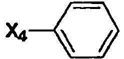 |
| 3 | H | H | 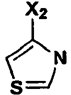 | H | 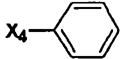 |
| 4 | H | H | 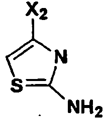 | H | 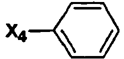 |
| 5 | H | H | 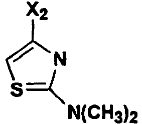 | H | 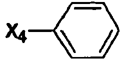 |
| 6 | H | H | 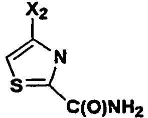 | H | 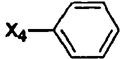 |
| 7 | H | H | 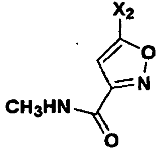 | H | 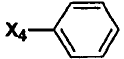 |
| 8 | H | H | 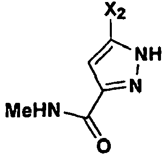 | H | 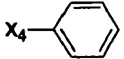 |
| 9 | H | H | 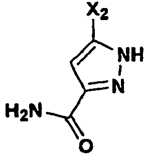 | H | 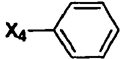 |

The compounds of Table 11 below were all found to be very potent in the assay described above using % inhibition as a criteria. In Table 11, X₂, X₄ etc. indicates the point of attachment. The vast majority of the compounds exhibited greater than 98% inhibition at a concentration of 10uM. The data at 10µM was calculated in the following manner:

### Method for extrapolating % inhibition at 10µM

The compounds of Table 11 below were all found to be very potent in the assay described above using % inhibition as a criteria. In Table 8 X₂, X₄ etc. indicates the point of attachment. The vast majority of the compounds exhibited greater than 98% inhibition at a concentration of 10µM. The data at 10µM was calculated in the following manner:

### Method for extrapolating % inhibition at 10µM

The data in Table 11 was obtained using the general procedures above and by the following methods. Data is not reported for all compounds since data for all the compounds is reported by the alternate method in Table 5. The percent inhibition for each compound was calculated by quantifying the level of luciferase expression in cells infected in the presence of compound as a percentage of that observed for cells infected in the absence of compound and subtracting such a determined value from 100. For compounds tested at concentrations less than 10 µM, the percent inhibition at 10 µM was determined by extrapolation using the XLfit curve fitting feature of the Microsoft Excel spreadsheet software. Curves were obtained from 10 data points (% inhibition determined at 10 concentrations of compound) by using a four parameter logistic model (XLfit model 205: y = A + ((B-A)/(1+((C/x)^{D})), where, A = minimum y, B = maximum y, C = logEC₅₀, D = slope factor, and x and y are known data values. Extrapolations were performed with the A and B parameters unlocked.

Thus the compounds of this invention are all potent antiviral inhibitors based on this assay.

**Table 11**

| | |
|---|---|
| 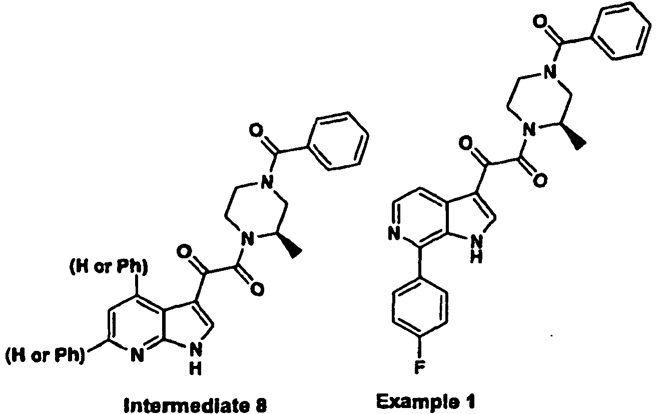 | |

| Compound # | Average % inhibition at 10 µM |
|---|---|
| | |
| Intermediate 8 | 85% |
| Example 1 | 56% |
| | |

The compounds of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating viral infections such as HIV infection and AIDS. The treatment involves administering to a patient in need of such treatment a pharmaceutical composition comprising a Pharmaceutical carrier and a therapeutically-effective amount of a compound of the present invention.

The pharmaceutical composition may be in the form of orally-administrable suspensions or tablets; nasal sprays, sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

The compounds of this invention can be administered orally to humans in a dosage range of 1 to 100 mg/kg body weight in divided doses. One preferred dosage range is 1 to 10 mg/kg body weight orally in divided doses. Another preferred dosage range is 1 to 20 mg/kg body weight orally in divided doses. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

Scheme 41a depicts methodology for converting a carboxylic acid to an alkynyl ketone. The alkynyl ketone intermediates can then be converted to pyrazoles or isoxazoles upon reaction with hydrazines or hydroxyl amines, respectively.

## Claims

1. A compound of Formula I, including pharmaceutically acceptable salts thereof, 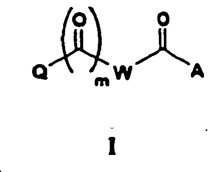 wherein:
Q is either 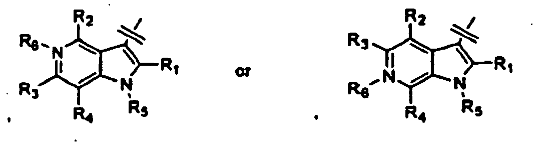 and then R² is selected from the group consisting of hydrogen, halogen and methoxy; and
R₃ is hydrogen;
or Q is: 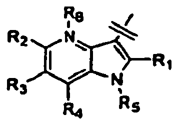 and R² is halogen or hydrogen and R³ is hydrogen;
R⁴ is B;
B is selected from the group consisting of substituted phenyl, heteroaryl, and C(O)R⁷ wherein said heteroaryl is optionally substituted and phenyl is substituted with one to three same or different halogens or from one to two same or different substituents selected from the group F;
F is selected from the group consisting of (C₁₋₆)alkyl, (C₃₋₆)cycloalkyl, aryl, heteroaryl, heteroalicyclic, hydroxy, (C₁₋₆)alkoxy, (C₁₋₆)thioalkoxy, cyano, halogen, carbonyl, benzyl, - NR⁴²C(O)-(C₁₋₆)alkyl, -NR⁴²C(O)-(C₃₋₆)cycloalkyl, -NR⁴²C(O)-aryl, -NR⁴²C(O)-heteroaryl, - NR⁴²C(O)-heteroalicyclic, a cyclic N-amido, -NR⁴²S(O)₂-(C₁₋₆)alkyl, -NR⁴²S(O)₂-(C₃₋₆)cycloalkyl, -NR⁴²S(O)₂-aryl, -NR⁴²S(O)₂-heteroaryl, -NR⁴²S(O)₂-heteroalicyclic, -S(O)₂ NR⁴²R⁴³, NR⁴²R⁴³, (C₁₋₆)alkylC(O)NR⁴²R⁴³, C(O)NR⁴²R⁴³, NHC(O)NR⁴²R⁴³, OC(O)NR⁴²R⁴³, NHC(O)OR⁵⁴, (C₁₋₆)alkylNR⁴²R⁴³, COOR⁵⁴,and (C₁₋₆)alkylCOOR⁵⁴ wherein said (C₁₋₆)alkyl, (C₃₋₆)cycloalkyl, aryl, heteroaryl, heteroalicyclic, (C₁₋₆)alkoxy, are optionally substituted with one to three same or different halogens or from one to two same or different substituents selected from the group G;
G is selected from the group consisting of (C₁₋₆)alkyl, (C₃₋₇)cycloalkyl, aryl, heteroaryl, heteroalicyclic, hydroxy, (C₁₆)alkoxy, (C₁₆)thioalkoxy, thioaryloxy, cyano, halogen, nitro, carbonyl, thiocarbonyl, benzyl, -NR⁴⁸(O)-(C₁₋₆)alkyl, -NR⁴⁸C(O)-(C₃₋₆)cycloalkyl, - NR⁴⁸C(O)-aryl, -NR⁴⁸C(O)-heteroaryl, -NR⁴⁸C(O)-heteroalicyclic, a cyclic N-amido, - NR⁴⁸S(O)₂-(C₁₋₆)alkyl, -NR⁴⁸S(O)₂-(C₃₋₆)cycloalkyl, -NR⁴⁸S(O)₂-aryl, -NR⁴⁸S(O)₂-heteroaryl, -NR⁴⁸S(O)₂-heteroalicyclic, sulfonyl, -S(O)2 NR⁴⁸R⁴⁹, NR⁴⁸R⁴⁹, (C₁₋₆)alkyl C(O)NR⁴⁸R⁴⁹, C(O)NR⁴⁸R⁴⁹, NHC(O)NR⁴⁸R⁴⁹, OC(O)NR⁴⁸R⁴⁹, NHC(O)OR⁵⁴, (C₁₋₆)alkylNR⁴⁸R⁴⁹, COOR⁵⁴, and (C₁₋₆)alkylCOOR⁵⁴;
R¹ is hydrogen;
m is 2;
R⁵ is hydrogen;
R⁶ does not exist;
R⁷ is selected from the group consisting of aryl, heteroaryl, and heteroalicyclic wherein said aryl, heteroaryl, and heteroalicyclic are optionally substituted with one to three same or different halogens or with from one to two same or different substituents selected from the group F;
A is selected from the group consisting of phenyl and heteroaryl; wherein heteroaryl is pyridinyl, furanyl or thienyl; and said phenyl or said heteroaryl is optionally substituted with one to two of the same or different amino, C₁₋₆alkyl, hydroxy, or halogen;
-W- is 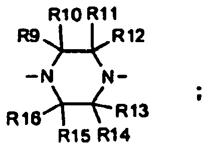
R⁹, R¹⁰, R¹¹, R¹², R¹³, and R¹⁴ are each hydrogen; and
R¹⁵ and R¹⁶ are each independently hydrogen or methyl with the proviso that only one is methyl;
R⁴² and R⁴³ are independently selected from the group consisting of hydrogen, (C₁₋₆)alkyl, (C₁₋₆)alkoxy, (C₃₋₇)cycloalkyl, aryl, heteroaryl and heteroalicyclic; or R⁴² and R⁴³ taken together with the nitrogen to which they are attached form a heteroaryl ring or a heteroalicyclic ring which may contain up to 2 additional heteroatoms selected from N, O, S(O)ₘ wherein m' is O, 1, or 2; and wherein said (C₁₋₆)alkyl, (C₁₋₆)alkoxy, (C₃₋₇)cycloalkyl, aryl, heteroaryl, and heteroalicyclic are optionally substituted with one to three same or different halogens or from one to two same or different substituents selected from the group G;
R⁴⁸ and R⁴⁹ are independently selected from the group consisting of hydrogen, (C₁₋₆)alkyl, (C₁₋₆)alkoxy, (C₃₋₇)cycloalkyl, allyl, aryl, heteroaryl, heteroalicyclic or R⁴⁸and R⁴⁹ taken together with the nitrogen to which they are attached form a heteroaryl ring or a heteroalicyclic ring which may contain up to two additional heteroatoms selected from N, O, S(O)_{m'} wherein m' is 0,1, or 2;
R⁵⁴ is selected from the group consisting of hydrogen, (C₁₋₆)alkyl, (C₃₋₇)cycloalkyl, aryl, heteroaryl, and heteroalicyclic wherein said (C₁₋₆)alkyl, (C₃₋₇)cycloalkyl, aryl, heteroaryl, and heteroalicyclic are optionally substituted with one to three same or different halogens or from one to two same or different substituents selected from the group consisting of: amino, OH, and NR⁵⁵R⁵⁶;
R^{54'} is selected from the group consisting of (C₁₋₆)alkyl, (C₃₋₇)cyloalkyl, aryl, heteroaryl, and heteroalicyclic wherein said (C₁₋₆)alkyl, (C₃₋₇)cycloalkyl, aryl, heteroaryl, and heteroalicyclic are optionally substituted with one to three same or different halogens or from one to two same or different substituents selected from the group consisting of: amino, OH, and NR⁵⁵R⁵⁶;
R⁵⁵ and R⁵⁶ are independently selected from the group consisting of hydrogen, (C₁₋₆)alkyl, or (C₃₋₇)cycloalkyl.

2. A compound of claim 1, including pharmaceutically acceptable salts thereof, wherein:
R⁴ is selected from the group consisting of B;
B is selected from the group consisting of substituted phenyl, or heteroaryl, wherein said phenyl is substituted and heteroaryl is optionally substituted with one to three same or different halogens or from one to two same or different substituents selected from the group F;
F is selected from the group consisting of (C₁₋₆)alkyl, (C₃₋₆)cycloalkyl, aryl, heteroaryl, heteroalicyclic, hydroxy, (C₁₋₆)alkoxy, (C₁₋₆)thioalkoxy, cyano, halogen, carbonyl, benzyl, - NR⁴²C(O)-(C₁₋₆)alkyl, -NR⁴²C(O)-(C₃₋₆)cycloalkyl, -NR⁴²C(O)-aryl, -NR⁴²C(O)heteroaryl, - NR⁴²C(O)-heteroalicyclic, a cyclic N-amido, -NR⁴²S(O)₂-(C₁₋₆)alkyl, -NR⁴²R⁴³, C(O)NR⁴²R⁴³, COOR⁵⁴, and wherein said (C₁₋₆)alkyl, (C₃₋₆)cycloalkyl, aryl, heteroaryl, heteroalicyclic, (C₁₋₆)alkoxy, are optionally substituted with one to three same or different halogens or from one to two same or different substituents selected from the group G;
G is selected from the group consisting of (C₁₋₆)alkyl, hydroxy, (C₁₋₆)alkoxy, halogen, - NR⁴⁸C(O)-(C₁₋₆)alkyl, -NR⁴⁸C(O)-(C₃)cycloalkyl, a cyclic N-amido, -NR⁴⁸S(O)₂-(C₁₋₆)alkyl, NR⁴⁸R⁴⁹, (C₁₋₆)alkyl C(O)NR⁴⁸R⁴⁹, C(O)NR⁴⁸R⁴⁹, (C₁₋₆)alkylNR⁴⁸R⁴⁹;
R⁴² and R⁴³ are independently selected from the group consisting of hydrogen, (C₁₋₆)alkyl, (C₁₋₆)alkoxy, (C₃₋₇)cycloalkyl, aryl, heteroaryl, heteroalicyclic or R⁴² and R⁴³ taken together with the nitrogen to which they are attached form a heteroaryl ring or a heteroalicyclic ring which may contain up to two additional heteroatoms selected from N, O, S(O)_{m'} wherein m' is 0, 1, or 2; and wherein said (C₁₋₆)alkyl, (C₁₋₆)alkoxy, (C₃₋₇)cycloalkyl, aryl, heteroaryl, and heteroalicyclic are optionally substituted with one to three same or different halogens or from one to two same or different substituents selected from the group G;
R⁴⁸ and R⁴⁹ are independently selected from the group consisting of hydrogen, (C₁₋₆)alkyl or R⁴⁸ and R⁴⁹ taken together with the nitrogen to which they are attached form a heteroaryl ring or a heteroalicyclic ring which may contain up to two additional heteroatoms selected from N, or O.

3. A compound of claim 1, including pharmaceutically acceptable salts thereof, wherein:
Q is 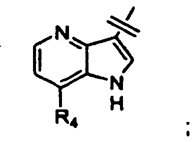
R⁴ is B;
A is Phenyl or 2-pyridyl;
B is heteroaryl, wherein said heteroaryl is optionally substituted with one to three same or different halogens or from one to two same or different substituents selected from the group F.

4. A compound of claim 2, including pharmaceutically acceptable salts thereof, wherein:
Q is 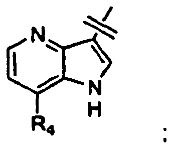
R⁴ is B;
A is Phenyl or 2-pyridyl;
B is heteroaryl, wherein said heteroaryl is optionally substituted with one to three same or different halogens or from one to two same or different substituents selected from the group F.

5. A compound of claim 3, including pharmaceutically acceptable salts thereof, wherein:
B is heteroaryl, wherein said heteroaryl is optionally substituted with one to three same or different halogens or from one to two same or different substituents selected from the group F.

6. A compound of claim 4, including pharmaceutically acceptable salts thereof, wherein:
B is heteroaryl, wherein said heteroaryl is optionally substituted with one to three same or different halogens or from one to two same or different substituents selected from the group F.

7. A compound of claim 1, including pharmaceutically acceptable salts thereof, wherein:
Q is 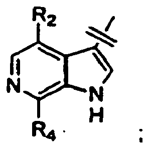
R² is selected from the group consisting of hydrogen, halogen, and methoxy;
R⁴ is B;
B is heteroaryl, wherein said heteroaryl is optionally substituted with one to three same or different halogens or from one to two same or different substituents selected from the group F.

8. A compound of claim 2, including pharmaceutically acceptable salts thereof, wherein:
Q is 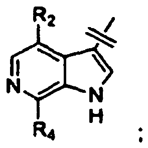
R² is selected from the group consisting of hydrogen, halogen, and methoxy;
R⁴ is B;
B is heteroaryl, wherein said heteroaryl is optionally substituted with one to three same or different halogens or from one to two same or different substituents selected from the group F.

9. A compound of claim 7 or 8, including pharmaceutically acceptable salts thereof, wherein:
A is Phenyl or 2-pyridyl.

10. A compound of claim 9 including pharmaceutically acceptable salts thereof, wherein:
B is heteroaryl, wherein said heteroaryl is optionally substituted with one to three same or different halogens or from one to two same or different substituents selected from the group F.

11. A compound of claim 1 or 2 in which:
R⁴ is B; and
F is selected from the group consisting of (C₁₋₆)alkyl, hydroxy, heteroaryl, heteroalicyclic, methoxy, methylthioalkoxy, halogen, carbonyl, C(O)NR⁴²R⁴³, -NR⁴²C(O)-(C₁₋₆)alkyl, - NR⁴²C(O)-(C₃₋₆)cycloalkyl, -NR⁴²C(O)-aryl, -NR⁴²C(O)-heteroaryl, -NR⁴²C(O)-heteroalicyclic, a cyclic N-amido, -NR⁴²S(O)₂-(C₁₋₆)alkyl, -NR⁴²S(O)₂-(C₃₋₆)cycloalkyl, -NR⁴²S(O)₂-aryl, -NR⁴²S(O)₂-heteroaryl, -NR⁴²S(O)₂-heteroalicyclic, NR⁴²R⁴³, COOH.

12. A compound of claim 11 in which A is Phenyl or 2-pyridyl.

13. A compound of claim 1 or 2, including pharmaceutically acceptable salts thereof, wherein:
Q is 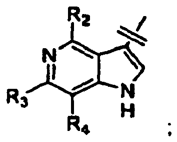
R² is selected from the group consisting of hydrogen or methoxy;
R³ is hydrogen;
R⁴ is B;
B is heteroaryl, wherein said heteroaryl is optionally substituted with one to three same or different halogens or from one to two same or different substituents selected from the group F.

14. A compound of claim 9 wherein R² is Fluoro.

15. A compound of claim 9 wherein R² is Methoxy.

16. A compound of any one of claims claim 3, 4, 7, 8, 9, or 13, wherein
B is selected from the group consisting of thiazole, pyridazine, pyrazine, pyrazole, isoxazole, isothiazole, imidazole, furyl, thienyl, oxazole, oxadiazole, thiadiazole, pyrimidine, triazine, triazole, tetrazole, pyridyl, wherein said heteroaryl is optionally substituted with one to three same or different halogens or from one to two same or different substituents selected from the group F.

17. A compound of claim 6 or 10 wherein
B is heteroaryl wherein said heteroaryl is optionally substituted with one to three same or different halogens or a substituent selected from the group (C₁-C₆ alkyl), amino, -NHC(O)-(C₁-C₆ alkyl), -NHS(O)₂-(C₁-C₆ alkyl), methoxy, -C(O)-NH₂, C(O)NHMe, C(O)NMe2, trifluoromethyl, -NHC(C₁-C₆ alkyl), -N(C₁-C₆ alkyl)₂, -heteroaryl, cyclic N-amido;

18. A compound of claim 16 wherein said heteroaryl is optionally substituted with one to three same or different halogens or a substituent selected from the group (C₁-C₆ alkyl), amino, -NHC(O)-(C₁-C₆ alkyl), -NHS(O)₂-(C₁-C₆ alkyl), methoxy, -C(O)-NH₂, C(O)NHMe, C(O)NMe2, trifluoromethyl, -NHC(C₁-C₆ alkyl), -N(C₁-C₆ alkyl)₂, -heteroaryl, cyclic N-amido.

19. A compound of claim 4 or 10 wherein B is thienyl.

20. A compound of claim 16 wherein B is thienyl which is optionally substituted with one to three same or different halogens or a substituent selected from the group (C₁-C₆ alkyl), amino, -NHC(O)-(C₁-C₆ alkyl), -NHS(O)₂-(C₁-C₆ alkyl), methoxy, -C(O)-NH₂, C(O)NHMe, C(O)NMe2, trifluoromethyl, -NHC(C₁-C₆ alkyl), -N(C₁-C₆ alkyl, -heteroaryl, cyclic N-amido.

21. A compound of claim 16 wherein B is selected from the group consisting of thiazole, pyridazine, pyrazine, pyrazole, isoxazole, isothiazole, imidazole, furyl, thienyl, oxazole, oxadiazole, thiadiazole, pyrimidine, triazine, triazole, tetrazole, pyridyl, wherein said heteroaryl is optionally substituted with one to three same or different halogens or a substituent selected from the group (C₁-C₆ alkyl), amino, -NHC(O)-(C₁-C₆ alkyl), -NHS(Oh-(C₁-C₆ alkyl), methoxy, -C(O)-NH₂, C(O)NHMe, C(O)NMe2, trifluoromethyl, -NHC(C₁-C₆ alkyl), -N(C₁-C₆ alkyl)₂, -heteroaryl, cyclic N-amido.

22. A compound of claim 1 which is depicted in Tables 5, 6, 7, and 8.

23. A compound of the formula: 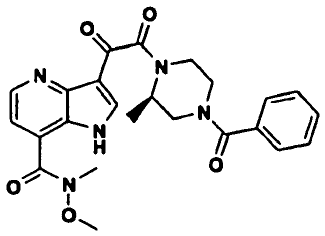 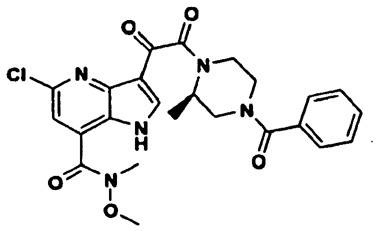 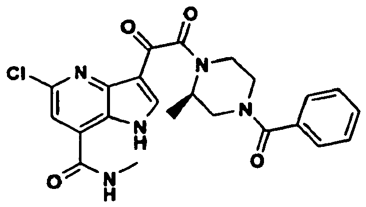 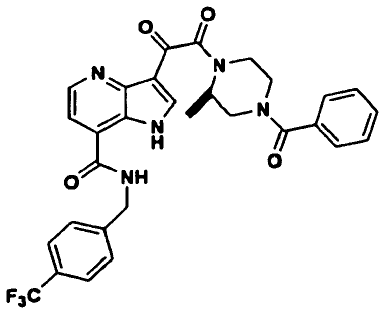 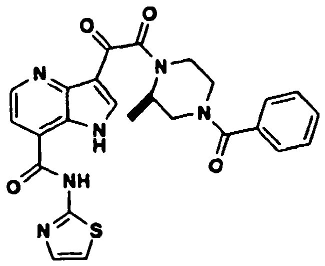 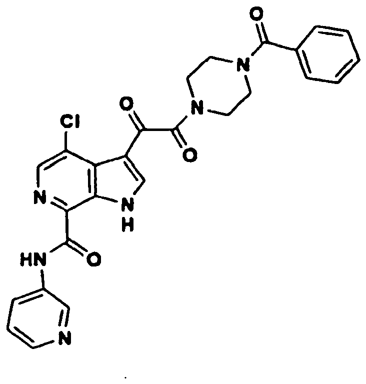 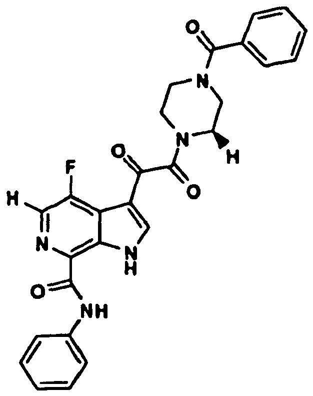 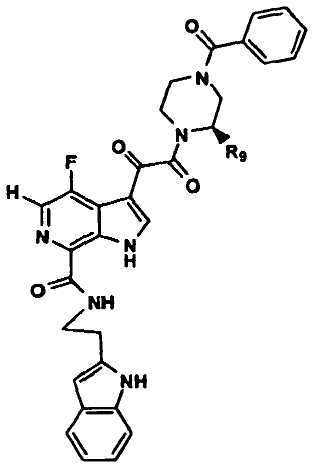 or a pharmaceutically acceptable salt thereof.

24. A pharmaceutical formulation which comprises an antiviral effective amount of a compound of Formula I, including pharmaceutically acceptable salts thereof, as claimed in any of claims 1-23, and a pharmaceutically acceptable carrier.

25. The pharmaceutical formulation of claim 24, useful for treating infection by HIV, which additionally comprises an antiviral effective amount of an AIDS treatment agent selected from the group consisting of:
(a) an AIDS antiviral agent;
(b) an anti-infective agent;
(c) an immunomodulator; and
(d) HIV entry inhibitors.

26. A compound of Formula I, including pharmaceutically acceptable salts thereof, as claimed in any of claims 1 - 23 for use in a method for treating mammals infected with a virus.

27. The compound of claim 26, comprising using a compound of Formula I in combination with an antiviral effective amount of an AIDS treatment agent selected from the group consisting of: an AIDS antiviral agent; an anti-infective agent; an immunomodulator; and HIV entry inhibitors, for use in a method for treatment of claim 26

28. The compound of claim 26 or 27, for use in a method for treatment of claim 26 wherein the virus is HIV.

## Patentansprüche

1. Verbindung von Formel I, enthaltend pharmazeutisch annehmbare Salze davon, 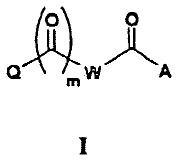 wobei:
Q entweder 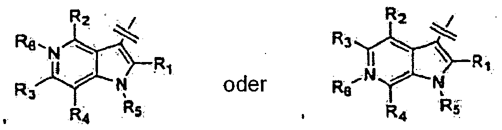 ist
und dann R² aus Wasserstoff, Halogen und Methoxy ausgewählt ist; und
R₃ Wasserstoff ist;
oder Q 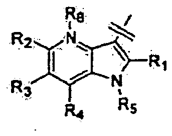 ist
und R² Halogen oder Wasserstoff ist und R³ Wasserstoff ist;
R⁴ B ist;
B aus substituiertem Phenyl, Heteroaryl und C(O)R⁷ ausgewählt ist, wobei das Heteroaryl optional substituiert ist und Phenyl mit einem bis drei gleichen oder verschiedenen Halogenen oder von einem bis zwei gleichen oder verschiedenen aus der Gruppe F ausgewählten Substituenten substituiert ist;
F aus (C₁₋₆)Alkyl, (C₃₋₆)Cycloalkyl, Aryl, Heteroaryl, Heteroalicyclic, Hydroxy, (C₁₋₆)Alkoxy, (C₁₋₆)Thioalkoxy, Cyano, Halogen, Carbonyl, Benzyl, -NR⁴²C(O)-(C₁₋₆)Alkyl, -NR⁴²C(O)-(C₃₋₆)Cycloalkyl, -NR⁴²C(O)-Aryl, -NR⁴²C(O)-Heteroaryl,-NR⁴²C(O)-Heteroalicyclic, einem zyklischen N-Amido, -NR⁴²S(O)₂-(C₁₋₆)Alkyl, - NR⁴²S(O)₂-(C₃₋₆)Cycloalkyl, -NR⁴²S(O)₂-Aryl, -NR⁴²S(O)₂-Heteroaryl, -NR⁴²S(O)₂-Heteroalicyclic, -S(O)₂NR⁴²R⁴³, NR⁴²R⁴³, (C₁₋₆)AlkylC(O)NR⁴²R⁴³, C(O)NR⁴²R⁴³, NHC(O)NR⁴²R⁴³, OC(O)NR⁴²R⁴³, NHC(O)OR^{54'}, (C₁₋₆)AlkylNR⁴²R⁴³, COOR⁵⁴ und (C₁₋₆)AlkylCOOR⁵⁴ ausgewählt ist, wobei das (C₁₋₆)Alkyl, (C₃₋₆)Cycloalkyl, Aryl, Heteroaryl, Heteroalicyclic, (C₁₋₆)Alkoxy optional mit einem bis drei gleichen oder verschiedenen Halogenen oder von einem bis zwei gleichen oder verschiedenen aus der Gruppe G ausgewählten Substituenten substituiert sind;
G aus (C₁₋₆)Alkyl, (C₃₋₇)Cycloalkyl, Aryl, Heteroaryl, Heteroalicyclic, Hydroxy, (C₁₋₆)Alkoxy, (C₁₋₆)Thioalkoxy, Thioaryloxy, Cyano, Halogen, Nitro, Carbonyl, Thiocarbonyl, Benzyl, -NR⁴⁸C(O)-(C₁₋₆)Alkyl, -NR⁴⁸C(O)-(C₃₋₆)Cycloalkyl, -NR⁴⁸C(O)-Aryl, -NR⁴⁸C(O)-Heteroaryl, -NR⁴⁸C(O)-Heteroalicyclic, einem zyklischen N-Amido, - NR⁴⁸S(O)₂-(C₁₋₆)Alkyl, -NR⁴⁸S(O)₂-(C₃₋₆)Cycloalkyl, -NR⁴⁸S(O)2-Aryl, -NR⁴⁸S(O)₂-Heteroaryl, -NR⁴⁸S(O)2-Heteroalicyclic, Sulfonyl, -S(O)2NR⁴⁸R⁴⁹, NR⁴⁸R⁴⁹, (C₁₋₆)AlkylC(O)NR⁴⁸R⁴⁹, C(O)NR⁴⁸R⁴⁹, NHC(O)NR⁴⁸R⁴⁹, OC(O)NR⁴⁸R⁴⁹, NHC(O)OR^{54'}, (C₁₋₆)AlkylNR⁴⁸R⁴⁹, COOR⁵⁴ und (C₁₋₆)AlkylCOOR^{54'}ausgewählt ist;
R¹ Wasserstoff ist;
m 2 ist;
R⁵ Wasserstoff ist;
R⁶ nicht existiert;
R⁷ aus Aryl, Heteroaryl und Heteroalicyclic ausgewählt ist, wobei das Aryl, Heteroaryl und Heteroalicyclic optional mit einem bis drei gleichen oder verschiedenen Halogenen oder mit von einem bis zwei gleichen oder verschiedenen aus der Gruppe F ausgewählten Substituenten substituiert sind;
A aus Phenyl und Heteroaryl ausgewählt ist; wobei Heteroaryl Pyridinyl, Furanyl oder Thienyl ist; und das Phenyl oder das Heteroaryl optional mit einem oder zwei gleichen oder verschiedenen Amino, C₁₋₆Alkyl, Hydroxy oder Halogen substituiert ist;
-W- 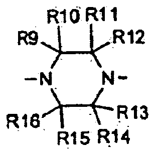 ist;
R⁹, R¹⁰, R¹¹, R¹², R¹³ und R¹⁴ jeweils Wasserstoff sind; und
R¹⁵ und R¹⁶ jeweils unabhängig Wasserstoff oder Methyl sind mit der Bedingung, dass nur eines Methyl ist;
R⁴² und R⁴³ unabhängig aus Wasserstoff, (C₁₋₆)Alkyl, (C₁₋₆)Alkoxy, (C₃₋₇)Cycloalkyl, Aryl, Heteroaryl und Heteroalicyclic ausgewählt sind; oder R⁴² und R⁴³ zusammen genommen mit dem Stickstoff, an dem sie angefügt sind, einen Heteroarylring oder einen Heteroalicyclicring bilden, der bis zu 2 zusätzliche Heteroatome enthalten kann, ausgewählt aus N, O, S(O)_{m'}, wobei m' 0, 1 oder 2 ist; und wobei das (C₁₋₆)Alkyl, (C₁₋₆)Alkoxy, (C₃₋₇)Cycloalkyl, Aryl, Heteroaryl und Heteroalicyclic optional mit einem bis drei gleichen oder verschiedenen Halogenen oder von einem bis zwei gleichen oder verschiedenen aus der Gruppe G ausgewählten Substituenten substituiert sind;
R⁴⁸ und R⁴⁹ unabhängig aus Wasserstoff, (C₁₋₆)Alkyl, (C₁₋₆)Alkoxy, (C₃₋₇)Cycloalkyl, Allyl, Aryl, Heteroaryl, Heteroalicyclic ausgewählt sind, oder R⁴⁸ und R⁴⁹ zusammen genommen mit dem Stickstoff, an dem sie angefügt sind, einen Heteroarylring oder einen Heteroalicyclicring bilden, der bis zu zwei zusätzliche Heteroatome enthalten kann, ausgewählt aus N, O, S(O)_{m'}, wobei m' 0, 1 oder 2 ist;
R⁵⁴ aus Wasserstoff, (C₁₋₆)Alkyl, (C₃₋₇)Cycloalkyl, Aryl, Heteroaryl und Heteroalicyclic ausgewählt ist, wobei das (C₁₋₆)Alkyl, (C₃₋₇)Cycloalkyl, Aryl, Heteroaryl und Heteroalicyclic optional mit einem bis drei gleichen oder verschiedenen Halogenen oder von einem bis zwei gleichen oder verschiedenen aus: Amino, OH und NR⁵⁵R⁵⁶, ausgewählten Substituenten substituiert sind;
R^{54'} aus (C₁₋₆)Alkyl, (C₃₋₇)Cycloalkyl, Aryl, Heteroaryl und Heteroalicyclic ausgewählt ist, wobei das (C₁₋₆)Alkyl, (C₃₋₇)Cycloalkyl, Aryl, Heteroaryl und Heteroalicyclic optional mit einem bis drei gleichen oder verschiedenen Halogenen oder von einem bis zwei gleichen oder verschiedenen aus: Amino, OH und NR⁵⁵R⁵⁶, ausgewählten Substituenten substituiert sind;
R⁵⁵ und R⁵⁶ unabhängig aus Wasserstoff, (C₁₋₆)Alkyl oder (C₃₋₇)Cycloalkyl ausgewählt sind.

2. Verbindung nach Anspruch 1, enthaltend pharmazeutisch annehmbare Salze davon, wobei:
R⁴ aus B ausgewählt ist;
B aus substituiertem Phenyl oder Heteroaryl ausgewählt ist, wobei das Phenyl substituiert ist und das Heteroaryl optional mit einem bis drei gleichen oder verschiedenen Halogenen oder von einem bis zwei gleichen oder verschiedenen aus der Gruppe F ausgewählten Substituenten substituiert ist;
F aus (C₁₋₆)Alkyl, (C₃₋₆)Cycloalkyl, Aryl, Heteroaryl, Heteroalicyclic, Hydroxy, (C₁₋₆)Alkoxy, (C₁₋₆)Thioalkoxy, Cyano, Halogen, Carbonyl, Benzyl, -NR⁴²C(O)-(C₁₋₆)Alkyl, -NR⁴²C(O)-(C₃₋₆)Cycloalkyl, -NR⁴²C(O)-Aryl, -NR⁴²C(O)-Heteroaryl,-NR⁴²C(O)-Heteroalicyclic, einem zyklischen N-Amido, -NR⁴²S(O)₂-(C₁₋₆)Alkyl, - NR⁴²R⁴³, C(O)NR⁴²R⁴³, COOR⁵⁴ ausgewählt ist, und wobei das (C₁₋₆)Alkyl, (C₃- ₆)Cycloalkyl, Aryl, Heteroaryl, Heteroalicyclic, (C₁₋₆)Alkoxy optional mit einem bis drei gleichen oder verschiedenen Halogenen oder von einem bis zwei gleichen oder verschiedenen aus der Gruppe G ausgewählten Substituenten substituiert sind;
G aus (C₁₋₆)Alkyl, Hydroxy, (C₁₋₆)Alkoxy, Halogen, -NR⁴⁸C(O)-(C₁₋₆)Alkyl, - NR⁴⁸C(O)-(C₃)Cycloalkyl, einem zyklischen N-Amido, -NR⁴⁸S(O)₂-(C₁₋₆)Alkyl, NR⁴⁸R⁴⁹, (C₁₋₆)Alkyl C(O)NR⁴⁸R⁴⁹, C(O)NR⁴⁸R⁴⁹, (C₁₋₆)AlkylNR⁴⁸R⁴⁹ ausgewählt ist;
R⁴² und R⁴³ unabhängig aus Wasserstoff, (C₁₋₆)Alkyl, (C₁₋₆)Alkoxy, (C₃-₇)Cycloalkyl, Aryl, Heteroaryl, Heteroalicyclic ausgewählt sind, oder R⁴² und R⁴³ zusammen genommen mit dem Stickstoff, an dem sie angefügt sind, einen Heteroarylring oder einen Heteroalicyclicring bilden, der bis zu zwei zusätzliche Heteroatome enthalten kann, ausgewählt aus N, O, S(O)_{m'}, wobei m' 0, 1 oder 2 ist; und wobei das (C₁₋₆)Alkyl, (C₁₋₆)Alkoxy, (C₃₋₇)Cycloalkyl, Aryl, Heteroaryl und Heteroalicyclic optional mit einem bis drei gleichen oder verschiedenen Halogenen oder von einem bis zwei gleichen oder verschiedenen aus der Gruppe G ausgewählten Substituenten substituiert sind;
R⁴⁸ und R⁴⁹ unabhängig aus Wasserstoff, (C₁₋₆)Alkyl ausgewählt sind, oder R⁴⁸ und R⁴⁹ zusammen genommen mit dem Stickstoff, an dem sie angefügt sind, einen Heteroarylring oder einen Heteroalicyclicring bilden, der bis zu zwei zusätzliche Heteroatome enthalten kann, ausgewählt aus N oder O.

3. Verbindung nach Anspruch 1, enthaltend pharmazeutisch annehmbare Salze davon, wobei:
Q 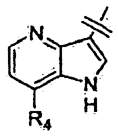 ist;
R⁴ B ist;
A Phenyl oder 2-Pyridyl ist;
B Heteroaryl ist, wobei das Heteroaryl optional mit einem bis drei gleichen oder verschiedenen Halogenen oder von einem bis zwei gleichen oder verschiedenen aus der Gruppe F ausgewählten Substituenten substituiert ist.

4. Verbindung nach Anspruch 2, enthaltend pharmazeutisch annehmbare Salze davon, wobei:
Q 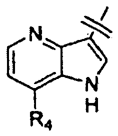 ist;
R⁴ B ist;
A Phenyl oder 2-Pyridyl ist;
B Heteroaryl ist, wobei das Heteroaryl optional mit einem bis drei gleichen oder verschiedenen Halogenen oder von einem bis zwei gleichen oder verschiedenen aus der Gruppe F ausgewählten Substituenten substituiert ist.

5. Verbindung nach Anspruch 3, enthaltend pharmazeutisch annehmbare Salze davon, wobei:
B Heteroaryl ist, wobei das Heteroaryl optional mit einem bis drei gleichen oder verschiedenen Halogenen oder von einem bis zwei gleichen oder verschiedenen aus der Gruppe F ausgewählten Substituenten substituiert ist.

6. Verbindung nach Anspruch 4, enthaltend pharmazeutisch annehmbare Salze davon, wobei:
B Heteroaryl ist, wobei das Heteroaryl optional mit einem bis drei gleichen oder verschiedenen Halogenen oder von einem bis zwei gleichen oder verschiedenen aus der Gruppe F ausgewählten Substituenten substituiert ist.

7. Verbindung nach Anspruch 1, enthaltend pharmazeutisch annehmbare Salze davon, wobei:
Q 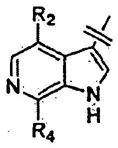 ist;
R² aus Wasserstoff, Halogen und Methoxy ausgewählt ist;
R⁴ B ist;
B Heteroaryl ist, wobei das Heteroaryl optional mit einem bis drei gleichen oder verschiedenen Halogenen oder von einem bis zwei gleichen oder verschiedenen aus der Gruppe F ausgewählten Substituenten substituiert ist.

8. Verbindung nach Anspruch 2, enthaltend pharmazeutisch annehmbare Salze davon, wobei:
Q 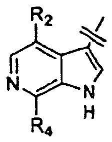 ist;
R² aus Wasserstoff, Halogen und Methoxy ausgewählt ist;
R⁴ B ist;
B Heteroaryl ist, wobei das Heteroaryl optional mit einem bis drei gleichen oder verschiedenen Halogenen oder von einem bis zwei gleichen oder verschiedenen aus der Gruppe F ausgewählten Substituenten substituiert ist.

9. Verbindung nach Anspruch 7 oder 8, enthaltend pharmazeutisch annehmbare Salze davon, wobei:
A Phenyl oder 2-Pyridyl ist.

10. Verbindung nach Anspruch 9, enthaltend pharmazeutisch annehmbare Salze davon, wobei:
B Heteroaryl ist, wobei das Heteroaryl optional mit einem bis drei gleichen oder verschiedenen Halogenen oder von einem bis zwei gleichen oder verschiedenen aus der Gruppe F ausgewählten Substituenten substituiert ist.

11. Verbindung nach Anspruch 1 oder 2, in der:
R⁴ B ist; und
F aus (C₁₋₆)Alkyl, Hydroxy, Heteroaryl, Heteroalicyclic, Methoxy, Methylthioalkoxy, Halogen, Carbonyl, C(O)NR⁴²R⁴³, -NR⁴²C(O)-(C₁₋₆)Alkyl, - NR⁴²C(O)-(C₃₋₆)Cycloalkyl, -NR⁴²C(O)-Aryl, -NR⁴²C(O)-Heteroaryl, -NR⁴²C(O)-Heteroalicyclic, einem zyklischen N-Amido, -NR⁴²S(O)₂-(C₁₋₆)Alkyl, -NR⁴²S(O)₂-(C₃₋₆)Cycloalkyl, -NR⁴²S(O)₂-Aryl, -NR⁴²S(O)₂-Heteroaryl, -NR⁴²S(O)₂-Heteroalicyclic, NR⁴²R⁴³, COOH ausgewählt ist.

12. Verbindung nach Anspruch 11, in der A Phenyl oder 2-Pyridyl ist.

13. Verbindung nach Anspruch 1 oder 2, enthaltend pharmazeutisch annehmbare Salze davon, wobei:
Q 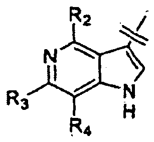 ist;
R² aus Wasserstoff oder Methoxy ausgewählt ist;
R³ Wasserstoff ist;
R⁴ B ist;
B Heteroaryl ist, wobei das Heteroaryl optional mit einem bis drei gleichen oder verschiedenen Halogenen oder von einem bis zwei gleichen oder verschiedenen aus der Gruppe F ausgewählten Substituenten substituiert ist.

14. Verbindung nach Anspruch 9, wobei R² Fluoro ist.

15. Verbindung nach Anspruch 9, wobei R² Methoxy ist.

16. Verbindung nach einem der Ansprüche 3, 4, 7, 8, 9 oder 13, wobei
B aus Thiazol, Pyridazin, Pyrazin, Pyrazol, Isoxazol, Isothiazol, Imidazol, Furyl, Thienyl, Oxazol, Oxadiazol, Thiadiazol, Pyrimidin, Triazin, Triazol, Tetrazol, Pyridyl ausgewählt ist, wobei das Heteroaryl optional mit einem bis drei gleichen oder verschiedenen Halogenen oder von einem bis zwei gleichen oder verschiedenen aus der Gruppe F ausgewählten Substituenten substituiert ist.

17. Verbindung nach Anspruch 6 oder 10, wobei
B Heteroaryl ist, wobei das Heteroaryl optional mit einem bis drei gleichen oder verschiedenen Halogenen oder von einem aus der Gruppe (C₁-C₆ Alkyl), Amino, - NHC(O)-(C₁-C₆ Alkyl), -NHS(O)₂-(C₁-C₆ Alkyl), Methoxy, -C(O)-NH₂, C(O)NHMe, C(O)NMe2, Trifluoromethyl, -NHC(C₁-C₆ Alkyl), -N(C₁-C₆ Alkyl)₂, -Heteroaryl, zyklisches N-Amido ausgewählten Substituenten substituiert ist.

18. Verbindung nach Anspruch 16, wobei das Heteroaryl optional mit einem bis drei gleichen oder verschiedenen Halogenen oder einem aus der Gruppe (C₁-C₆ Alkyl), Amino, -NHC(O)-(C₁-C₆ Alkyl), -NHS(O)₂-(C₁-C₆ Alkyl), Methoxy, -C(O)-NH₂, C(O)NHMe, C(O)NMe2, Trifluoromethyl, -NHC(C₁-C₆ Alkyl), -N(C₁-C₆ Alkyl)₂, - Heteroaryl, zyklisches N-Amido ausgewählten Substituenten substituiert ist.

19. Verbindung nach Anspruch 4 oder 10, wobei B Thienyl ist.

20. Verbindung nach Anspruch 16, wobei B Thienyl ist, das optional mit einem bis drei gleichen oder verschiedenen Halogenen oder einem aus der Gruppe (C₁-C₆ Alkyl), Amino, -NHC(O)-(C₁-C₆ Alkyl), -NHS(O)₂-(C₁-C₆ Alkyl), Methoxy, -C(O)-NH₂, C(O)NHMe, C(O)NMe2, Trifluoromethyl, -NHC(C₁-C₆ Alkyl), -N(C₁-C₆ Alkyl)₂, - Heteroaryl, zyklisches N-Amido ausgewählten Substituenten substituiert ist.

21. Verbindung nach Anspruch 16, wobei B aus Thiazol, Pyridazin, Pyrazin, Pyrazol, Isoxazol, Isothiazol, Imidazol, Furyl, Thienyl, Oxazol, Oxadiazol, Thiadiazol, Pyrimidin, Triazin, Triazol, Tetrazol, Pyridyl ausgewählt ist, wobei das Heteroaryl optional mit einem bis drei gleichen oder verschiedenen Halogenen oder einem aus der Gruppe (C₁-C₆ Alkyl), Amino, -NHC(O)-(C₁-C₆ Alkyl), -NHS(O)₂-(C₁-C₆ Alkyl), Methoxy, -C(O)-NH₂, C(O)NHMe, C(O)NMe2, Trifluoromethyl, -NHC(C₁-C₆ Alkyl), -N(C₁-C₆ Alkyl)₂, - Heteroaryl, zyklisches N-Amido ausgewählten Substituenten substituiert ist.

22. Verbindung nach Anspruch 1, die in den Tabellen 5, 6, 7 und 8 dargestellt ist.

23. Verbindung der Formel: 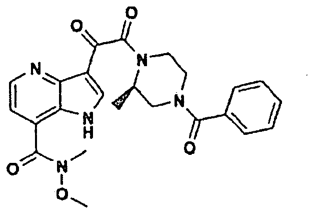 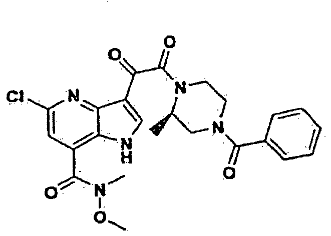 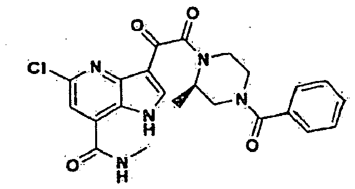 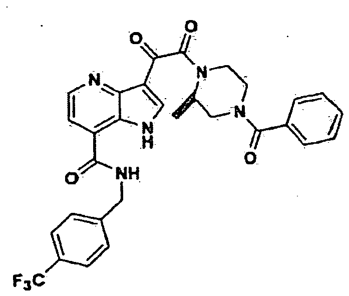 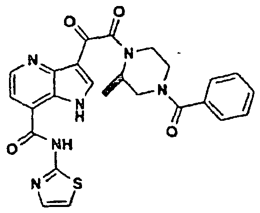 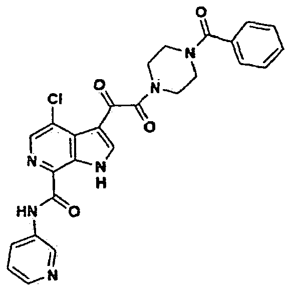 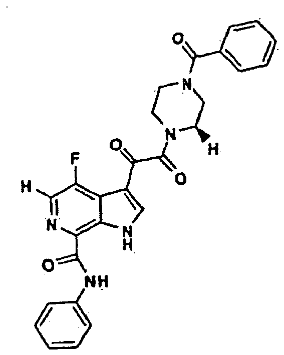 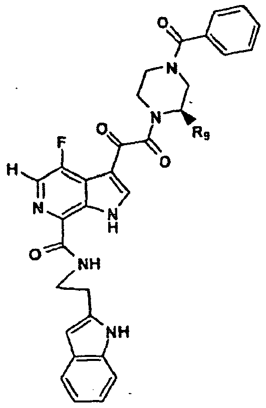 oder ein pharmazeutisch annehmbares Salz davon.

24. Pharmazeutische Formulierung, die eine antiviral wirksame Menge einer Verbindung von Formel I, enthaltend pharmazeutisch annehmbare Salze davon, wie in einem der Ansprüche 1-23 beansprucht, und einen pharmazeutisch annehmbaren Träger umfasst.

25. Pharmazeutische Formulierung nach Anspruch 24, nützlich zur Behandlung einer Infektion durch HIV, die zusätzlich eine antiviral wirksame Menge eines Mittels zur Behandlung von AIDS umfasst, ausgewählt aus:
(a) einem AIDS-Antivirusmittel;
(b) einem antiinfektiösen Mittel;
(c) einem Immunmodulator; und
(d) HIV-Eintrittsinhibitoren.

26. Verbindung von Formel I, enthaltend pharmazeutisch annehmbare Salze davon, wie in einem der Ansprüche 1-23 beansprucht, zur Verwendung in einem Verfahren zur Behandlung von mit einem Virus infizierten Säugetieren.

27. Verbindung nach Anspruch 26, umfassend, eine Verbindung von Formel I in Kombination mit einer antiviral wirksamen Menge eines Mittels zur Behandlung von AIDS, ausgewählt aus: einem AIDS-Antivirusmittel; einem antiinfektiösen Mittel; einem Immunmodulator und HIV-Eintrittsinhibitoren, zur Verwendung in einem Verfahren zur Behandlung nach Anspruch 26 zu verwenden.

28. Verbindung nach Anspruch 26 oder 27 zur Verwendung in einem Verfahren zur Behandlung nach Anspruch 26, wobei der Virus HIV ist.

## Revendications

1. Composé de formule I, incluant certains de ses sels pharmaceutiquement acceptables, 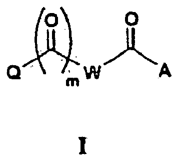 dans laquelle:
Q représente 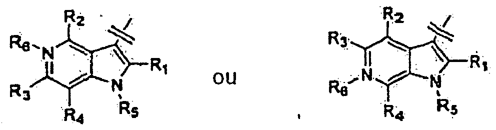
et alors R² est choisi dans le groupe constitué d'un hydrogène, d'un halogène et d'un méthoxy; et
R³ est un hydrogène;
ou Q représente: 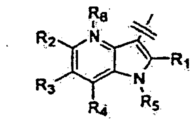 et R² est un halogène ou un hydrogène et R³ est un hydrogène;
R⁴ est B;
B est choisi dans le groupe constitué d'un phényle substitué, d'un hétéroaryle, et de C(O)R⁷, ledit hétéroaryle étant éventuellement substitué et le phényle étant substitué par un à trois halogènes identiques ou différents ou par un à deux substituants identiques ou différents choisis dans le groupe F;
F est choisi dans l'ensemble constitué des groupes alkyle en C₁₋₆, cycloalkyle en C₃₋₆, aryle, hétéroaryle, hétéroalicycliques, hydroxy, alcoxy en C₁₋₆, thioalcoxy en C₁₋₆, cyano, halogènes, carbonyle, benzyle, -NR⁴²C(O)-alkyle en C₁₋₆, -NR⁴²C(O)-cycloalkyle en C₃₋₆, -NR⁴²C(O)-aryle, -NR⁴²C(O)-hétéroaryle, -NR⁴²C(O)-hétéroalicycliques, N-amido cyclique, -NR⁴²S(O)₂-alkyle en C₁₋₆, -NR⁴²S(O)₂-cycloalkyle en C₃₋₆, -NR⁴²S(O)₂-aryle, -NR⁴²S(O)₂-hétéroaryle, -NR⁴²S(O)₂-heteroalicycliques, -S(O)₂NR⁴²R⁴³, NR⁴²R⁴³, (alkyle en C₁₋₆)-C(O)NR⁴²R⁴³, C(O)NR⁴²R⁴³, NHC(O)NR⁴²R⁴³, OC(O)NR⁴²R⁴³, NHC(O)OR^{54'}, (alkyle en C₁₋₆)-NR⁴²R⁴³, COOR⁵⁴, et (alkyle en C₁₋₆)-COOR⁵⁴, lesdits groupes alkyle en C₁₋₆, cycloalkyle en C₃₋₆, aryle, hétéroaryle, hétéroalicycliques, alcoxy en C₁₋₆, sont éventuellement substitués par un à trois halogènes identiques ou différents ou par un à deux substituants identiques ou différents choisis dans le groupe G;
G est choisi dans l'ensemble constitué des groupes alkyle en C₁₋₆, cycloalkyle en C₃₋₇, aryle, hétéroaryle, hétéroalicycliques, hydroxy, alcoxy en C₁₋₆, thioalcoxy en C₁₋₆, thioaryloxy, cyano, halogènes, nitro, carbonyle, thiocarbonyle, benzyle, -NR⁴⁸C(O)-alkyle en C₁₋₆, -NR⁴⁸C(O)-cycloalkyle en C₃₋₆, -NR⁴⁸C(O)-aryle, -NR⁴⁸C(O)-hétéroaryle, -NR⁴⁸C(O)-hétéroalicycliques, N-amido cyclique, -NR⁴⁸S(O)₂-alkyle en C₁₋₆, - NR⁴⁸S(O)₂-cycloalkyle en C₃₋₆, -NR⁴⁸S(O)₂-aryle, -NR⁴⁸S(O)₂-hétéroaryle, -NR⁴⁸S(O)₂-heteroalicycliques, sulfonyle, -S(O)₂NR⁴⁸R⁴⁹, NR⁴⁸R⁴⁹, (alkyle en C₁₋₆)-C(O)NR⁴⁸R⁴⁹, C(O)NR⁴⁸R⁴⁹, NHC(O)NR⁴⁸R⁴⁹, OC(O)NR⁴⁸R⁴⁹, NHC(O)OR^{54'}, (alkyle en C₁₋₆)-NR⁴⁸R⁴⁹, COOR⁵⁴, et (alkyle en C₁₋₆)-COOR^{54'};
R¹ est un hydrogène;
m vaut 2;
R⁵ est un hydrogène;
R⁶ n'existe pas;
R⁷ est choisi dans l'ensemble constitué des groupes aryle, hétéroaryle, et hétéroalicycliques, lesdits groupes aryle, hétéroaryle, et hétéroalicycliques étant éventuellement substitués par un à trois halogènes identiques ou différents ou par un à deux substituants identiques ou différents choisis dans le groupe F;
A est choisi dans l'ensemble constitué des groupes phényle et hétéroaryle; ledit hétéroaryle étant un pyridinyle, furanyle ou thiényle; et ledit phényle ou ledit hétéroaryle étant éventuellement substitué par un à deux groupes identiques ou différents parmi un amino, un alkyle en C₁₋₆, un hydroxy, ou un halogène;
-W- est 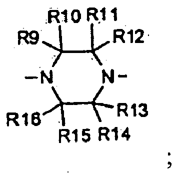
R⁹, R¹⁰, R¹¹, R¹², R¹³, et R¹⁴ sont chacun un hydrogène; et
R¹⁵ et R¹⁶ sont chacun indépendamment un hydrogène ou un méthyle, sous réserve qu'un seul soit un méthyle;
R⁴² et R⁴³ sont indépendamment choisis dans l'ensemble constitué d'un hydrogène et des groupes alkyle en C₁₋₆, alcoxy en C₁₋₆, cycloalkyle en C₃₋₇, aryle, hétéroaryle et hétéroalicycliques; ou R⁴² et R⁴³ pris ensemble avec l'azote auquel ils sont fixés forment un noyau hétéroaryle ou un noyau hétéroalicyclique qui peut contenir jusqu'à 2 hétéroatomes supplémentaires choisis parmi N, O, S(O)_{m'} où m' vaut 0, 1, ou 2; et où lesdits groupes alkyle en C₁₋₆, alcoxy en C₁₋₆, cycloalkyle en C₃₋₇, aryle, hétéroaryle et hétéroalicycliques sont éventuellement substitués par un à trois halogènes identiques ou différents ou par un à deux substituants identiques ou différents choisis dans le groupe G;
R⁴⁸ et R⁴⁹ sont indépendamment choisis dans l'ensemble constitué d'un hydrogène et des groupes alkyle en C₁₋₆, alcoxy en C₁₋₆, cycloalkyle en C₃₋₇, allyle, aryle, hétéroaryle, hétéroalicycliques, ou R⁴⁸ et R⁴⁹ pris ensemble avec l'azote auquel ils sont fixés forment un noyau hétéroaryle ou un noyau hétéroalicyclique qui peut contenir jusqu'à deux hétéroatomes supplémentaires choisis parmi N, O, S(O)_{m'} où m' vaut 0, 1, ou 2;
R⁵⁴ est choisi dans l'ensemble constitué d'un hydrogène et des groupes alkyle en C₁₋₆, cycloalkyle en C₃₋₇, aryle, hétéroaryle et hétéroalicycliques, lesdits groupes alkyle en C₁₋₆, cycloalkyle en C₃₋₇, aryle, hétéroaryle et hétéroalicycliques étant éventuellement substitués par un à trois halogènes identiques ou différents ou par un à deux substituants identiques ou différents choisis dans le groupe constitué de: un amino, OH, et NR⁵⁵R⁵⁶;
R^{54'} est choisi dans l'ensemble constitué des groupes alkyle en C₁₋₆, cycloalkyle en C₃₋₇, aryle, hétéroaryle et hétéroalicycliques, lesdits groupes alkyle en C₁₋₆, cycloalkyle en C₃₋₇, aryle, hétéroaryle et hétéroalicycliques étant éventuellement substitués par un à trois halogènes identiques ou différents ou par un à deux substituants identiques ou différents choisis dans l'ensemble constitué de: un amino, OH, et NR⁵⁵R⁵⁶;
R⁵⁵ et R⁵⁶ sont indépendamment choisis dans le groupe constitué d'un hydrogène, d'un alkyle en C₁₋₆, ou d'un cycloalkyle en C₃₋₇.

2. Composé selon la revendication 1, incluant certains de ses sels pharmaceutiquement acceptables, dans lequel:
R⁴ est choisi dans le groupe constitué de B;
B est choisi dans le groupe constitué d'un phényle substitué, ou d'un hétéroaryle, ledit phényle étant substitué et l'hétéroaryle étant éventuellement substitué par un à trois halogènes identiques ou différents ou par un à deux substituants identiques ou différents choisis dans le groupe F;
F est choisi dans l'ensemble constitué des groupes alkyle en C₁₋₆, cycloalkyle en C₃₋₆, aryle, hétéroaryle, hétéroalicycliques, hydroxy, alcoxy en C₁₋₆, thioalcoxy en C₁₋₆, cyano, halogènes, carbonyle, benzyle, -NR⁴²C(O)-alkyle en C₁₋₆, -NR⁴²C(O)-cycloalkyle en C₃₋₆, -NR⁴²C(O)-aryle, -NR⁴²C(O)-hétéroaryle, -NR⁴²C(O)-hétéroalicycliques, N-amido cyclique, -NR⁴²S(O)₂-alkyle en C₁₋₆, -NR⁴²R⁴³, C(O)NR⁴²R⁴³, COOR⁵⁴, lesdits groupes alkyle en C₁₋₆, cycloalkyle en C₃₋₆, aryle, hétéroaryle, hétéroalicycliques, alcoxy en C₁₋₆, sont éventuellement substitués par un à trois halogènes identiques ou différents ou par un à deux substituants identiques ou différents choisis dans le groupe G;
G est choisi dans l'ensemble constitué des groupes alkyle en C₁₋₆, hydroxy, alcoxy en C₁₋₆, halogènes, -NR⁴⁸C(O)-alkyle en C₁₋₆, -NR⁴⁸C(O)-cycloalkyle en C₃, N-amido cyclique, -NR⁴⁸S(O)₂-alkyle en C₁₋₆, NR⁴⁸R⁴⁹, (alkyle en C₁₋₆)-C(O)NR⁴⁸R⁴⁹, C(O)NR⁴⁸R⁴⁹, (alkyle en C₁₋₆)-NR⁴⁸R⁴⁹;
R⁴² et R⁴³ sont indépendamment choisis dans l'ensemble constitué d'un hydrogène et des groupes alkyle en C₁₋₆, alcoxy en C₁₋₆, cycloalkyle en C₃₋₇, aryle, hétéroaryle, hétéroalicycliques, ou R⁴² et R⁴³ pris ensemble avec l'azote auquel ils sont fixés forment un noyau hétéroaryle ou un noyau hétéroalicyclique qui peut contenir jusqu'à deux hétéroatomes supplémentaires choisis parmi N, 0, S(O)_{m'} où m' vaut 0, 1, ou 2; et où lesdits groupes alkyle en C₁₋₆, alcoxy en C₁₋₆, cycloalkyle en C₃₋₇, aryle, hétéroaryle et hétéroalicycliques sont éventuellement substitués par un à trois halogènes identiques ou différents ou par un à deux substituants identiques ou différents choisis dans le groupe G;
R⁴⁸ et R⁴⁹ sont indépendamment choisis dans l'ensemble constitué d'un hydrogène et des groupes alkyle en C₁₋₆, ou R⁴⁸ et R⁴⁹ pris ensemble avec l'azote auquel ils sont fixés forment un noyau hétéroaryle ou un noyau hétéroalicyclique qui peut contenir jusqu'à deux hétéroatomes supplémentaires choisis parmi N, ou O.

3. Composé selon la revendication 1, incluant certains de ses sels pharmaceutiquement acceptables, dans lequel:
Q est 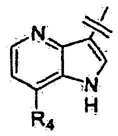
R⁴ est B;
A est un phényle ou 2-pyridyle;
B est un hétéroaryle, ledit hétéroaryle étant éventuellement substitué par un à trois halogènes identiques ou différents ou par un à deux substituants identiques ou différents choisis dans le groupe F.

4. Composé selon la revendication 2, incluant certains de ses sels pharmaceutiquement acceptables, dans lequel:
Q est 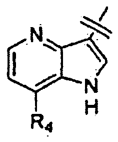
R⁴ est B;
A est un phényle ou 2-pyridyle;
B est un hétéroaryle, ledit hétéroaryle étant éventuellement substitué par un à trois halogènes identiques ou différents ou par un à deux substituants identiques ou différents choisis dans le groupe F.

5. Composé selon la revendication 3, incluant certains de ses sels pharmaceutiquement acceptables, dans lequel: B est un hétéroaryle, ledit hétéroaryle étant éventuellement substitué par un à trois halogènes identiques ou différents ou par un à deux substituants identiques ou différents choisis dans le groupe F.

6. Composé selon la revendication 4, incluant certains de ses sels pharmaceutiquement acceptables, dans lequel: B est un hétéroaryle, ledit hétéroaryle étant éventuellement substitué par un à trois halogènes identiques ou différents ou par un à deux substituants identiques ou différents choisis dans le groupe F.

7. Composé selon la revendication 1, incluant certains de ses sels pharmaceutiquement acceptables, dans lequel:
Q est 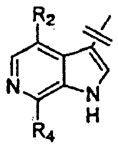
R² est choisi dans le groupe constitué d'un hydrogène, d'un halogène, et d'un méthoxy;
R⁴ est B;
B est un hétéroaryle, ledit hétéroaryle étant éventuellement substitué par un à trois halogènes identiques ou différents ou par un à deux substituants identiques ou différents choisis dans le groupe F.

8. Composé selon la revendication 2, incluant certains de ses sels pharmaceutiquement acceptables, dans lequel:
Q est 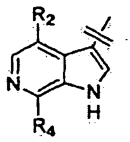
R² est choisi dans le groupe constitué d'un hydrogène, d'un halogène, et d'un méthoxy;
R⁴ est B;
B est un hétéroaryle, ledit hétéroaryle étant éventuellement substitué par un à trois halogènes identiques ou différents ou par un à deux substituants identiques ou différents choisis dans le groupe F.

9. Composé selon la revendication 7 ou 8, incluant certains de ses sels pharmaceutiquement acceptables, dans lequel:
A est un phényle ou 2-pyridyle;

10. Composé selon la revendication 9 incluant certains de ses sels pharmaceutiquement acceptables, dans lequel: B est un hétéroaryle, ledit hétéroaryle étant éventuellement substitué par un à trois halogènes identiques ou différents ou par un à deux substituants identiques ou différents choisis dans le groupe F.

11. Composé selon la revendication 1 ou 2 dans lequel:
R⁴ est B; et
F est choisi dans l'ensemble constitué des groupes alkyle en C₁₋₆, hydroxy, hétéroaryle, hétéroalicycliques, méthoxy, méthylthioalcoxy, halogènes, carbonyle, C(O)NR⁴²R⁴³, -NR⁴²C(O)-alkyle en C₁₋₆, -NR⁴²C(O)-cycloalkyle en C₃₋₆, -NR⁴²C(O)-aryle, -NR⁴²C(O)-hétéroaryle, -NR⁴²C(O)-hétéroalicycliques, N-amido cyclique, - NR⁴²S(O)₂-alkyle en C₁₋₆, -NR⁴²S(O)₂-cycloalkyle en C₃₋₆, -NR⁴²S(O)₂-aryle, - NR⁴²S(O)₂-hétéroaryle, -NR⁴²S(O)₂-heteroalicycliques, NR⁴²R⁴³, COOH.

12. Composé selon la revendication 11 dans lequel A est un phényle ou 2-pyridyle.

13. Composé selon la revendication 1 ou 2, incluant certains de ses sels pharmaceutiquement acceptables, dans lequel:
Q est 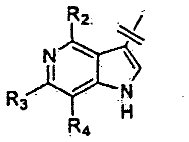
R² est choisi dans le groupe constitué d'un hydrogène ou d'un méthoxy;
R³ est un hydrogène;
R⁴ est B;
B est un hétéroaryle, ledit hétéroaryle étant éventuellement substitué par un à trois halogènes identiques ou différents ou par un à deux substituants identiques ou différents choisis dans le groupe F.

14. Composé selon la revendication 9 dans lequel R² est un fluoro.

15. Composé selon la revendication 9 dans lequel R² est un méthoxy.

16. Composé selon l'une quelconque des revendications 3, 4, 7, 8, 9, ou 13, dans lequel
B est choisi dans le groupe constitué des thiazole, pyridazine, pyrazine, pyrazole, isoxazole, isothiazole, imidazole, furyle, thiényle, oxazole, oxadiazole, thiadiazole, pyrimidine, triazine, triazole, tétrazole, pyridyle, ledit hétéroaryle étant éventuellement substitué par un à trois halogènes identiques ou différents ou par un à deux substituants identiques ou différents choisis dans le groupe F.

17. Composé selon la revendication 6 ou 10 dans lequel
B est un hétéroaryle, ledit hétéroaryle étant éventuellement substitué par un à trois halogènes identiques ou différents ou un substituant choisi dans l'ensemble constitué des groupes alkyle en C₁-C₆, amino, -NHC(O)-alkyle en C₁-C₆, -NHS(O)₂-alkyle en C₁-C₆, méthoxy, -C(O)-NH₂, C(O)NHMe, C(O)NMe₂, trifluorométhyle, -NHC(alkyle en C₁-C₆), -N(alkyle en C₁-C₆)₂, -hétéroaryle, N-amido cyclique.

18. Composé selon la revendication 16 dans lequel ledit hétéroaryle est éventuellement substitué par un à trois halogènes identiques ou différents ou un substituant choisi dans l'ensemble constitué des groupes alkyle en C₁-C₆, amino, -NHC(O)-alkyle en C₁-C₆, -NHS(O)₂-alkyle en C₁-C₆, méthoxy, -C(O)-NH₂, C(O)NHMe, C(O)NMe₂, trifluorométhyle, -NHC(alkyle en C₁-C₆), -N(alkyle en C₁-C₆)₂, -hétéroaryle, N-amido cyclique.

19. Composé selon la revendication 4 ou 10 dans lequel B est un thiényle.

20. Composé selon la revendication 16 dans lequel B est un thiényle qui est éventuellement substitué par un à trois halogènes identiques ou différents ou un substituant choisi dans l'ensemble constitué des groupes alkyle en C₁-C₆, amino, -NHC(O)-alkyle en C₁-C₆, -NHS(O)₂-alkyle en C₁-C₆, méthoxy, -C(O)-NH₂, C(O)NHMe, C(O)NMe₂, trifluorométhyle, -NHC(alkyle en C₁-C₆), -N(alkyle en C₁-C₆)₂, -hétéroaryle, N-amido cyclique.

21. Composé selon la revendication 16 dans lequel B est choisi dans le groupe constitué des thiazole, pyridazine, pyrazine, pyrazole, isoxazole, isothiazole, imidazole, furyle, thiényle, oxazole, oxadiazole, thiadiazole, pyrimidine, triazine, triazole, tétrazole, pyridyle, ledit hétéroaryle étant éventuellement substitué par un à trois halogènes identiques ou différents ou un substituant choisi dans l'ensemble constitué des groupes alkyle en C₁-C₆, amino, -NHC(O)-alkyle en C₁-C₆, -NHS(O)₂-alkyle en C₁-C₆, méthoxy, - C(O)-NH₂, C(O)NHMe, C(O)NMe₂, trifluorométhyle, -NHC(alkyle en C₁-C₆), -N(alkyle en C₁-C₆)₂, -hétéroaryle, N-amido cyclique.

22. Composé selon la revendication 1 qui est illustré dans les tableaux 5, 6, 7, et 8.

23. Composé de formule: 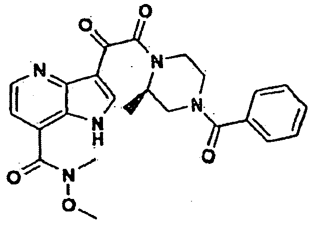 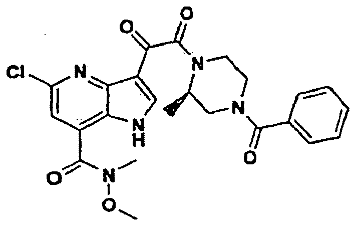 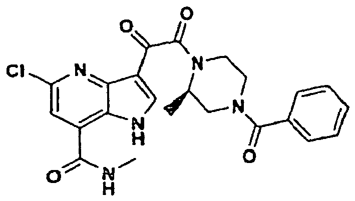 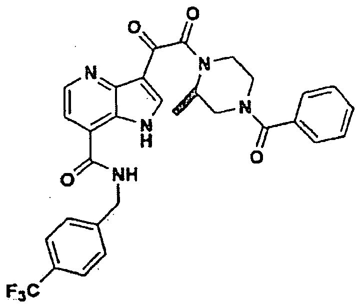 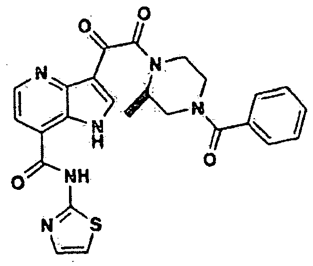 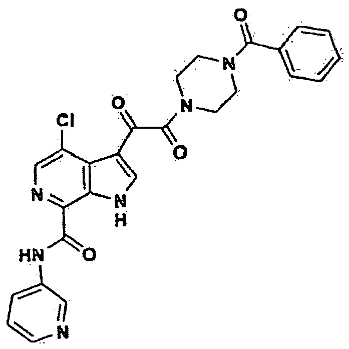 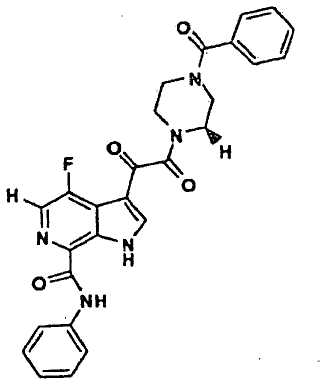 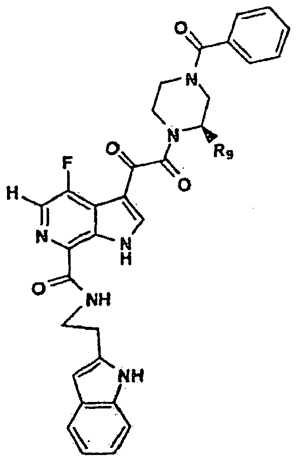 ou un sel pharmaceutiquement acceptable de celui-ci.

24. Formulation pharmaceutique qui comprend une quantité antivirale efficace d'un composé de formule I, incluant certains de ses sels pharmaceutiquement acceptables, tel que revendiqué dans n'importe lesquelles des revendications 1-23, et un véhicule pharmaceutiquement acceptable.

25. Formulation pharmaceutique selon la revendication 24, utile pour traiter une infection par le VIH, qui comprend en plus une quantité antivirale efficace d'un agent de traitement du SIDA choisi dans le groupe constitué de:
(a) un agent antiviral contre le SIDA;
(b) un agent anti-infectieux;
(c) un immunomodulateur; et
(d) des inhibiteurs d'entrée du VIH.

26. Composé de formule I, incluant certains de ses sels pharmaceutiquement acceptables, tel que revendiqué dans n'importe lesquelles des revendications 1-23, destiné à être utilisé dans une méthode de traitement de mammifères infectés par un virus.

27. Composé selon la revendication 26, comprenant l'utilisation d'un composé de formule I en combinaison avec une quantité antivirale efficace d'un agent de traitement du SIDA choisi dans le groupe constitué de: un agent antiviral contre le SIDA; un agent anti-infectieux; un immunomodulateur; et des inhibiteurs d'entrée du VIH, destiné à être utilisé dans une méthode de traitement selon la revendication 26.

28. Composé selon la revendication 26 ou 27 destiné à être utilisé dans une méthode de traitement selon la revendication 26, où le virus est le VIH.
